# EUROPEAN PATENT APPLICATION

(11) **EP 1 302 462 A1**
(43) Date of publication of application: **16.04.2003**
(21) Application number: 01948032.6
(22) Date of filing: 17.07.2001
(51) Int. Cl.: C07D 213/74, C07D 401/04, C07D 401/14, C07D 409/14, C07D 417/14, C07D 471/04, C07D 471/10

(54) **SULFONE DERIVATIVES, PROCESS FOR THEIR PRODUCTION AND USE THEREOF**

(30) Priority: 17.07.2000 JP 2000221065
(71) Applicant: Takeda Chemical Industries, Ltd., Osaka-shi, Osaka 541-0045 (JP)
(72) Inventor: KUBO, Keiji, Minoo-shi, Osaka 562-0044 (JP); MIYAWAKI, Toshio, Nishinomiya-shi, Hyogo 662-0084 (JP); KAWAMURA, Masaki, Ikeda-shi, Osaka 563-0024 (JP)
(74) Representative: Rickard, Timothy Mark Adrian
(86) International application number: JP0106148
(87) International publication number: WO02006234

(57) **Abstract**

A compound represented by the formula: 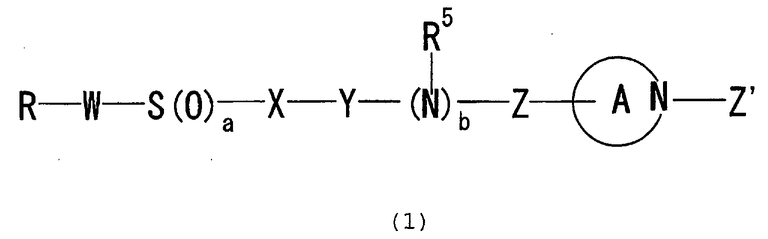 wherein R is a cyclic hydrocarbon group, or the like; W is a bond, or the like; X is a divalent hydrocarbon group, or the like; Y and Z are each independently -N(R⁶)- or the like; ring A is a nitrogen-containing heterocyclic ring, or the like; R5 and R6 are independently hydrogen atom, a hydrocarbon group, or the like; Z' is imidoyl group, or the like; a is 0, 1 or 2; and b is 0 or 1, or a salt thereof.

## Description

### TECHNICAL FIELD

The present invention relates to novel sulfone derivatives which inhibit activated coagulation factor X (FXa) to exhibit anti-coagulative effect and antithrombotic effect and which are useful for preventing and/or treating thromboembolic diseases of arteries and veins, inflammation, cancers, and the like, as well as their production and use.

### BACKGROUND ART

It is important to inhibit the formation of a thrombus in preventing and/or treating cardiac infarctionion, cerebral thrombosis and the like, and various anti-thombotic agents such as anti-thrombin agents and platelet aggregation inhibitors have been developed. Nevertheless, platelet aggregation inhibitors as well as anti-thrombin agents have bleeding side effects and problems in their safety, since these agents posses a platelet aggregation-inhibiting activity in combination with anti-coagulative effect. On the other hand, FXa inhibitors specifically inhibit a coagulation factor, and thus are considered to be useful as anticoagulant.

So far, compounds having FXa-inhibiting effects are disclosed for example in JP 7-112970 A, JP 5-208946 A, WO-96/16940, WO96/4067 and WO 96/10022, as well as Journal of Medicinal Chemistry, Vol.41, page 3357 (1998), etc.

Since such compounds having FXa-inhibiting effects described above have low absorbaility by oral administration and lasting of their activity is short, they do not exhibit sufficient activities when clinically using as an agent for treating thrombosis.

### SUMMARY OF THE INVENTION

The present inventors thought that a sulfone derivative having strong FXa specific inhibiting activity can exerts sustained and sufficient effect by oral administration, and is useful for preventing and/or treating thromboembolic diseases of arteries and veins, inflammation and cancers, and continued to intensively study.

As a result, the present inventors found that a novel sulfone derivative represented by the following formula (I) or a salt thereof [hereinafter referred to as compound (I) in some cases] has specific strong FXa inhibiting activity, has high safety, and exerts sustained and sufficient effect by oral administration, and further studied to complete the present invention.

That is, the present invention provides:
(1) a compound represented by the formula (I) 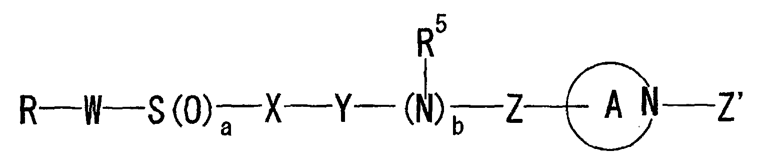 wherein R is an optionally substituted cyclic hydrocarbon group or an optionally substituted heterocyclic group, W is a bond or an optionally substituted divalent chainlike hydrocarbon group, X is an optionally substituted divalent hydrocarbon group, Y and Z are independently -N(R⁶)-, -CO-, -S(O)-, -S(O)₂-, -CH₂-, -N(R⁶)-CO-, -CO-CH₂- or a bond [preferably -N(R⁶)-, -CO-, S(O), -S(O)₂-, -CH₂- or a bond], ring A is an optionally substituted nitrogen-containing heterocyclic ring, R⁵ and R⁶ are independently hydrogen atom, an optionally substituted hydrocarbon group, an optionally substituted alkoxy group, an optionally esterified or amidated carboxyl or an optionally substituted acyl group, R⁵ may bind to a substituent of X or a substituent of ring A to form a ring, Z' is an optionally substituted imidoyl group or an optionally substituted nitrogen-containing heterocyclic group, a is 0, 1 or 2, and b is 0 or 1, or a salt thereof;
(2) a prodrug of the compound described in the above (1) or a salt thereof;
(3) the compound described in the above (1), wherein R is an optionally substituted aryl group;
(4) the compound described in the above (1), wherein R is an aryl group optionally substituted with a substituent selected from a halogen atom, C₁₋₆ alkyl, C₂₋₆ alkenyl, C₂₋₆ alkynyl, optionally substituted amino, nitro, cyano, optionally substituted amidino or optionally esterified or amidated carboxyl;
(5) the compound described in the above (1), wherein R is an optionally substituted heterocyclic group;
(6) the compound described in the above (1), wherein R is a heterocyclic group optionally substituted with a substituent selected from a halogen atom, C₁₋₆ alkyl, C₂₋₆ alkenyl, C₂₋₆ alkynyl, optionally substituted amino, nitro, cyano, optionally substituted amidino or optionally esterified or amidated carboxyl;
(7) the compound described in the above (1), wherein R is naphthyl or benzopyranyl optionally substituted with a halogen atom (preferably naphthyl optionally substituted with a halogen atom);
(8) the compound described in the above (1), wherein W is a bond;
(9) the compound described in the above (1), wherein X is an optionally substituted divalent chainlike hydrocarbon group;
(10) the compound described in the above (1), wherein X is an optionally substituted phenylene group;
(11) the compound described in the above (1), wherein Y is -CO- or -SO₂-, and Z is a bond;
(12) the compound described in the above (1), wherein Y is a bond, and Z is -CO-;
(13) the compound described in the above (1), wherein ring A is an optionally substituted piperazine ring or an optionally substituted piperidine ring;
(14) the compound described in the above (1), wherein Z' is an optionally substituted nitrogen-containing heterocyclic group;
(15) the compound described in the above (1), wherein Z' is a nitrogen-containing heterocyclic group optionally substituted with a substituent selected from optionally substituted C₁₋₄ alkyl and optionally substituted amino;
(16) the compound described in the above (1), wherein Z' is an optionally substituted pyridyl group;
(17) the compound described in the above (16), wherein Z' is bound to ring A at 4-position of the pyridine ring;
(18) the compound described in the above (1), wherein R⁵ is hydrogen atom or optionally substituted C₁₋₆ alkyl;
(19) the compound described in the above (1), wherein R⁵ binds to a substituent of ring A to form a ring;
(20) the compound described in the above (1), wherein a is 2;
(21) the compound described in the above (1), wherein b is 1;
(22) a compound selected from the group consisting of N-[3-[(6-chloro-2-naphthyl)sulfonyl]propyl]-N-methyl-1-(4-pyridyl)-4-piperidinecarboxamide, methyl 2-[N-[3-[(6-chloro-2-naphthyl)sulfonyl]propyl]-N-[1-(4-pyridyl)-4-piperidyl]carbonylamino]acetate, 3-[(6-chloro-2-naphtyl)sulfonyl]-N-methyl-N-[1-(4-pyridyl)-4-piperidyl]propanamide, ethyl 2-[N-[3-[(6-chloro-2-naphthyl)sulfonyl]propanoyl]-N-[1-(4-pyridyl)-4-piperidyl]amino]acetate, ethyl 3-[N-[3-[(6-chloro-2-naphthyl)sulfonyl]propanoyl]-N-[1-(4-pyridyl)-4-piperidyl]amino]propionate, 3-[(6-chloro-2-naphthyl)sulfonyl]-N-methyl-N-[1-(2-methyl-4-pyridyl)-4-piperidyl]propanamide, N-[2-(acetylamino)ethyl]-3-[(6-chloro-2-naphthyl)sulfonyl]-N-[1-(2-methyl-4-pyridyl)-4-piperidyl]propanamide, N-(2-aminoethyl)-3-[(6-chloro-2-naphthyl)sulfonyl]-N-[1-(4-pyridyl)-4-piperidyl]propanamide, N-[2-(acetylamino)ethyl]-3-[(6-chloro-2-naphthyl)sulfonyl]-N-[1-(4-pyridyl)-4-piperidylpropanamide, 3-[(6-chloro-2-naphthyl)sulfonyl]-N-[2-[(methanesulfonyl)amino]ethyl]-N-[1-(4-pyridyl)-4-piperidyl]propanamide, 3-[(6-bromo-2-naphthyl)sulfonyl]-N-methyl-N-[1-(2-methyl-4-pyridyl)-4-piperidyl]propanamide, 3-[(6-chloro-2-naphthyl)sulfonyl]-N-[1-(2-methyl-4-pyridyl)-4-piperidyl]-N-[3-(1-oxide-4-thiomorpholinyl)-3-oxopropyl]propanamide, N-[2-(N-acetyl-N-methylamino)ethyl]-3-[(6-chloro-2-naphthyl)sulfonyl]-N-[1-(2-methyl-4-pyridyl)-4-piperidyl]propanamide, 3-[(6-chloro-2-naphthyl)sulfonyl]-N-methyl-N-[1-(2,6-dimethyl-4-pyridyl)-4-piperidyl]propanamide and 1-[3-[(6-chloro-2-naphthyl) sulfonyl]propanoyl-4-(2-methyl-4-pyridyl)piperazine or a salt thereof;
(23) a prodrug of the compound described in the above (22) or a salt thereof;
(24) a pharmaceutical composition comprising the compound described in the above (1) or a salt or a prodrug thereof;
(25) the composition described in the above (24) which is an anticoagulant;
(26) the composition described in the above (24) which is an activated coagulation factor X inhibiting agent;
(27) the composition described in the above (24) which is an agent for preventing and treating cardiac infarction, cerebral thrombosis, deep vein thrombosis, pulmonary thrombotic embolus or thrombotic embolus during operation or after operation;
(28) a process for preparing the compound (1) described in the above (1) or a salt thereof, which comprises:
   reacting a compound represented by the formula (II):

      **L**^{**1**}**―Z'**
   wherein L¹ is a leaving group, and the other symbol is the same as defined in the above (1), or a salt thereof, with a compound represented by the formula (III): 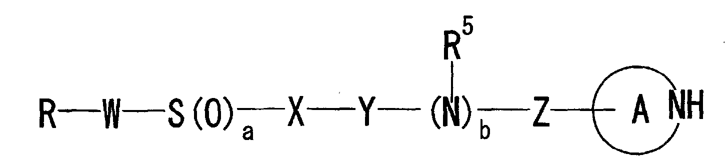 wherein the symbols are the same as defined in the above (1), or a salt thereof, or
   reacting a compound represented by the formula (IV):

   R-W-S(O)ₐ-X-Y-L²

   wherein L² is a leaving group, and the other symbols are the same as defined in the above (1), or a salt thereof, with a compound represented by the formula (V): 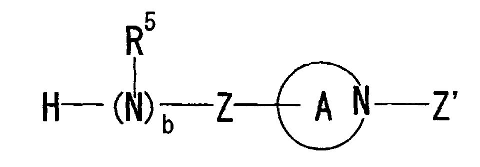 wherein the symbols are the same as defined in the above (1), or a salt thereof, or
   reacting a compound represented by the formula (VI): 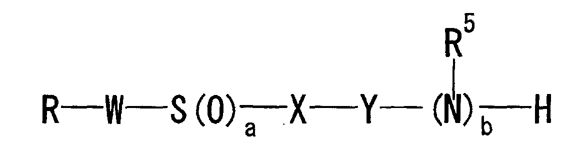 wherein the symbols are the same as defined in the above (1), or a salt thereof, with a compound represented by the formula (VII): 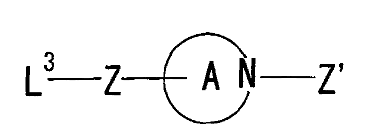 wherein L³ is a leaving group, and the other symbols are the same as defined in the above (1), or a salt thereof, or reacting a compound represented by the formula (Ia): 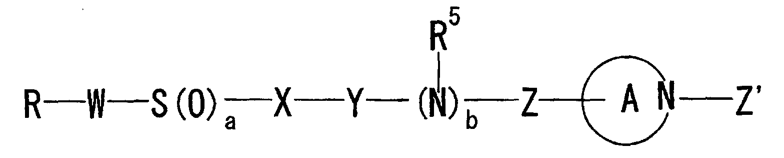 wherein a is 0, and the other symbols are the same as defined in the above (1), or a salt thereof, with an oxidizing reagent (provided that a in the reaction product is 1 or 2), or
   reacting a compound represented by the formula (VIII):

   R⁵-L⁴

   wherein L⁴ is a leaving group, and the other symbol is the same as defined in the above (1), or a salt thereof, with a compound represented by the formula (Ib): 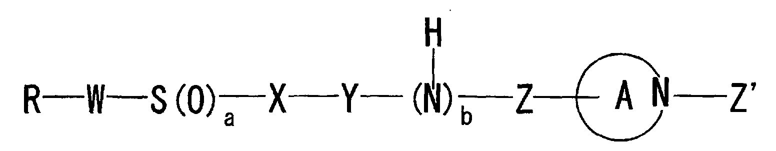 wherein the symbols are the same as defined in the above (1), or a salt thereof, or
   reacting a compound represented by the formula (IX):

   R-W-S(Oₐ)-M

   wherein M is hydrogen atom, an alkali metal, an alkaline earth metal or a leaving group, and the other symbols are the same as defined in the above (1), or a salt thereof, with a compound represented by the formula (X): 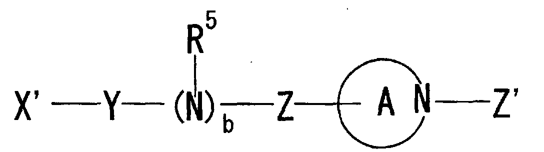 wherein X' is alkenyl or alkynyl, or alkyl having a leaving group, and the other symbols are the same as defined in the above (1), or a salt thereof, and
   if necessary, further subjecting the compound obtained in the above reaction to hydrolysis, esterification, amidation, alkylation, acylation, reduction, oxidation and/or deprotection;
(29) 3-(6-halogeno-2-naphthyl)sulfonylpropionic acid, or an ester or an amide or a salt thereof;
(30) 3-(6-chloro-2-naphthyl)sulfonylpropionic acid, or an ester, an amide or a salt thereof;
(31) a method for inhibiting coagulation in a mammal, which comprises administering an effective amount of the compound described in the above (1) or a salt thereof, or a prodrug thereof to the mammal;
(32) a method for inhibiting activated coagulation factor X in a mammal, which comprises an effective amount of the compound described in the above (1) or a salt thereof, or a prodrug thereof to the mammal;
(33) a method for preventing and/or treating cardiac infarction, cerebral thrombosis, deep vein thrombosis, pulmonary thrombotic embolus or thrombotic embolus during operation or after operation in a mammal, which comprises administering an effective amount of the compound described in the above (1) or a salt thereof, or a prodrug thereof to the mammal;
(34) use of the compound described in the above (1) or a salt thereof, or a prodrug thereof for manufacturing a medicament for inhibiting coagulation;
(35) use of the compound described in the above (1) or a salt thereof, or a prodrug thereof for manufacturing a medicament for inhibiting activated coagulation factor X;
(36) use of the compound described in the above (1) or a salt thereof, or a prodrug thereof for manufacturing a medicament for preventing or treating cardiac infarction, cerebral thrombosis, deep vein thrombosis, pulmonary thrombotic embolus or thrombotic embolus during operation or after operation; and the like.

### EMBODIMENTS FOR CARRYING OUT THE INVENTION

In the above formulas, R is an optionally substituted cyclic hydrocarbon group or an optionally substituted heterocyclic group (preferably an optionally substituted aryl group or an optionally substituted heterocyclic group).

Examples of the cyclic hydrocarbon group in the "optionally substituted cyclic hydrocarbon group" represented by R include an alicyclic hydrocarbon group, an aryl group, etc., with an aryl group being preferred.

Examples of the "alicyclic hydrocarbon group" as an example of the cyclic hydrocarbon group include a saturated or unsaturated alicyclic hydrocarbon group such as a cycloalkyl group, a cycloalkenyl group, a cycloalkadienyl group.and the like.

The "cycloalkyl group" mentioned here may for example be a C₃₋₉ cycloalkyl group such as cyclopropyl, cyclobutyl, cyclopentyl, cyclohexyl, cycloheptyl, cyclooctyl, cyclononyl and the like.

The "cycloalkenyl group" may for example be a C₃₋₉ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, 1-cyclohexen-1-yl, 1-cyclohepten-1-yl and the like.

The "cycloalkadienyl group" may for example be a C₄₋₆ cycloalkadienyl group such as 2,4-cyclopentadien-1-yl, 2,4-cyclohexadien-1-yl, 2,5-cyclohexadien-1-yl and the like.

The "aryl group" as an example of the cyclic hydrocarbon group may for example be a monocyclic or fused polycyclic aromatic hydrocarbon group, for example, with a C₆₋₁₄ aryl group such as phenyl, naphthyl, anthryl, phenanthryl, acenaphthylenyl and the like being preferred, and with phenyl, 1-naphthyl, 2-naphthyl and the like being preferred especially.

Examples of the cyclic hydrocarbon group also include a bicyclic or tricyclic hydrocarbon group resulted from a condensation of two to three groups (preferably 2 or more types of the groups) which may be same or different and selected from the group consisting of the alicyclic hydrocarbon groups and the aromatic hydrocarbon groups described above, such as 1,2-dihydronaphthyl, 1,2,3,4-tetrahydronaphthyl, indenyl, dihydrobenzocycloheptenyl, fluorenyl, etc., and the like.

The heterocyclic group in the "optionally substituted heterocyclic group" represented by R may for example be an aromatic heterocyclic group, a saturated or unsaturated non-aromatic heterocyclic group (aliphatic heterocyclic group) containing, as an atom constituting the ring system (ring atom), at least one (preferably 1 to 4, more preferably 1 to 2) atom of 1 to 3 species (preferably 1 to 2 species) of the heteroatoms selected from oxygen, sulfur and nitrogen atoms, and the like.

The "aromatic heterocyclic group" may for example be a 5- to 6-membered aromatic monocyclic heterocyclic group such as an aromatic monocyclic heterocyclic group, for example, furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.; a 8- to 16-membered (preferably 8- to 12-membered) aromatic fused heterocyclic group such as an aromatic fused heterocyclic group, for example, benzofuranyl, isobenzofuranyl, benzo[b]thienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, puteridinyl, carbazolyl, α-carbolinyl, β-carbolinyl, γ-carbolinyl, acridinyl, phenoxazinyl, phenothiazinyl, phenazinyl, phenoxathiynyl, thianthrenyl, phenathridinyl, phenathrolinyl, indolidinyl, pyrrolo[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.; and the like (preferably a heterocyclic ring formed by a condensation of one or two (preferably one) 5- to 6-membered aromatic monocyclic heterocyclic groups described above with one or two (preferably one) benzene rings , or a heterocyclic ring formed by a condensation of two or three (preferably two) same or different heterocyclic rings of 5-to 6-membered aromatic monocyclic heterocyclic groups described above, more preferably a heterocyclic ring formed by a condensation of the 5- to 6-membered aromatic monocyclic heterocyclic group described above, particularly, benzofuranyl, benzopyranyl, benzo[b]thienyl, etc.).

The "non-aromatic heterocyclic group" may for example be a 3- to 8-membered (preferably 5- to 6-membered) saturated or non-saturated (preferably saturated) non-aromatic heterocyclic group (aliphatic heterocyclic group) such as oxiranyl, azetidinyl, oxetanyl, thietanyl, pyrrolidinyl, tetrahydrofuryl, thioranyl, piperidyl, tetrahydropyranyl, morpholinyl, thiomorpholinyl, piperazinyl and the like, as well as a non-aromatic heterocyclic group formed as a result of a saturation of a part or all of the double bonds of an aromatic monocyclic heterocyclic group or an aromatic fused heterocyclic group described above such as 1,2,3,4-tetrahydroquiolyl, 1,2,3,4-tetrahydroisoquinolyl and the like.

Examples of the substituent on the "optionally substituted cyclic hydrocarbon group" and the "optionally substituted heterocyclic group" represented by R include an optionally substituted alkyl group, an optionally substituted alkenyl group, an optionally substituted alkynyl group, an optionally substituted aryl group, an optionally substituted cycloalkyl group, an optionally substituted cycloalkenyl group, an optionally substituted heterocyclic group, an optionally substituted amino group, an optionally substituted imidoyl group (for example a group represented by the formula -C(U')=N-U wherein each of U and U' is hydrogen atom or a substituent (U is preferably hydrogen atom)), an optionally substituted amidino group (for example a group represented by the formula -C(NT'T")=N-T wherein each of T, T' and T" is hydrogen atom or a substituent (T is preferably hydrogen atom)), an optionally substituted hydroxyl group, an optionally substituted thiol group, an optionally substituted alkylsulfinyl, an optionally substituted alkylsulfonyl, an optionally esterified carboxyl group, an optionally substituted carbamoyl group, an optionally substituted thiocarbamoyl group, an optionally substituted sulfamoyl group, a halogen atom (for example, fluoro, chloro, iodo and the like, preferably chloro, bromo and the like), cyano group, nitro group, a sulfonic acid-derived acyl group, a carboxylic acid-derived acyl group and the like, and any of these substituents may occur 1 to 5 times (preferably 1 to 3 times) in any possible positions. It may also possible that the "optionally substituted cyclic hydrocarbon group" and the "optionally substituted heterocyclic group" represented by R have oxo group, and when R is benzopyranyl then R may form benzo-α-pyronyl, benzo-γ-pyronyl and the like.

The aryl group in the "optionally substituted aryl group" as the substituent may for example be a C₆₋₁₄ aryl group such as phenyl, naphthyl, anthryl, phenanthryl, acenaphthylenyl and the like. The substituent on the aryl group mentioned here may for example be a lower alkoxy group (for example a C₁₋₆ alkoxy group such as methoxy, ethoxy, propoxy, etc.), a halogen atom (for example fluoro, chloro, bromo, iodo, etc.), a lower alkyl group (such as a C₁₋₆ alkyl group such as methyl, ethyl, propyl, etc.), a lower alkenyl group (for example a C₂₋₆ alkenyl group such as vinyl, allyl, etc.), a lower alkynyl group (for example a C₂₋₆ alkynyl group such as ethynyl, propargyl, etc.), an optionally substituted amino group, an optionally substituted hydroxyl group, cyano group, an optionally substituted amidino group, carboxyl group,. a lower alkoxycarbonyl group (for example a C₁₋₆ alkoxy-carbonyl group such as methoxycarbonyl, ethoxycarbonyl, etc.), an optionally substituted carbamoyl group (for example a carbamoyl group which may be substituted with a C₁₋₆ alkyl or acyl group (for example, formyl, C₂₋₆ alkanoyl, benzoyl, an optionally halogenated C₁₋₆ alkoxy-carbonyl, an optionally halogenated C₁₋₆ alkyl-sulfonyl, benzenesulfonyl, etc.) which may be substituted with a 5- to 6-membered aromatic monocyclic heterocyclic group (for example pyridinyl, etc.)), 1-azetidinylcarbonyl, 1-pyrrolidinylcarbonyl, piperidinocarbonyl, morpholinocarbonyl, thiomorpholinocarbonyl (sulfur atom may be oxidized), 1-piperazinylcarbonyl, etc.), and the like, and any of these substituents may occur 1 to 3 times in any possible positions.
Examples of the "optionally substituted amino group", "optionally substituted hydroxyl group" and "optionally substituted amidino group" as the substituents include those similar to the "optionally substituted amino group", "optionally substituted hydroxyl group" and "optionally substituted amidino group" as the substituents which may be possessed by the "optionally substituted cyclic hydrocarbon group" and "optionally substituted heterocyclic group" represented by R mentioned hereinafter.

The cycloalkyl group in the "optionally substituted cycloalkyl group" as the substituent may for example be a C₃₋₇ cycloalkyl group such as cyclopropyl, cyclobutyl, cyclopentyl, cyclohexyl, cycloheptyl, and the like. Examples of the substituent on the cycloalkyl group mentioned here include those similar to the substituent on the "optionally substituted aryl group" described above and may occur similar times.

The cycloalkenyl group in the "optionally substituted cycloalkenyl group" as the substituent may for example be a C₃₋₆ cycloalkenyl group such as cyclopropenyl, cyclobutenyl, cyclopentenyl, cyclohexenyl, and the like, and examples of the substituent on the optionally substituted cycloalkenyl group mentioned here include those similar to the substituent on the "optionally substituted aryl group" described above and may occur similar times.

The alkyl group in the "optionally substituted alkyl group" as the substituent may for example be a C₁₋₆ alkyl group such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 2,2-dimethylpropyl and the like. Examples of the substituent on the alkyl group mentioned here include those similar to the substituent on the "optionally substituted aryl group" described above and may occur similar times.

The alkenyl group in the "optionally substituted alkenyl group" as the substituent may for example be a C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like. Examples of the substituent on the alkenyl group mentioned here include those of the substituent on the "optionally substituted aryl group" described above and may occur similar times.

The alkynyl group in the "optionally substituted alkynyl group" as a substituent may for example be a C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like. Examples of the substituent on the alkynyl group mentioned here include those similar to the substituent on the "optionally substituted aryl group" described above and may occur similar times.

The heterocyclic group in the "optionally substituted heterocyclic group" as the substituent may for example be an aromatic heterocyclic group, a saturated or unsaturated non-aromatic heterocyclic group (aliphatic heterocyclic group) containing as an atom constituting a ring system (ring atom) at least one (preferably 1 to 4, more preferably 1 to 2) atom of 1 to 3 species (preferably 1 to 2 species) of the heteroatoms selected from oxygen, sulfur and nitrogen atoms.

The "aromatic heterocyclic group" may for example be a 5- to 6-membered aromatic monocyclic heterocyclic group such as an aromatic monocyclic heterocyclic group, for example, furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like; a 8- to 16-membered (preferably 8- to 12-memberd) aromatic fused heterocyclic group such as an aromatic fused heterocyclic group, for example, benzofuranyl, isobenzofuranyl, benzo[b]thienyl, indolyl, isoindolyl, 1H-indazolyl, benzindazolyl, benzoxazolyl, 1,2-benzoisooxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, puteridinyl, carbazolyl, α-carbolinyl, β-carbolinyl, γ-carbolinyl, acridinyl, phenoxazinyl, phenothiazinyl, phenazinyl, phenoxathinyl, , thianthrenyl, phenathridinyl, phenathrolinyl, indolidinyl, pyrrolo[1,2-b]pyridazinyl, pyrrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.; and the like (preferably a heterocyclic ring formed by a condensation of one or two (preferably one) 5- to 6-membered aromatic monocyclic heterocyclic groups described above with one or two (preferably one) benzene rings , or a heterocyclic ring formed by a condensation of two or three (preferably two) same or different heterocyclic rings of 5- to 6-membered aromatic monocyclic heterocyclic groups described above).

The "non-aromatic heterocyclic group" may for example be a 3- to 8-membered (preferred 5- to 6-membered) saturated or non-saturated (preferably saturated) non-aromatic heterocyclic group (aliphatic heterocyclic group) such as oxylanyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl and the like, as well as a non-aromatic heterocyclic group formed as a result of a saturation of a part or all of the double bonds of an aromatic monocyclic heterocyclic group or an aromatic fused heterocyclic group described above such as 1,2,3,4-tetrahydroquinolyl, 1,2,3,4-tetrahydroisoquinolyl and the like.

The substituent which may be possessed by the "optionally substituted heterocyclic group" as the substituent may for example be a lower alkyl group (for example a C₁₋₆ alkyl group such as methyl, ethyl, propyl, etc.), a lower alkenyl group (for example a C₂₋₆ alkenyl group such as vinyl, allyl, etc.), a lower alkynyl group (for example a C₂₋₆ alkynyl group such as ethynyl, propargyl, etc.), an acyl group (for example a C₁₋₆ alkanoyl group such as formyl, acetyl, propionyl, pivaloyl, etc., benzoyl, etc.), an optionally substituted amino group, an optionally substituted hydroxyl group, a halogen atom (for example, fluoro, chloro, bromo, iodo, etc., preferably chloro, bromo, etc.), an optionally substituted imidoyl group, an optionally substituted amidino group, and the like.

Examples of the "optionally substituted amino group", "optionally substituted hydroxyl group", "optionally substituted imidoyl group" and "optionally substituted amidino group" which may be possessed by the "optionally substituted heterocyclic group" as the substituent include those similar to the "optionally substituted amino group", "optionally substituted hydroxyl group", "optionally substituted imidoyl group" and "optionally substituted amidino group" as the substituent which may be possessed by "optionally substituted cyclic hydrocarbon group" and "optionally substituted heterocyclic group" represented by R.

The substituent on the "optionally substituted amino group", "optionally substituted imidoyl group", "optionally substituted amidino group", "optionally substituted hydroxyl group", "optionally substituted thiol group", "optionally substituted alkylsulfinyl" and "optionally substituted alkylsulfonyl" as the substituents which may be possessed by the "optionally substituted cyclic hydrocarbon group" and "optionally substituted heterocyclic group" represented by R may for example a lower alkyl group (for example a C₁₋₆ alkyl group such as methyl, ethyl, propyl, isopropyl, butyl, isobutyl, t-butyl, pentyl, hexyl, etc.) which may be substituted by a substituent selected from a halogen atom (for example fluoro, chloro, bromo, iodo, etc.) and an optionally halogenated C₁₋₆ alkoxy group (for example methoxy, ethoxy, trifluoromethoxy, 2,2,2-trifluoroethoxy, trichloromethoxy, 2,2,2-trichloroethoxy, etc.), an acyl group (a C₁₋₆ alkanoyl (for example formyl, acetyl, propionyl, pivaloyl, etc.), benzoyl, C₁₋₆ alkylsulfonyl (for example methanesulfonyl, etc.), benzenesulfonyl, etc.), an optionally halogenated C₁₋₆ alkoxy-carbonyl (for example, methoxycarbonyl, ethoxycarbonyl, trifluoromethoxycarbonyl, 2,2,2-trifluoroethoxycarbonyl, trichloromethoxycarbonyl, 2,2,2-trichloroethoxycarbonyl, etc.), a C₁₋₆ alkoxy-carbonyl which may be substituted with phenyl (for example benzyloxycarbonyl, etc.), a heterocyclic group (those similar to the "heterocyclic group" in the "optionally substituted heterocyclic group" represented by R, preferably pyridyl, more preferably 4-pyridyl, etc.), and the "amino group" in the "optionally substituted amino group" as the substituent may be substituted by an optionally substituted imidoyl group (for example a C₁₋₆ alkylimidoyl (for example formyl imidoyl, acetylimidoyl), a C₁₋₆ alkoxyimidoyl, a C₁₋₆ alkylthioimidoyl, amidino, etc.) and an amino group which may be substituted by 1 to 2 C₁₋₆ alkyls, or two substituents are taken together with a nitrogen atom to form a cyclic amino group, and in such case such cyclic amino group may for example be a 3- to 8-membered (preferably 5- to 6-membered) cyclic amino group such as 1-azetidinyl; 1-pyrrolidinyl; piperidino; thiomorpholino; morpholino; 1-piperazinyl; 1-piperazinyl which may have at its 4-position a lower alkyl group (for example a C₁₋₆ alkyl group such as methyl, ethyl, propyl, isopropyl, butyl, t-butyl, pentyl, hexyl, etc.), an aralkyl group (for example a C₇₋₁₀ aralkyl group such as benzyl, phenethyl, etc.), an aryl group (for example a C₆₋₁₀ aryl group such as phenyl, 1-naphthyl, 2-naphthyl, etc.); 1-pyrrolyl; 1-imidazolyl; etc.

The "optionally substituted carbamoyl group" may for example be unsubstituted carbamoyl as well as N-monosubstituted carbamoyl group and N,N-disubstituted carbamoyl group.

Examples of the substituent of the "N-monosubstituted carbamoyl group" include a lower alkyl group (for example a C₁₋₆ alkyl group such as methyl, ethyl, propyl, isopropyl, butyl, isobutyl, t-butyl, pentyl, hexyl, etc.), a lower alkenyl group (for example a C₂₋₆ alkenyl group such as vinyl, allyl, isopropenyl, propenyl, butenyl, pentenyl, hexenyl, etc.), a cycloalkyl group (for example a C₃₋₆ cycloalkyl group such as cyclopropyl, cyclobutyl, cyclopentyl, cyclohexyl, etc.), an aryl group (for example a C₆₋₁₀ aryl group such as phenyl, 1-naphthyl, 2-naphthyl, etc.), an aralkyl group (for example a C₇₋₁₀ aralkyl group such as benzyl and phenethyl, preferably a phenyl-C₁₋₄ alkyl group, etc.), an arylalkenyl group (for example a C₈₋₁₀ arylalkenyl group such as cinnamyl, preferably a phenyl-C₂₋₄ alkenyl group, etc.), a heterocyclic group (for example those similar to "heterocyclic ring" as the substituent on the "optionally substituted cyclic hydrocarbon group" represented by R described above), amino optionally substituted with one or two C₁₋₆ alkyls (for example a C₁₋₆ alkyl group such as methyl, ethyl, propyl, isopropyl, butyl, isobutyl, t-butyl, pentyl, hexyl, etc.), and the like. Each of such lower alkyl group, a lower alkenyl group, a cycloalkyl group, an aryl group, an aralkyl group, an arylalkenyl group and a heterocyclic group may have a substituent, and such substituent may for example be hydroxyl group, an optionally substituted amino group [such amino group may have as its substituents one or two groups selected from a lower alkyl group (for example a C₁₋₆ alkyl group such as methyl, ethyl, propyl, isopropyl, butyl, isobutyl, t-butyl, pentyl, hexyl, etc.), an acyl group (for example a C₁₋₆ alkanoyl group such as formyl, acetyl, propionyl, pivaloyl, etc., as well as benzoyl, etc.), carboxyl group, a C₁₋₆ alkoxy-carbonyl group and the like], a halogen atom (for example fluoro, chloro, bromo, iodo, etc.), nitro group, cyano group, a lower alkyl group which may be substituted by 1 to 5 halogen atoms (for example, fluoro, chloro, bromo, iodo, etc.), and the like. The lower alkyl group mentioned above may for example be a C₁₋₆ alkyl group such as methyl, ethyl, n-propyl, isopropyl, n-butyl, isobutyl, sec-butyl, tert-butyl, pentyl, hexyl, etc., with methyl, ethyl, etc. being preferred. The lower alkoxy group mentioned above may for example be a C₁₋₆ alkoxy group such as methoxy, ethoxy, n-propoxy, isopropoxy, n-butoxy, isobutoxy, sec-butoxy, tert-butoxy, etc., with methoxy, ethoxy, etc., being preferred. Any of these substituents may be the same or different and occurs preferably 1 or 2 to 3 times (preferably 1 or 2 times).

"N,N-Disubstituted carbamoyl group" means carbamoyl group having two substituents on the nitrogen atom, and examples of one of these substituents include those similar to the substituent on "N-monosubstituted carbamoyl group" described above, and examples of the other include a lower alkyl group (for example a C₁₋₆ alkyl group such as methyl, ethyl, propyl, isopropyl, butyl, t-butyl, pentyl, hexyl, etc.), a C₃₋₆ cycloalkyl group (for example cyclopropyl, cyclobutyl, cyclopentyl, cyclohexyl, etc.), a C₇₋₁₀ aralkyl group (for example benzyl, phenethyl, etc., preferably a phenyl-C₁₋₄ alkyl group, etc.), and the like. Alternatively, two substituents may be taken together with the nitrogen atom to form a cyclic amino group, and in such case a cyclic aminocarbamoyl group may for example be a 3- to 8-membered (preferably 5- to 6-membered) cyclic aminocarbonyl such as 1-azetidinylcarbonyl, 1-pyrrolidinylcarbonyl, piperidinocarbonyl, morpholinocarbonyl, thiomorpholinocarbonyl (the sulfur atom may be oxidized), 1-piperazinylcarbonyl as well as piperazinylcarbonyl which may have at its 4-position a lower alkyl group (for example a C₁₋₆ alkyl group such as methyl, ethyl, propyl, isopropyl, butyl, t-butyl, pentyl, hexyl, etc.), an aralkyl group (for example a C₇₋₁₀ aralkyl group such as benzyl, phenethyl, etc.), an aryl group (for example a C₆₋₁₀ aryl group such as phenyl, 1-naphthyl, 2-. naphthyl, etc.), etc., and the like

Examples of the substituent on the "optionally substituted thiocarbamoyl group" and "optionally substituted sulfamoyl group" include those similar to the substituent on the "optionally substituted carbamoyl group" described above.

The optionally esterified carboxyl group may for example be free carboxyl group as well as a lower alkoxycarbonyl group, an aryloxycarbonyl group, an aralkyloxycarbonyl group and the like.

The "lower alkoxycarbonyl group" may for example be a C₁₋₆ alkoxy-carbonyl group such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, etc., with a C₁₋₃ alkoxy-carbonyl group such as methoxycarbonyl, ethoxycarbonyl, propoxycarbonyl, etc., being preferred.

The "aryloxycarbonyl group" is preferably, for example, a C₇₋₁₂ aryloxy-carbonyl group such as phenoxycarbonyl, 1-naphthoxycarbpnyl, 2-naphthoxycarbonyl and the like.

The "aralkyloxycarbonyl group" is preferably, for example, a C₇₋₁₀ aralkyloxy-carbonyl group such as benzyloxycarbonyl and phenethyloxycarbony (preferably a C₆₋₁₀ aryl-C₁₋₄ alkoxy-carbonyl, etc.).

Each of such "aryloxycarbonyl group" and "aralkoxycarbonyl group" may have a substituent, and examples of such substituent include those similar to the substituent on the aryl group or the aralkyl group as the examples of a substituent on an N-monosubstituted carbamoyl group described above and may occur similar times.

The "sulfonic acid-derived acyl group" as the substituent may for example be sulfonyl bound to one substituent occurring on the nitrogen atom of "N-monosubstituted carbamoyl group" described above, and is preferably an acyl such as a C₁₋₆ alkylsulfonyl including methanesulfonyl, ethanesulfonyl, etc.

The "carboxylic acid-derived acyl group" as the substituent may for example be carbonyl bound to one substituent occurring on the nitrogen atom of "N-monosubstituted carbamoyl group" described above, and is preferably an acyl such as a C₁₋₆ alkanoyl such as formyl, acetyl, propionyl, pivaloyl, etc. as well as benzoyl, etc.

R is preferably an aryl group which may be substituted with a substituent selected from a halogen atom, C₁₋₆ alkyl, C₂₋₆ alkenyl, C₂₋₆ alkynyl, optionally substituted amino, nitro, cyano, optionally substituted amidino and optionally esterified or amidated carboxyl; or a heterocyclic group which may be substituted with a substituent selected from a halogen atom, C₁₋₆ alkyl, C₂₋₆ alkenyl, C₂₋₆ alkynyl, optionally substituted amino, nitro, cyano, optionally substituted amidino and optionally esterified or amidated carboxyl.

In particular, R is preferably optionally substituted aryl and, among them, preferred is aryl optionally substituted with a halogen atom or C₂₋₄ alkenyl (preferably a halogen atom) (preferably C₆₋₁₄ aryl such as phenyl, 1-naphthyl, 2-naphthyl, etc.).

It is also preferable that R is an optionally substituted heterocyclic group, especially a heterocyclic group which may be substituted by a halogen atom (preferably, benzofuranyl, benzopyranyl, etc., more preferably benzopyranyl).

Particularly preferred R is naphthyl which may be substituted with a halogen atom.

In the above formulas, W is a bond or an optionally substituted divalent chainlike hydrocarbon group.

Example of the "optionally substituted divalent chainlike hydrocarbon group" represented by W include C₁₋₆ alkylyne such as methylene, ethylene, trimethylene, tetramethylene, etc., C₂₋₆ alkenylyne such as vinylene, propylene, 1- or 2-butenylyne, butadienylene, etc., and the like.

Examples of the substituent which may be possessed by the divalent chainlike hydrocarbon group in the optionally substituted divalent chainlike hydrocarbon group" represented by W include the same substituents as those which may be possessed by the "cyclic hydrocarbon group" in the "optionally substituted cyclic hydrocarbon group" represented by the above R.

As W, a bond or C₁₋₆ alkylene are preferable and, inter alia, a bond is preferably used.

In the above formulas, X is an optionally substituted divalent hydrocarbon group (preferably an optionally substituted divalent chainlike hydrocarbon group or an optionally substituted phenylene group).

Examples of the "optionally substituted divalent hydrocarbon group" represented by X include an "optionally substituted divalent chainlike hydrocarbon group" and an "optionally substituted divalent cyclic hydrocarbon".

Examples of the "optionally substituted divalent chainlike hydrocarbon group" represented by X include C₁₋₈ alkylene such as methylene, trimethylene, tetramethylene and the like, C₂₋₈ alkenylene such as vinylene, propylene, 1- or 2-butenylene, butadienylene and the like, and C₂₋₈ alkynylene such as ethynylene, 1- or 2-propynylene, 1- or 2-butynylene and the like.

Examples of the "optionally substituted divalent cyclic hydrocarbon group" represented by X include an "optionally substituted divalent cyclic hydrocarbon group" formed by removing an optional hydrogen atom from the above-described "optionally substituted cyclic hydrocarbon group" represented by R and, inter alia, an "optionally substituted divalent aryl group", particularly, an "optionally substituted phenylene group" is preferred and, as the "optionally substituted phenylene group", there are 1,2-phenylyne, 1,3-phenylyne and 1,4-phenylene.

As the "optionally substituted divalent hydrocarbon group" represented by X, an optionally substituted divalent chainlike hydrocarbon group and an optionally substituted phenylene group are preferred and, inter alia, optionally substituted C₁₋₆ lower alkylene is preferred.

Examples of the substituent which may be possessed by the "optionally substituted cyclic hydrocarbon group" represented by X include the same substituents as those of the above-described "optionally substituted cyclic hydrocarbon group" represented by R and, inter alia, examples thereof include lower alkyl (e.g. C₁₋₆alkyl such as methyl, ethyl, propyl, etc.), lower alkenyl (e.g. C₂₋₆alkenyl such as vinyl, allyl, etc.), lower alkynyl (e.g. C₂₋₆alkynyl such as ethynyl, propargyl, etc.), optionally substituted amino, optionally substituted hydroxyl, cyano, optionally substituted amidino, carboxyl, lower alkoxycarbonyl (e.g. C₁₋₆ alkoxy-carbonyl such as methoxycarbonyl, ethoxycarbonyl, etc.), optionally substituted carbamoyl group (e.g. carbamoyl group optionally substituted with C₁₋₆ alkyl or acyl (e.g. formyl, C₂₋₆ alkanoyl, benzoyl, optionally halogenated C₁₋₆ alkoxycarbonyl, optionally haologenated C₁₋₆ alkylsulfonyl, benzenesulfonyl, etc.) and the like) and an oxo group, and these substituents may located at any 1 to 3 possible positions.

In the above-described formulas, Y and Z are independently -N(R⁶)-, -CO-, -S(O)-, -S(O)₂-, -CH₂-, -N(R⁶)-CO-, -CO-CH₂- or a bond (preferably -N(R⁶)-, -CO-, -S(O)-, -S(O)₂-, -CH₂- or a bond). Examples of a combination of Y and Z include:
the case where Y is -N(R⁶)- and Z is -CO-;
the case where Y is -N(R⁶)- and Z is -S(O)-;
the case where Y is -N(R⁶)- and Z is -S(O)₂-;
the case where Y is -N(R⁶)- and Z is -CH₂-;
the case where Y is -N(R⁶)- and Z is a bond;
the case where Y is -CO- and Z is -N(R⁶)-;
the case where Y is -CO- and Z is -CO-;
the case where Y is -CO- and Z is -S(O)-;
the case where Y is -CO- and Z is -S(O)₂-;
the case where Y is -CO- and Z is -CH₂-;
the case where Y is -CO- and Z is a bond;
the case where Y is -S(O)- and Z is -N(R⁶)-;
the case where Y is -S(O)- and Z is -CO-;
the case where Y is -S(O)- and Z is -S(O)-;
the case where Y is -S(O)- and Z is -S(O)₂-;
the case where Y is -S(O)- and Z is -CH₂-;
the case where Y is -S(O)- and Z is a bond:
the case where Y is -S(O)₂- and Z is -N(R⁶)-;
the case where Y is -S(O)₂- and Z is -CO-;
the case where Y is -S(O)₂- and Z is -S(O)-;
the case where Y is -S(O)₂- and Z is -S(O)₂-;
the case where Y is -S(O)₂- and Z is -CH₂-;
the case where Y is -S(O)₂- and Z is a bond;
the case where Y is -CH₂- and Z is -N(R⁶)-;
the case where Y is -CH₂- and Z is -CO-;
the case where Y is -CH₂- and Z is -S(O)-;
the case where Y is -CH₂- and Z is -S(O)₂-;
the case where Y is -CH₂- and Z is -CH₂-;
the case where Y is -CH₂- and Z is a bond;
the case where Y is a bond and Z is -N(R⁶)-;
the case where Y is a bond and Z is -CO-;
the case where Y is a bond and Z is -S(O)-;
the case where Y is a bond and Z is -S(O)₂-;
the case where Y is a bond and Z is -CH₂-;
the case where Y is a bond and Z is a bond.

In any of the above cases, any combination is possible regardless of b in the above formulae being 0 or 1, provided that when Y and Z are -N(R⁶)-, b is preferably 0.

Inter alia, the case where Y is -CO-, -S(O)₂- or a bond, and Z is -CO- or a bond is preferable and, particularly, the case where Y is -CO- or -S(O)₂- and Z is a bond; the case where Y is a bond and Z is -CO- and the like are preferred.

In addition, the case where Z is not -CO- is a preferable example.

In the above-described formulas, ring A is an optionally substituted nitrogen-containing heterocyclic ring.

Examples of the "nitrogen-containing heterocyclic ring" in the "optionally substituted nitrogen-containing heterocyclic ring" represented by A include the "heterocyclic ring" containing at least one nitrogen atom among the "heterocyclic rings" exemplified as the "heterocyclic ring" constituting the above-described "optionally substituted heterocyclic group" represented by R, for example, a nitrogen-containing aromatic heterocyclic group, saturated or unsaturated nitrogen-containing non-aromatic heterocyclic group (nitrogen-containing aliphatic heterocyclic group) and the like containing at least one nitrogen atom as an atom (ring atom) constituting a ring system and further optionally containing 1 to 3 kinds of (preferably 1 to 2 kinds of) 1 to 3 (preferably 1 to 2) hetero atom(s) selected from oxygen, sulfur and nitrogen atoms, and a nitrogen-containing aliphatic heterocyclic group (nitrogen-containing nonaromatic heterocyclic group) and the like are preferably used.

As the "nitrogen-containing heterocyclic ring" in the "optionally substituted nitrogen-containing heterocyclic ring" represented by ring A, a "monocyclic or bicyclic 5-to 12-membered nitrogen-containing heterocyclic ring" is preferred and, inter alia, a "monocyclic or bicyclic 5- to 12-membered nitrogen-containing aliphatic heterocyclic group" is preferable.

Examples of the "nitrogen-containing aliphatic heterocyclic ring" include, among those exemplified as the above-described "aromatic heterocyclic ring" constituting an aromatic monocyclic heterocyclic group or an aromatic fused heterocyclic group as the above R, a nitrogen-containing aliphatic heterocyclic group wherein a part or a whole of double bonds of a "nitrogen-containing aromatic heterocyclic ring" containing at least one nitrogen atom, such as 3- to 8-memebred (preferably 5- to 6-membered) saturated or unsaturated (preferably saturated) monocyclic nitrogen-containing aliphatic heterocyclic group such as azetidine, pyrrolidine, piperidine, morpholine, thiomorpholine, piperazine, homopiperazine, etc., 8- to 12-membered (preferably 10- to 12-membered) saturated or unsaturated bicyclic nitrogen-containing aliphatic heterocyclic group such as 1,2,3,4-tetrahydroquinoline, 1,2,3,4-tetrahydroisoquinoline, 4,5,6,7,-tetrahydrothioazolo[5,4-c]pyridine, 4,5,6,7-tetrahydropyrazolo[4,3-c]pyridine, 4,5,6,7-tetrahydroimidazo[4,5-c]pyridine, etc., and the like and, inter alia, piperazine and piperidine are preferably used.

Examples of the substituent which may be possessed by the "nitrogen-containing heterocyclic ring" in the "optionally substituted nitrogen-containing heterocyclic ring" represented by ring A include the same substituents as those which may be possessed by the "heterocyclic group" in the above-described "optionally substituted heterocyclic group" represented by R, as well as oxy group and thioxo group.

In addition, as a structure of the "optionally substituted nitrogen-containing heterocyclic ring" represented by ring A as well as a binding mode between Z and Z', those represented by the formula: 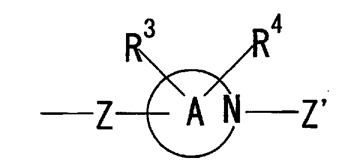 wherein ring A is the same as defined above, R³ and R⁴ are independently hydrogen atom, optionally substituted alkyl, optionally substituted alkenyl, optionally substituted alkynyl, optionally substituted hydroxyl, optionally substituted thiol, optionally substituted alkylsulfinyl, optionally substituted alkylsulfonyl, optionally substituted amino, optionally substituted carbamoyl, optionally esterified carboxyl, a halogen atom or cyano group, or R³ and R⁴ or R³ and R⁵ may be taken together to form a ring, etc., are preferably used.

In the above-described formulas, as the "optionally substituted alkyl", the "optionally substituted alkenyl", the "optionally substituted alkynyl", the "optionally substituted hydroxyl group", the "optionally substituted thiol group", the "optionally substituted alkylsulfinyl", the "optionally substituted alkylsulfonyl", the "optionally substituted amino", the "optionally substituted carbamoyl" and the "optionally esterified carboxyl" represented by R³ and R⁴, there are the same "optionally substituted alkyl", "optionally substituted alkenyl", "optionally substituted alkynyl", "optionally substituted hydroxyl group", "optionally substituted thiol group", "optionally substituted alkylsulfinyl", "optionally substituted alkylsulfonyl", "optionally substituted amino", "optionally substituted carbamoyl" and "optionally esterified carboxyl" as those represented by R and, inter alia, as R³ and R⁴, hydrogen atom, optionally substituted amino, optionally substituted carbamoyl and optionally esterified carboxyl are preferred.

In the above-described formulas, R⁵ is hydrogen atom, an optionally substituted hydrocarbon group, an optionally substituted alkoxy group, optionally esterified or amidated carboxyl or an optionally substituted acyl group (preferably a hydrogen atom, optionally substituted alkyl, optionally substituted alkenyl, optionally substituted alkynyl, optionally substituted aryl, optionally substituted alkoxy, optionally esterified carboxyl, optionally substituted carbamoyl (amidated carboxyl) or optionally substituted acyl), and R⁵ may bind to a substituent of X or a substituent of ring A to form a ring.

In the above-described formulas, examples of the "hydrocarbon group" in the "optionally substituted hydrocarbon group" represented by R⁵ include a "hydrocarbon group" formed by adding one hydrogen atom to one bond of the "divalent hydrocarbon group" in the above-described "optionally substituted divalent hydrocarbon group" represented by X and, examples of the substituent which may be possessed by the "hydrocarbon group" include the same substituents as the substituents which may be possessed by the "divalent hydrocarbon group" in the above-described "optionally substituted divalent hydrocarbon group" representd by X.

In the above-described formulas, as the "optionally substituted alkyl", the "optionally substituted alkenyl", the "optionally substituted alkynyl", the "optionally substituted alkoxy group", the "optionally substituted aryl group", the "optionally esterified carboxyl", the "optionally substituted carbamoyl" and the "optionally substituted acyl" represented by R⁵, there are the same "optionally substituted alkyl", "optionally substituted alkenyl", "optionally substituted alkynyl", "optionally substituted alkoxy group", "optionally substituted aryl group", "optionally esterified carboxyl", "optionally substituted carbamoyl" and "optionally substituted acyl" represented by R. Inter alia, as R⁵, hydrogen atom or optionally substituted C₁₋₆alkyl is preferred.

In addition, when R⁵ binds to a substituent (preferably R³ as the substituent of ring A in the above-described formulas) of ring A to form a ring, ring A may be taken together with -N-Z- (Z is carbon atom or nitrogen atom which binds to ring A) to form an "optionally substituted spiro ring" or an "optionally substituted nitrogen-containing fused heterocyclic ring". That is, when the replacing position of a substituent of ring A to which R⁵ binds is on such carbon atom that Z binds to ring A, an "optionally substituted spiro ring" is formed, and, when the replacing position of a substituent of ring A to which R⁵ binds is not such an atom (carbon atom or nitrogen atom) that Z binds to ring A, an "optionally substituted nitrogen-containing fused heterocyclic ring" is formed.

Examples of the "spiro ring" in the "optionally substituted spiro ring" include spiro rings formed by binding of two rings such as saturated or unsaturated nitrogen-containing aliphatic heterocyclic ring optionally containing 1 to 3 kinds of (preferably 1 to 2 kinds of) 1 to 4 (preferably 1 to 2) hetero atom(s) selected from oxygen, sulfur and nitrogen atoms as an atom constituting a ring (ring atom) besides one nitrogen atom contained in a ring, etc. Examples thereof include spiro rings formed by binding of the same or different two rings selected from 3-to 8-membered (preferably 5- to 6-membered) saturated or unsaturated nitrogen-containing aliphatic heterocyclics such as azetidine, pyrrolidine, pyrazolidine, oxazolidine, isoxazolidine, imidazolidine, thiazolidine, isothiazolidine, pyrazoline, 1,2,4-oxadiazoline, oxazoline, isoxazoline, imidazoline, thiazoline, isothiazoline, piperidine, morpholine, thiomorpholine, piperazine, and the like.

Examples of the "nitrogen-containing fused heterocyclic ring" in the "optionally substituted fused heterocyclic ring" include rings formed by fusing the same heterocyclic rings as those (preferable monocyclic heterocyclic rings) in the "optionally substituted heterocyclic rings" represented by R, with ring A.

When R⁵ binds to a substituent (preferably R³ as the substituent of ring A in the above-described formulas) to form a ring, that is, when ring A is taken together with -N-Z- (Z is carbon atom or nitrogen atom binding to ring A) to form an "optionally substituted spiro ring" or an "optionally substituted nitrogen-containing fused heterocyclic ring", examples of the substituent of the "optionally substituted spiro ring" and the "optionally substituted nitrogen-containing heterocyclic ring" include the same substituents as those which may be possessed by the "heterocyclic group" in the above-described "optionally substituted heterocyclic group" represented by R, as well as oxo group and thioxo group and, inter alia, the same "optionally substituted alkyl", "optionally substituted alkenyl", "optionally substituted alkynyl", "optionally substituted amino", "optionally esterified carboxyl" and "optionally substituted carbamoyl" as those as the substituent which may be possessed by the "optionally substituted heterocyclic group" represented by R as well as oxo group and thioxo group are preferred.

These substituents may replace at any replaceable 1 to 3 positions and, inter alia, optionally substituted C₁₋₆ alkyl group, oxo group and thioxo group are preferably used as the substituent.

In addition, R⁵ may bind to the substituent of X to form a ring, and examples of the ring include the same "optionally substituted nitrogen-containing heterocyclic ring" as that represented by ring A (preferably piperidine ring).

In the above-described formulas, R⁶ is hydrogen atom, an optionally substituted hydrocarbon group, an optionally substituted alkoxy group, optionally esterified or amidated carboxyl or an optionally substituted acyl group (preferably hydrogen atom, an optionally substituted alkyl, optionally substituted alkenyl, optionally substituted alkynyl, optionally esterified carboxyl, optionally substituted acyl and the like).

Examples of the "hydrocarbon group" in the "optionally substituted hydrocarbon group" represented by R⁶ in the above-described formulas include a "hydrocarbon group" formed by adding one hydrogen atom to the "divalent hydrocarbon group" in the above-described "optionally substituted divalent hydrocarbon group" represented by X, and examples of the substituent which may be possessed by the "hydrocarbon group" include the same substituents as those which may be possessed by the "divalent hydrocarbon group" in the above-described "optionally substituted divalent hydrocarbon group" represented by X.

In the above-described formulas, examples of the "optionally substituted alkyl", the "optionally substituted alkenyl", the "optionally substituted alkynyl", the "optionally substituted alkoxy", the "optionally substituted aryl", the "optionally esterified carboxyl", the "optionally substituted carbamoyl (amidated carboxyl)" and the "optionally substituted acyl" include the same "optionally substituted alkyl", "optionally substituted alkenyl", "optionally substituted alkynyl", "optionally substituted alkoxy", "optionally substituted aryl", "optionally esterified carboxyl", "optionally substituted carbamoyl (amidated carboxyl)" and "optionally substituted acyl" as the substituent which may be possessed by the "optionally substituted heterocyclic group" represented by R.

In the above-described formulas, a is 0, 1 or 2 (preferably 2).

In the above-described formulas, b is 0 or 1 (preferably 1).

In the above-described formulas, Z' is an optionally substituted imdoyl group or an optionally substituted nitrogen-containing heterocyclic group.

Examples of the "optionally substituted imidoyl group" represented by Z' include a group represented by the formula -C(R')=N-R" [wherein R" is hydrogen atom, an optionally substituted hydroxyl group, an optionally substituted hydrocarbon group or an acyl group derived from carboxylic acid, and R' is hydrogen atom, an optionally substituted hydrocarbon group, an acyl group derived from carboxylic acid, an optionally substituted amino group, an optionally substituted thiol group or an optionally substituted hydroxyl group] and the like.

In addition, in the "optionally substituted imidoyl group", when R' is thiol group or hydroxyl group, and R" is hydrogen atom, the "optionally substituted imidoyl group" may be a group represented by the formula -C(=O)-NH₂ or -C(=S)-NH₂, respectively.

In the above-described formulas, examples of the "optionally substituted hydrocarbon group" represented by R' and R" include the same "optionally substituted hydrocarbon group" as that represented by the above-described R⁵; examples of the "acyl group derived from carboxylic acid" represented by R' and R" include the same "acyl group derived from carboxylic acid" as that as the substituent which may be possessed by the above-described "optionally substituted cyclic hydrocarbon group" represented by R; examples of the "optionally substituted hydroxyl group" represented by R' and R" include the same "optionally substituted hydroxyl group" as that as the substituent which may be possessed by the above-described "optionally substituted cyclic hydrocarbon group" represented by R; examples of the "optionally substituted thiol group" represented by R' include the same "optionally substituted thio group" as that as the substituent which may be possessed by the above-described "optionally substituted cyclic hydrocarbon group" represented by R; and examples of the "optionally substituted amino group" represented by R' include the same "optionally substituted amino group" as that as the substituent which may be possessed by the above-described "optionally substituted cyclic hydrocarbon group" represented by R, or an amino group optionally having 1 to 2 "optionally substituted hydrocarbon group" represented by the above-described R⁵, etc.

In the compound represented by the formula (I), the compound wherein R" is an acyl group derived from carboxylic acid is useful as a prodrug of the compound wherein R" is hydrogen atom.

Examples of the "acyl group derived from carboxylic acid" represented by R" include the same "acyl group derived from carboxylic acid" as the substituent which may be possessed by the above-described "optionally substituted cyclic hydrocarbon group" represented by R, and the "acyl group derived from carboxylic acid" represented by R may be optionally esterified carboxyl such as a group represented by the formula -COOR"' [wherein R"' is an optionally substituted hydrocarbon group].

Examples of the "optionally substituted hydrocarbon group" represented by R"' include the same "optionally substituted hydrocarbon group" as that representd by above-described R⁵.

Preferred examples of the "hydrocarbon group" in the "optionally substituted hydrocarbon group" represented by R"' include C₁₋₆ alkyl, C₂₋₆ alkenyl, C₃₋₆ cycloalkyl, C₆₋₁₀ aryl, C₆₋₁₀ aryl-C₁₋₄ alkyl, etc. Examples of the substituent which may be possessed by the "hydrocarbon group" include the same number of the same substituents as those which may be possessed by the above-described "optionally substituted hydrocarbon group" represented by R⁵.

Examples of the group represented by the formula -COOR"' include, inter alia, a C₁₋₆ alkoxy-carbonyl group (e.g. methoxycarbonyl, ethoxycarbonyl, isopropoxycarbonyl etc.), C₁₋₆ alkanoyloxy-C₁₋₆ alkoxy-carbonyl group (e.g. pivaloyloxymethoxycarbonyl, 1-(acetoxy)ethoxycarbonyl, acetoxy-tert-butoxycarbonyl etc.), C₁₋₆ alkoxy-carbonyloxy-C₁₋₆ alkoxy-carbonyl group (e.g. ethoxycarbonyloxymethoxycarbonyl etc.), 5-C₁₋₄ alkyl-2-oxo-dioxolen-4-yl-C₁₋₆ alkoxy-carbonyl group (e.g. 5-methyl-2-oxo-dioxolen-4-ylmethoxycarbonyl, etc.), and the like.

Here, when R' is an optionally substituted amino group (preferably, amino, methylamino, ethylamino, propylamino, dimethylamino, diethylamino, hydrazino, piperidino, piperazino, morpholino, thiomorpholino, etc.), the "optionally substituted imidoyl group" represented by Z' forms an optionally substituted amidino group. Examples of such the optionally substituted amidino group include an amidino group which may be substituted with 1 to 2 lower (C₁₋₆) alkyl group(s), lower (C₁₋₆) alkanoyl group(s) or benzoyl group(s) (e.g. amidino, N-methylamidino, N-ethylamidino, N-propylamidino, N,N'-dimethylamidino, N,N'-diethylamidino, N-methyl-N'-diethylamidino, N-formylamidino, N-acetylamidino, etc.), and the like.

In the above-described formulas, preferred examples of R" include hydrogen atom, a lower alkyl group (e.g. C₁₋₆ alkyl group such as methyl, ethyl, propyl, isopropyl, butyl, isobutyl etc.), an acyl group (e.g. C₁₋₆ alkanoyl such as formyl, acetyl, propionyl, pivaloyl etc.; benzoyl; C₁₋₈ alkoxycarbonyl such as methoxycarbonyl, ethoxycarbonyl, propoxycarbonyl, isopropoxycarbonyl, butoxycarbonyl, isobutoxycarbonyl etc.; C₇₋₁₀ aralkyloxycarbonyl such as benzyloxycarbonyl, phenethyloxycarbonyl etc.), hydroxyl group and the like, and preferred examples of R' include hydrogen atom, lower alkyl (e.g. C₁₋₆ alkyl such as methyl, ethyl, propyl, isopropyl, butyl, isobutyl etc.), an optionally substituted amino group (e.g. amino group optionally substituted with 1 to 2 the same or different lower alkyl group (e.g. C₁₋₆ alkyl group such as methyl, ethyl, propyl, isopropyl, butyl, isobutyl etc.,) or an amino group optionally substituted with an acyl group (e.g. C₁₋₆ alkanoyl such as formyl, acetyl, propionyl, pivaloyl etc., benzoyl etc.), hydrazine group, 5- to 6-membered cyclic amino group (e,g, piperidino, thiomorpholino, morpholino, piperazino etc.) etc.), hydroxyl group, a lower alkoxy group (e.g. C₁₋₆ alkoxy group such as methoxy, ethoxy, propoxy, isopropoxy, butoxy, isobutoxy etc.), mercapto group, a lower alkylthio group (e.g. C₁₋₆ alkylthio group such as methylthio, ethylthio, propylthio, isopropylthio, butylthio, isobutylthio, etc.), and the like.

In the above-described formulas, as R", hydrogen atom is preferred.

In the above-described formulas, as R', hydrogen atom, a lower alkyl group or an optionally substituted amino group is preferred and, inter alia, a lower alkyl group or an optionally substituted amino group is preferred, particularly, an optionally substituted amino group (preferably amino optionally substituted with C₁₋₄ alkyl) is preferred.

Examples of the "nitrogen-containing heterocyclic group" in the "optionally substituted nitrogen-containing heterocyclic group" represented by Z' include an aromatic nitrogen-containing heterocyclic group and a saturated or unsaturated nonaromatic nitrogen-containing heterocyclic group (aliphatic nitrogen-containing heterocyclic group) which contain at least one (preferably 1 to 4, more preferably 1 to 3) nitrogen atom in addition to carbon atoms as an atom constituting a ring system (ring atom) and may contain further 1 to 3 hetero atom(s) selected from oxygen atom and sulfur atom.

Examples of the "aromatic nitrogen-containing heterocyclic group" include an aromatic monocyclic nitrogen-containing heterocyclic group such as pyrrolyl, oxazolyl, isooxazolyl, thiazolyl, isothiazolyl, imidazolyl, (1H-imidazol-1-yl, 1H-imidazol-4-yl etc.), pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl (1,2,4-triazolyl-1-yl, 1,2,4-triazolyl-4-yl etc.), tetrazolyl, pyridyl (2-, 3- or 4-pyridyl), piridazinyl, pyrimidinyl, pyrazinyl, triazinyl and the like, and a N-oxide thereof, as well as 8- to 16-membered (preferably 8- to 12-membered) aromatic fused nitrogen-containing heterocyclic group such as indolyl, isoindolyl, 1H-indazolyl, benzindazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenatrizinyl, phenathrolinyl, indolizinyl, pyrrolo[1,2-b]pyridazinyl, pyrazolo[1,5-a]pyridyl, imidazo[1,2-a]pyrizyl, imidazo[1,5-a]pyrizyl, imidazo[1,2-b]pyridazinyl, imidazo[1,2-a]pyrimizinyl, 1,2,4-triazolo[4,3-a]pyridyl, 1,2,4-triazolo[4,3-b]pyridazinyl and the like, and N-oxide thereof (preferably a heterocyclic ring in which 1-2 (preferably one) above-described 5- to 6-membered aromatic monocyclic nitrogen-containing heterocyclic group(s) are fused with 1 to 2 (preferably one) benzene ring(s), or a heterocyclic ring in which same or different 2 to 3 (preferably 2) heterocyclic rings of 5- to 6-membered above-described aromatic monocyclic nitrogen-containing heterocyclic groups are fused, more preferably a heterocyclic ring in which the above-described 5- to 6-membered aromatic monocyclic nitrogen-containing heterocyclic group is fused with benzene ring), and the like.

Inter alia, a 5- to 6-membered aromatic monocyclic nitrogen-containing heterocyclic group is preferred and, in particular, imidazolyl and pyridyl are preferred.

In addition, as pyridyl, 4-pyridyl is preferred, and this 4-pyridyl may have a substituent (e.g,. optionally substituted lower alkyl group) at the 2-position.

Examples of the "non-aromatic nitrogen-containing heterocyclic group" include, in addition to partially reduced above-described "aromatic nitrogen-containing heterocyclic group" (e.g. imidazolinyl, tetrahydropyrimidinyl etc.), azetidinyl, pyrrolidinyl, piperidyl (2-, 3- or 4-piperidyl), morpholinyl, thiomorpholinyl, piperazinyl (1-piperazinyl etc.), homopiperazinyl and the like and, inter alia, a 5- to 6-membered nonaromatic monocyclic nitrogen-containing heterocyclic group is preferred.

As the substituent of the "nitrogen-containing heterocyclic group" represented by Z', the same substituent as that of the "heterocyclic group" represented by R is used. In addition, the nitrogen atom constituting the nitrogen-containing heterocyclic group may be oxidized. In addition, the substituents of the "nitrogen-containing heterocyclic group" represented by Z may bind to each other to form a ring (e.g. benzene ring).

As Z', an optionally substituted nitrogen-containing heterocyclic group is preferred and, inter alia, a nitrogen-containing heterocyclic group optionally substituted with a substituent selected from an optionally substituted C₁₋₄ alkyl and optionally substituted amino is preferred.

In addition, as Z', an optionally substituted aromatic nitrogen-containing heterocyclic group is preferred and, inter alia, an optionally substituted pyridyl group is preferred and, in particular, an optionally substituted pyridyl group which binds to ring A at 4-position of pyridine ring is preferred.

As a preferred aspect of the optionally substituted nitrogen-containing heterocyclic group as Z', there is a group represented by the formula: 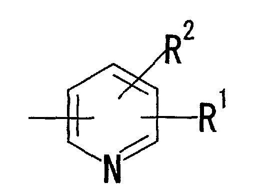 wherein R¹ and R² are independently hydrogen atom, optionally substituted alkyl, optionally substituted alkenyl, optionally substituted alkynyl, optionally substituted amino, optionally esterified carboxyl, optionally substituted carbamoyl, a halogen atom, cyano group or nitro group, or R¹ and R² may bind to each other to form a ring.

In the above-described formula, examples of the "optionally substituted alkyl", the "optionally substituted alkenyl", the "optionally substituted alkynyl", the "optionally substituted amino", the "optionally esterified carboxyl" and the "optionally substituted carbamoyl" include the same "optionally substituted alkyl", "optionally substituted alkenyl", "optionally substituted alkynyl", "optionally substituted amino", "optionally esterified carboxyl" and "optionally substituted carbamoyl" as those represented by R. Inter alia, optionally substituted C₁₋₄ lower alkyl or optionally substituted amino is preferred, and, more preferably, one of R¹ and R² is hydrogen atom.

As the compound (I), N-[3-[(6-chloro-2-naphthyl)sulfonyl]propyl]-N-methyl-1-(4-pyridyl)-4-piperidinecarboxamide, methyl 2-[N-[3-[(6-chloro-2-naphthyl)sulfonyl]propyl]-N-[1-(4-pyridyl)-4-piperidyl]carbonylamino]acetate, 3-[(6-chloro-2-naphtyl)sulfonyl]-N-methyl-N-[1-(4-pyridyl)-4-piperidyl]propanamide, ethyl 2-[N-[3-[(6-chloro-2-naphthyl)sulfonyl]propanoyl]-N-[1-(4-pyridyl)-4-piperidyl]amino]acetate, ethyl 3-[N-[3-[(6-chloro-2-naphthyl)sulfonyl]propanoyl]-N-[1-(4-pyridyl)-4-piperidyl]amino]propionate, 3-[[6-chloro-2-naphthyl]sulfonyl]-N-methyl-N-[1-(2-methyl-4-pyridyl)-4-piperidyl]propanamide, N-[2-(acetylamino)ethyl]-3-[(6-chloro-2-naphthyl)sulfonyl]-N-[1-(2-methyl-4-pyridyl)-4-piperidyl]propanamide, N-(2-aminoethyl)-3-[(6-chloro-2-naphthyl)sulfonyl]-N-[1-(4-pyridyl)-4-piperidyl]propanamide (preferably ditrifluoroacetate thereof), N-[2-(acetylamino)ethyl]-3-[(6-chloro-2-naphthyl)sulfonyl]-N-[1-(4-pyridyl)-4-piperidyl]propanamide, 3-[(6-chloro-2-naphthyl)sulfonyl]-N-[2-[(methanesulfonyl)amino]ethyl]-N-[1-(4-pyridyl)-4-piperidyl]propanamide, 3-[(6-bromo-2-naphthyl)sulfonyl]-N-methyl-N-[1-(2-methyl-4-pyridyl)-4-piperidyl]propanamide, 3-[(6-chloro-2-naphthyl)sulfonyl]-N-[1-(2-methyl-4-pyridyl)-4-piperidyl]-N-[3-(1-oxide-4-thiomorpholinyl)-3-oxopropyl]propanamide, N-[2-(N-acetyl-N-methylamino)ethyl]-3-[(6-chloro-2-naphthyl)sulfonyl]-N-[1-(2-methyl-4-pyridyl)-4-piperidyl]propanamide, 3-[(6-chloro-2-naphthyl)sulfonyl]-N-methyl-N-[1-(2,6-dimethyl-4-pyridyl)-4-piperidyl]propanamide and 1-[3-[(6-chloro-2-naphthyl)sulfonyl]propanoyl-4-(2-methyl-4-pyridyl)piperazine or salts thereof are particularly preferably used.

A prodrug for compound (I) means a compound which is converted into compound (I) by a reaction with an enzyme or a gastric acid under an in vivo physiological condition, i.e. a compound which undergoes an enzymatic oxidation, reduction or hydrolysis to form compound (I) and a compound which is hydrolyzed by a gastric acid to form compound (I). A prodrug for compound (I) may for example be a compound resulting from an acylation, an alkylation or a phosporylation of amino group of compound (I) (for example, a compound resulting from eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation of an amino acid of compound (I)), a compound resulting from an acylation, an alkylation, a phosphorylation and a boration of hydroxyl group of compound (I) (for example a compound resulting from acetylation, palmitoylation, propanoylation, pivaloylation, succinylation, fumarylation, alanylation, dimethylaminomethylcarbonylation of hydroxyl group of compound (I)), or a compound resulting from an esterification or an amidation of carboxyl group of compound (I) (for example a compound resulting from ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, 1-(cyclohexyloxycarbonyl)ethyl esterification, methylamidation of carboxyl group of compound (I)) and the like. Any of these compounds can be produced from compound (I) by a method known per se.

A prodrug for compound (I) may also be a compound which is changed into compound (I) under a physiological condition described in "IYAKUHIN NO KAIHATSU (Pharmaceutical development)", Vol.7, Molecular design, p163-198, HIROKAWA SHOTEN, 1990.

A salt of compound (I) may for example be a pharmaceutically acceptable salt such as an acid addition salt with trifluoroacetic acid, acetic acid, lactic acid, succinic acid, maleic acid, tartaric acid, citric acid, gluconic acid, ascorbic acid, benzoic acid, methanesulfonic acid, p-toluenesulfonic acid, cinnamic acid, fumaric acid, phosphonic acid, hydrochloric acid, nitric acid, hydrobromic acid, hydroiodic acid, sulfamic acid, sulfuric acid and the like, a metal salt with sodium, potassium, magnesium, calcium and the like, an organic salt with trimethylamine, triethylamine, pyridine, picolin, N-methylpyrrolidine, N-methylpiperidine, N-methylmorpholine and the like.

When an optically active form of compound (I) is required, for example, it can be obtained by using an optically active starting material, or resolution of a racemic form of the compound by using a conventional method.

Compound (I) or a salt thereof can be produced, for example, by the following processes A to F. Each of compounds described in the following reaction formulas may form a salt in so far as it does not interfere with the reaction and, as such a salt, there are the same salts as those of compound (I).

### Process A

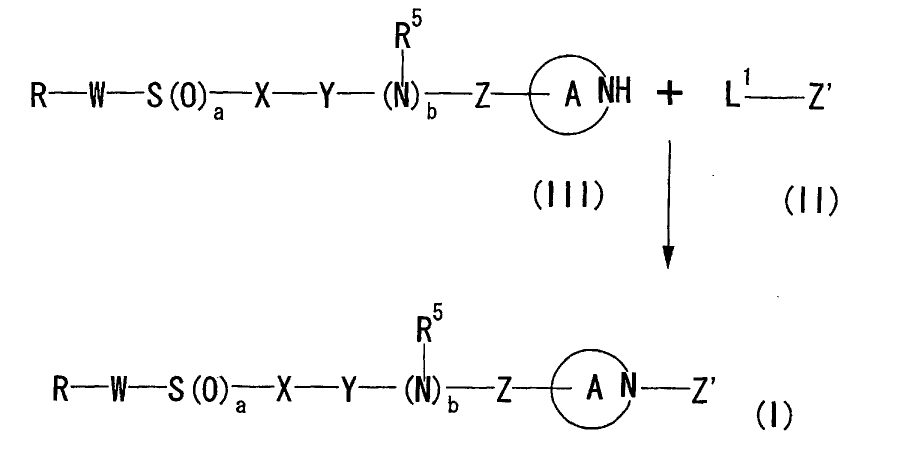

### Process B

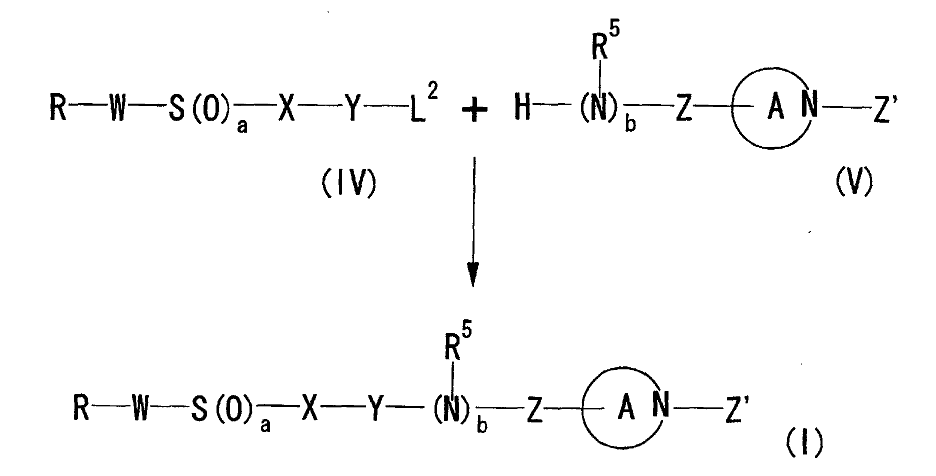

### Process C

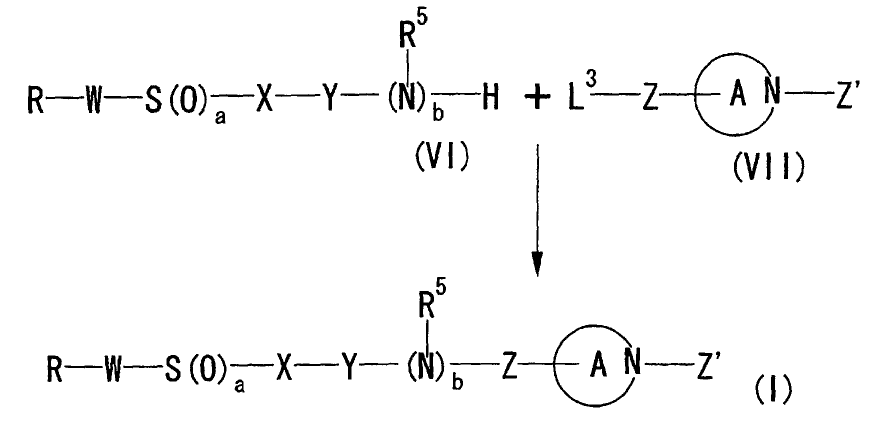

### Process D

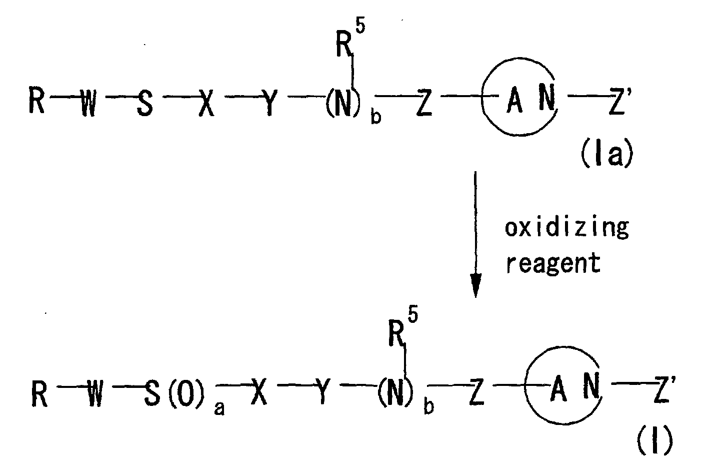

### Process E

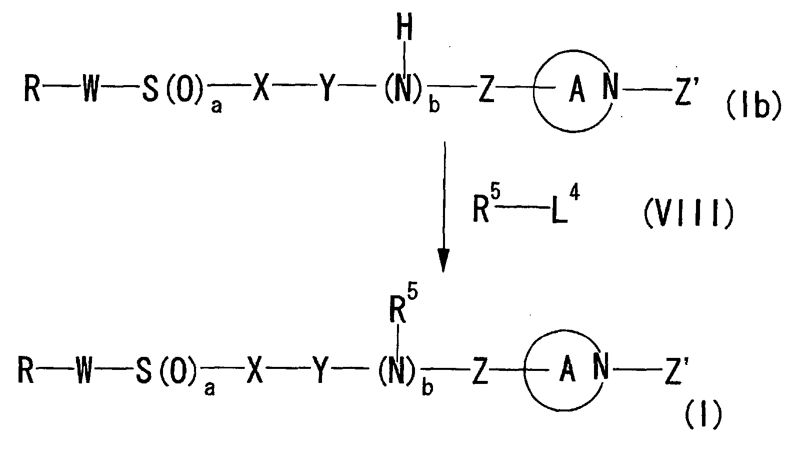

### Process F

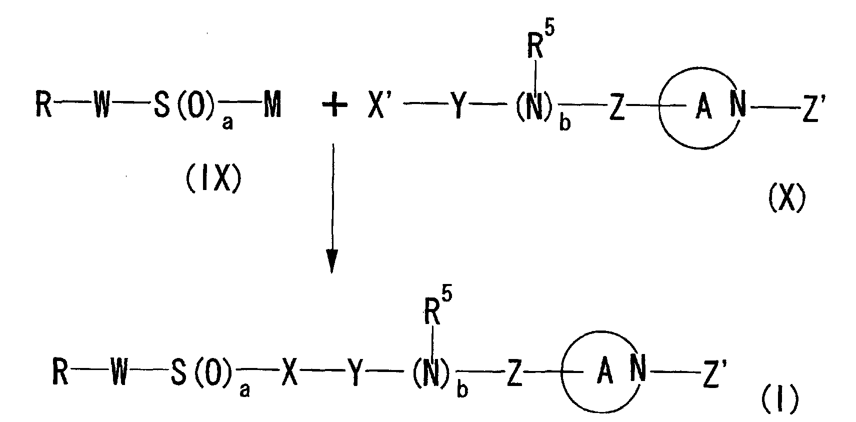

### Process A

Compound (I) can be produced by reacting compound (II) represented by the formula (II):

**L**^{**1**}**―Z'**

wherein L¹ is a leaving group (e.g. a halogen atom (e.g. fluoro, chloro, bromo, iodo etc.) or a reactive derivative of sulfonic acid (e.g. a group forming sulfonic acid ester, active sulfonic acid amide (e.g. 1,2,4-triazolide, imidazolide etc.), quaternary amine sulfonyl (e.g. N-methylpyrrolidium salt etc.), bissulfonylimide (e.g. N-phenylbissulfonylimide etc.) etc.), and the other symbols are the same as defined above, or a salt thereof, with compound (III) represented by the formula (III): 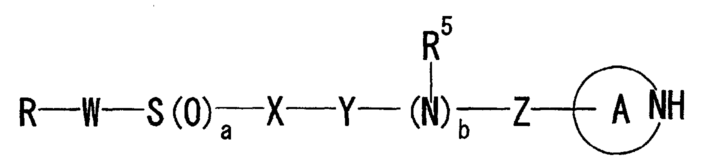 wherein the symbols are the same as defined above, or a salt thereof. Examples of the salts of compound (II) and compound (III) include the above-described acid addition salts with acids which form an acid addition salt with compound (I).

This reaction is generally carried out in a solvent, and a solvent which does not interfere with the reaction is selected. As such a solvent, there are alcohols (e.g. methanol, ethanol, propanol, isopropanol, butanol, tert-butanol etc.), ethers (e.g. dioxane, tetrahydrofuran, diethyl ether, tert-butyl methyl ether, diisopropyl ether, ethylene glycol dimethyl ether, etc.), esters (e.g. ethyl formate, ethyl acetate, n-butyl acetate etc.), carboxylic acids (e.g. formic acid, acetic acid, propionic acid etc.), halogenated hydrocarbons (e.g. dichloromethane, chloroform, carbon tetrachloride, trichloroethylene, 1,2-dichloroethane, chlorobenzene etc.), hydrocarbons (e.g. n-hexane, benzene, toluene etc.), amides (e.g. formamide, N,N-dimethylformamide, N,N-dimethylacetamide etc.), ketones (e.g. acetone, methyl ethyl ketone, methyl isobutyl ketone etc.), nitriles (e.g. acetonitrile, propionitrile etc.), and dimethyl sufoxide, sulfolane, hexamethylphosphoramide, water and the like, and they are used alone or in combination thereof.

This reaction may be carried out in the presence of a base, if necessary, and as such a base, there are inorganic bases such as lithium hydroxide, potassium hydroxide, sodium hydroxide, calcium hydroxide, sodium carbonate, potassium carbonate, sodium bicarbonate, potassium bicarbonate and the like, alkali metal salts of a C₁₋₆lower aliphatic fatty acid such as sodium formate, sodium acetate, potassium acetate and the like, and tertiary amine such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-diazabicyclo[4.3.0]non-5-ene (DBN), 1,8-diazabicyclo[5.4.0]-7-endecene (DBU), 1,4-diazabicyclo[2.2.2]octane and the like.

The reaction uses compound (II) in an amount of 0.5 to 5 equivalents, preferably 0.8 to 2 equivalents based on the compound (III).

The reaction temperature is -20 to 200°C, preferably 0 to 170°C.

The reaction time is varied depending upon kinds of compound (II) and compound (III), a kind of the solvent, the reaction temperature and the like, and is usually about 1 minute to about 72 hours, preferably about 15 minutes to about 24 hours.

### Process B

Compound (I) can be produced by reacting compound (IV) represented by the formula (IV):

R-W-S(O)ₐ-X-Y-L²

wherein L² is a leaving group (e.g. a halogen atom (e.g. fluoro, chloro, bromo, iodo, etc.), a C₁₋₆ alkylsulfonyloxy group optionally substituted with 1 to 3 halogen atom(s) (e.g. methanesulfonyloxy, ethanesulfonyloxy, trifluoromethanesulfonyloxy etc.), an optionally substituted arylsulfonyloxy group (e.g. benzenesulfonyloxy group, p-toluenesulfonyloxy group, p-bromobenzenesulfonyloxy group etc.) or hydroxyl group, which is a group forming a free carboxylic acid, a salt thereof (inorganic salt, organic acid etc.) or a reactive derivative (e.g. acid halide, ester, acid azide, acid anhydride, mixed acid anhydride, active amide, active ester, active thioester etc.) etc.), and the other symbols are the same as defined above, with compound (IV) represented by the formula (V): 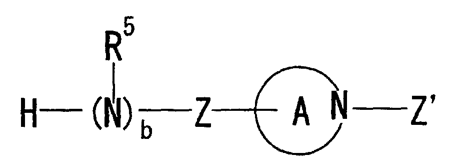 wherein the symbols are the same as defined above.

This process is carried out by reacting compound (V) or a salt thereof with the free acid (IV) or a salt thereof (inorganic acid, organic acid etc.) or a reactive derivative thereof (e.g. acid halide, ester, acid azide, acid anhydride, mixed acid anhydride, active azide, active ester, active thioester etc.). Examples of the salt of compound (V) include an acid addition salt with an acid which form an acid addition salt with the above-described compound (I).

As the inorganic salt used for compound (IV), there are alkali metal salts (e.g. sodium, potassium etc.), alkaline earth metal salts (e.g. potassium salt etc.) and, as the organic salt, there are, for example, a trimethylamine salt, a triethylamine salt, a tert-butyldimethylamine salt, a dibenzylmethylamine salt, a benzyldimethylamine salt, a N,N-dimethylaniline salt, a pyridine salt, a quinoline salt and the like. In addition, examples of acid halide include acid chloride, acid bromide and the like; examples of an ester include lower alkyl esters such as methyl, ethyl and the like; examples of mixed acid anhydride include monoC₁₋₄ alkyl carbonic acid mixed acid anhydride (e.g. a mixed acid anhydride of a free acid (IV) with monomethyl carbonate, monoethyl carbonate, monoisopropyl carbonate, monoisobutyl carbonate, mono-tert-butyl carbonate, monobenzyl carbionate, mono(p-nitrobenzyl) carbonate, monoallyl carbonate and the like), C₁₋₆ apliphatic carboxylic acid mixed acid anhydride (e.g. a mixed acid anhydride of the free acid (IV) with acetic acid, cyanoacetic acid, propionic acid, butyric acid, isobutyric acid, valeric acid, isovaleric acid, pivalic acid, trifluoroacetic acid, trichloroacetic acid, acetoacetic acid and the like), C₇₋₁₁ aromatic carboxylic acid mixed acid anhydride (e.g. a mixed acid anhydride of the free acid (IV) with benzoic acid, p-toluic acid, p-chlorobenzoic acid and the like), and organic sulfonic acid mixed acid anhydride (e.g. a mixed acid anhydride of the free acid (IV) with methanesulfonic acid, ethanesulfonic acid, benzenesulfionic acid, p-tulenesulfonic acid and the like); and examples of an active amide include amide with a nitrogen-containing heterocyclic compound (e.g. acid amide of a free acid (IV) with pyrazole, imidazole, benzotriazole and the like, and the nitrogen-containing heterocyclic compound may be substituted with C₁₋₆alkyl (e.g. methyl, ethyl, propyl, isopropyl, butyl, isobutyl, sec-butyl, tert-butyl etc.), C₁₋₆alkoxy (e.g. methoxy, ethoxy, propoxy, isopropoxy, butoxy, tert-butoxy etc.), a halogen atom (e.g. fluoro, chloro, bromo, etc.), oxo, thioxo, C₁₋₆ alkylthio (e.g. methylthio, ethylthio, propylthio, butylthio etc.) and the like).

Examples of an active ester include organic phosphate ester (e.g. diethoxyphosphate ester, diphenoxyphosphate ester etc.) as well as p-nitrophenyl ester, 2,4-dinitrophenyl ester, cyanomethyl ester, pentachlorophenyl ester, N-hydroxysuccineimide ester, pentachlorophenyl ester, N-hydroxysuccineimide ester, N-hydroxyphthalimide ester, 1-hydroxybenzotiazole ester, 6-chloro-1-hydroxybenzotriazole ester, 1-hydroxy-1H-2-pyridone ester and the like. Examples of active thioester include esters with aromatic heterocyclic thiol compounds (the heterocyclic ring may be substituted with C₁₋₆ alkyl (e.g. methyl, ethyl, propyl, isopropyl, butyl, isobutyl, sec-butyl, tert-butyl etc.), C₁₋₆alkoxy (e.g. methoxy, ethoxy, propoxy, isopropoxy, butoxy, tert-butoxy etc.), halogen atom (e.g. fluoro, chloro, bromo, etc.), C₁₋₆ alkylthio (e.g. methylthio, ethylthio, propylthio, butylthio etc.) and the like) [e.g. 2-pyridylthiol ester, 2-benzothiazolylthiol ester] and the like.

This reaction is generally carried out in a solvent and, if necessary, in the presence of a base or a condensing reagent (e.g. carbodiimides (DCC, WSC, DIC etc.), phosphate derivative (e.g. diethyl cyanophosphate, DPPA, BOP-CI etc.), 4-(4,6-dimethoxy-1,3,5-triazin-2-yl)-4-methylmorpholinium chloride (DMTMM: Kunishima et al., Tetrahedron, 1999, 55, 13159) etc.). As such the solvent and base, those described in the above-described process A are used as they are.

In this reaction, compound (V) is used in an amount of 0.5 to 5 equivalents, preferably 0.8 to 2 equivalents based on compound (IV).

The reaction temperature is -50 to 150°C, preferably -20 to 100°C.

The reaction time is varied depending on kinds of compound (IV) and (V), kinds of the solvent and base, the reaction temperature and the like, but is usually about 1 minute to about 100 hours, preferably about 15 minutes to about 48 hours.

### Process C

Compound (I) or a salt thereof can be produced by reacting a compound represented by the formula (VI): 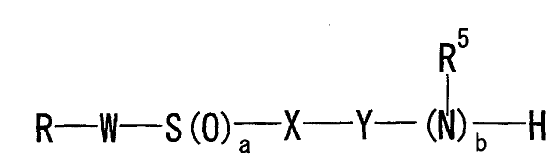 wherein the symbols are the same as defined above, or a salt, with a compound represented by the formula (VII): 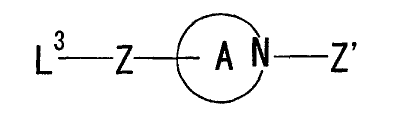 wherein L³ is the same as defined with respect to L² of the formula (IV), and the other symbols are the same as defined above, or a salt thereof.

The reaction of this process is generally carried out in a solvent, and a solvent which does not interfere with the reaction is appropriately selected. As such the solvent, there are alcohols (e.g. methanol, ethanol, propanol, isopropanol, butanol, tert-butanol etc.), ethers (e.g. dioxane, tetrahydrofuran, diethyl ether, tert-butyl methyl ether, diisopropyl ether, ethylene glycol-dimethyl ether etc.), esters (e.g. ethyl formate, ethyl acetate, n-butyl acetate etc.), halogenated hydrocarbons (e.g. dichloromethane, chloroform, carbon tetrachloride, trichloroethylene, 1,2-dichloroethane etc.), hydrocarbons (e.g. n-hexane, benzene, toluene etc.), amides such as formamide, N,N-dimethylformamide, N,N-dimethylacetamide and the like, nitriles such as acetonitrile, propionitrile and the like, as well as dimethyl sulfoxide, sulfolane, hexamethylphosphoramide, water and the like and they are used alone or as a mixed solvent thereof.

In addition, this reaction may be carried out in the presence of a base and, as such a base, there are alkali metal hydrides such as potassium hydride, sodium hydride, metal alkoxides having a 1 to 6 carbon atoms such as lithium ethoxide, lithium tert-butoxide, sodium methoxide, sodium ethoxide, potassium tert-butoxide and the like, inorganic bases such as lithium hydroxide, potassium hydroxide, sodium hydroxide, sodium carbonate, potassium carbonate, sodium bicarbonate and the like, and tertiary amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-diazabicyclo[4.3.0]non-5-ene (DBN), 1,8-diazabicyclo[5.4.0]-7-undecene (DBU), 1,4-diazabicyclo[2.2.2]octane and the like..

In this reaction, compound (VII) is used in an amount of 0.5 to 10 equivalents, preferably 0.8 to 3 equivalents based on compound (VI).

The reaction temperature is -30 to 250°C, preferably -10 to 150°C.

The reaction time is varied depending on kinds of compounds (VI) and (VII), a kind of the solvent, the reaction temperature and the like, but is usually about 1 minute to about 72 hours, preferably about 15 minutes to about 24 hours.

### Process D

Compound (I) can be produced by oxidizing compound (Ia) represented by the formula (Ia): 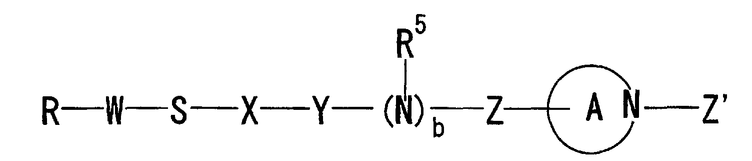 wherein the symbols are the same as defined above, or a salt thereof.

This oxidizing reaction is carried out in the presence of an oxidizing reagent. Examples of the oxidizing reagent include oxygen, hydrogen peroxide, organic peracids such as perbenzoic acid, m-chloroperbenzoic acid, peracetic acid and the like, perchlorate such as lithium perchlorate, silver perchlorate, tetrabutylammonium perchlorate and the like, periodate such as sodium periodate and the like, periodic acid, manganese dioxide, lead tetraacetate, permanganate such as potassium permanganate and the like, halogen such as iodine, bromine, chlorine and the like, N-bromosuccinimide, N-chlorosuccinimide, sulfuryl chloride, chloramine T and the like.

This reaction is generally carried out in a solvent, and a solvent which does not interfere with the reaction is appropriately selected. As such the solvent, there are alcohols (e.g. methanol, ethanol, propanol, isopropanol, butanol, tert-butanol etc.), esters (e.g. dioxane, tetrahydrofuran, diethyl ether, tert-butyl methyl ether, diisopropyl ether, ethylene glycol dimethyl ether etc.), esters (e.g. ethyl formate, ethyl acetate, n-butyl acetate etc.), carboxylic acids (e.g. formic acid, acetic acid, propionic acid etc.), halogenated hydrocarbons (e.g. dichloromethane, chloroform, carbon tetrachloride, trichloroethylene, 1,2-dichloroethane, chlorobenzene etc.), hydrocarbons (e.g. n-hexane, benzene, toluene etc.), amides (e.g. formamide, N,N-dimethylformamide, N,N-dimethylacetamide etc.), ketones (e.g. acetone, methyl ethyl ketone, methyl isobutyl ketone etc.), nitriles (e.g. acetonitrile, propionitrile etc.), as well as sulfolane, hexamethylphosphoramide, water and the like and they are used alone or as a mixed solvent thereof.

This reaction may also be carried out in the presence of a base. As such the base, there are inorganic bases such as alkali metal hydroxides such as lithium hydroxide, sodium hydroxide, potassium hydroxide and the like, alkaline earth metal hydroxides such as magnesium hydroxide, calcium hydroxide and the like, alkali metal carbonates such as sodium carbonate, potassium carbonate and the like, alkali metal bicarbonates such as sodium bicarbonate, potassium bicarbonate and the like.

In the reaction, the oxidizing reagent is used in an amount of 0.1 to 20 equivalents, preferably about 0.4 to 10 equivalents, and the base is used in an amount of 0.1 to 20 equivalents, preferably 0.4 to 10 equivalents based on compound (Ia).

Alternatively, this reaction may be carried out in the presence of an acid if necessary and, as such the acid, there are mineral acids such as hydrochloric acid, hydrobromic acid, sulfuric acid, phosphoric acid, perchloric acid and the like, sulfonic acids such as methanesulfonic acid, ethanesulfonic acid, benzenesulfonic acid, toluenesulfonic acid, camphorsulfonic acid and the like, and organic acids such as formic acid, acetic acid, propionic acid, trifluoroacetic acid and the like. The amount of the acid to be used are 0.1 to 20 equivalents, preferably 0.5 to 10 equivalents based on compound (Ia).

The reaction temperature is about -10°C to about 250°C, preferably about -5°C to about 150°C.

The reaction time is varied depending on kinds of compound (Ia), kinds of the base and solvent, the reaction temperature and the like, but is usually about 1 minute to about 50 hours, preferably about 5 minutes to about 24 hours.

### Process E

Compound (I) can be produced by reacting compound (VIII) represented by the formula (VIII):

R⁵-L⁴

wherein L⁴ is a leaving group (e.g. a halogen atom (e.g. fluoro, chloro, bromo, iodo, etc.), a C₁₋₆ alkylsulfonyloxy group optionally substituted with 1 to 3 halogen atom(s) (e.g. methanesulfonyloxy, ethanesulfonyloxy, trifluoromethanesulfonyloxy etc.), an optionally substituted arylsulfonyloxy group (e.g. benzenesulfonyloxy, p-toluenesulfonyloxy, p-bromobenzenesulfonyloxy etc.) or hydroxyl group, which is a group forming a free carboxylic acid or a salt thereof (inorganic salt, organic salt etc.) or a reactive derivative thereof (e.g. acid halide, ester, acid azide, acid anhydride, mixed acid anhydride, active amide, active ester, active thioester etc.) etc.), and the other symbols are the same as defined above, with compound (Ib) represented by the formula (Ib): 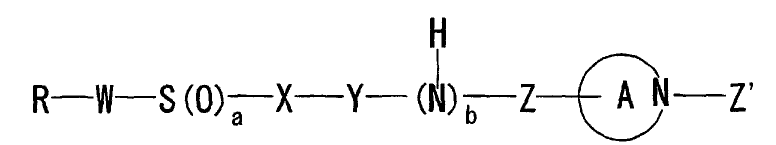 wherein the symbols are the same as defined above.

This process can be carried out by reacting compound (Ib) or a salt thereof with the free acid (VIII) or a salt thereof (inorganic salt, organic salt etc.) or a reactive derivative thereof (e.g. acid halide, ester, acid azide, acid anhydride, mixed acid anhydride, active amide, active ester, active thioester etc.). Examples of the salt of compound (Ib) include acid addition salts with acids which form an acid addition salt with the above-described compound (I).

As the inorganic salt used in the compound (VIII), there are alkali metal salts (e.g. sodium salt, potassium salt etc.), alkaline earth metal salts (e.g. calcium salt etc.) and the like are used and, as the organic salt, a trimethylamine salt, a triethylamine salt, a tert-butyldimethylamine salt, a dibenzylmethylamine salt, a benzyldimethylamine salt, N,N-dimethylaniline salt, a pyridine salt, a quinoline salt and the like. In addition, examples of acid halide include acid chloride, acid bromide and the like; examples of ester include lower alkyl esters such as methyl, ethyl and the like; examples of mixed acid anhydride include monoC₁₋₄ alkylcarbonic acid mixed acid anhydrides (e.g. a mixed acid anhydride of the free acid (VIII) with monomethyl carbonate, monoethyl carbonate, monoisopropyl carbonate, monoisobutyl carbonate, mono-tert-butyl carbonate, monobenzyl carbonate, mono(p-nitrobenzyl) carbonate, monoallyl carbonate and the like), C₁₋₆ aliphatic carboxylic acid mixed acid anhydrides (e.g. a mixed acid anhydride of the free acid (VIII) with acetic acid, cyanoacetic acid, propionic acid, butyric acid, isobutyric acid, valeric acid, isovaleric acid, pivalic acid, trifluoroacetic acid, trichloroacetic acid, acetoacetic acid and the like), C₇₋₁₁ aromatic carboxylic acid mixed acid anhydrides (e.g. a mixed acid anhydride of a free acid (VIII) with benzoic acid, p-toluic acid, p-chlorobenzoic acid and the like), and organic sulfonic acid mixed acid anhydrides (a mixed acid anhydride with methanesulfonic acid, ethanesulfonic acid, benzenesulfonic acid, p-toluenesulfonic acid and the like); examples of active amide include amides with a nitrogen-containing heterocyclic compound (e.g. acid amide of a free acid (VIII) with pyrazole, imidazole, benzotriazole and the like, the nitrogen-containing heterocyclic compound may be substituted with C₁₋₆ alkyl (e.g. methyl, ethyl, propyl, isopropyl, butyl, isobutyl, sec-butyl, tert-butyl, etc), C₁₋₆ alkoxy (e.g. methoxy, ethoxy, propoxy, isopropoxy, butoxy, tert-butoxy etc), a halogen atom (e.g. fluoro, chloro, bromo, etc.), oxo, thioxo, C₁₋₆ alkylthio (e.g. methylthio, ethylthio, propylthio, butylthio etc.) and the like), and the like.

Examples of the active ester include organic phosphate esters (e.g. diethoxyphosphate ester, diphenoxyphosphate ester etc.) as well as p-nitrophenyl ester, 2,4-dinitrophenyl ester, cyanomethyl ester, pentachlorophenyl ester, N-hydroxysuccinimide ester, N-hydroxyphthalimide ester, 1-hydroxybenzotriazole ester, 6-chloro-1-hydroxybenzotriazole ester, 1-hydroxy-1H-2-pyridone ester and the like. Examples of the active thioester include esters [e.g. 2-pyridylthiol ester, 2-benzothiazolylthiol ester] which were derived from aromatic heterocyclic thiol compounds [the heterocyclic ring may be substituted with C₁₋₆ alkyl (e.g. methyl, ethyl, propyl, isopropyl, butyl, isobutyl, sec-butyl, tert-butyl etc.), C₁₋₆ alkoxy (e.g. methoxy, ethoxy, propoxy, isopropoxy, butoxy, tert-butoxy etc.), a halogen atom (e.g. fluoro, chloro, bromo, etc.), C₁₋₆ alkylthio (e.g. methylthio, ethylthio, propylthio, butylthio etc.) and the like].

This reaction is generally carried out in a solvent and, if necessary, in the presence of a base or a condensing reagent (e.g. carbodiimides (DCC, WSC, DIC etc.), phosphate derivatives (e.g. diethyl cyanophosphate, DPPA, BOP-Cl etc.), DMTMM etc.). As such the solvent and base, those described for the above-described process A are used as they are.

In this reaction, compound (Ib) is used in an amount of 0.5 to 5 equivalents, preferably 0.8 to 2 equivalents based on compound (VIII).

The reaction temperature is -50 to 150°C, preferably -20 to 100°C.

The reaction time is varied depending on kinds of compounds (VIII) and (Ib), kinds of the solvent and the base, the reaction temperature and the like, but is usually about 1 minute to about 100 hours, preferably about 15 minutes to about 48 hours.

### Process F

Compound (I) can be produced by reacting a compound represented by the formula (IX):

R-W-S(O)ₐ-M

wherein M is hydrogen atom, hydroxyl group, an alkali metal, an alkaline earth metal or a leaving group (e.g. a halogen atom (e.g. fluoro, chloro, bromo, iodo, etc.), and the other symbols are the same as defined above, or a salt thereof, with compound (Ib) represented by the formula (X): 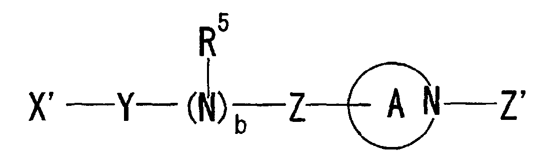 wherein X' is alkenyl or alkynyl (preferably C₂₋₈ alkenyl or C₂₋₈ alkynyl), or alkyl (preferably C₁₋₈ alkyl) having a leaving group (e.g. a halogen atom (e.g. fluoro, chloro, bromo, iodo, etc.), a C₁₋₆ alkylsulfonyloxy group optionally substituted with 1 to 3 halogen atom(s) (e.g. methanesulfonyloxy, ethanesulfonyloxy, trifluoromethanesulfonyloxy etc.), an optionally substituted arylsulfonyloxy group (e.g. benzenesulfonyloxy, p-toluenesulfonyloxy, p-bromobenzenesulfonyloxy etc.) or hydroxyl group), and the other symbols are the same as defined above.

This process is generally carried out in a solvent and, if necessary, in the presence of a base. As such the solvent and base, those described for the above-described process A are used as they are.

In this reaction, compound (X) is used in an amount of 0.5 to 3 equivalents, preferably 0.8 to 2 equivalents based on compound (IX).

The reaction temperature is -50 to 150°C, preferably -20 to 120°C.

The reaction time is varied depending on kinds of compounds (IX) or (X), kinds of the solvent and base, the reaction temperature and the like, but is usually about 1 minute to about 100 hours, preferably about 15 minutes to about 24 hours.

Staring compounds (III), (VI) and (X) used in the above-described respective reactions can be synthesized, for example, by the following methods.

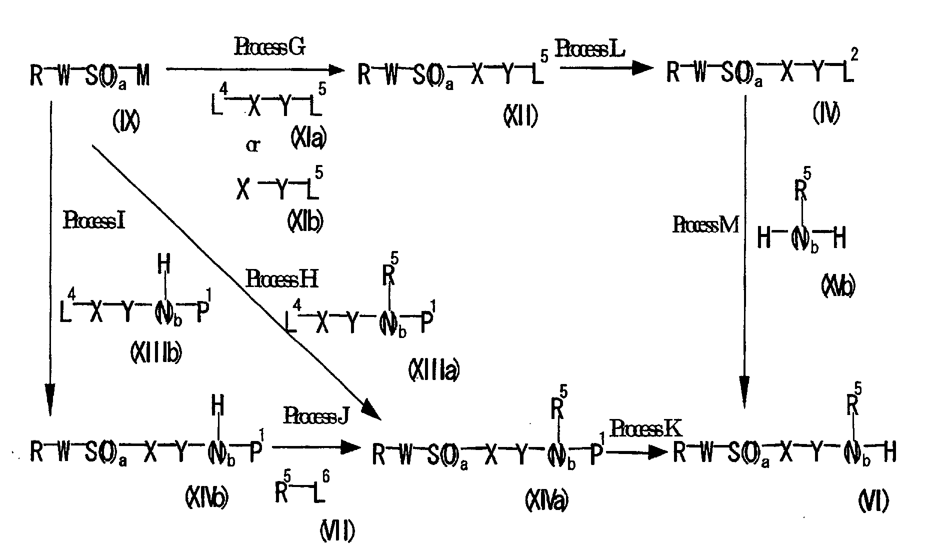

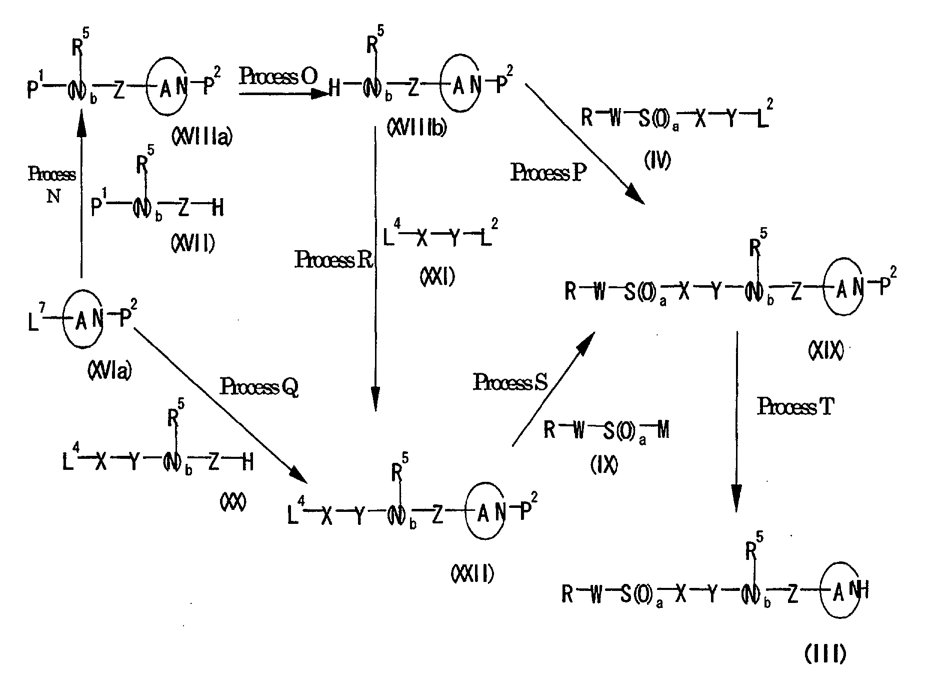

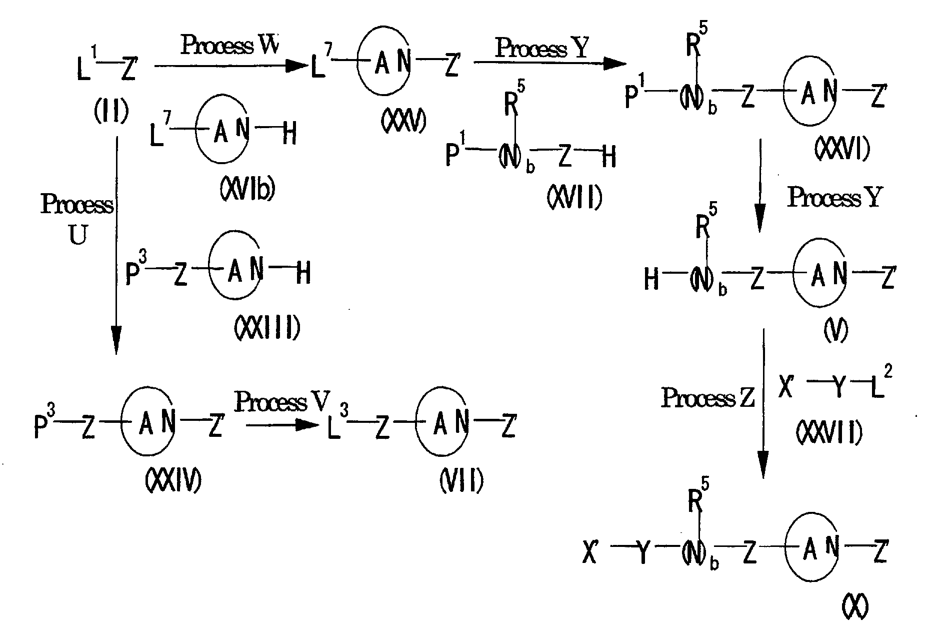

### Process G

Compound (XII) represented by the formula (XII):

R-W-S(O)ₐ-X-Y-L⁵

wherein the symbols are the same as defined above, is produced by reacting compound (IX) represented by the formula (IX):

R-W-S(O)ₐM

wherein the symbols are the same as defined above, or a salt thereof, with compound (XIa) or compound (XIb) represented by the formula (XIa):

L⁴-X-Y-L⁵

or the formula (XIb):

X'-Y-L⁵

wherein L⁵ is hydrogen atom, hydroxyl group, C₁₋₆ alkoxy (e.g. methoxy, ethoxy, propoxy, tert-butoxy etc.) or C₆₋₁₀ aryl-C₁₋₄ alkoxy (e.g. benzyloxy, phenethyloxy etc.), and the other symbols are the same as defined above, or a salt thereof. If necessary, the product can be oxidized to increase the oxidation number of the sulfur atom.

This reaction is a replacement reaction of the leaving group L⁴ of compound (XIa), or an addition reaction to a multiple bond between carbon atoms of compound (XIb), and the reaction conditions, reaction solvent, reaction time and the like in such the reaction are the same as or similar to those explained for the reaction between compound (II) and compound (III) in the process A. The reaction conditions, reaction solvent, reaction time and the like in the reaction of oxidizing the sulfur atom are the same as or similar to those explained for the reaction of oxidizing compound (Ia) in the process D.

### Process H

Compound (XIVa) represented by the formula (XIVa): 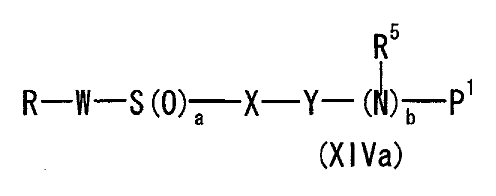 wherein the symbols are the same as defined above, is produced by reacting compound (IX) represented by the formula (IX):

R-W-S(O)ₐ-M

wherein the symbols are the same as defined above, or a salt thereof, with compound (XIIIa) represented by the formula (XIIIa): 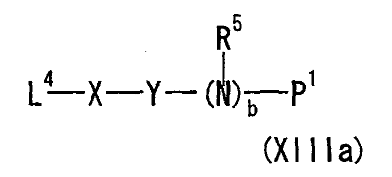 wherein P¹ is a protecting group for amino group, and the other symbols are the same as defined above, or a salt thereof.

Examples of the protecting group represented by P¹ in the above-described formulas (XIIIa) and (XIVa) include protecting groups described in T.W. Green et al. "Protective Groups in Organic Synthesis", John Wiley & Sons, Inc. New York, 1991 and, for example, C₁₋₈ acyl (e.g. formyl, acetyl, propionyl etc.), C₁₋₆ alkoxy-carbonyl (e.g. methoxycarbonyl, ethoxycarbonyl, tert-butoxycarbonyl etc.), C₇₋₁₅ aralkoxy-carbonyl (e.g. benzyloxycarbonyl, phenethyloxycarbonyl, (1-naphthyl)methoxycarbonyl, fluorenylmethoxycarbonyl etc.), allyloxycarbonyl, C₆₋₁₀ arylcarbonyl (e.g. benzoyl, naphthylcarbonyl etc.), C₇₋₁₉ aralkyl (e.g. benzyl, diphenylmethyl, trityl etc.), C₁₋₆ alkyl-sulfonyl (e.g. methanesulfonyl, ethanesulfonyl etc.), C₁₋₆ alkyl-sulfonyl (e.g. methanesulfonyl, ethanesulfonyl etc.), C₆₋₁₀ arylsulfonyl (e.g. phenylsulfonyl, naphthylsulfonyl etc.) and the like are used. These groups may be substituted with 1 to 3 C₁₋₄ alkyl(s), 1 to 3 halogen atom(s) (e.g. fluorine atom, chlorine atom, bromine atom etc.), 1 to 3 nitro group(s), or 1 to 3 cyano group(s).

The reaction conditions, reaction solvent, reaction time and the like in this reaction are the same or similar to those explained for the reaction between compound (IX) and compound (XIIa) or compound (XIIb) in the process G.

### Process I

Compound (XIVb) represented by the formula (XIVb): 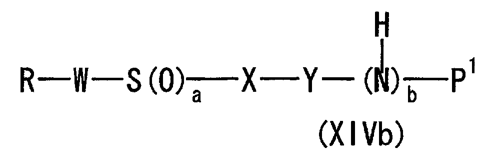 wherein the symbols are the same as defined above, or a salt thereof, can be produced by reacting compound (IX) represented by the formula (IX):

R-W-S(O)ₐ-M

wherein the symbols are the same as defined above, or a salt thereof, with compound (XIIIb) represented by the formula (XIIIb): 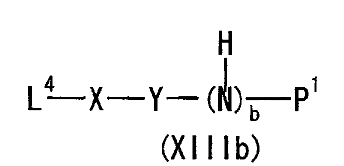 wherein the symbols are the same as defined above, or a salt thereof.

The reaction conditions, reaction solvent, reaction time and the like in this reaction are the same or similar to those explained for the reaction between compound (IX) and compound (XIIa) or compound (XIIb) in the process G.

### Process J

Compound (XIVa) represented by the formula (XIVa): 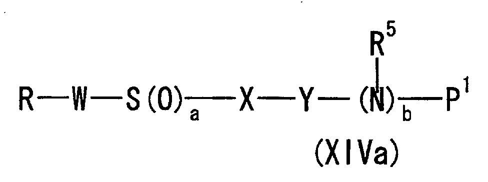 wherein the symbols are the same as defined above, or a salt thereof, can be produced by reacting a compound (XIVb) represented by the formula (XIVb): 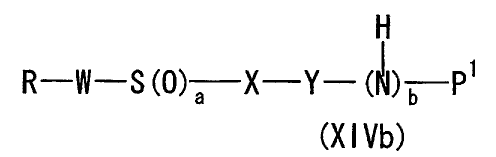 wherein the symbols are the same as defined above, or a salt thereof, with compound (VIII) represented by the formula (VIII):

R⁵-L⁶

wherein the symbols are the same as defined above, or a salt thereof.

The reaction conditions, reaction solvent, reaction time and the like in this reaction are the same or similar to those explained for the reaction between compound (Ib) and compound (VIII) in the process E.

### Process K

Compound (IV) represented by the formula (VI): 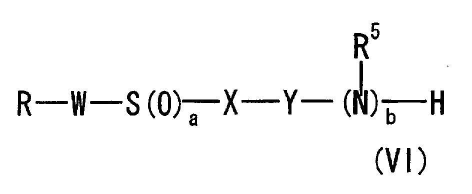 wherein the symbols are the same as defined above, or a salt thereof, can be produced by removing a protecting group for amino group of compound (XIVa) represented by the formula (XIVa): 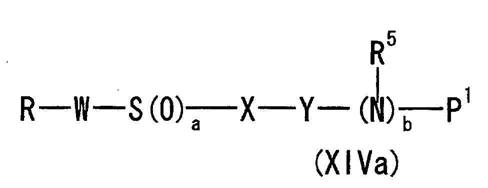 wherein the symbols are the same as defined above, or a salt thereof.

Removal of the protecting group for amino group can be carried out by the method described in, for example, T. W. Green et al. "Protective Groups in Organic Synthesis", John Wiley & Sons, Inc. New York, 1991 or a similar method. For example, a method using an acid, a base, reduction, ultraviolet light, palladium acetate or the like is used.

### Process L

Compound (IV) represented by the formula (IV):

R-S(O)ₐ-X-Y-L²

wherein the symbols are the same as defined above, or a salt thereof, can be prepared by converting the leaving group L⁵ of compound (XII) represented by the formula (XII):

R-W-S(O)ₐ-X-Y-L⁵

wherein the symbols are the same as defined above, or a salt thereof, into the other leaving group L², or optionally, by oxidizing the sulfur atom before or after the reaction of converting the leaving group.

This leaving group conversion is carried out by a reaction such as acid hydrolysis (e.g. using hydrochloric acid, hydrobromic acid, sulfuric acid etc.), alkali hydrolysis (e.g. using sodium hydroxide, potassium hydroxide, lithium hydroxide, sodium carbonate, potassium carbonate, sodium bicarbonate etc.), halogenation (e.g. using thionyl chloride, thionyl bromide, phosphorus oxychloride, phosphorus pentachloride, phosphorus trichloride, phosphorus oxybromide etc.), C₁₋₆ alkylsulfonation (e.g. using methanesulfonyl chloride, ethanesulfonyl chloride, trifluoromethanesulfonic anhydride, etc.) or arylsulfonation (using benzenesulfonyl chloride, p-toluenesulfonyl chloride, p-bromobenzenesulfonyl chloride and the like in the presence or absence of a base (e.g. triethylamine, diisopropylethylamine, pyridine, N,N-dimethylaminopyridine, sodium carbonate, potassium carbonate, sodium bicarbonate etc.)) and the like. The reaction solvent, reaction time, and reaction temperature are the same as or similar to those explained for the process A.

### Process M

Compound (VI) represented by the formula (VI): 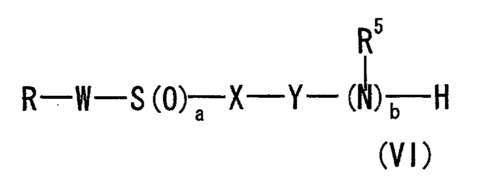 wherein the symbols are the same as defined above, or a salt thereof, can be produced by reacting compound (IV) represented by the formula (IV): R-W-S(O)ₐ-X-Y-L²
wherein the symbols are the same as defined above, or a salt thereof, with compound (XVb) represented by the formula (XVb): 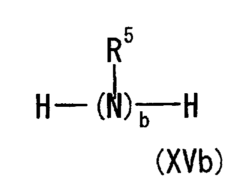 wherein the symbols are the same as defined above, or a salt thereof.

The reaction conditions, reaction solvent, reaction time and the like in this reaction are the same as or similar to those explained for the reaction between compound (IV) and compound (V) in the process B.

### Process N

Compound (XVIIIa) represented by the formula (XVIIIa): 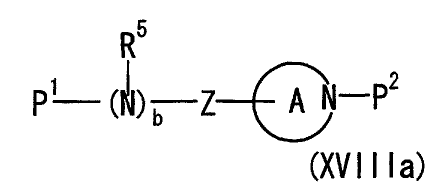 wherein the symbols are the same as defined above, or a salt thereof, can be produced by reacting compound (XVIa) represented by the formula (XVIa): 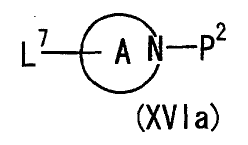 wherein the symbols are the same as define above, or a salt thereof, with compound (XVII) represented by the formula (XVII): 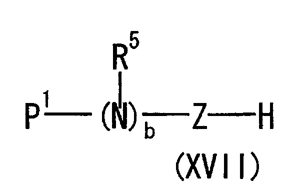 wherein the symbols are the same as define above, or a salt thereof.

The reaction conditions, reaction solvent, reaction time and the like in this reaction are the same as or similar to those explained for the reaction between compound (IV) and compound (V) in the process B.

### Process O

Compound (XVIIIb) represented by the formula (XVIIIb): 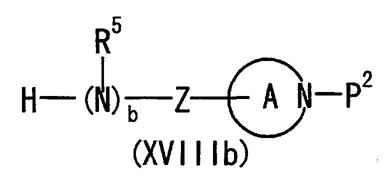 wherein the symbols are the same as define above, or a salt thereof, can be produced by removing the protecting group for amino group of compound (XVIa) represented by the formula (XVIIIa): 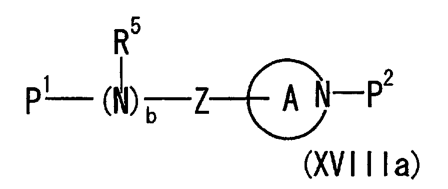 wherein the symbols are the same as define above, or a salt thereof.

The reaction conditions, reaction solvent, reaction time and the like in the present reaction are the same as or similar to those explained for the reaction of deprotecting compound (XIVa) in the process K.

### Process P

Compound (XIX) represented by the formula (XIX): 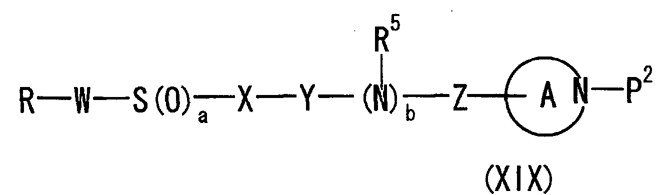 wherein the symbols are the same as defined above, or a salt thereof, can be produced by reacting compound (XVIIIb) represented by the formula (XVIIIb): 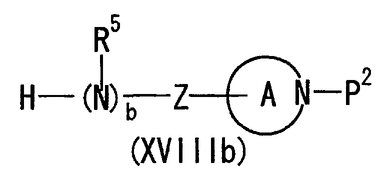 wherein the symbols are the same as defined above, or a salt thereof, with compound (IV) represented by the formula (IV): R-W-S(O)ₐ-X-Y-L² [wherein the symbols are the same as defined above] or a salt thereof.

The reaction conditions, reaction solvent, reaction time and the like in this reaction are the same or similar to those explained for the reaction between compound (IV) and compound (V) in the process B.

### Process Q

Compound (XXII) represented by the formula (XXII): 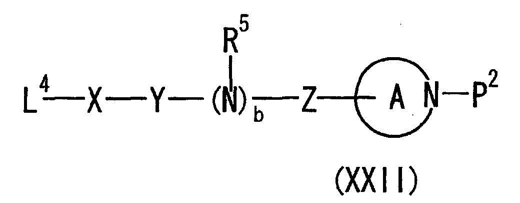 wherein the symbols are the same as defined above, or a salt thereof, can be produced by reacting compound (XVIa) represented by the formula (XVIa): 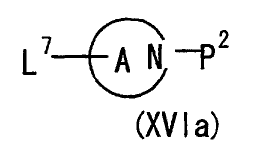 wherein the symbols are the same as defined above, or a salt thereof, with compound (XX) represented by the formula (XX): 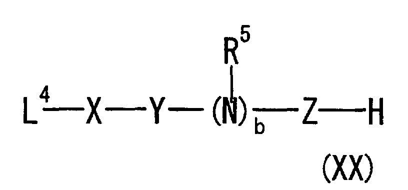 wherein the symbols are the same as defined above, or a salt thereof.

The reaction conditions, reaction solvent, reaction time and the like in this reaction are the same or similar to those explained for the reaction between compound (XVIa) and compound (XVII) in the process N.

### Process R

Compound (XXII) represented by the formula (XXII): 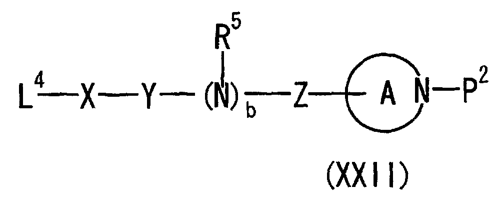 wherein the symbols are the same as defined above, or a salt thereof, can be produced by reacting compound (XVIIIb) represented by the formula (XVIIIb): 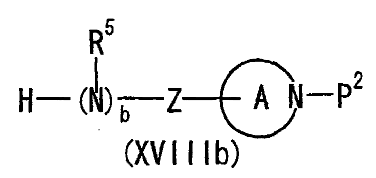 wherein the symbols are the same as defined above, or a salt thereof, with compound (XXI) represented by the formula (XXI): L⁴-X-Y-L² [wherein the symbols are the same as defined above] or a salt thereof.

The reaction conditions, reaction solvents, a reaction time and the like in this reaction are the same or similar to those explained for the reaction between compound (IV) and compound (V) in the process B.

### Process S

Compound (XIX) represented by the formula (XIX): 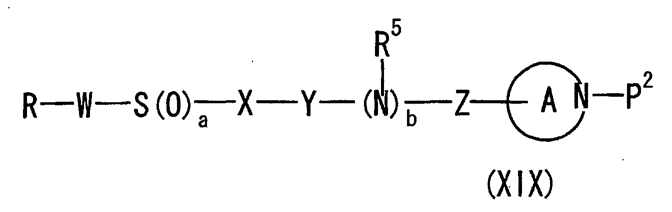 wherein the symbols are the same as defined above, or a salt thereof, can be produced by reacting compound (XXII) represented by the formula (XXII): 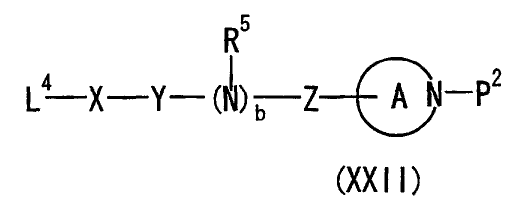 wherein the symbols are the same as defined above, or a salt thereof, with compound (IX) represented by the formula (IX): R-W-S(O)ₐ M [wherein the symbols are the same as defined above] or a salt thereof.

The reaction conditions, reaction solvent, reaction time and the like in this reaction are the same or similar to those explained for the reaction between compound (IX) and compound (X) in the process F.

### Process T

Compound (III) represented by the formula (III): 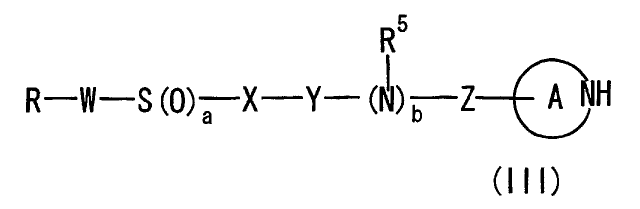 wherein the symbols are the same as defined above, or a salt thereof, can be produced by removing the protecting group on the nitrogen atom in ring A of compound (XIX) represented by the formula (XIX): 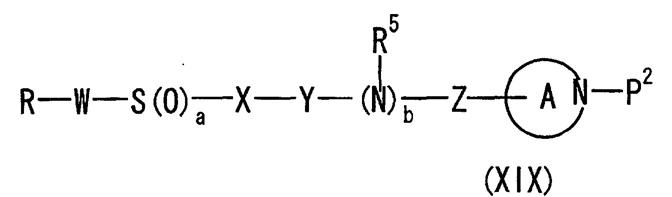 wherein the symbols are the same as defined above, or a salt thereof.

The reaction conditions, reaction solvent, reaction time and the like in this reaction are same as or similar to those explained for the reaction of deprotecting compound (XIVa) in the process K.

### Process U

Compound (XXIV) represented by the formula (XXIV): 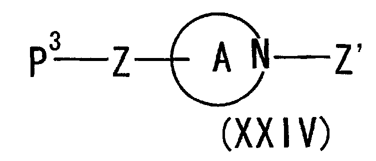 wherein the symbols are the same as defined above, or a salt thereof, can be produced by reacting compound (II) represented by the formula (II): 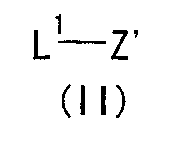 wherein the symbols are the same as defined above, or a salt thereof, with compound (XXIII) represented by the formula (XXIII): 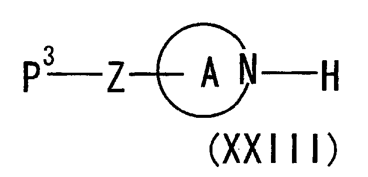 wherein P³ is hydrogen atom, hydroxyl group, C₁₋₆ alkoxyl (e.g. methoxy, ethoxy, tert-butoxy etc.) or benzyloxy, and the other symbols are the same as defined above, or a salt thereof.

The reaction conditions, reaction solvent, reaction time and the like in this reaction are the same or similar to those explained for the reaction between compound (II) and compound (III) in the process A.

### Process V

Compound (VII) represented by the formula (VII): 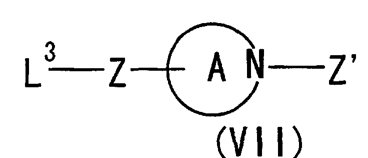 wherein the symbols are the same as defined above, or a salt thereof, can be produced by converting the protecting group P³ of compound (XXIV) represented by the formula (XXIV): 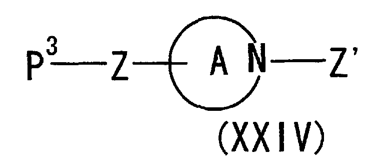 wherein the symbols are the same as defined above, or a salt thereof, into the leaving group L³.

The reaction conditions, reaction solvent, reaction time and the like in this reaction are the same as or similar to those explained for the reaction of converting compound (XII) into compound (IV) in the process L.

### Process W

Compound (XXV) represented by the formula (XXV): 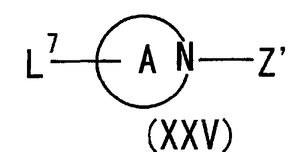 wherein the symbols are the same as defined above, or a salt thereof, can be produced by reacting compound (III) represented by the formula (II) 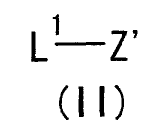 wherein the symbols are the same as defined above, or a salt thereof, with compound (XVIb) represented by the formula (XVIb): 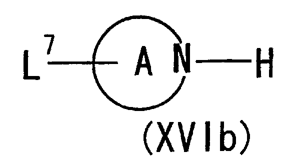 wherein the symbols are the same as defined above, or a salt thereof.

The reaction conditions, reaction solvent, reaction time and the like in this reaction are the same as or similar to those explained for the reaction between compound (II) and compound (III) in the process A.

### Process X

Compound (XXVI) represented by the formula (XXVI): 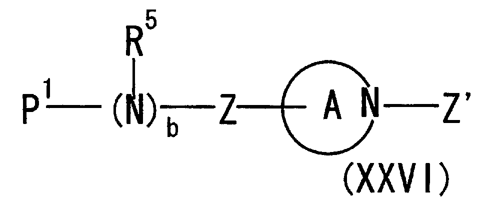 wherein the symbols are the same as defined above, or a salt thereof, can be produced by reacting compound (XXV) represented by the formula (XXV): 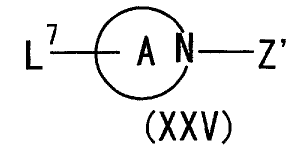 wherein the symbols are the same as defined above, or a salt thereof, with compound (XVII) represented by the formula (XVII): 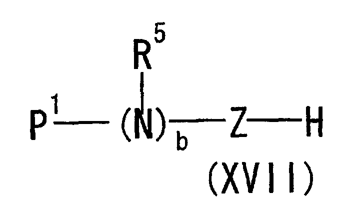 wherein the symbols are the same as defined above, or a salt thereof.

The reaction conditions, reaction solvent, reaction time and the like in this reaction are the same as or similar to those explained for the reaction between compound (XVIa) and compound (XVII) in the process N.

### Process Y

Compound (V) represented by the formula (V): 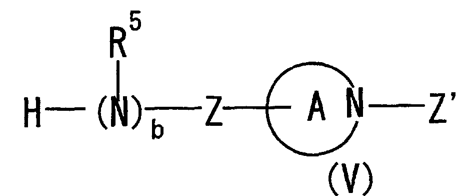 wherein the symbols are the same as defined above, or a salt thereof, can be produced by removing the protecting group for amino group of compound (XXVI) represented by the formula (XXVI): 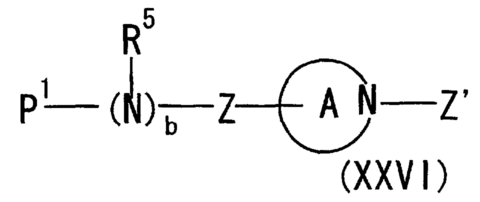 wherein the symbols are the same as defined above, or a salt thereof.

The reaction conditions, reaction solvent, reaction time and the like in this reaction are the same as or similar to those explained for the reaction of deprotecting compound (XIVa) in the process K.

### Process Z

Compound (X) represented by the formula (X): 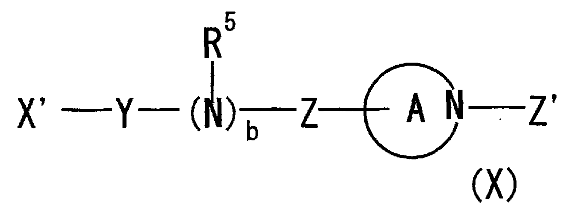 wherein the symbols are the same as defined above, or a salt thereof, can be produced by reacting compound (V) represented by the formula (V): 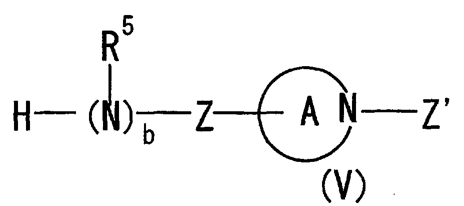 wherein the symbols are the same as defined above, or a salt thereof, with compound (XXVII) represented by the formula (XXVII) :

X'-Y-L²

[wherein the symbols are the same as defined above] or a salt thereof.

The reaction conditions, reaction solvent, reaction time and the like in this reaction are the same as or similar to those explained for the reaction between compound (IV) and compound (V) in the process B.

The starting compound (II) used in the above-described production processes A to Z can be produced by the methods described, for example, in A. Weissberger, "The Chemistry of Heterocyclic Compounds, 14 Part 2", 1961, Intersciences Publishers, Inc., New York or A. Weissberger, E.C. Tayler, "The Chemistry of Heterocyclic Compounds, 14 Part 2", 1974, John Wiley & Sons, New York, or similar methods. In addition, the other starting compounds (VIII), (IX), (XIa), (XIb), (XIIIa), (XIIIb), (XVb), (XVII), (XX), (XXI) and (XXIII) can be produced by per se known methods or similar methods.

When the compound is obtained in the free state in each of the above-described reactions of the present invention, it may be converted into a salt according to a conventional method, or when the compound is obtained as a salt, it may be converted into a free compound or another salt according to a conventional method.

Among synthetic intermediates used for the above-described reactions, 3-(6-halogeno-2-naphthyl)sulfonylpropionic acid, an ester, an amide or a salt thereof [preferably 3-(6-chloro-2-naphthyl)sulfonylpropionic acid, an ester, an amide or a salt thereof] are novel compounds, and are used advantageously for synthesizing the compound (I).

Here, any salts may be used as far as they do not interfere with the reaction, and examples thereof include the same as those used for the compound (I).

Any esters may be used in so far as they do not interfere with the reaction, and examples of the ester include (1) lower alkyl C₁₋₆esters such as methyl, ethyl, tert-butyl and the like, (2) organic phosphate esters (e.g. diethoxyphosphate ester, diphenoxyphosphate ester etc.), (3) p-nitrophenyl ester, (4) 2,4-dinitrophenyl ester, (5) cyanomethyl ester, (6) pentachlorophenyl ester, (7) N-hydroxysuccinimido ester, (8) N-hydroxyphthalimide ester, (9) 1-hydroxybenzotriazole ester, (10) 6-chloro-1-hydroxybenzotriazole ester, (11) 1-hydroxy-1H-2-pyridone ester, (12) thioester [e.g. esters with aromatic heterocyclic thiol compounds [these heterocyclic rings may be substituted with C₁₋₆ alkyl (e.g. methyl, ethyl, propyl, isopropyl, butyl, isobutyl, sec-butyl, tert-butyl etc.), C₁₋₆ alkoxy (e.g. methoxy, ethoxy, propoxy, isopropoxy, butoxy, tert-butoxy etc.), a halogen atom (e.g. fluorine, chlorine, bromine etc.), C₁₋₆ alkylthio (e.g. methylthio, ethylthio, propylthio, butylthio etc.) and the like] [e.g. 2-pyridylthio ester, 2-benzothiazolylthio ester] etc.].

Any amides may be used in so far as they do not interfere with the reaction, and examples thereof include amides with a nitrogen-containing heterocyclic compound [e.g. acid amide derived from pyrazole, imidazole, benzotriazole and the like, and these nitrogen-containing heterocyclic compounds may be substituted with C₁₋₆ alkyl (e.g. methyl, ethyl, propyl, isopropyl, butyl, isobutyl, sec-butyl, tert-butyl etc.), C₁₋₆ alkoxy (e.g. methoxy, ethoxy, propoxy, isopropoxy, butoxy, tert-butoxy etc.), a halogen atom (e.g. fluoro, chloro, bromo, etc.), oxo, thioxo, C₁₋₆ alkylthio (e.g. methylthio, ethylthio, propylthio, butylthio etc.) etd.].

3-(6-Halogeno-2-naohthyl)sulfoynlpropionic acid, or an ester, an amide or a salt thereof may be used for a reaction for synthesizing the compound (I) after derivatization to an acid halide or a mixed acid anhydride, and examples of the acid halide include acid chloride and acid bromide, examples of the mixed acid anhydride include monoC₁₋₄ alkyl carbonic acid mixed anhydride (e.g. mixed acid anhydride with monomethylcarbonic acid, monoethylcarbonic acid, monoisopropylcarbonic acid, monoisobutylcarbonic acid, mono-tert-butylcarbonic acid, monobenzylcarbonic acid, mono(p-nitrobenzyl)carbonic acid, imonoallycarbonic acid etc.), C₁₋₆ aliphatic carboxylic acid mixed anhydride (e.g. mixed acid anhydride with acetic acid, cyanoacetic acid, propionic acid, butyric acid, isobutyric acid, valeric acid, isovaleric acid, pivalic avid, trifluoroacetic acid, trichloroacetic acid, acetoacetic acid etc.), C₇₋₁₁ aromatic carboxylic acid mixed acid anhydride (e.g. mixed acid anhydride with benzoic acid, p-toluic acid, p-chlorobenzoic acid etc.), organic sulfonic acid mixed acid anhydride (e.g. mixed acid anhydride with methanesulfonic acid, ethanesulfonic acid, benzenesulfonic acid, p-toluenesulfonic acid etc.) and the like.

The thus-obtained compound (I) can be isolated and purified from the reaction mixture using a per se known method, for example, means such as extraction, concentration, neutralization, filtration, recrystallization, column chromatography, thin chromatography and the like.

A salt of compound (I) can be prepared by a per se known method, for example, by adding an inorganic acid or an organic acid to compound (I).

When optical isomers are present in compound (I), these individual optical isomers and mixtures thereof are all included in the scope of the present invention and, if needed, these isomers can be optically resolved according to a per se known method, or can be individually prepared.

In addition, compound (I) or a salt thereof may be a hydrate, and both of a hydrate and a non-hydrate are included in the scope of the present invention.

Since compound (I) according to the present invention or a salt thereof has a low toxicity and is safe and it inhibits an FXa and has an anticoagulative effect, it is useful in preventing and/or treating diseases, for example, cardiac infarction, cerebral thrombosis, deep vein thrombosis, pulmonary thromboembolism, thromboembolism during or after surjery, economy-class syndromes, inflammation, cancers, etc., as well as those listed below in animals especially in mammals (for example, human, monkey, cat, pig, horse, cattle, mouse, rat, guinea pig, dog, rabbit, etc.), and is preferred especially when being used in preventing and/or treating cardiogenic embolus such as atrial fibrillation, etc., cerebral infarction due to embolus derived from arteriosclerotic lesion at carotid, etc., deep vein thrombosis, pulmonary thromboembolism, and the like.

### Brain:

atrial fibrillation-induced cerebral infarction, acute ischemic apoplexy, acute cerebral thrombosis, cerebrovascular spasm after subarachnoid hemorrhage, Alzheimer's disease, transient cerebral ischemic attack (TIA), mixed dementia, cerebrovascular/multi-infarct dementia

### Heart:

acute coronary artery disease, acute cardiac infarction, sequela of myocardial infarction, improvement of prognosis and/or prevention of secondary crisis of myocardial infarction, unstable angina, angina pectris, vascular reocclusion and restenosis after coronary intervention such as stenting, PTCA (percutaneous transluminal coronary angioplasty) and atherectomy

### Peripheral organs:

Deep vein thrombosis, prevention of crisis and/or prevnetion of secondary crisis of deep vain thrombosis, chronic arterial obstruction, peripheral blood disease, adult respiratory distress syndrome, chronic renal disease (for example, diabetic nephrosis, chronic glomerulonephritis, IgA nephrosis), diabetic circulatory disorder, pain, neuropathy

### Others:

Dialysis-induced thrombocytopenia, thrombocytopenia after major surgery, arterial sclerosis, cancer metastasis, systemic inflammatory response syndrome (SIRS) or pancreatitis- or cancer-related disseminated intravascular coagulation (DIC), congestive chronic heart faliure, implant rejection, implant organ protection or improvement, shock- or DIC-related various organ failures (for example, pulmonary insufficiency, hepatic insufficiency, renal insufficiency, cardiac insufficiency), prevention of coagulation of perfusing blood during blood extracorporeal circulation

Compound (I) of the present invention or a salt thereof can orally or parenterally be administered as it is or in combination with a pharmaceutically acceptable carrier.

A formulation containing compound (I) or a salt thereof can be given orally in a dosage form such as tablets (including sugar-coated tablets and film-coated tablets), pills, granules, powders, capsules (including soft capsules, microcapsules), syrups, emulsions and suspensions, while it can be given parenterally in a dosage form such as injection, infusion and dripping formulations as well as suppositories. Further, a sustained release preparation prepared by combining with a suitable base (e.g., a polymer of butyric acids, a polymer of glycolic acids, a copolymer of butyric acid-glycolic acid, a mixture of a polymer of butyric acids and a polymer of glycolic acids, a polyglycerol fatty acid ester, etc.) is also advantageous.

While the amount of compound (I) or a salt thereof in a formulation of the invention may vary depending on the form of the formulation, it is usually 2 to 85 % by weight, preferably 5 to 70 % by weight based on the entire amount of the formulation.

A method for formulating compound (I) or a salt thereof into a dosage form described above, a known method employed generally in the art can be applied. Also for producing a dosage form described above, appropriate amounts of appropriate additives employed usually in the pharmaceutical field such as excipients, binders, disintegrants, lubricants, sweeteners, surfactants, suspending agents, emulsifiers and the like can be incorporated.

For example, compound (I) or a salt can be formulated into a tablet by incorporating an excipient, a binder, a disintegrant, a lubricant and the like, while it can be formulated into a pill or a granule by incorporating an excipient, a binder, a disintegrant and the like. It can be formulated also into a powder or a capsule by incorporating an excipient, into a syrup by incorporating a sweetener, into an emulsion or a suspension by incorporating a suspending agent, a surfactant, an emulsifier and the like.

An excipient may for example be lactose, sugar, glucose, starch, sucrose, microcrystalline cellulose, licorice powder, mannitol, sodium hydrogen carbonate, calcium phosphate, calcium sulfate and the like.

A binder may for example be 5 to 10 % by weight starch glue, 10 to 20 % by weight gum arabic or gelatin, 1 to 5 % by weight tragacanth gum, carboxymethyl cellulose, sodium alginate, glycerin and the like.

A disintegrant may for example be a starch, calcium carbonate and the like.

A lubricant may for example be magnesium stearate, stearic acid, calcium stearate, purified talc and the like.

A sweetener may for example be glucose, fructose, inverted sugar, sorbitol, xylitol, glycerin, syrups simplex and the like.

A surfactant may for example be sodium laurylsulfate, polysorbate 80, sorbitan monofatty ester, polyoxyl stearate 40 and the like.

A suspending agent may for example be gum arabic, sodium alginate, sodium carboxymethyl cellulose, methyl cellulose, bentonite and the like.

An emulsified may for example be gum arabic, tragacanth gum, gelatin, polysorbate 80 and the like.

Also for formulating compound (I) or a salt thereof into a dosage form described above, appropriate amounts of appropriate additives employed usually in the pharmaceutical field such as colorants, preservatives, flavors, seasonings, stabilizers, thickening agents and the like can be incorporated if necessary.

A formulation according to the invention containing compound (I) or a salt thereof is stable and has a low toxicity, and can be used safely. Its daily dose may vary depending on the condition and the body weight of the patient, the type of the compound and the administration route, and is usually about 1 to 1000 mg as an active ingredient (Compound (I) or a salt thereof) per day in an adult weighing about 60 kg when given orally to a patient having a thrombosis, preferably about 3 to 300 mg, more preferably about 10 to 200 mg, which can be given at once, or divided into two or 3 dosages.

When compound (I) of the present invention or a salt thereof is given parenterally, it is given usually in a liquid formulation (for example, injection formulation). In such case, a single dosage may vary depending on the target organ, the condition and the administration mode, and is usually about 0.01 mg to about 100 mg per kg body weight when given in an injection formulation, preferably about 0.01 to about 50 mg, more preferably about 0.01 to about 20 mg, which is given conveniently via an intravenous injection. In addition to the intravenous injection formulation, a subcutaneous injection formulation, an intradermal injection formulation, an intramuscular injection formulation and a dripping injection formulation may also included in the injection formulation, and an iontophoresis percutaneous formulation is included in a sustained release formulation. Any of such injection formulations can be prepared by a method known per se, i.e., by dissolving, suspending or emulsifying compound (I) of the invention or a salt thereof in an aseptic aqueous or oily liquid. An aqueous liquid for an injection may for example be a physiological saline and an isotonic solution containing glucose or other auxiliary agents (for example, D-sorbitol, D-mannitol, sodium chloride and the like), which may be used in combination with a suitable solubilizing aid such as an alcohol (for example, ethanol), a polyalcohol (for example, propylene glycol, polyethylene glycol), a nonionic surfactant (for example, polysorbate 80, HCO-50) and the like. An oily liquid may for example be a sesame oil and a soybean oil, which may be used in combination with a solubilizing aid such as benzyl benzoate and benzyl alcohol. Those which may also be incorporated are a buffering agent (for example, phosphate buffer and sodium acetate buffer), an analgesic (for example, benzalkonium chloride and procaine hydrochloride), a stabilizer (for example, human serum albumin and polyethylene glycol), a preservative (for example, benzyl alcohol and phenol) and the like. An injection formulation thus prepared is contained usually in an ampule.

The formulation of the present invention may appropriately be used in combination with a thrombolytic agent (for example, TPA, urokinase. etc.), an Alzheimer treating agent (for example, Avan, Calan, etc.), a cholesterol treating agent (for example, HMG-CoA reductase inhibitor such as simvastatin, pravastatin, etc.), a TG reducing agent (for example, clofibrate, etc.), an All antagonist (for example, candesartan, cilexetil, losartan, etc.), an antiplatelet agent (for example, clopidogrel, abciximab, aspirin, etc.), a Ca antagonist (for example, calslot, amlodipine, etc.), an ACE inhibitor (for example, enalapril, captopril, etc.), a β blocker (for example, metoprolol, carvedilol, etc.), an antiarrhythmic agent (for example, procaine amide, etc.), and the like, or these medicinal components can appropriately formulated in a preparation.

The present invention is further detailed in the following Examples, Formulation Examples and Experiments, which serve only as examples and are not intended to restrict the present invention and can be modified without departing the scope of the present invention.

An elution of a column chromatography in Examples was conducted with observing by a TLC (thin layer chromatography). In the observation of a TLC, a TLC plate employed was a 60F254 manufactured by Merck and NH manufactured by Fuji Silicial Chemical Co., Ltd., and a development was performed using a solvent which was employed as an eluent in a column chromatography, while an UV detector was used for a detection. Silica gel employed was kieselgel 60 (70 to 230 mesh) or kieselgel 60 (230 to 400 mesh) manufactured by Merck. Basic silica gel employed was basic silica gel NH-DM1020 (100 to 200 mesh) manufactured by Fuji Silicial Chemical Co., Ltd. An NMR spectrum was determined by a spectrometer model Varian Gemini 200 or 300 using tetramethylsilane as an internal or external standard and the chemical shift data were represented as total δ values in ppm. An IR spectrum was determined by a Shimadzu spectrometer model FTZR-8200. A figure in a bracket indicated in conjunction with a mixed solvent is a volume ratio of the constituent solvents. A % value indicated in conjunction with a solution is an amount in gram contained in 100 ml of the solution. The following abbreviations are employed in Reference Examples and Examples.
s: singlet
d: doublet
t: triplet
q: quartet
dd: double doublet
m: multiplet
br: broad
brs: broad singlet
J: coupling constant
WSC: water-soluble carbodiimide
THF: tetrahydrofuran
DMF: dimethylformamide
DMSO: dimethylsulfoxide
HOBt: 1-hydroxybenzotriazole

### Example 1

### 3-[(6-Chloro-2-naphthyl)sulfonyl]-N-methyl-N-[[1-(4-pyridyl)-4-piperidinyl]methyl]propanamide

### 1a) N-Methyl-1-(4-pyridyl)piperidine-4-carboxamide

A mixture of 1-(4-pyridyl)piperidine-4-carboxylic acid (1.03 g) and thionyl chloride (6 ml) was refluxed for 30 minutes, and concentrated under reduced pressure. The residue was suspended in THF (50 ml), and methylamine hydrochloride (0.41 g) and triethylamine (3.1 ml) were added thereto. The reaction mixture was stirred at room temperature for 14 hours. The precipitates were filtered, the filtrate was concentrated under reduced pressure, the residue was purified by column (basic silica gel, ethyl acetate/methanol=5/1), and recrystallized from ethyl acetate-methanol to obtain the title compound as a pale brown solid (0.70 g, 64%).
NMR (CDCl₃) δ: 1.69-2.05 (4H, m), 2.26-2.39 (1H, m), 2.83 (3H, d, J = 4.4), 2.82-2.96 (3H, m), 3.92 (2H, dm, J = 13.2), 5.66 (1H, br s), 6.66 (2H, d, J = 6.1), 8.25 (2H, d, J = 6.1).

### 1b) 4-[4-(N-Methylamino)methyl-1-piperidyl]pyridine

N-Methyl-1-(4-pyridyl)piperidine-4-carboxamide (0.50 g) obtained in Example 1a) was added to a suspension of lithium aluminum hydride (LAH) (0.13 g) in THF (12 ml), and the mixture was refluxed for 5 hours under the argon atmosphere. Water (0.54 ml) and a 6N aqueous sodium hydroxide solution (0.07 ml) were added to the reaction mixture. The precipitates were filtered, the filtrate was concentrated under reduced pressure, the residue was purified by column (silica gel, ethyl acetate/methanol=20/1), and recrystrallized from ethyl acetate-methanol to obtain the title compound as brown oil (0.29 g, 62%).
NMR (CDCl₃) δ: 1.18-1.38 (2H, m), 1.66-1.75 (1H, m), 1.78-1.86 (2H, m), 2.45 (3H, s), 2.49 (2H, d, J = 6.6), 2.84 (2H, dt, J = 2.4 and 12.8), 3.89 (2H, dm, J = 12.8), 6.65 (2H, d, J = 6.6), 8.24 (2H, d, J = 6.6).

### 1c) 6-Chloronaphthlane-2-sulfonyl chloride

Sodium carbonate (11.1 g) was added to a suspension of 6-aminonaphthalene-2-sulfonic acid (44.6 g) in water (200 ml), and the resulting solution was ice-cooled.
Concentrated hydrochloric acid (43 ml) was added slowly and, subsequently, a solution of sodium nitrite (16.6 g) in water (100 ml) was added dropwise at 5°C or lower over 30 minutes. The reaction mixture was stirred at 0 to 5°C for 1 hour, and the pale orange precipitates were filtered.
The resulting solid was added to ice-cooled cuprous chloride (23.8 g) in 28% hydrochloric acid (100 ml), and the mixture was stirred at room temperature for 1 hour and, further, at 60°C for 30 minutes. The reaction mixture was cooled to 0°C, pH was adjusted to 1 to 2 with a 50% aqueous solution potassium hydroxide (50 g). The precipitates were filtered, and suspended in hot water (about 200 ml). The suspension was made alkaline with a 50% aqueous potassium hydroxide solution, the insolubles were filtered, and dried to obtain a solid (16.8 g). Further, concentration of the filtrate under reduced pressure gave a solid (19.7 g).

The resulting solid was heated with phosphorus pentachloride (74 g) at 100 to 110°C for 4 hours. The mixture was cooled to room temperature, an ice (40 g) was added, the precipitates were filtered, and purified by the column (silica gel, chloroform/hexane=1/3) to obtain the title compound (12.1 g, 23%).
NMR (CDMCl₃) δ: 7.66 (1H, dd, J = 2.1 and 8.7), 7.97-8.07 (4H, m), 8.59 (1H, s).

### 1d) 6-Chloro-2-mercaptonaphthalene

A solution of 6-chloronaphthalene-2-sulfonyl chloride (20.2 g) obtained in Example 1c) in THF (130 ml) was added dropwise to a solution of LAH (7.07 g) in THF (130 ml) suspension under refluxing, and the mixture was further continued to be refluxed for 1 hour. Ethyl acetate (50 ml) was added dropwise to the reaction mixture and, subsequently, 2N hydrochloric acid (360 ml) was added, and the mixture was extracted with ethyl acetate. The extract was purified by subjecting to silica gel column as it was, to obtain the title compound (13.1 g, 88%).
NMR (CDCl₃) δ: 3.60 (1H, s), 7.34-7.48 (2H, m), 7.61 (1H, s), 7.66 (1H, s), 7.68-7.81 (2H, m).

### 1e) 3-(6-Chloro-2-naphthyl)thiopropionic acid

Methyl 3-bromopropionate (0.12 ml) was added to a solution of 6-chloro-2-mercaptonaphthalene (0.20 g) obtained in Example 1d) and 1N sodium hydroxide (1.1 ml) in methanol (15 ml), the mixture was stirred at room temperature for 1 hour, and 1 N aqueous sodium hydroxide solution (2.2 ml) was additionally added to reflux for 22 hours. The reaction mixture was concentrated under reduced pressure, and the residue was diluted with water, and made acidic with 1 N hydrochloric acid. The precipitates were filtered, and purified by silica gel column to obtain the title compound (0.14 g, 52%).
NMR (CDCl₃) δ: 2.72 (2H, t, J = 7.3), 3.26 (2H, t, J = 7.3), 7.40-7.49 (2H, m), 7.65-7.72 (2H, m), 7.77-7.80 (2H, m) .

### 1f) 3-(6-Chloro-2-naphthyl)sulfonylpropionic acid

Metachloroperbenzoic acid (mCPBA) (0.20 g) was added to a solution of 3-(6-chloro-2-naphthyl)thiopropionic acid (0.14 g) obtained in Example 1e) in methanol (10 ml), and the mixture was stirred at room temperature for 5 hours, and concentrated under reduced pressure. The residue was purified by silica gel column to obtain the title compound (0.11 g, 69%).
NMR (CDCl₃ + two drops of DMSO-d₆) δ: 2.67 (2H, t, J = 7.7), 3.51 (2H, t, J = 7.7), 7.85 (1H, dd, J = 1.9 and 8.7), 7.86-8.01 (4H, m), 8.46 (1H, s).

### 1g) 3-[(6-Chloro-2-naphthyl)sulfonyl]-N-methyl-N-[[1-(4-pyridyl)-4-piperidyl]methyl]propanamide

A mixture of 3-(6-chloro-2-naphthyl)sulfonylpropionic acid (0.11 g) obtained in Example 1f) and thionyl chloride (1 ml) was refluxed for 30 minutes, and concentrated under reduced pressure. The residue was dissolved in THF (3 ml), and added to a solution of 4-[4-(N-methylamino)methyl-piperidyl]pyridine (76 mg) obtained in Example 1b) and triethylamine (56 ml) in THF (2 ml) under ice-cooling. The reaction mixture was stirred at room temperature for 3 hours, and concentrated under reduced pressure, and the residue was diluted with saturated brine and extracted with THF. The extract was dried over anhydrous magnesium sulfate, the solvent was distilled off under reduced pressure, and the residue was purified by silica gel column to obtain the title compound as a pale yellow amorphous material (20 mg, 11%).
NMR (CDCl₃) δ: 1.15-1.35 (2H, m), 1.62-1.90 (3H, m), 2.71-2.93 (4H, m), 3.04 (3H, s, Me), 3.18-3.24 (2H, m), 3.57 (2H, t, J = 7.5), 3.80-3.93 (2H, m), 6.60-6.67 (2H, m), 7.59 (1H, dd, J = 2.0 and 8.8), 7.94-7.97 (4H, m), 8.21-8.29 (2H, m), 8.48 (1H, s). IR (KBr): 1640, 1597, 1310, 1148, 1127 cm⁻¹.

| Elemental Analysis: C₂₅H₂₈N₃O₃SCl | | | |
|---|---|---|---|
| Calcd (%) | C, 61.78; | H, 5.81; | N, 8.65 |
| Found (%) | C, 61.63; | H, 6.09; | N, 8.59 |

### Example 2

### N-[2-[(6-Chloro-2-naphthyl)sulfonyl]ethyl]-N-methyl-1-(4-pyridyl)-4-piperidinecarboxamide

### 2a) 2-(6-Chloro-2-naphthyl)thioethanol

2-Bromoethanol (0.60 ml) was added to a solution of 6-chloro-2-mercaptonaphthalene (0.14 g) obtained in Example 1d) and 1N sodium hydroxide (0.84 ml) in methanol (10 ml), and the mixture was stirred at room temperature for 2 hours. The reaction mixture was concentrated under reduced pressure, and the residue was diluted with water. The precipitated precipitates were filtered, and dried to obtain the title compound (163 mg 98%).
NMR (CDCl₃) δ: 2.02 (1H, t, J = 6.2), 3.22 (2H, t, J = 6.0), 3.80 (2H, q-like, J = 6.0), 7.39-7.51 (2H, m), 7.66-7.70 (2H, m), 7.77-7.79 (2H, m).

### 2b) 2-(6-Chloro-2-naphthyl)sulfonylethanol

mCPBA (0.28 g) was added to a solution of the 2-(6-chloro-2-naphthyl)thioethanol (160 mg) obtained in Example 2a) in methanol (5 ml)-THF (10 ml), and the mixture was stirred at room temperature for 3 hours, and concentrated under reduced pressure. The residue was dissolved in ethyl acetate, washed with saturated sodium bicarbonate water, and dried over anhydrous magnesium sulfate. The solvent was distilled off under reduced pressure, and the residue was purified by silica gel column to obtain the title compound as a colorless solid (0.15 g, 82%).
NMR (CDCl₃) δ: 2.71 (1H, t, J = 6.5), 3.43 (2H, t, J = 5.4), 4.50 (2H, q-like, J = 5.4), 7.61 (1H, dd, J = 2.1 and 8.7), 7.89-7.99 (4H, m), 8.50 (1H, s).

### 2c) 6-Chloro-2-(2-chloroethyl)sulfonylnaphthalene

A solution of 2-(6-chloro-2-naphthyl)sulfonylethanol (0.15 g) obtained in Example 2b), thionyl chloride (0.16 ml) and DMF (catalytic amount) in chloroform (3 ml) was refluxed for 3 hours, and concentrated under reduced pressure. The residue was washed with isopropyl ether to obtain the title compound (0.14 g, 90%).
NMR (CDCl₃) δ: 3.57-3.64 (2H, m), 3.76-3.84 (2H, m), 7.61 (1H, dd, J = 1.9 and 8.9), 7.92-7.98 (4H, m), 8.48 (1H, s).

### 2d) N-[2-[(6-Chloro-2-naphthyl)sulfonyl]ethyl]-N-methyl-1-(4-pyridyl)-4-piperidinecarboxamide

Sodium hydride (60% oily; 22 mg) was added to a solution of N-methyl-1-(4-pyridyl)piperidine-4-carboxamide (110 mg) obtained in Example 1a) in DMF (2 ml) under ice-cooling, the mixture was stirred at room temperature for 1 hour, and 6-chloro-2-(2-chloroethyl)sulfonylnaphthalene (144 mg) obtained in Example 2c) was added thereto at 0°C, and the mixture was stirred at room temperature for 4 hours. The reaction mixture was concentrated under reduced pressure, and the residue was purified by basic silica gel column to obtain the title compound as a pale yellow amorphous material (4.9 mg, 2%).
NMR (CDCl₃) δ: 1.71-1.82 (4H, m), 2.58-2.73 (1H, m), 2.80-2.94 (2H, m), 3.22 (3H, s, NMe), 3.47 (2H, t, J = 6.5), 3.79 (2H, t, J = 6.5), 3.88 (2H, dm, J = 13.2), 6.64 (2H, d, J = 6.5), 7.59 (1H, dd, J = 2.0 and 8.8), 7.90-7.97 (4H, m), 8.25 (2H, d, J = 6.5), 8.46 (1H, s).

| Elemental Analysis: C₂₄H₂₆N₃O₃SCl·0.7H₂O | | | |
|---|---|---|---|
| Calcd (%) | C, 59.48; | H, 5.70; | N, 8.67 |
| Found (%) | C, 59.47; | H, 5.68; | N, 8.53 |

### Example 3

### N-[3-(6-Chloro-2-naphthyl)thiopropyl]-N-methyl-1-(4-pyridyl)-4-piperidinecarboxamide

### 3a) tert-Butyl N-[3-(6-chloro-2-naphthyl)thiopropyl]-N-methylcarbamate

A 28% methanolic solution of sodium methoxide (0.87 g) was added to a suspension of 6-chloro-2-mercaptonaphthalene (0.58 g) obtained in Example 1d) in methanol (50 ml), tert-butyl N-(3-chloropropyl)-N-methylcarbamate (Vedejs, E., Stults, S., J.Org.Chem., 1988, 53, 2226) (0.75 g) was added thereto, and the mixture was stirred at 40°C for 10 hours and, further, at 65°C for 8 hours. The reaction mixture was concentrated under reduced pressure, extracted with ethyl acetate, washed with saturated brine, and dried over anhydrous sodium sulfate, concentration under reduced pressure and purification by silica gel column afforded the title compound as colorless oil (1.0 g, 91%).
NMR(CDCl₃) δ: 1.45(9H, s), 1.89 (2H, tt, J = 6.9 and 7.3), 2.84 (3H, s), 3.01 (2H, t, J = 7.3), 3.37 (2H, t, J = 6.9), 7.35-7.46 (2H, m), 7.64 (1H, s), 7.69 (2H, s), 7.77 (1H, d, J = 2.0).

### 3b) N-[3-(6-Chloro-2-naphthyl)thiopropyl]-N-methyl-1-(4-pyridyl)-4-piperidinecarboxamide

Trifluoroacetic acid (2 ml) was added to a solution of tert-butyl N-[3-(6-chloro-2-naphthyl)thiopropyl]-N-methylcarbamate (0.5 g) obtained in Example 3a) in toluene (10 ml), and the mixture was stirred for 30 minutes. Toluene was added to the reaction mixture, and the mixture was concentrated to dryness under reduced pressure. Dichloromethane (20 ml) and triethylamine (4 ml) were added to the residue, and 1-(4-pyridyl-4-piperidinecarbonyl chloride hydrochloride (0.26 g), followed by stirring for 15 hours. The reaction mixture was concentrated, extracted with ethyl acetate, washed successively with water, aqueous sodium bicarbonate and saturated brine, and dried over anhydrous sodium sulfate. The extract was concentrated, and the residue was purified by basic silica gel column, and crystallized from ethyl acetate/ether to obtain the title compound (36 mg, 19%).
NMR (CDCl₃) δ: 1.58-2.10 (5H, m), 2.59-3.10 (9H, m), 3.53 (2H, t, J = 7.5), 3.70-4.00 (2H, m), 6.55-6.70 (2H, m), 7.35-7.50 (2H, m), 7.60-7.80 (4H, m), 8.25 (2H, d, J = 6.2).

| Elemental Analysis: C₂₅H₂₈ClN₃OS | | | |
|---|---|---|---|
| Calcd (%) | C, 66.13; | H, 6.14; | N, 9.19 |
| Found (%) | C, 65.99; | H, 6.14; | N, 9.19 |

### Example 4

### N-[3-(6-Chloro-2-naphthyl)sulfinylpropyl]-N-methyl-1-(4-pyridyl)-4-piperidinecarboxamide

1 N Hydrochloric acid (0.5 ml) was added to a solution of N-[3-(6-chloro-2-naphthyl)thiopropyl]-N-methyl-1-(4-pyridyl)-4-piperidinecarboxamide (90 mg) obtained in Example 3b) in methanol (10 ml) under ice-cooling, and mCPBA (38 mg) was added thereto, followed by stirring for 1 hour. The reaction mixture was concentrated under reduced pressure, and the residue was dissolved in methylene chloride, and purified by basic silica gel column to obtain the title compound as a colorless powdery solid (50 mg, 47%).
NMR (CDCl₃) δ: 1.60-2.25 (6H, m), 2.60-3.05 (5H, m), 3.08 (3H, s), 3.40-3.65 (2H, m), 3.75-4.00 (2H, m), 6.65 (2H, d, J = 6.6), 7.50-7.65 (2H, m), 7.80-7.98 (3H, m), 8.15 (1H, s), 8.2 (2H, d, J = 6.6).

| Elemental Analysis: C₂₅H₂₈ClN₃O₂S·0.75H₂O | | | |
|---|---|---|---|
| Calcd (%) | C, 62.10; | H, 6.15; | N, 8.69 |
| Found (%) | C, 62.36; | H, 6.08; | N, 8.54 |

### Example 5

### N-[3-(6-Chloro-2-naphthyl)sulfonylpropyl]-N-methyl-1-(4-pyridyl)-4-piperidinecarboxamide

### 5a) tert-Butyl N-(3-(6-chloro-2-naphthyl)sulfonylpropyl)-N-methylcarbamate

mCPBA (0.52 g) was added to a solution of N-[3-(6-chloro-2-naphthyl)thiopropyl]-N-methylcarbamate (0.5 g) obtained in Example 3a) in ethyl acetate (50 ml), the mixture was stirred at room temperature for 1 hour, washed with saturated sodium bicarbonate water twice and, further, with sodium thiosulfate, and dried over anhydrous sodium sulfate. The extract was concentrated under reduced pressure. Hexane/ethyl acetate (8:1) was added to the residue, and crystallization was performed to obtain the title compound as colorless crystals (0.37 g, 69%).
NMR (CDCl₃) δ: 1.39 (9H, s), 1.90-2.08 (2H, m), 2.80 (3H, s), 3.10-3.22 (2H, m), 3.31 (2H, t, J = 6.8), 7.60 (1H, dd, J = 2.0 and 8.8), 7.88-8.00 (4H, m), 8.47 (1H, s). IR (KBr) : 1694, 1395, 1312, 1146, 1132 cm⁻¹.

### 5b) N-[3-(6-Chloro-2-naphthyl)sulfonylpropyl]-N-methyl-1-(4-pyridyl)-4-piperidinecarboxamide

According to the same manner as that of Example 3b), the title compound was obtained as a colorless powdery solid (58 mg, 14%) from tert-butyl N-[3-(6-chloro-2-naphthyl)sulfonylpropyl]-N-methylcarbamate (0.33 g) obtained in Example 5a).
NMR (CDCl₃) δ: 1.70-2.20 (5H, m), 2.60-3.00 (4H, m), 3.09 (3H, s), 3.09-3.22 (2H, m), 3.40-3.65 (2H, m), 3.80-4.00 (2H, m), 6.65 (2H, d, J = 6.6), 7.55-7.68 (1H, m), 7.82-8.00 (4H, m), 8.24 (2H, d, J = 6.6), 8.46 (1H, s).

| Elemental Analysis: C₂₅H₂₈ClN₃O₃S·0.1H₂O | | | |
|---|---|---|---|
| Calcd (%) | C, 61.55; | H, 5.83; | N, 8.61 |
| Found (%) | C, 61.29; | H, 5.85; | N, 8.54 |

### Example 6

### N-[2-[(6-Chloro-2-naphthyl)thio]ethyl-N-methyl-1-(4 pyridyl)-4-piperidinecarboxamide

### 6a) tert-Butyl N-(2-bromoethyl)carbamate

A 2 N aqueous sodium hydroxide solution (25 ml) and di-tert-butyl dicarbonate (4.8 g) were added to a solution of 2-bromoethylamine hydrobromide (4.1 g) in water (25 ml) and acetonitrile (25 ml), and the mixture was stirred for 15 hours. The reaction mixture was concentrated, water was added to the residue, the mixture was extracted with ethyl acetate, washed successively with an aqueous dilute potassium hydrogensulfate solution and saturated brine, and dried over anhydrous sodium sulfate. The extract was concentrated to obtain the title compound as colorless oil (3.8 g, 85%).
NMR (CDCl₃) δ: 1.46 (9H, s), 3.40-3.60 (4H, m), 4.98 (1H, bs).

### 6b) tert-Butyl N-[2-(6-chloro-2-naphthyl)thioethyl]-carbamate

According to the same manner as that of Example 3a), the title compound was obtained as colorless crystals (0.12 g, 69%) from tert-butyl N-(2-bromoethyl)carbamate (0.18 g) obtained in Example 6a).
NMR (CDCl₃) δ: 1.43 (9H, s), 3.15 (2H, t, J = 6.4), 3.30-3.45 (2H, m), 4.90 (1H, bs), 7.36-7.52 (2H, m), 7.62-7.80 (4H, m).

### 6c) tert-Butyl N-[2-(6-chloro-2-naphthyl)thioethyl]-N methylcarbamate

Sodium hydride (60% oily; 0.17 g) was added to a solution of tert-butyl N-[2-(6-chloro-2-naphthyl)-thioethyl]carbamate (0.12 g) obtained in Example 6b) in DMF (5 ml), and the mixture was stirred at room temperature for 1 hour. Then, methyl iodide (0.15 g) was added, and the mixture was stirred for 15 hours. Ice-water was added to the reaction mixture, and the mixture was extracted with ethyl acetate, washed successively with an aqueous dilute potassium hydrogensulfate solution and saturated brine, and dried over anhydrous sodium sulfate. The extract was concentrated, and purified by silica gel column to obtain the title compound as colorless oil (0.08 g, 64%).
NMR (CDCl₃) δ: 1.42 (9H, s), 2.89 (3H, s), 3.06 (2H, t, J = 7.0), 3.38 (2H, t, J = 7.2), 7.36-7.52 (2H, m), 7.62-7.82 (4H, m).

### 6d) N-[2-(6-Chloro-2-naphthyl)thioethyl]-N-methyl-1-(4-pyridyl)-4-piperidinecarboxamide

Trifluoroacetic acid (1 ml) was added to a solution of tert-butyl N-[2-(6-chloro-2-naphthyl)thiomethyl]-N-methylcarbamate (0.19 g) obtained in Example 6c) in toluene (5 ml), and the mixture was stirred for 1 hour. Toluene was added to the reaction mixture, and the mixture was concentrated to dryness under reduced pressure. Methylene chloride (20 ml) was added to the residue, diisopropylethylamine (0.47 ml) was added thereto, 1-(4-pyridinyl)-4-piperadznecarbonyl chloride hydrochloride (0.21 g) was added thereto, and the mixture was stirred for 15 hours. The reaction mixture was concentrated, extracted with ethyl acetate, washed successively with water, sodium bicarbonate water and saturated brine, and dried over anhydrous sodium sulfate. The solvent was distilled off, and the residue was dissolved in ethyl acetate, and purified by basic silica gel column to obtain the title compound as a colorless powdery solid (0.23 g, 95%).
NMR (CDCl₃) δ: 1.44-2.00 (5H, m), 2.15-2.40 (1H, m), 2.58-2.98 (3H, m), 3.05-3.30 (4H, m), 3.45-3.98 (3H, m), 6.45-6.65 (2H, m), 7.35-7.55 (2H, m), 7.62-7.88 (4H, m), 8.23 (2H, d, J = 5.2).

| Elemental Analysis: C₂₄H₂₆ClN₃OS·0.5H₂O | | | |
|---|---|---|---|
| Calcd (%) | C, 64.20; | H, 6.06; | N, 9.36 |
| Found (%) | C, 64.34; | H, 6.04; | N, 9.26 |

### Example 7

### N-[4-(6-Chloro-2-naphthyl)sulfonylbutyl]-1-(4-pyridyl)-4-piperidinecarhoxamide

### 7a) tert-Butyl 4-hydroxybutylcarbamate

A 2N aqueous sodium hydroxide solution (16 ml) was added to a solution of 4-amino-1-butanol (2.7 g) in acetonitrile (30 ml), and di-tert-butyl dicarbonate (6.7 g) was added dropwise under water-cooling while stirring. A temperature was returned to a room temperature, the mixture was stirred for 3 hours, concentrated under reduced pressure, extracted with ethyl acetate, washed successively with water, an aqueous dilute potassium hydrogensulfate solution and saturated brine, and dried over anhydrous sodium sulfate. Concentration under reduced pressure afforded the title compound as colorless oil (7.9 g, 77%).
NMR (CDCl₃) δ: 1.44 (9H, m), 1.5-1.68 (4H, m), 3.10-3.22 (2H, m), 3.62-3.74 (2H, m).

### 7b) tert-Butyl 4-bromobutylcarbamate

Carbon tetrabromide (5 g) was added at once to a solution of tert-butyl 4-hydroxybutylcarbamate (1.89 g) obtained in Example 7a) and triphenylphosphine (3.15 g) in methylene chloride (20 ml), and the mixture was further stirred at room temperature for 2 minutes. The reaction mixture was washed by addition of saturated sodium bicarbonate water, followed by further washing with saturated brine. The extract was concentrated, and the residue was purified by silica gel column to obtain the title compound as colorless oil (1.76 g, 70%).
NMR (CDCl₃) δ: 1.46 (9H, s), 1.50-2.00 (4H, m), 3.08-3.12 (2H, m), 3.43 (2H, t, J = 6.6).

### 7c) tert-Butyl N-[4-(6-chloro-2-naphthyl)thiobutyl]-carbamate

According to the same manner as that of Example 3a), the title compound was obtained as colorless crystals (0.74 g, 79%) from tert-butyl 4-bromobutylcarbamate (0.76 g) obtained in Example 7b).
NMR (CDCl₃) δ: 1.42 (9H, s), 1.50-1.80 (4H, m), 3.03 (2H, t, J = 7.0), 3.05-3.20 (2H, m), 4.50 (1H, bs), 7.35-7.46 (2H, m), 7.60-7.74 (3H, m), 7.75 (1H, s).

### 7d) tert-Butyl N-[4-(6-chloro-2-naphthyl)sulfonylbutyl]-carbamate

mCPBA (0.35 g) was added to a solution of tert-butyl N-[4-(6-chloro-2-naphthyl)thiobutyl]carbamate (0.35 g) obtained in Example 7c) in ethyl acetate (50 ml), the mixture was stirred at room temperature for 1 hour, extracted with ethyl acetate, washed with saturated sodium bicarbonate water twice and further with an aqueous sodium thiosulfate solution, and dried over anhydrous sodium sulfate. The extract was concentrated under reduced pressure, and the residue was crystallized from hexane/ethyl acetate to obtain the title compound as colorless crystals (0.35 g, 92%).
NMR (CDCl₃) δ: 1.39 (9H, s), 1.48-1.90 (4H, m), 3.00-3.28 (4H, m), 4.54 (1H, bs) 7.59 (1H, dd, J = 1.8 and 8.8), 7.82-8.00 (4H, m), 8.46 (1H.s).

### 7e) N-[4-(6-Chloro-2-naphthyl)sulfonylbutyl]-1-(4-pyridyl)-4-piperidinecarboxamide

According to the same manner as that of Example 6d), the title compound was obtained as colorless crystals (0.2 g, 77%) from tert-butyl N-[4-(6-chloro-2-naphthyl)sulfonyl-butyl]carbamate (0.21 g) obtained in Example 7d).
NMR (CDCl₃) δ: 1.55-1.90 (8H, m), 2.15-2.36 (1H, m), 2.74-2.95 (2H, m), 3.21 (2H, t, J = 7.5), 3.27 (2H, t, J = 6.2), 3.76-3.95 (2H, m), 5.84 (1H, t, J = 5.7), 6.63 (1H, dd, J = 1.4 and 5.0), 7.59 (1H, dd, J = 2.2 and 8.8), 7.82-8.00 (4H, m), 8.24 (2H, dd, J = 1.4 and 5.0), 8.45 (1H, s).

| Elemental Analysis: C₂₅H₂₈ClN₃O₃S | | | |
|---|---|---|---|
| Calcd (%) | C, 61.78; | H, 5.81; | N, 8.65 |
| Found (%) | C, 61.70; | H, 5.76; | N, 8.69 |

### Example 8

### N-[4-[(6-Chloro-2-naphthyl)sulfonyl]butyl-N-methyl-1-(4-pyridyl)-4-piperidinecarboxamide

### 8a) tert-Butyl N-[4-(6-chloro-2-naphthyl)sulfonylbutyl]-N-methylcarbamate

According to the same manner as that of Example 6c), the title compound was obtained as colorless oil (0.14 g, 50%) from tert-butyl N-[4-(6-chloro-2-naphthyl)sulfonyl-butyl]carbamate obtained in Example 7d).

### 8b) N-[4-[(6-Chloro-2-naphthyl)sulfonyl]butyl-N-methyl-1-(4-pyridyl)-4-piperidinecarboxamide

According to the same manner as that of Example 6d), the title compound was obtained as a colorless powdery solid (0.15 g, 88%) from tert-butyl N-[4-(6-chloro-2-naphthyl)sulfonylbutyl]-N-methylcarbamate (0.14 g) obtained in Example 8a).
NMR (CDCl₃) δ: 1.55-1.90 (8H, m), 2.54-3.00 (3H, m), 3.02 (3H, s), 3.25 (2H, t, J = 7.5), 3.36 (2H, t, J = 6.4), 3.74-3.98 (2H, m), 6.65 (2H, d, J = 6.6), 7.58 (1H, dd, J = 2.2 and 8.8), 7.82-8.00 (4H, m), 8.25 (2H, d, J = 6.5), 8.45 (1H, s).

| Elemental Analysis: C₂₆H₃₀ClN₃O₃S | | | |
|---|---|---|---|
| Calcd (%) | C, 62.54; | H, 6.05; | N, 8.40 |
| Found (%) | C, 62.27; | H, 6.05; | N, 8.49 |

### Example 9

### N-[3-(6-Chloro-2-naphthyl)sulfonylpropyl]-N-methyl-1-(4-pyridyl)-4-piperidinecarboxamide

### 9a) tert-Butyl N-(3-bromopropyl)carbamate

According to the same manner as that of Example 6a), the title compound was obtained as colorless oil (4.37 g, 91%) from 3-bromopropylamine hydrobromide (4.37 g).
NMR (CDCl₃) δ: 1.45 (9H, s), 1.96-2.15 (2H, m), 3.27 (2H, q, J = 6.6), 3.44 (2H, t, J = 6.4), 4.65 (1H, bs).

### 9b) tert-Butyl N-[3-(6-chloro-2-naphthyl)thiopropyl]carbamate

According to the same manner as that of Example 3a), the title compound was obtained as colorless crystals (0.28 g, 69%) from tert-butyl N-(3-bromopropyl)carbamate (0.18 g) obtained in Example 9a).
NMR (CDCl₃) δ: 1.44 (9H, s), 1.78-1.96 (2H, m), 3.05 (2H, t, J = 7.2), 3.27 (2H, q, J = 6.6), 4.60 (1H, bs), 7.35-7.48 (2H, m), 7.65 (1H, d, J = 2.4), 7.68-7.80 (2H, m), 7.76 (1H, d, J = 2.2).

### 9c) tert-Butyl N-[3-(6-chloro-2-naphthyl)sulfonylpropyl]-carbamate

According to the same manner as that of Example 7d), the title compound was obtained as colorless crystals (1.07 g, 98%) from tert-butyl N-[3-(6-chloro-2-naphthyl)thiopropyl]carbamate (1.1 g) obtained in Example 9b).
NMR (CDCl₃) δ: 1.40 (9H, s), 1.85-2.05 (2H, m), 3.15-3.35 (4H, m), 4.68 (1H, bs), 7.59 (1H, dd, J = 2.2 and 8.4), 7.85-8.00 (4H, m), 8.46 (1H, s).

### 9d) N-[3-(6-Chloro-2-naphthyl)sulfonylpropyl]-1-(4-pyridyl)-4-piperidinecarboxamide

According to the same manner as that of Example 6d), the title compound was obtained as colorless crystals (0.13 g, 85%) from tert-butyl N-[3-(6-chloro-2-naphthyl)sulfonylpropyl]carbamate (0.12 g) obtained in Example 9c).
NMR (CDCl₃) δ: 1.28-1.78 (5H, m), 2.15-2.40 (1H, m), 2.68-2.90 (2H, m), 2.98-3.18 (2H, m), 3.20-3.48 (3H, m), 3.78-3.98 (2H, m), 6.76 (2H, d, J = 6.6), 7.70-8.00 (3H, m), 8.05-8.35 (5H, m), 8.62 (1H, s).

| Elemental Analysis: C₂₄H₂₆ClN₃O₃S·0.4H₂O | | | |
|---|---|---|---|
| Calcd (%) | C, 60.15; | H, 5.64; | N, 8.77 |
| Found (%) | C, 60.11; | H, 5.43; | N, 8.57 |

### Example 10

### 4-(6-Chloro-2-naphthyl)thio-1-[[1-(4-pyridyl)-4-piperidyl]carbonyl]piperidine

### 10a) tert-Butyl 4-hydroxy-1-pyperidinecarboxlate

According to the same manner as that of Example 7a), the title compound was obtained as colorless crystals (6.2 g, quantitative) from 4-hydroxypiperidine (3.03 g).
NMR (CDCl₃) δ: 1.30-1.56 (2H, m), 1.46 (9H, s), 1.75-1.95 (2H, m), 2.95-3.14 (2H, m), 3.75-3.95 (3H, m).

### 10b) tert-Butyl 4-bromo-1-piperidinecarboxylate

According to the same manner as that of Example 7b), the title compound was obtained as colorless oil (0.89 g, 67%) from tert-butyl 4-hydroxy-1-piperidinecarboxylate (1.89 g) obtained in Example 10a).
NMR (CDCl₃) δ: 1.46 (9H, s), 1.82-2.20 (4H, m), 3.22-3.40 (2H, m), 3.60-3.78 (2H, m), 4.25-4.42 (1H, m).

### 10c) tert-Butyl 4-(6-chloro-2-naphthyl)thio-1-piperidinecarboxylate

Triethylamine (0.6 ml) was added to a suspension of 6-chloro-2-mercaptonaphthalene (0.23 g) obtained in Example 1d) in acetonitrile (10 ml), the mixture was stirred for 5 minutes, tert-butyl 4-bromo-1-piperidinecarboxlate (0.80 g) obtained in Example 10b) was added, and the mixture was stirred for 20 hours while maintaining at 80°C. The reaction mixture was poured into water, the precipitated crystals were filtered, washed with water, dissolved in ethyl acetate, and dried over anhydrous sodium sulfate. The solvent was distilled off, the residue was purified by silica gel column to obtain colorless oil, which was crystallized with hexane to obtain the title compound as colorless crystals (0.38 g, 38%).
NMR (CDCl₃) δ: 1.44 (9H, s), 1.50-1.70 (2H, m), 1.88-2.06 (2H, m), 2.86-3.06 (2H, m), 3.24-3.46 (1H, m), 3.90-4.06 (2H, m), 7.38-7.55 (2H, m), 7.66-7.76 (2H, m), 7.80 (1H, d, J = 2.2), 7.85 (1H, s).

### 10d) 4-(6-Chloro-2-naphthyl)thio-1-[[1-(4-pyridyl)-4-piperidyl]carbonyl]piperidine

According to the same manner as that of Example 6d), the title compound was obtained as colorless crystals (0.13 g, 85%) from tert-butyl 4-(6-chloro-2-naphthyl)thio-1-piperidinecarboxylate (0.12 g) obtained in Example 10c).
NMR (CDCl₃+DCl) δ: 1.40-2.20 (8H, m), 2.90-3.48 (5H, m), 3.50-3.72 (1H, m), 4.00-4.40 (4H, m), 7.15 (2H, d, J = 7.6), 7.40-7.62 (2H, m), 7.75-7.92 (3H, m), 7.95 (1H, s), 8.09 (2H, d, J = 7.6).

| Elemental Analysis: C₂₆H₂₈ClN₃OS·0.6H₂O | | | |
|---|---|---|---|
| Calcd (%) | C, 65.49; | H, 6.17; | N, 8.83 |
| Found (%) | C, 65.49; | H, 6.26; | N, 8.75 |

### Example 11

### 4-(6-Chloro-2-naphthyl)sulfonyl-1-[[1-(4-pyridyl)-4-piperidyl]carbonyl]piperidine

### 11a) tert-Butyl 4-(6-chloro-2-naphthyl)sulfonyl-1-piperidinecarboxylate

According to the same manner as that of Example 7d), the title compound was obtained as the colorless crystals (0.15 g, 92%) from tert-butyl 4-(6-chloro-2-naphthyl)thio-1-piperidinecarboxylate (0.15 g) obtained in Example 10c).
NMR (CDCl₃) δ: 1.42 (9H, s), 1.55-1.78 (2H, m), 1.94-2.10 (2H, m), 2.55-2.78 (2H, m), 3.02-3.20 (1H, m), 4.15-4.34 (2H, m), 7.60 (1H, dd, J = 1.8 and 8.8), 7.81-8.00 (4H, m), 8.43 (1H, s).

### 11b) 4-(6-Chloro-2-naphthyl)sulfonyl-1-[[1-(4-pyridyl)-4-piperidyl]carbonyl]piperidine

According to the same manner as that of Example 6d), the title compound was obtained as the colorless crystals (58 mg, 33%) from tert-butyl 4-(6-chloro-2-naphthyl)sulfonyl-1-piperidinecarboxylate (0.14 g) obtained in Example 11a).
NMR (DMSO-d₆) δ: 1.38-1.80 (6H, m), 1.82-2.15 (2H, m), 2.40-3.62 (6H, m), 3.80-4.00 (2H, m), 4.00-4.22 (2H, m), 4.50-4.70 (1H, m), 6.71 (2H, d, J = 5.8), 7.64 (1H, dd, J = 2.2 and 8.8), 8.52 (1H, s).

| Elemental Analysis: C₂₆H₂₈ClN₃O₃S·0.5H₂O | | | |
|---|---|---|---|
| Calcd (%) | C, 61.59; | H, 5.76; | N, 8.29 |
| Found (%) | C, 61.33; | H, 5.83; | N, 8.27 |

### Example 12

### 1-[5-(6-Chloro-2-naphthyl)sulfonylpentanoyl]-4-(4-pyridyl)piperazine

### 12a) Ethyl 5-(6-chloro-2-naphthyl)sulfonylvalerate

Sodium ethoxide (0.41 g) and ethyl 5-bromovalerate were added to a suspension of 6-chloro-2-mercaptonaphthalene (0.4 g) obtained in Example 1d) in ethanol (20 ml), and the mixture was stirred at 80°C for 16 hours. The reaction mixture was concentrated, water was added to adjust to pH 2, the solution was extracted with ethyl acetate, washed with saturated brine, and dried over anhydrous sodium sulfate. The extract was concentrated to dryness, the residue was dissolved in ethanol (30 ml), 0.3 ml of concentrated sulfuric acid was added, and the mixture was heated to reflux for 3 hours to perform esterification. The reaction mixture was concentrated, extracted with ethyl acetate, washed with saturated brine, and dried over anhydrous sodium sulfate. mCPBA (0.85 g) was added to the extract, and the mixture was stirred at room temperature for 1 hour. The reaction mixture was washed successively with dilute sodium bicarbonate water and saturated brine, and dried over anhydrous sodium sulfate. After concentration to dryness, the residue was purified by silica gel column to obtain the title compound as colorless crystals (0.59 g, 83%).
NMR (CDCl₃) δ: 1.20 (3H, t, J = 7.0), 1.60-1.90 (4H, m), 2.26 (2H, t, J = 7.5), 3.18 (2H, t-like), 4.08 (2H, q, J = 7.0), 7.59 (1H, dd, J = 1.8 and 8.8), 7.88-8.00 (4H, m), 8.46

### 12b) 5-(6-Chloro-2-naphthyl)sulfonylvaleric acid

A 2 N aqueous sodium hydroxide solution (4 ml) was added to a solution of ethyl 5-(6-chloro-2-naphthyl)sulfonylvalarate (0.59 g) obtained in Example 12a) in methanol (10 ml), and the mixture was stirred at room temperature for 3 hours. The reaction mixture was concentrated, and water was added to the residue to dissolve it. pH was adjusted to 2 with 3 N hydrochloric acid, and the precipitated crystals were filtered and washed with water. This solid was dissolved in a mixed solvent of ethyl acetate and THF, dried over anhydrous sodium sulfate, concentrated, and the residue was crystallized with ethyl acetate and ether to obtain the title compound as colorless needles (0.47 g, 86%).
NMR (CDCl₃+DMSO-d₆) δ: 1.60-1.90 (4H, m), 2.28 (2H, t, J = 6.7), 3.19 (2H, t, J = 7.5), 5.10 (1H, bs), 7.59 (1H, dd, J = 2.2 and 8.8), 7.82-8.00 (4H, m), 8.46 (1H, s).

### 12c) 1-[5-(6-Chloro-2-naphthyl)sulfonylpentanoyl]-4-(4-pyridyl)piperazine

A solution of the 5-(6-chloro-2-naphthyl)sulfonyl-valerate (0.33 g) obtained in Example 12b), HOBt (0.17 g) and WSC (0.29 g) in acetonitrile (30 ml) was stirred for 1 hour, 4-pyridylpiperazine (0.17 g) was added thereto, and the mixture was stirred at room temperature for 16 hours. The reaction mixture was concentrated, extracted with ethyl acetate, washed successively with water, sodium bicarbonate water and saturated brine, and dried over anhydrous sodium sulfate. The extract was concentrated, and the residue was crystallized from ethyl acetate to obtain the title compound as colorless crystals (0.42 g, 88%).
NMR (DMSO-d₆) δ: 1.42-1.74 (4H, m), 2.20-2.42 (2H, m), 3.10-3.60 (10H, m), 6.80 (2H, d, J = 6.6), 7.71 (1H, dd, J = 2.2 and 8.8), 7.97 (1H, dd, J = 1.4 and 8.8), 8.10-8.36 (5H, m), 8.62 (1H, s).

| Elemental Analysis: C₂₄H₂₆ClN₃O₃S | | | |
|---|---|---|---|
| Calcd (%) | C, 61.07; | H, 5.55; | N, 8.80 |
| Found (%) | C, 60.29; | H, 5.53; | N, 8.68 |

### Example 13

### 1-[5-(6-Chloro-2-naphthyl)sulfonylpentyl]-4-(4-pyridyl)piperazine

1 M Diborane-THF complex (1 ml) was added to a suspension of 1-[5-(6-chloro-2-naphthyl)sulfonyl-pentanoyl]-4-(4-pyridyl)piperazine (0.15 g) obtained in Example 12c) in THF (15 ml), and the mixture was heated to reflux for 3 hours under a nitrogen stream. The mixture was degraded by addition of 3 N hydrochloric acid, the mixture was made alkaline with saturated sodium bicarbonate water, extracted with ethyl acetate, washed with saturated brine, and dried over anhydrous sodium sulfate. The extract was concentrated, and the residue was crystallized from methanol/hexane to obtain the title compound (11 mg, 24%).
NMR (CDCl₃) δ: 1.30-1.60 (4H, m), 1.65-1.90 (2H, m), 2.33 (2H, t, J = 7.0), 2.48 (4H, t, J = 5.0), 3.10-3.35 (6H, m), 6.63 (2H, d, J = 6.0), 7.59 (1H, dd, J = 1.8 and 8.8), 7.80-8.00 (4H, m), 8.26 (2H, bs), 8.46 (1H, s).

| Elemental Analysis: C₂₄H₂₈ClN₃O₂S·1.75H₂O | | | |
|---|---|---|---|
| Calcd (%) | C, 58.88; | H, 6.49; | N, 8.58 |
| Found (%) | C, 58.83; | H, 6.30; | N, 8.43 |

### Example 14

### Methyl 2-[N-[3-(6-chloro-2-naphthyl)sulfonylpropyl]-N-[[1-(4-pyridyl)-4-piperidinyl]carbonyl]amino]acetate

### 14a) N-[3-(6-Chloro-2-naphthyl)sulfonylpropyl]-2,4-dinitrobenzenesulfonamide

Trifluoroacetic acid (2 ml) was added to a suspension of tert-butyl N-[3-(6-chloro-2-naphthyl)sulfonylpropyl]-carbamate (0.77 g) obtained in Example 9c) in toluene, and the mixture was stirred at room temperature for 2 hours. Toluene was added to the reaction mixture, concentration to dryness was repeated twice. Methylene chloride (40 ml) was added to the residue to suspend it, diisopropylamine (0.7 ml) was added, and 2,4-dinitrobenzenesulfonyl chloride (0.6 g) was added, followed by stirring at room temperature for 30 minutes. The mixture was extracted with methylene chloride, washed with water, washed successively with dilute hydrochloric acid and saturated brine, and dried over anhydrous sodium sulfate. The extract was concentrated, and the residue was purified by silica gel column to obtain the title compound as a yellow powder (0.5 g, 48%).
NMR (CDCl₃+DMSO-d₆) δ: 1.90-2.10 (2H, m), 3.10-3.40 (4H, m), 7.61 (1H, dd, J = 1.8 and 8.8), 7.80-8.10 (4H, m), 8.28 (1H, d, J=8.8), 8.40-8.60 (3H, m).

### 14b) N-[3-(6-Chloro-2-naphthyl)sulfonylpropyl]-N-methoxycarbonylmethyl-2,4-dinitrobenzenesulfonylamide

Potassium carbonate (0.48 g) was added to a solution of N-[3-(6-chloro-2-naphthyl)sulfonylpropyl]-2,4-dinitrobenzenesulfoneamine (0.36 g) obtained in Example 14a) in DMF (7 ml), methyl bromoacetate (0.095 ml) was added, and the mixture was stirred at room temperature for 2 hours. The mixture was extracted with ethyl acetate, washed with water and saturated brine, and dried over anhydrous sodium sulfate. The solvent was distilled off under reduced pressure to obtain the title compound. This crude purified material was used in the next reaction as it was.
NMR (CDCl₃) δ: 2.00-2.20 (2H, m), 3.27 (2H, t, J = 7.4), 3.60 (2H, t, J = 7.4), 3.65 (3H, s), 4.19 (2H, s), 7.60 (1H, dd, J = 1.8 and 8.8), 7.85-8.05 (4H, m), 8.25 (1H, d, J = 8.8), 8.38-8.55 (3H, m).

### 14c) Methyl 2-[N-[3-(6-chloro-2-naphthyl)sulfonylpropyl]-amino]acetate

Isopropylamine (1 ml) was added to a solution of N-[3-(6-chloro-2-naphthyl)sulfonylpropyl]-N-methoxycarbonylmethyl-2,4-dinitrobenzenesulfonylamide obtained in Example 14b) in methylene chloride (7 ml), and the mixture was stirred at room temperature for 30 minutes. The reaction mixture was concentrated, extracted with ethyl acetate, washed successively with sodium bicarbonate water and saturated brine, and dried over anhydrous sodium sulfate. The extract was concentrated, and the residue was purified by silica gel column to obtain the title compound as colorless oil (0.13 g, 52%).
NMR (CDCl₃) δ: 1.80-2.00 (2H, m), 2.71 (2H, t, J = 6.6), 3.22-3.35 (2H, m), 3.33 (2H, s), 3.70 (3H, s), 7.59 (1H, dd, J = 2.0 and 8.8), 7.85-8.00 (4H, m), 8.47 (1H, s).

### 14d) Methyl 2-[N-[3-(6-chloro-2-naphthyl)sulfonylpropyl]-N-[[1-(4-pyridyl)-4-piperidinyl]carbonyl]amino]acetate

Diisopropylethylamine (0.38 ml) was added to a solution of methyl 2-[N-[3-(6-chloro-2-naphthyl)-sulfonylpropyl]amino]acetate (0.13 g) obtained in Example 14c) in methylene chloride (20 ml), 1-(4-pyridinyl)-4-piperazinecarbonyl chloride hydrochloride (0.19 g) was added, and the mixture was stirred for 15 hours. The reaction mixture was concentrated, extracted with ethyl acetate, washed successively with water, sodium bicarbonate water and saturated brine, and dried over anhydrous sodium sulfate. The extract was concentrated, the residue was purified by basic silica gel column, and crystallized from ethyl acetate/ether to obtain the title compound as colorless crystals (84 mg, 42%).
NMR (CDCl₃) δ: 1.65-2.20 (6H, m), 2.35-3.00 (3H, m), 3.15-3.32 (2H, m), 3.40-4.18 (9H, m), 6.64 (2H, d, J = 5.2), 7.55-7.70 (1H, m), 7.80-8.00 (4H, m), 8.25 (2H, d, J = 5.2), 8.46 (1H, s).

| Elemental Analysis: C₂₇H₃₀ClN₃O₅S | | | |
|---|---|---|---|
| Calcd (%) | C, 59.61; | H, 5.56; | N, 7.72 |
| Found (%) | C, 59.61; | H, 5.69; | N, 7.78 |

### Example 15

### 2-[N-[3-(6-Chloro-2-naphthyl)sulfonylpropyl]-N-[[1-(4-pyridyl)-4-piperidinyl]carbonyl]amino]acetic acid

According to the same manner as that of Example 12b), the title compound was obtained as a colorless powder (0.05 g, 76%) from methyl 2-[N-[3-(6-chloro-2-naphthyl)sulfonylpropyl]-N-[[1-(4-pyridyl)-4-piperidinyl]carbonyl]amino]acetate (84 mg) obtained in Example 14d).
NMR (CD₃OD) δ: 1.50-2.10 (6H, m), 2.75-3.75 (7H, m), 3.91 (2H, s), 4.10-4.30 (2H, m), 7.09 (2H, d, J = 7.8), 7.65 (1H, dd, J = 1.8 and 8.8), 7.90-8.20 (6H, m), 8.54 (1H, s).

| Elemental Analysis: C₂₆H₂₈ClN₃O₅S·1.0H₂O | | | |
|---|---|---|---|
| Calcd (%) | C, 56.98; | H, 5.52; | N, 7.67 |
| Found (%) | C, 57.21; | H, 5.29; | N, 7.84 |

### Example 16

### N-[3-(6-Chloro-2-naphthyl)sulfonylpropyl]-N-ethyl-1-(4-pyridyl)-4-piperidinecarboxamide

### 16a) N-[3-(6-Chloro-2-naphthyl)sulfonylpropyl]-N-ethyl-2,4-dinitrobenzenesulfonylamide

According to the same manner as that of Example 14b), the title compound was obtained as a yellow powder (0.42 g, 94%) from N-[3-(6-chloro-2-naphthyl)sulfonylpropyl]-2,4-dinitrobenzenesulfonamide (0.42 g) obtained in Example 14a).
NMR (CDCl₃) δ: 1.14 (3H, t, J = 7.2), 2.00-2.20 (2H, m), 3.15-3.30 (2H, m), 3.38 (2H, q, J = 7.2), 3.51 (2H, t, J = 7.2), 7.60 (1H, dd, J = 1.8 and 8.8), 7.82-8.00 (5H, m), 8.20-8.60 (3H,m ).

### 16b) 3-(6-Chloro-2-naphthyl)sulfonyl-N-ethylpropylamine

According to the same manner as that of Example 14c), the title compound was obtained as colorless oil (0.15 g, 62%) from N-[3-(6-chloro-2-naphthyl)sulfonylpropyl]-N-ethyl-2,4-dinitrobenzenesulfonylamide (0.42 g) obtained in Example 16a).
NMR (CDCl₃) δ: 1.04 (3H, t, J = 7.1), 1.80-2.00 (2H, m), 2.57 (2H, q, J = 7.1), 2.69 (2H, t, J = 7.0), 3.20-3.35 (2H, m), 7.58 (1H, dd, J = 1.8 and 8.8), 7.55-8.00 (4H, m), 8.46 (1H, s).

### 16c) N-[3-(6-Chloro-2-naphthyl)sulfonylpropyl]-N-ethyl-1-(4-pyridyl)-4-piperidinecarboxamide

According to the same manner as that of Example 6d), the title compound was obtained as a colorless powder (0.23 g, 92%) from 3-(6-chloro-2-naphthyl)sulfonyl-N-ethylpropylamine (0.15 g) obtained in Example 16b).
NMR (CDCl₃) δ: 1.07 (0.75H, t, J = 7.2), 1.22 (2.25H, t, J = 7.2), 1.60-2.20 (6H, m), 2.55-2.78 (1H, m), 2.78-3.00 (2H, m), 3.10-3.26 (2H, m), 3.26-3.60 (4H, m), 3.78-4.00 (2H, m), 6.64 (2H, d, J = 6.6), 7.55-7.70 (1H, m), 7.80-8.10 (4H, m), 8.24 (2H, d, J = 6.6), 8.46 (1H, s).

| Elemental Analysis: C₂₆H₃₀ClN₃O₃S·1.0H₂O | | | |
|---|---|---|---|
| Calcd (%) | C, 60.28; | H, 6.23; | N, 8.11 |
| Found (%) | C, 60.41; | H, 6.16; | N, 8.18 |

### Example 17

### N-[3-(6-Chloro-2-naphthyl)sulfonylpropyl]-N-[1-(4-pyridyl)-4-piperidyl] methylacetamide

1M borane-THF complex (1 ml) was added to a suspension of N-[3-[(6-chloro-2-naphthyl)sulfonyl]propyl-N-methyl-1-(4-pyridyl)-4-piperidinecarboxamide (81 mg) obtained in Example 9d) in anhydrous THF (6 ml) under a nitrogen stream, and the mixture was heated to reflux for 8 hours. After degradation by addition of 3 N hydrochloric acid, the reaction mixture was concentrated, made alkaline with sodium bicarbonate water, extracted with ethyl acetate, washed with saturated brine, and dried over anhydrous sodium sulfate. The extract was concentrated to dryness, the residue was dissolved in methylene chloride (10 ml), acetic anhydride (0.1 ml) and diisopropylamine (0.2 ml) were added, and the mixture was stirred at room temperature for 16 hours. The reaction mixture was concentrated, made alkaline with sodium bicarbonate water, extracted with ethyl acetate, washed with saturated brine, and dried over anhydrous sodium sulfate. The extract was concentrated to dryness, and the residue was purified with basic silica gel column to obtain the title compound as a colorless powder (47 mg, 55%).
NMR (CDCl₃) δ: 1.10-1.40 (2H, m), 1.60-2.20 (7H, m), 2.36-2.96 (3H, m), 3.10-3.36 (4H, m), 3.40-3.76 (2H, m), 3.76-4.00 (2H, m), 6.58-6.72 (2H, m), 7.55-7.68 (1H, m), 7.84-8.00 (4H, m), 8.17-8.33 (2H, m), 8.46 (1H, s).

| Elemental Analysis: C₂₆H₃₀ClN₃O₃S·0.5H₂O | | | |
|---|---|---|---|
| Calcd (%) | C, 61.34; | H, 6.14; | N, 8.25 |
| Found (%) | C, 61.29; | H, 6.18; | N, 8.34 |

### Example 18

### N-[3-(6-Chloro-2-naphthyl) sulfonylpropyl]-N-isopropyl-1-(4-pyridyl)-4-piperidinecarboxamide

### 18a) 2,4-Dinitro-N-isopropylbenzenesulfonamide

2,4-Dinitrobenzenesulfonyl chloride (1.07 g) was added to a solution of isopropylamine (0.36 g) and pyridine (0.53 ml) in methylene chloride (15 ml), and the mixture was stirred at room temperature for 30 minutes. After adjusted to pH 2, the mixture was concentrated, extracted with ethyl acetate, and washed with water and saturated brine.
Extract was dried over anhydrous sodium sulfate, concentrated, and the residue was purified by silica gel column to obtain the title compound as colorless needles (0.46 g, 40%).
NMR (CDCl₃) δ: 1.19 (6H, d, J = 6.6), 3.62-3.82 (1H, m), 5.18 (1H, d, J = 7.2), 8.40 (1H, d, J = 8.8), 8.56 (1H, dd, J = 2.2 and 8.8), 8.68 (1H, d, J = 2.2).

### 18b) 3-(6-Chloro-2-naphthyl)sulfonylpropanol

Sodium methoxide (0.49 g) and 3-bromopropanol (0.93 g) were added to a solution of 6-chloro-2-mercaptonaphthalene (0.87 g) obtained in Example 1d) in methanol (90 ml), and the mixture was heated to reflux for 15 hours. The mixture was cooled, the insolubles were filtered, and the filtrate was concentrated. Water was added to the residue, pH was adjusted to 2 with dilute hydrochloric acid, and the mixture was extracted with ethyl acetate, and washed with water and saturated brine. The extract was dried over anhydrous sodium sulfate, and concentrated. Ethyl acetate (50 ml) was added to the residue to dissolve it, and mCPBA (2.1 g) was added thereto, followed by stirring at room temperature for 1 hour. The reaction mixture was washed with sodium bicarbonate water twice, and washed with saturated brine. The extract was dried over anhydrous sodium sulfate, and concentrated, the residue was purified by silica gel column, hexane-ethyl acetate (1:1) was added, the precipitated crystals were filtered to obtain the title compound as colorless crystals (0.7 g, 55%).
NMR (CDCl₃) δ: 1.94-2.10 (2H, m), 3.25-3.38 (2H, m), 3.76 (2H, t, J = 6.0), 7.59 (1H, dd, J = 2.2 and 8.8), 7.80-8.00 (4H, m), 8.49 (1H, s).

### 18c) N-[3-(6-Chloro-2-naphthyl)sulfonylpropyl]-2,4-dinitro-N-isopropylbenzenesulfoneamine

Diethyl azodicarboxylate (DEAD) (40% toluene solution, 0.3 ml) was added to a solution of 3-(6-chloro-2-naphthyl)sulfonylpropanol (0.28 g) obtained in Example 18b), 2,4-dinitro-N-isopropylbenzenesulfonamide (0.29 g) obtained in Example 18a) and triphenylphosphine (0.32 g) in THF (5 ml), and the mixture was stirred at room temperature for 5 hours. The reaction mixture was concentrated, adjusted to pH 2 with dilute hydrochloric acid, the mixture was extracted with ethyl acetate, and washed with water and saturated brine. The extract was dried over anhydrous sodium sulfate, and concentrated. The residue was purified by silica gel column, ethyl acetate-ether (1:2) was added, and the precipitated crystals were filtered to obtain the title compound as colorless crystals (0.49 g, 88%).
NMR (CDCl₃) δ: 1.18 (6H, d, J = 6.6), 2.05-2.25 (2H, m), 3.24 (2H, t, J = 7.3), 3.49 (2H, t, J = 7.5), 4.00-4.20 (1H, m), 7.61 (1H, dd, J = 1.8 and 8.8), 7.85-8.00 (4H, m), 8.26 (1H, d, J = 8.4), 8.40-8.52 (3H, m).

### 18d) 3-(6-Chloro-2-naphthyl)sulfonyl-N-isopropylpropylaimne

According to the same manner as that of Example 14c), the title compound was obtained as colorless oil (0.19 g, 81%) from N-[3-(6-chloro-2-naphthyl)sulfonylpropyl]-2,4-dinitro-N-isopropylbenzenesulfonamide (0.4 g) obtained in Example 18c).
NMR (CDCl₃) δ: 1.08 (6H, d, J = 6.2), 1.92-2.12 (2H, m), 2.75-3.00 (1H, m), 2.80 (2H, t, J = 7.0), 3.25-3.38 (2H, m), 7.57 (1H, dd, J = 2.0 and 8.8), 7.90-8.00 (4H, m), 8.48( 1H,s).

### 18e) N-[3-(6-Chloro-2-naphthyl)sulfonylpropyl]-N-isopropyl-1-(4-pyridyl)-4-piperidinecarboxamide

According to the same manner as that of Example 14d), the title compound (0.17 g, 54%) was obtained from 3-(6-chloro-2-naphthyl)sulfonyl-N-isopropylpropylamine (0.19 g) obtained in Example 18d).
NMR (CDCl₃) δ: 1.12 (1.2H, d, J = 6.6), 1.25 (4.8H, d, J = 6.6), 1.60-2.20 (6H, m), 2.60-2.80 (1H, m), 2.80-3.00 (2H, m), 3.21 (2H, t, J = 7.5), 3.35 (2H, t, J = 7.7), 3.80-4.00 (2H, m), 4.00-4.20 (1H, m), 6.60-6.70 (2H, m), 7.56 (1H, dd, J = 1.8 and 8.8), 7.80-8.00 (4H, m), 8.20-8.30 (2H, m), 8.47 (1H, s).

| Elemental Analysis: C₂₇H₃₂ClN₃O₃S·0.5H₂O | | | |
|---|---|---|---|
| Calcd (%) | C, 62.00; | H, 6.36; | N, 8.03 |
| Found (%) | C, 62.29; | H, 6.34; | N, 8.34 |

### Example 19

### N-[3-(6-Chloro-2-naphthyl)sulfonylpropyl]-N-phenyl-1-(4-pyridyl)-4-piperidinecarboxamide

### 19a) 2,4-Dinitro-N-phenylbenzenesulfonamide

According to the same manner as that of Example 18a), the title compound was obtained as pale yellow needles (77%) from aniline.
NMR (CDCl₃) δ: 7.16-7.38 (5H, m), 8.04 (1H, d, J = 8.4), 8.37 (1H, dd, J = 2.2 and 8.4), 8.66 (1H, d, J = 2.2).

### 19b) N-[3-(6-Chloro-2-naphthyl)sulfonylpropyl]-2,4-dinitro-N-phenylbenzenesulfonamide

According to the same manner as that of Example 18c), the title compound was obtained as colorless crystals (96%) from 2,4-dinitro-N-phenylbenzenesulfoneaimde obtained in Example 19a).
NMR (DMSO-d₆) δ: 1.52-1.78 (2H, m), 3.46 (2H, t, J = 7.7), 3.82 (2H, t, J = 6.4), 7.02-7.38 (2H, m), 7.73 (1H, dd, J = 1.8 and 8.8), 7.78-7.97 (2H, m), 8.10-8.30 (3H, m), 8.49 (1H, dd, J = 2.6 and 8.8), 8.55 (1H, s), 8.95 (1H, d, J = 2.2).

### 19c) 3-(6-Chloro-2-naphthyl)sulfonyl-N-phenylpropylamine

According to the same manner as that of Example 18d), the title compound was obtained as pale yellow crystals (82%) from N-[3-(6-chloro-2-naphthyl)sulfonylpropyl]-2,4-dinitro-N-phenylbenzenesulfonamide obtained in Example 19b).
NMR (CDCl₃) δ: 2.00-2.18 (2H, m), 3.20-3.40 (4H, m), 3.69 (1H, bs), 6.54 (2H, d, J = 7.4), 6.69 (1H, t, J = 7.4), 7.11 (1H, d, J = 7.2), 7.15 (1H, d, J = 7.2), 7.58 (1H, dd, J = 1.8 and 8.8), 7.80-8.00 (4H, m), 8.46 (1H, s).

### 19d) N-[3-(6-Chloro-2-naphthyl)sulfonylpropyl]-N-phenyl-1-(4-pyridyl)-4-piperidinecarboxamide

According to the same manner as that of Example 18e), the title compound (51%) was obtained from 3-(6-chloro-2-naphthyl)sulfonyl-N-phenylpropylamine obtained in Example 19c).
NMR (CDCl₃) δ: 1.55-2.05 (6H, m), 2.25-2.44 (1H, m), 2.44-2.68 (2H, m), 3.15-3.30 (2H, m), 3.78 (1H, t, J = 7.0), 6.67 (2H, d , J = 5.0), 7.05-7.20 (2H, m), 7.35-7.50 (3H, m), 7.59 (1H, dd, J = 1.8 and 8.8), 7.80-8.00 (4H, m), 8.21 (2H, d, J = 5.0), 8.42 (1H, s).

| Elemental Analysis: C₃₀H₃₀ClN₃O₃S | | | |
|---|---|---|---|
| Calcd (%) | C, 65.74; | H, 5.52; | N, 7.67 |
| Found (%) | C, 65.04; | H, 6.39; | N, 8.19 |

### Example 20

### Ethyl 2-[N-[3-(6-chloro-2-naphthyl)sulfonylpropyl]-N-[[1-(4-pyridyl)-4-piperidyl]carbonyl]amino]acetate

### 20a) Ethyl 2-(2,4-dinitrophenyl)sulfonylamino]acetate

According to the same manner as that of Example 18a), the title compound was obtained as pale yellow needles (53%) from glycine ethyl ester hydrochloride.
NMR (CDCl₃) δ: 1.19 (3H, t, J = 7.2), 4.07 (2H, q, J = 7.2), 4.08 (2H, d, J = 5.8), 6.14 (1H, t, J = 5.8), 8.31 (1H, d, J = 8.8), 8.55 (1H, dd, J = 2.2 and 8.8), 8.76 (1H, d, J = 2.2).

### 20b) Ethyl2-[N-[3-(6-chloro-2-naphthyl)sulfonylpropyl]-N-[(2,4-dinitrophenyl)sulfonyl]amino]acetate

According to the same manner as that of Example 18c), the title compound was obtained as a colorless powder (quantitative) from ethyl 2-[(2,4-dinitrophenyl)-sulfonylamino]acetate obtained in Example 20a).
NMR (DMSO-d₆) δ: 1.52-1.78 (2H, m), 3.46 (2H, t, J = 7.7), 3.82 (2H, t, J = 6.4), 7.02-7.38 (2H, m), 7.73 (1H, dd, J = 1.8 and 8.8), 7.78-7.97 (2H, m), 8.10-8.30 (3H, m), 8.49 (1H, dd, J = 2.6 and 8.8), 8.55 (1H, s), 8.95 (1H, d, J = 2.2).

### 20c) 3-(6-chloro-2-naphthyl)sulfonyl-N-phenylpropylamine

According to the same manner as that of Example 18d), the title compound was obtained as the pale yellow crystals (82%) from ethyl 2-[N-[3-(6-chloro-2-naphthyl)sulfonyl-propyl]-N-[(2,4-dinitrophenyl)sulfonyl]amino]acetate obtained in Example 20b).
NMR (CDCl₃) δ: 1.28 (3H, t, J = 7.2), 2.00-2.20 (2H, m), 3.27 (2H, t, J = 7.5), 3.61 (2H, t, J = 6.8), 4.18 (2H, s), 4.21 (2H, q, J = 7.2), 7.61 (1H, dd, J = 1.8 and 8.8), 7.88-8.00 (4H, m), 8.25 (1H, d, J = 8.8), 8.38-8.55 (3H, m).

### 20d) Ethyl 2-[N-[3-(6-chloro-2-naphthyl)sulfonylpropyl]-N-[[1-(4-pyridyl)-4-piperidyl]carbonyl]amino]acetate

According to the same manner as that of Example 18e), the title compound (61%) was obtained from 3-(6-chloro-2-naphthyl)sulfonyl-N-phenylpropylaimne obtained in Example 20c).
NMR (CDCl₃) δ: 1.23 (1.5H, t, J = 7.2), 1.29 (1.5H, t, J = 7.2), 1.65-2.15 (6H, m), 2.35-2.60 (0.5H, m), 2.70-3.02 (2.5H, m), 3.15-3.32 (2H, m), 3.54 (1H, t, J = 6.6), 3.67 (1H, t, J = 7.4), 3.80-3.96 (2H, m), 3.99 (1H, s), 4.10 (1H, s), 4.12 (1H, q, J = 7.2), 4.21 (1H, q, J = 7.2), 6.64 (2H, d, J = 4.8), 7.55-7.68 (1H, m), 7.82-8.00 (4H, m), 8.25 (2H, d, J = 5.8), 8.45 (0.5H, s), 8.47 (0.5H, s).

| Elemental Analysis: C₂₈R₃₂ClN₃O₅S | | | |
|---|---|---|---|
| Calcd (%) | C, 60.26; | H, 5.78; | N, 7.53 |
| Found (%) | C, 60.28; | H, 6.05; | N, 7.63 |

### Example 21

### N-(2-Amino-2-oxoethyl)-N-[3-(6-chloro-2-naphthyl)sulfonyl-propyl]-1-(4-pyridyl)-4-piperidinecarboxamide

WSC (47 mg) was added to a solution of 2-[N-[3-(6-chloro-2-naphthyl)sulfonylpropyl]-N-[[1-(4-pyridyl)-4-piperidinyl]carbonyl]]amino]acetic acid (90 mg) obtained in Example 15 and HOBt (25 mg) in DMF (3 ml), and the mixture was stirred at room temperature for 1 hour. Then, 25% aqueous ammonia (0.1 ml) was added, the mixture was stirred for 18 hours, and WSC (47 mg) was further added, and the mixture was stirred for 7 hours. The reaction mixture was concentrated, extracted with ethyl acetate, washed with saturated brine, dried over anhydrous sodium sulfate, the solvent was distilled off, and the residue was crystallized from DMF-ethyl acetate to obtain the title compound as colorless crystals (51 mg, 57%).
NMR (DMSO-d₆+D₂O) δ: 1.35-2.00 (6H, m), 2.50-3.00 (3H, m), 3.20-4.00 (6H, m), 3.78 (1H, s), 4.00 (1H, s), 6.70 (1H, d, J = 6.0), 6.76 (1H, d, J = 6.0), 7.69 (1H, dd, J = 2.0 and 8.8), 7.88-8.02 (1H, m), 8.05-8.40 (5H, m), 8.57 (0.5H, s), 8.60 (0.5H, s).

| Elemental Analysis: C₂₆R₂₉ClN₄O₄S·0.5H₂O | | | |
|---|---|---|---|
| Calcd (%) | C, 58.04; | H, 5.62; | N, 10.41 |
| Found (%) | C, 58.32; | H, 5.59; | N, 10.23 |

### Example 22

### N-[3-(6-Chloro-2-naphthyl)sulfonylpropyl]-N-[2-(4-morpholinyl)-2-oxoethyl]-1-(4-pyridyl)-4-piperidinecarboxamide

According to the same manner of Example 21, the title compound (79%) was obtained from 2-[N-[3-(6-chloro-2-naphthyl)sulfonylpropyl]-N-[[1-(4-pyridyl)-4-piperidinyl]carbonyl]amino]acetic acid obtained in Example 15 and morpholine.
NMR (DMSO-d₆+DCl) δ: 1.35-2.00 (6H, m), 2.55-4.30 (19H, m), 7.13 (2H, d, J = 6.4), 7.72 (1H, dd, J = 2.2 and 8.8), 7.95 (1H, dt, J = 1.8 and 9.6), 8.10-8.36 (5H, m), 8.61 (1H, s).

| Elemental Analysis: C₃₀H₃₅ClN₄O₅S | | | |
|---|---|---|---|
| Calcd (%) | C, 60.14; | H, 5.89; | N, 9.35 |
| Found (%) | C, 59.86; | H, 5.88; | N, 9.13 |

### Example 23

### N-[3-(6-Chloro-2-naphtyl)sulfonylpropyl]-N-[[2-oxo-2-(4-pyridyl)methylamino]ethyl]-1-(4-pyridyl)-4-piperidinecarboxamide

According to the same manner of Example 21, the title compound (6.0%) was obtained from 2-[N-[3-(6-chloro-2-naphthyl)sulfonylpropyl]-N-[[1-(4-pyridyl)-4-piperidinyl]carbonyl]amino]acetic acid obtained in Example 15 and 4-aminomethylpyridine.
NMR (DMSO-d₆+DCl) δ: 1.70-2.40 (7H, m), 2.70-3.06 (2H, m), 3.08-3.30 (2H, m), 3.50-4.20 (6H, m), 4.38 and 4.46 (total 2H, each d, J = 6.2), 6.65 (2H, d, J = 6.6), 7.13 (1H, d, J = 6.2), 7.15-7.30 (1H, m), 7.55-8.00 (5H, m), 8.23 (2H, d, J = 6.0), 8.40-8.60 (3H, m).

| Elemental Analysis: C₃₂H₃₄ClN₅O₄S·1.25H₂O | | | |
|---|---|---|---|
| Calcd (%) | C, 59.80; | H, 5.72; | N, 10.90 |
| Found (%) | C, 59.75; | H, 5.77; | N, 10.90 |

### Example 24

### Ethyl 2-[N-[4-(6-chloro-2-naphthyl)sulfonylbutanoyl]-N-[1-(4-pyridyl)-4-piperidyl]amino]acetate

### 24a) Ethyl 4-(6-chloro-2-naphtyl)thiobutyrate

Ethyl 4-bromobutyrate (1.07 g) was added to a solution of 6-chloro-2-mercaptonaphthalene (0.97 g) obtained in Example 1d) and sodium ethoxide (0.48 g) in ethanol (20 ml), and the mixture was stirred at 60°C for 1 hour. The reaction mixture was concentrated under reduced pressure, water was added to the residue, the mixture was extracted with ethyl acetate, washed with water, and dried over anhydrous magnesium sulfate. The solvent was distilled off to obtain the title compound as colorless crystals (1.41 g, 92%).
NMR (CDCl₃) δ: 1.24 (3H, t, J = 7.1), 2.01 (2H, m), 2.49 (2H, t, J = 7.2), 3.07 (2H, t, J = 7.2), 4.13 (2H, q, J = 7.1), 7.40 (1H, dd, J = 8.7 and 2.1), 7.43 (1H, dd, J = 8.8 and 1.7), 7.66 (1H, d, J = 8.4), 7.68 (1H, d, J = 8.8), 7.72 (1H, d, J = 1.7), 7.76 (1H, d, J = 2.1).

### 24b) Ethyl 4-(6-chloro-2-naphthyl)sulfonylbutyrate

A solution of mCPBA (2.38 g) in ethyl acetate (20 ml) was added dropwise to a solution of ethyl 4-(6-chloro-2-naphthyl)thiobutyrate (1.41 g) obtained in Example 24a) in ethyl acetate (20 ml). The reaction mixture was stirred at room temperature for 1.5 hours, washed with saturated sodium bicarbonate water and saturated brine, and dried over anhydrous magnesium sulfate. The solvent was distilled off to obtain the title compound as colorless oil (1.54 g, quantitative).
NMR (CDCl₃) δ: 1.21 (3H, t, J = 7.3), 1.98-2.13 (2H, m), 2.46 (2H, t, J = 7.1), 3.23-3.30 (2H, m), 4.11 (2H, q, J = 7.3), 7.59 (1H, dd, J = 8.8 and 1.8), 7.87-7.99 (4H, m), 8.47 (1H, s).

### 24c) 4-(6-Chloro-2-naphthyl)sulfonylbutyric acid

According to the same manner as that of Example 12b), the title compound was obtained as colorless needles (1.21 g, 83%) from ethyl 4-(6-chloro-2-naphthyl)sulfonylbutyrate (1.54 g) obtained in Example 24b).
NMR (CDCl₃) δ: 1.97-2.15 (2H, m), 2.55 (2H, t, J = 7.0), 3.27 (2H, t, J = 7.6), 7.60 (1H, dd, J = 8.7 and 1.7), 7.87-7.98 (4H, m), 8.48 (1H, s).

### 24d) Ethyl 2-[1-(4-pyridyl)-4-piperidyl]aminoacetate

Sodium cyanoborohydrate (63 mg) was added to a solution of 1-(4-pyridyl)-4-piperidone (0.18 g) and glycine ethyl ester hydrochloride (0.14 g) in ethanol (6 ml), the mixture was stirred for 30 minute, and concentrated to dryness. The residue was dissolved in water and purified by CHP-20 column. 1 N Hydrochloric acid was added to the end fraction solution to adjust to pH 2.0, and concentrated to dryness to obtain the title compound (0.21 g).
NMR (CD₃OD) δ: 1.33 (3H, t, J = 7.2), 1.60-1.95 (2H, m), 2.25-2.40 (2H, m), 3.18- 40 (2H, m), 3.55-3.80 (1H, m), 4.09 (2H, s), 4.33 (2H, q, J = 7.2), 4.35-4.55 (2H, m), 7.25 (2H, d, J = 8.0), 8.17 (2H, d, J = 8.0).

### 24e) Ethyl 2-[N-[4-(6-chloro-2-naphthyl)sulfonylbutanoyl]-N-[1-(4-pyridyl)-4-piperidyl]amino]acetate

Thionyl chloride (1 ml) was added to a suspension of 4-(6-chloro-2-naphthyl)sulfonylbutyric acid (0.31 g) obtained in Example 24c) in toluene (5 ml), the mixture was stirred for 2 hours while maintaining at 90°C. The reaction mixture was cooled, and concentrated to dryness to obtain the acid chloride, which was added to a suspension of ethyl 2-[1-(4-pyridyl)-4-piperidinyl]amino]acetate (0.25 g) obtained in Example 24d) and diisopropylamine (0.7 ml) in methylene chloride (20 ml). The reaction mixture was stirred at room temperature for 16 hours, concentrated, and extracted with ethyl acetate. The extract was washed with sodium bicarbonate water and saturated brine, and dried over anhydrous sodium sulfate. The solvent was distilled off, and the residue was purified by basic silica gel column to obtain the title compound as a colorless powder (0. 13 g, 31%).
NMR (CDCl₃) δ: 1.25 (1.5H, t, J = 7.3), 1.28 (1.5H, t, J = 7.3), 1.35-1.96 (5H, m), 2.02-2.28 (2H, m), 2.45 (1H, t, J = 6.4), 2.73 (1H, t, J = 6.4), 2.80-3.02 (2H, m), 3.30 (1H, t, J = 6.8), 3.33 (1H, t, J = 6.8), 3.80-4.05 (2H, m), 3.88 (1H, s), 3.91 (1H, s), 4.14 (1H, q, J = 7.3), 4.21 (1H, q, J = 7.3), 6.60-6.70 (2H, m), 7.59 (1H, dd, J = 1.8 and 8.8), 8.20-8.32 (2H, m), 8.77 (1H, s).

| Elemental Analysis: C₂₈H₃₂ClN₃O₅S | | | |
|---|---|---|---|
| Calcd (%) | C, 60.26; | H, 5.78; | N, 7.53 |
| Found (%) | C, 60.01; | H, 5.22; | N, 7.33 |

### Example 25

### N-[4-(6-Chloro-2-naphthyl)sulfonylbutyl]-1-(4-pyridyl)-4-piperidineamine

### 25a) 4-(6-Chloro-2-naphthyl)sulfonylbutylamine trifluoroacetate

Trifluoroacetic acid (3 ml) was added to a suspension of tert-butyl N-[4-(6-chloro-2-naphthyl)sulfonylbutyl]-carbamate (0.75 g) obtained in Example 7d) in toluene (3 ml), and the mixture was stirred at room temperature for 2 hours. Toluene was added, the mixture was concentrated under reduced pressure, hexane was added to the residue, and the precipitated crystals were filtered to obtain the title compound as colorless prisms (0.72 g, 93%).
NMR (CDCl₃+DMSO-d₆) δ: 1.70-1.95 (4H, m), 2.75-3.05 (2H, m), 3.10-3.30 (2H, m), 7.61 (1H, dd, J = 1.8 and 8.8), 7.85-8.10 (4H, m), 8.20-8.60 (3H, bs), 8.46 (1H, s).

### 25b) N-[4-(6-Chloro-2-naphthyl)sulfonylbutyl]-1-(4-pyridyl)-4-piperidineamine

Sodium cyanoborohydride (77 mg) was added to a solution of 4-(6-chloro-2-naphthyl)sulfonylbutylamine trifluoroacetate (0.41 g) obtained in Example 25a) and 1-(4-pyridyl)-4-piperidone (0.18 g) in methanol (10 ml), and the mixture was stirred for 2 hours. The reaction mixture was concentrated, the residue was dissolved in water, purified by CHP-20 column, and crystallized with methanol/ether to obtain the title compound as colorless crystals (0.19 g, 41%).
NMR (CDCl₃) δ: 1.20-1.45 (2H, m), 1.45-1.68 (2H, m), 1.70-1.98 (4H, m), 2.56-2.75 (1H, m), 2.63 (2H, t, J = 7.0), 2.80-3.00 (2H, m), 3.12-3.25 (2H, m), 3.70-3.86 (2H, m), 6.63 (2H, d, J = 6.6), 7.59 (1H, dd, J = 1.8 and 8.8), 7.83-8.00 (4H, m), 8.24 (2H, d, J = 6.6), 8.47 (1H, s).

| Elemental Analysis: C₂₄H₂₈ClN₃O₂S·1.25H₂O | | | |
|---|---|---|---|
| Calcd (%) | C, 59.99; | H, 6.40; | N, 8.74 |
| Found (%) | C, 59.93; | H, 6.15; | N, 8.57 |

### Example 26

### Ethyl N-[4-(6-chloro-2-naphthyl)sulfonylbutyl]-N-[1-(4-pyridyl)-4-piperidine]carbamate

Ethyl chloroformate (0.2 ml) and triethylamine (0.15 ml) were added to a solution of N-[4-(6-chloro-2-naphthyl)sulfonylbutyl]-1-(4-pyridyl)-4-piperidineamine (0.11 g) obtained in Example 25b) in chloroform (8 ml) under ice-cooling, the mixture was stirred for 30 minutes and allowed to warm to room temperature, and the mixture was stirred for 2 hours. The reaction mixture was made alkaline with dilute sodium bicarbonate water, extracted with ethyl acetate, and washed with saturated brine. This was dried over anhydrous sodium sulfate, concentrated, and the residue was purified by basic silica gel column to obtain the title compound as colorless powder (0.12g, 93%).
NMR (CDCl₃) δ: 1.21 (3H, t, J = 7.0), 1.30-1.82 (8H, m), 2.70-2.98 (2H, m), 3.07 (2H, t, J = 6.7), 3.18 (2H, t, J = 7.5), 3.80-4.20 (3H, m), 4.10 (2H, q, J = 7.0), 6.63 (2H, d, J = 4.8), 7.59 (1H, dd, J = 2.0 and 8.8), 7.90-8.00 (4H, m), 8.25 (2H, d, J = 4.8), 8.44 (1H, s).

| Elemental Analysis: C₂₇H₃₂ClN₃O₄S·0.25H₂O | | | |
|---|---|---|---|
| Calcd (%) | C, 60.66; | H, 6.13; | N, 7.86 |
| Found (%) | C, 60.59; | H, 6.08; | N, 7.84 |

### Example 27

### 3-(6-Chloro-2-naphthyl)sulfonyl-N'-[1-(4-pyridyl)-4-piperidyl]propanehydrazide

### 27a) Methyl 3-(6-chloro-2-naphthyl)thiopropionate

A solution of 6-chloro-2-mercaptonaphthalene (6.3 g) obtained in Example 1d), methyl acrylate (2.9 g) and triethylamine (0.9 ml) in ethyl acetate (75 ml) was stirred at room temperature for 2.5 hours. The solvent was distilled off under reduced pressure, and the residue was washed with hexane to obtain the title compound as a colorless solid (8.88 g, 98%).
NMR (CDCl₃) δ: 2.68 (2H, t, J = 7.4), 3.27 (2H, t, J = 7.4), 3.68 (3H, s, OMe), 7.39-7.48 (2H, m), 7.65-7.71 (2H, m), 7.74-7.77 (2H, m).

### 27b) 3-(6-Chloro-2-naphthyl)sulfonylpropionic acid

A solution of methyl 3-(6-chloro-2-naphthyl)thiopropionate (8.88 g) obtained in Example 27a) and 30% hydrogen peroxide in water (6 ml) in acetic acid (60 ml) was refluxed for 30 minutes, concentrated sulfuric acid (6 ml) was added, and the mixture was further refluxed for 1.5 hours. The reaction mixture was diluted with water, the precipitated precipitates were filtered, dried, purified by silica gel column, and recrystallzied from isopropyl ether/hexane to obtain the title compound (8.20 g, 87%).
NMR (CDCl₃) δ: 2.82 (2H, t, J = 7.5), 3.248 (2H, t, J = 7.5), 7.60 (1H, dd, J = 2.0 and 9.0), 7.91-7.97 (4H, m), 8.47 (1H, s).

### 27c) 1-(4-Pyridyl)-4-piperidone hydrazone

Hydrazine monohydrate (1 ml) was added to a solution of 1-(4-pyridyl)-4-piperidone (1.76 g) in methanol (18 ml), and the mixture was stirred at room temperature for 1 hour. The solvent was distilled off, and the residue was crystallized with methanol-ether to obtain the title compound as pale yellow crystals (1.76 g, 92%).
NMR (CDCl₃) δ: 2.50 (2H, t, J = 6.2), 2.61 (2H, t, J = 6.2), 3.54 (2H, t, J = 6.2), 3.63 (2H, t, J = 6.2), 4.99 (2H, bs), 6.60 (2H, d, J = 6.6), 8.27 (2H, d, J = 6.6).

### 27d) 3-(6-Chloro-2-naphthyl)sulfonyl-N'-[1-(4-pyridyl)-4-piperidyl]propanehydrazide

WSC (0.3 g) was added to a solution of 3-(6-chloro-2-naphthyl)sulfonylpropionic acid (0.3 g) obtained in Example 27b) and HOBt (0.17 g) in DMF (6 ml), and the mixture was stirred for 1 hour. Then, 1-(4-pyridyl)-4-piperidone hydrazone (0.19 g) obtained in Example 27c), and the mixture was stirred at room temperature for 4 hours. The reaction mixture was concentrated under reduced pressure, sodium bicarbonate water was added to make alkaline, the solution was extracted with methylene chloride, and dried over anhydrous sodium sulfate. The extract was concentrated, methanol (10 ml) and acetic acid (0.2 ml) were added to the residue, and sodium cyanoborohydride (0.15 g) was added under water-cooling. The mixture was stirred at room temperature for 4 hours, adjusted to pH 1 with 3 N hydrochloric acid, and purified by CHP-20 column to obtain the title compound as pale yellow powder (90 mg, 16%).
NMR (CD₃OD) δ: 1.65-1.95 (2H, m), 2.20-2.24 (2H, m), 2.80 (2H, t, J = 6.6), 3.16-3.40 (2H, m), 3.71 (2H, t, J = 6.6), 3.75-4.00 (1H, m), 4.32-4.52 (2H, m), 7.24 (2H, d, J = 7.2), 7.66 (1H, dd, J = 1.8 and 8.8), 7.90-8.20 (3H, m), 8.15 (2H, d, J = 7.2), 8.57 (1H, s).

| Elemental Analysis: C₂₃H₂₅ClN₄O₃S·HCl·2.75H₂O·0.2MeOH | | | |
|---|---|---|---|
| Calcd (%) | C, 49.28; | H, 5.76; | N, 9.91 |
| Found (%) | C, 49.44; | H, 5.91; | N, 9.62 |

### Example 28

### 3-(6-Chloro-2-naphthyl)sulfonyl-N-methyl-N'-[1-(4-pyridyl)-4-piperidyl]propanehydrazide

Methylhydrazine (0.2 ml) was added to a solution of 1-(4-pyridyl)-4-piperidone (0.36 g) in methanol (7 ml), the mixture was stirred for 24 hours, and the reaction mixture was concentrated under reduced pressure. Toluene was added to the residue, and the mixture was concentrated to dryness again. On the other hand, WSC (0.3 g) was added to a solution of 3-(6-chloro-2-naphthyl)sulfonylpropionic acid (0.3 g) obtained in Example 27b) and HOBt (0.17 g) in DMF (10 ml), the mixture was stirred for 1 hour, the aforementioned hydrazone was added, and the mixture was stirred at room temperature for 4 hours. The reaction mixture was concentrated, sodium bicarbonate water was added to make alkaline, and the solution was extracted with methylene chloride. The extract was dried over anhydrous sodium sulfate, the solvent was distilled off, the residue was dissolved in methanol (10 ml), sodium cyanoborohydride (0.15 g) was added under ice-cooling, and the mixture was stirred at room temperature for 24 hours. The reaction mixture was concentrated, a 1 N aqueous sodium hydroxide solution was added to make alkaline, the precipitated precipitates were dissolved by addition of methanol, which was purified by CHP-20 column to obtain the title compound as a pale yellow powder (0.16 g, 32%).
NMR (CDCl₃) δ: 0.90-1.15 (2H, m), 1.55-1.74 (2H, m), 2.75-3.20 (5H, m), 2.99 (3H, m), 3.48-3.72 (4H, m), 6.72 (2H, d, J = 6.6), 7.53 (1H, dd, J = 2.2 and 8.8), 7.88-8.16 (4H, m), 8.12 (2H, J = 6.6), 8.52 (1H, s).

| Elemental Analysis: C₂₄H₂₇ClN₄O₃S·0.5H₂O | | | |
|---|---|---|---|
| Calcd (%) | C, 58.11; | H, 5.69; | N, 11.30 |
| Found (%) | C, 57.85; | H, 5.85; | N, 11.49 |

### Example 29

### 3-(6-Chloro-2-naphthyl)sulfonyl-N'-methyl-N'-[1-(4-pyridyl)-4-piperidyl]propanehydrazide hydrochloride

36% Formalin (0.2 ml) was added to a solution of 3-(6-chloro-2-naphthyl)sulfonyl-N'-[1-(4-pyridyl)-4-piperidyl]propanehydrazide (0.18 g) obtained in Example 27d) in formic acid (2 ml), and the mixture was stirred at 100°C for 5 hours. The reaction mixture was concentrated, made alkaline with a 1 N aqueous sodium hydroxide solution, and extracted with methylene chloride. The extract was dried over anhydrous sodium sulfate, and concentrated, and the residue was purified by silica gel column and CHP-20 column to obtain the title compound as the colorless powder (85 mg, 40%).
NMR (CDCl₃) δ: 1.50-1.84 (2H, m), 1.85-2.25 (2H, m), 2.50-2.90 (5H, bs), 3.10-3.40 (2H, m), 3.52-3.72 (1H, m), 3.64 (2H, t, J = 6.6), 4.15-4.36 (2H, m), 7.15 (2H, d, J = 7.6), 7.66 (1H, dd, J = 2.2 and 8.8), 7.88-8.16 (7H, m), 8.54 (1H, s).

| Elemental Analysis: C₂₄H₂₇ClN₄O₃S·HCl·1.5H₂O | | | |
|---|---|---|---|
| Calcd (%) | C, 52.36; | H, 5.68; | N, 10.18 |
| Found (%) | C, 52.52; | H, 5.78; | N, 10.06 |

### Example 30

### 4-(6-Chloro-2-naphthyl)sulfonyl-N-methyl-N-[1-(4-pyridyl)-4-piperidinyl]butaneamide

### 30a) 4-Methylamino-1-(4-pyridyl)piperidine

Acetic acid (0.34 g) was added to a solution of 1-(4-pyridyl)-4-piperidone (0.88 g) and methylamine hydrochloride (0.37 g) in methanol (10 ml), sodium cyanoborohydride (0.34 g) was added, and the mixture was stirred at room temperature for 17 hours. The reaction mixture was concentrated, a 1 N aqueous sodium hydroxide solution was added to the residue to make alkaline, and the solution was extracted with chloroform. The extract was dried over anhydrous sodium sulfate, and the solvent was distilled off to obtain the title compound as pale brown oil (0.96 g, quantitative).
NMR (CDCl₃) δ: 1.22-1.50 (2H, m), 1.90-2.10 (2H, m), 2.47 (3H, s), 2.51-2.70 (1H, m), 2.84-3.02 (2H, m),3.75-3.92 (2H, m), 6.66 (2H, d, J = 6.6), 8.25 (2H, d, J = 6.6).

### 30b) 4-(6-Chloro-2-naphthyl)sulfonyl-N-methyl-N-[1-(4-pyridyl)-4-piperidyl]butaneamide

WSC (0.15 g) was added to a solution of 4-(6-chloro-2-naphthyl)sulfonylbutyric acid (0.16 g) obtained in Example 24c) and HOBt (80 mg) in DMF (5 ml), the mixture was stirred for 1 hour, 4-methylamino-1-(4-pyridyl)piperidine (0.12 g) obtained in Example 30a) was added, and the mixture was stirred at room temperature for 14 hours. DMF was distilled off, the solution was made alkaline with a 1 N aqueous sodium hydroxide solution, and extracted with ethyl acetate. The extracted was washed with saturated brine, and dried over anhydrous sodium sulfate. The solvent was distilled off, ether was added to the residue, and the crystals were filtered to obtain the title compound as colorless crystals (0.22 g, 90%).
NMR (CDCl₃) δ: 1.55-1.90 (4H, m), 2.00-2.25 (2H, m), 2.45-2.74 (2H, m), 2.79 (3H, s), 2.82-3.02 (2H, m), 3.34 (2H, t, J = 7.2), 3.88-4.05 (2H, m), 4.52-4.80 (1H, m), 6.65 (2H, d, J = 6.6), 7.59 (1H, dd, J = 1.8 and 8.8), 7.80-8.00 (4H, m), 8.26 (2H, d, J = 6.6), 8.47 (1H, s).

| Elemental Analysis: C₂₅H₂₈ClN₃O₃S·0.1H₂O | | | |
|---|---|---|---|
| Calcd (%) | C, 61.55; | H, 5.83; | N, 8.61 |
| Found (%) | C, 61.44; | H, 5.70; | N, 8.76 |

### Example 31

### 4-(6-Chloro-2-naphthyl)sulfonyl-N-methyl-N-[1-(4-pyridyl)-4-piperidyl]propanamide

According to the same manner as that of Example 30b), the title compound (0.12 g, 60%) was obtained from 3-(6-chloro-2-naphthyl)sulfonylpropionic acid obtained in Example 27b) and 4-methylamino-1-(4-pyridyl)piperidine obtained in Example 30a).
NMR (CDCl₃) δ: 1.50-1.95 (4H, m), 2.70-3.08 (4H, m), 2.83 (3H, s), 3.58 (2H, t, J = 7.9), 3.80-4.10 (2H, m), 4.46-4.72 (1H, m), 6.65 (2H, d, J = 6.6), 7.60 (1H, dd, J = 1.8 and 8.8), 7.80-8.00 (4H, m), 8.26 (2H, d, J = 6.6), 8.49 (1H, s).

| Elemental Analysis: C₂₄H₂₆ClN₃O₃S·0.2H₂O·0.1Et₂O | | | |
|---|---|---|---|
| Calcd (%) | C, 60.67; | H, 5.75; | N, 8.78 |
| Found (%) | C, 60.69; | H, 5.72; | N, 8.88 |

### Example 32

### 4-(6-Chloro-2-naphthyl)sulfonyl-N-methyl-N-[1-(4-pyridyl)-4-piperidyl]propaneamine hydrochloride

A solution of 4 N hydrogen chloride in acetic acid was added to a solution of 4-(6-chloro-2-naphthyl)sulfonyl-N-methyl-N-[1-(4-pyridyl)-4-piperidyl]propioneamide obtained in Example 31 in methanol, to make the solution acidic. The solvent was distilled off to obtain the title compound as a pale yellow powder (56%).
NMR (CD₃OD) δ: 1.40-2.00 (4H, m), 2.50-3.40 (3H, m), 2.85 (3H, s), 3.65 (2H, bs), 4.00-4.70 (4H, m), 7.15 (2H, bs), 7.55-7.75 (1H, m), 7.85-8.30 (6H, m), 8.56 (1H, s).

| Elemental Analysis: C₂₄H₂₆ClN₃O₃S·HCl·2.75H₂O | | | |
|---|---|---|---|
| Calcd (%) | C, 51.66; | H, 5.87; | N, 7.53 |
| Found (%) | C, 51.59; | H, 5.79; | N, 7.48 |

### Example 33

### Ethyl 2-[N-[3-(6-chloro-2-naphthyl)sulfonylpropioyl]-N-[1-(4-pyridyl)-4-piperidyl]amino]acetate

According to the same manner as that of Example 30b), the title compound was obtained as a colorless powder from ethyl 2-[1-(4-pyridyl)-4-piperidinyl]amino]acetate obtained in Example 24d) and 3-(6-chloro-2-naphthyl)sulfonyl-propionic acid obtained in Example 27b).
NMR (CDCl₃) δ:1.23 and 1.27 (3H, each t, J = 7.3), 1.25-2.00 (5H, m), 2.70-3.17 (4H, m), 3.50-3.65 (2H, m), 3.70-4.10 (2H, m), 3.87, 3.93 (2H, each s), 4.13 and 4.18(2H, each q, J = 7.3), 6.58-6.72 (2H, m), 7.55-7.65 (1H, m), 7.80-8.00 (4H, m), 8.20-8.34 (2H, m), 8.49 (1H, s).

| Elemental Analysis: C₂₇H₃₀ClN₃O₅S·0.55H₂O | | | |
|---|---|---|---|
| Calcd (%) | C, 58.54; | H, 5.66; | N, 7.59 |
| Found (%) | C, 58.27; | H, 5.77; | N, 7.87 |

### Example 34

### Ethyl 3-[N-[3-(6-chloro-2-naphthyl)sulfonylpropioyl]-N-[1-(4-pyridyl)-4-piperidyl]amino]propionate

### 34a) Ethyl (3-[1-(4-pyridyl)-4-piperidyl]aminopropionate

According to the same manner as that of Example 24d), the title compound (1.44 g, quantitative) was obtained from 1-(4-pyridyl)-4-piperidone (0.88g) and β-alanine ethyl ester hydrochloride (0.93 g).
NMR (CDCl₃) δ: 1.26 (3H, t, J = 7.1), 1.28-1.55 (2H, m), 1.88-2.08 (2H, m), 2.51 (2H, t, = 6.4), 2.64-2.85 (1H, m), 2.85-3.05 (4H, m), 3.50-4.00 (1H, bs), 3.75-3.92 (2H, m), 4.15 (2H, q, J = 7.2), 6.66 (2H, d, J = 6.4), 8.23 (2H, d, J = 6.4).

### Example 34b) Ethyl 3-[N-[3-(6-chloro-2-naphthyl)sulfonylpropionyl]-N-[1-(4-pyridyl)-4 piperidyl]aminolpropionate

Thionyl chloride (2 ml) was added to 3-(6-chloro-2-naphthyl)sulfonylpropionic acid (0.30 g) obtained in Example 27b), and the mixture was stirred for 1 hour while maintaining at 90°C. Toluene was added, the solution was concentrated to dryness to obtain the acid chloride, which was added to a solution of ethyl 3-[1-(4-pyridyl)-4-piperidyl]aminopropionate (0.2 g) obtained in Example 34a) and diisopropylethylamine (0.4 ml) in methylene chloride (20 ml), the mixture was stirred for 1 hour under water-cooling, and further stirred at room temperature for 2 hours. The mixture was washed by addition of water, and dried over anhydrous sodium sulfate. The solvent was distilled off, and the residue was purified by basic silica gel column and LH-20 column to obtain the title compound as a colorless powder (45 mg, 8%).
NMR (CDCl₃) δ: 1.22 and 1.28 (3H, each t, J = 7.0), 1.50-2.00 (5H, m), 2.35-2.60 (2H, m), 2.72-3.08 (4H, m), 3.32-3.68 (4H, m), 3.70-4.20 (4H, m), 6.58-6.74 (2H, m), 7.61 (1H, dd, J = 2.2 and 8.8), 7.80-8.05 (4H, m), 8.20-8.40 (2H, m), 8.49 (1H, s).

| Elemental Analysis: C₂₈H₃₂ClN₃O₅S·0.5H₂O | | | |
|---|---|---|---|
| Calcd (%) | C, 59.30; | H, 5.87; | N, 7.41 |
| Found (%) | C, 59.38; | H, 5.61; | N, 7.51 |

### Example 35

### 2-(6-Chloro-2-naphthyl)sulfonyl-N-methyl-N-[1-(4-pyridyl)-4-piperidyl]acetamide

### 35a) Methyl 2-(6-chloro-2-naphthyl)thioacetate

Sodium methoxide (0.81 g) was added to a suspension of 6-chloro-2-mercaptonaphthalene (1.95 g) obtained in Example 1d) in methanol (40 ml), the mixture was stirred for 10 minutes, methyl bromoacetate (1.14 ml) was added, and the mixture was stirred at room temperature for 2 hours. The insolubles were filtered, the filtrate was concentrated, adjusted to pH 1 with 3 N hydrochloric acid, and extracted with ethyl acetate. The extract was washed with saturated brine, and dried over anhydrous sodium sulfate. The solvent was distilled off, hexane containing a small amount of ethyl acetate was added to the residue, and the crystallized title compound was obtained as colorless prisms (0.91 g, 34%). Further, 1.7 g of the title compound was obtained as oil from the mother liquid.
NMR (CDCl₃) δ: 3.72 (3H, s), 3.75 (2H, s), 7.42 (1H, dd, J = 2.2 and 8.8), 7.49 (1H, dd, J = 2.2 and 8.8), 7.64-7.85 (4H, m).

### 35b) Methyl 2-(6-chloro-2-naphthyl)sulfonylacetate

According to the same manner as that of Example 24b), the title compound was obtained as colorless crystals (2.5 g, 84%) from methyl 2-(6-chloro-2-naphthyl)thioacetate (2.6 g) obtained in Example 35a).
NMR (CDCl₃) δ: 3.70 (3H, s), 4.21 (2H, s), 7.60 (1H, dd, J = 1.8 and 8.8), 7.90-8.00 (4H, m), 8.51 (1H, s).

### 35c) 2-(6-Chloro-2-naphthyl)sulfonylacetic acid

According to the same manner as that of Example 12b), the title compound was obtained as colorless crystals (2.25 g, 93%) from methyl 2-(6-chloro-2-naphthyl)sulfonylacetate (2.45 g) obtained in Example 35b).
NMR (CDCl₃+DMSO-d₆) δ: 4.20 (2H, s), 7.59 (1H, dd, J = 2.2 and 8.8), 7.90-8.05 (4H, m), 8.54 (1H, s).

### 35d) 2-(6-Chloro-2-naphthyl)sulfonyl-N-methyl-N-[2-(4-pyridyl)-4-piperidyl]acetamide

According to the same manner as that of Example 34b), the title compound was obtained as colorless crystals from 2-(6-chloro-2-naphthyl)sulfonylacetic acid obtained in Example 35c) and 4-methylamino-1-(4-pyridyl)piperidine obtained in Example 30a).
NMR (CDCl₃) δ: 1.60-2.00 (4H, m), 2.81, 3.02 (3H, each s), 2.82-3.16 (2H, m), 3.88-4.20 (2H, m), 4.34 and 4.41 (2H, each s), 4.45-4.75 (1H, m), 6.60-6.76 (2H, m), 7.59 (1H. dd, J = 2.0 and 8.8), 7.90-8.00 (4H, m), 8.25-8.36 (2H, m), 8.49 (1H, s).

| Elemental Analysis: C₂₃H₂₄ClN₃O₃S | | | |
|---|---|---|---|
| Calcd (%) | C, 60.32; | H, 5.28; | N, 9.18 |
| Found (%) | C, 60.17; | H, 5.25; | N, 9.19 |

### Example 36

### N-[3-(6-Chloro-2-naphthyl)sulfonylpropyl]-N-methyl-N'-[1-(4-pyridyl)-4-piperidyl]urea

Diphenylphosphoryl azide (DPPA) (0.22 ml) was added to a solution of 4-(6-chloro-2-naphthyl)sulfonylbutyric acid (0.31 g) obtained in Example 34c) and triethylamine (0.15 ml) in toluene (10 ml), the mixture was stirred at room temperature for 15 minutes, and stirred at 110°C for 1.5 hours. After cooling, 4-methylamino-1-(4-pyridyl)piperidine (0.2 g) obtained in Example 30a) and methylene chloride (8 ml) were added, and the mixture was stirred at room temperature for 3 hours. The solvent was distilled off, the solution was made alkaline with a 1N aqueous sodium hydroxide solution, and extracted with chloroform. After drying with anhydrous sodium sulfate, the solvent was distilled off, the residue was purified by silica gel column, and recrystallized from methanol/ethyl acetate to obtain the title compound as colorless crystals (0.27 g, 53%).
NMR (CDCl₃+DMSO-d₆) δ: 1.54-1.75 (4H,m), 1.87-2.08 (2H, m), 2.67 (3H, s), 2.80-3.04 (2H, m), 3.20-3.42(4H, m), 3.88-4.05(2H, m), 4.24-4.50(1H, m), 5.58(1H, t, J = 5.8), 6.67 (2H, d, J = 6.6), 7.60 (1H, dd, J = 1.8 and 8.8), 7.82-8.02 (4H, m), 8.22 (2H,d, J = 6.6), 8.47 (1H, s).

| Elemental Analysis: C₂₅H₂₉ClN₄O₃S | | | |
|---|---|---|---|
| Calcd (%) | C, 59.93; | H, 5.83; | N, 11.18 |
| Found (%) | C, 59.71; | H, 5.86; | N, 11.09 |

### Example 37

### N-[3-(6-Chloro-2-naphthyl)sulfonylethyl]-N'-methyl-N'-[1-(4-pyridyl)-4-piperidyl]urea

According to the same manner as that of Example 36, the title compound was obtained as colorless crystals from 3-(6-chloro-2-naphthyl)sulfonylpropionic acid obtained in Example 27b) and 4-methylamino-1-(4-pyridyl)piperidine obtained in Example 30a).
NMR (CDCl₃) δ: 1.52-1.75 (4H, m), 2.67 (1H, m), 2.67 (3H, s), 2.80-3.05 (2H, m), 3.36-3.50 (2H, m), 3.68-3.82 (2H, m), 3.87-4.0 5(2H, m), 4.24-4.50 (1H, m), 5.34 (1H, t, J = 5.7), 6.65 (2H, d, J = 6.6), 7.61 (1H, dd, J = 1.8 and 8.8), 7.85-8.00 (4H, m), 8.26 (2H, d, J = 6.6), 8.47 (1H, s).

| Elemental Analysis: C₂₄H₂₇ClN₄O₃S | | | |
|---|---|---|---|
| Calcd (%) | C, 59.19; | H, 5.59; | N, 11.50 |
| Found (%) | C, 58.95; | H, 5.77; | N, 11.46 |

### Example 38

### 3-[(4-Chlorophenyl)methylsulfonyl]-N-methyl-N-[1-(4-pyridyl)-4-piperidyl]propanamide

### 38a) Methyl-3-(4-chlorophenyl)methylthiopropionate

4-Chlorobenzyl chloride (1.61 g) was added to a solution of methyl 3-mercaptopropionate (1.26 g) and a 2 N aqueous sodium hydroxide solution (5.5 ml) in methanol (20 ml), and the mixture was stirred at room temperature for 20 hours. The reaction mixture was concentrated under reduced pressure, water was added, and the mixture was extracted with ethyl acetate. The extract was washed with water, dried over anhydrous magnesium sulfate, and the solvent was distilled off to obtain the title compound as colorless oil (2.01 g, 82%).
NMR (CDCl₃) δ: 2.51-2.72 (4H, m), 3.69 (3H, s), 3.70 (2H, s), 7.28 (4H, m).

### 38b) Methyl 3-(4-chlorophenyl)methylsulfonylpropionate

According to the same manner as that of Example 24b), the title compound was obtained as colorless leaflets (90%) from methyl 3-(4-chlorophenyl)methylthiopropionate obtained in Example 38a).
NMR (CDCl₃) δ: 2.84 (2H, t, J = 7.4), 3.20 (2H, t, J = 7.4), 3.74 (3H, s), 4.26 (2H, s), 7.39 (4H, s-like).

### 38c) 3-(4-Chlorophenyl)methylsulfonylpropionate

A solution of the methyl 3-(4-chlorophenyl)-methylsulfonylpropionate (2.04 g) obtained in Example 38b) and concentrated sulfuric acid (3 ml) in formic acid (20 ml) was refluxed for 14 hours, and the reaction mixture was diluted with water. The precipitated crystals were filtered, dried, and recrystallized from acetic acid/hexane to obtain the title compound as colorless needles (1.71 g, 88%).
NMR (CDCl₃) δ: 2.89 (2H, t, J = 7.1), 3.21 (2H, t, J = 7.1), 4.26 (2H, s), 7.39 (4H, s-like).

### 38d) 3-[(4-Chlorophenyl)methylsulfonyl]-N-methyl-N-[1-(4-pyridyl)-4-piperidyl]propanamide

According to the same manner as that of Example 30b), the title compound was obtained as colorless needles (48%) from 3-(4-chlorophenyl)methylsulfonyl-propionic acid obtained in Example 38c) and 4-methylamino-1-(4-pyridyl)piperidine obtained in Example 30a).
NMR (CDCl₃) δ: 1.63-1.79 (4H, m), 2.79-3.03 (7H, m), 3.29 (2H, t, J = 6.8), 3.93-4.02 (2H, m), 4.30 (2H, s), 4.65-4.74 (1H, m), 6.67 (2H, d, J = 6.5), 7.41 (4H, s-like), 8.27 (2H, d, J = 6.5).

| Elemental Analysis: C₂₁H₂₆N₃O₃SCl | | | |
|---|---|---|---|
| Calcd (%) | C, 57.85; | H, 6.01; | N, 9.64 |
| Found (%) | C, 57.56; | H, 6.07; | N, 9.68 |

### Example 39

### 3-[(2'-Cyano-4-biphenyl)methylsulfonyl]-N-methyl-N-[1-(4-pyridyl)-4-piperidyl]propanamide

### 39a) Methyl 3-[(2'-cyano-4-biphenyl)methylthio]propionate

According to the same manner as that of Example 38a), the title compound was obtained as colorless oil (quantitative) from methyl 3-mercaptopropionate and 4-bromomethyl-2'-cyanobiphenyl.
NMR (CDCl₃) δ: 2.56-2.64 (2H, m), 2.71-2.79 (2H, m), 3.70 (3H, s), 3.80 (2H, s), 7.40-7.55 (6H, m), 7.61-7.69 (1H, m), 7.77 (1H, dd, J = 0.9 and 7.5).

### 39b) Methyl 3-[(2'-cyano-4-biphenyl)methylsulfonyl]-propionate

According to the same manner as that of Example 24b), the title compound was obtained as colorless prisms (79%) from methyl 3-[(2'-cyano-4-biphenyl)-methylthio]propionate obtained in Example 39a).
NMR (CDCl₃) δ: 2.86 (2H, t, J = 7.4), 3.27 (2H, t, J = 7.4), 3.74 (3H, s), 4.36 (2H, s), 7.44-7.72 (7H, m), 7.79 (1H, dd, J = 1.4 and 7.6).

### 39c) 3-[(2'-Cyano-4-biphenyl)methylsulfonyl]propionic acid

According to the same manner as that or Example 38c), the title compound was obtained as colorless prisms (71%) from methyl 3-[(2'-cyano-4-bipheyl)methylsulfonyl]propionate obtained in Example 39b). NMR (DMSO-d₆) δ: 2.72 (2H, t, J = 7.4), 3.35 (2H, t, J = 7.4), 4.65 (2H, s), 7.54-7.68 (6H, m), 7.78-7.95 (1H, m), 7.98 (1H, dd, J = 1.2 and 7.4).

### 39d) 3-[(4-Chlorobenzyl)sulfonyl]-N-methyl-N-[1-(4-pyridyl)-4-piperidyl]propanamide

According to the same manner as that of Example 30b), the title compound was obtained as colorless prisms (71%) from 3-[(2'-cyano-4-biphenyl)-methylsulfonyl]propionic acid obtained in Example 39c) and 4-methylamino-1-(4-pyridyl)piperidine obtained in Example 30a).
NMR (CDCl₃) δ: 1.63-1.88 (4H, m), 2.80-3.03 (7H, m), 3.37 (2H, t, J = 7.2), 3.95-4.01 (2H, m), 4.40 (2H, s), 4.62-4.79 (1H, m), 6.66 (2H, d, J = 6.5), 7.44-7.53 (2H, m), 7.61-7.72 (5H, m), 7.79 (1H, d, J = 7.6), 8.27 (2H, d, J = 6.5).

| Elemental Analysis: C₂₈H₃₀N₄O₃S | | | |
|---|---|---|---|
| Calcd (%) | C, 66.91; | H, 6.02; | N, 11.15 |
| Found (%) | C, 65.44; | H, 5.96; | N, 10.51 |

### Example 40

### 3-[(6-Chloro-2-naphthyl)methylsulfonyl]-N-methyl-N-[1-(4-pyridyl)-4-piperidyl]propanamide

### 40a) Methyl 3-[(6-chloro-2-naphthyl)methylthio]propionate

According to the same manner as that of Example 38a), the title compound was obtained as colorless oil (42%) from methyl 4-mercaptopropionate and 2-chloro-6-chloromethylnaphthalene (Haydock D.B.et al., Eur. J.Med.Chem.Chim.Ther, 1984, 19, 205).
NMR (CDCl₃) δ: 2.52-2.73 (4H, m), 3.67 (3H, s), 3.87 (2H, s), 7.42 (1H,dd, J = 1.8 and 8.6), 7.51 (1H, dd, J = 1.6 and 8.4), 7.69-7.80 (4H, m).

### 40b) Methyl 3-[(6-chloro-2-naphthyl)methylsulfonyl]-propionate

According to the same manner of that of Example 24b), the title compound was obtained as colorless prisms (43%) from methyl 3-[(6-chloro-2-naphthyl)methylthio]propionate obtained in Example 40a).
NMR (CDCl₃) δ: 2.83 (2H, t, J = 7.4), 3.23 (2H, t, J = 7.4), 3.72 (3H, s), 4.44 (2H, s), 7.47 (1H, dd, J = 1.8 and 8.8), 7.57 (1H, dd, J = 1.4 and 8.4), 7.79 (1H, s), 7.83 (1H, s), 7.85-7.90 (2H, m).

### 40c) 3-[(6-Chloro-2-naphthyl)methylsulfonyl]propionic acid

According to the same manner as that of Example 38c), the title compound was obtained as a pale brown solid (quantitative) from methyl 3-[(6-chloro-2-naphthyl)methylsulfonyl]propionate obtained in Example 40b). NMR (DMSO-d₆) δ: 2.65 (2H, t, J = 7.4), 3.33 (2H, t, J = 7.4), 4.72 (2H, s), 7.54-7.62 (2H, m), 7.93-8.03 (3H, m), 8.08 (1H, d, J = 1.8).

### 40d) 3-[(6-Chloro-2-naphthyl)methylsulfonyl]-N-methyl-N-[1-(pyridyl)-4-piperidyl]propanamide

According to the same manner as of that of Example 30b), the title compound was obtained as colorless prisms (71%) from 3-[(6-chloro-2-naphthyl)methylsulfonyl]propionic acid obtained in Example 40c) and 4-methylamino-1-(4-pyridyl)piperidine obtained in Example 30a).
NMR (CDCl₃) δ: 1.67-1.76 (4H, m), 2.78-2.85 (5H, m), 2.91-3.13 (2H, m), 3.32 (2H, t, J = 7.5), 3.93-4.14 (2H, m), 4.48 (2H, s), 4.67-4.76 (1H, m), 6.73 (2H, d, J = 7.0), 7.47 (1H, dd, J = 2.0 and 8.8), 7.60 (1H, dd, J = 1.4 and 8.2), 7.79 -7.84 (3H, m), 7.93 (1H, s), 8.22 (2H, d, I = 7.0).

| Elemental Analysis: C₂₅H₂₈N₃O₃SCl | | | |
|---|---|---|---|
| Calcd (%) | C, 57.85; | H, 6.01; | N, 9.64 |
| Found (%) | C, 57.56; | H, 6.07; | N, 9.68 |

### Example 41

### Ethyl 3-[N-[3-(6-chloro-2-naphthyl)sulfonylpropionyl]-N-[1-(4-pyridyl)-4-piperidyl]amino]propionate

### 41a) tert-Butyl 4-(2-ethoxycarbonylethyl)amino-1-piperidinecarboxylate

Acetic acid (1.2 g) was added to a solution of tert-butyl 4-oxo-1-piperidinecarboxylate (1.99 g) and β-alanine ethyl ester hydrochloride (1.69 g) in methanol (50 ml), sodium cyanoborohydride (1.2 g) was added in portions under ice-cooling, and the mixture was stirred at room temperature for 16 hours. The reaction mixture was concentrated, made alkaline by addition of sodium bicarbonate water, and extracted with ethyl acetate. The extract was dried over anhydrous sodium sulfate, and the solvent was distilled off to obtain the title compound as colorless oil (2.8 g, 93%).
NMR (CDCl₃) δ: 1.2-1.45 (2H, m), 1.23 (3H, t,J = 7.2), 1.45 (9H, s), 1.80-2.00 (2H, m), 2.40 (1H, br s), 2.58 (2H, t, J = 6.4), 2.60-2.90 (3H, m), 2.98 (2H, t, J = 6.4), 3.95-4.15 (2H, m), 4.16 (2H, q, J = 7.2).

### 41b) Ethyl 3-[N-[3-(6-chloro-2-naphthyl)sulfonylpropionyl]-N-(1-tert-butoxycarbonyl-4-piperidyl)amino]propionate

A solution of 3-(6-chloro-2-naphthyl)sulfonylpropionic acid (0.15 g) obtained in Example 27b) and ethyl 3-(1-tert-butoxycarbonyl-4-piperidyl)aminopropionate (0.2 g) in THF (10 ml) was stirred for 5 minutes, 4-(4,6-dimethoxy-1,3,5-triazin-2-yl)-4-methylmorpholinium chloride (DMTMM: Kunishima, M. et al., Tetrahedron, 1999, 55, 13159) (0.15 g) was added, and the mixture was stirred at room temperature for 3 hours. The reaction mixture was concentrated, extracted with ethyl acetate, and washed with dilute potassium hydrogensulfate, sodium bicarbonate water and saturated brine. The extract was dried over anhydrous sodium sulfate, concentrated, and the residue was purified by silica gel column to obtain the title compound as a colorless powder (0.28 g, 96%).
NMR (CDCl₃) δ: 1.15-1.35 (5H, m), 1.45 (4.5H, s), 1.48 (4.5H, s), 1.50-1.80 (2H, m), 2.35-3.05 (6H, m), 3.35-3.80 (5H, m), 4.00-4.40 (4H, m), 7.54-7.68 (1H, m), 7.88-8.00 (4H, m), 8.48 (1H, s).

### 41c) Ethyl 3-[N-[3-(6-chloro-2-naphthyl)sulfonylpropionyl]-N-[1-(4-pyridyl)-4-piperidyl]amino]propionate

A mixture of ethyl 3-[N-[3-(6-chloro-2-naphthyl)-sulfonylpropionyl]-N-(1-tert-butoxycarbonyl-4-piperidyl)amino]propionate (1.57 g) obtained in Example 41a), toluene (2 ml) and trifluoroacetic acid (4 ml) was stirred at room temperature for 1 hour. Toluene was added to the reaction mixture, and concentration to dryness was performed twice. Isopropyl alcohol (30 ml), 4-bromopyridine hydrochloride (0.58 g) and diisopropylethylamine (7.8 g) were added to the residue, and the mixture was heated to reflux for 48 hours. The reaction mixture was concentrated to dryness, made alkaline with an aqueous sodium carbonate solution, and extracted with methylene chloride, and the extract was dried over anhydrous sodium sulfate, concentrated, and the residue was purified by basic silica gel column to obtain the title compound as a colorless powder (0.44 g, 29%).
NMR (CDCl₃) δ: 1.22 and 1.28 (3H, each t, J = 7.0), 1.50-2.00 (5H, m), 2.35-2.60 (2H, m), 2.72-3.08 (4H, m), 3.32-3.68 (4H, m), 3.70-4.20 (4H, m), 6.58-6.74 (2H, m), 7.61 (1H, dd, J = 2.2 and 8.8), 7.80-8.05 (4H, m), 8.20-8.40 (2H, m), 8.49 (1H, s).

### Example 42

### 4-(6-Chloro-2-naphthyl)sulfonyl-N-methyl-N-[1-(2-methyl-4-pyridyl)-4-piperidinyl]propanamide

### 42a) 1-(2-Methyl-4-pyridyl)-4-piperidone

A solution of 4-piperidone hydrochloride monohydrate (1.53 g) and 4-chloro-2-methylpyridine (1.27 g) in acetic acid (5 ml) was refluxed for 22 hours. The reaction mixture was concentrated under reduced pressure, water was added to the residue, the solution was made alkaline with potassium carbonate, and extracted with ethyl acetate. The extract was dried over anhydrous magnesium sulfate, the solvent was distilled off, and the residue was purified by silica gel column to obtain the title compound as a yellow solid (0.89 g, 47%).
NMR (CDCl₃) δ: 2.49 (3H, s, Me), 2.56 (4H, t, J = 6.3), 3.74 (4H, t, J = 6.3), 6.54-6.61 (2H, m), 8.23 (1H, d, J = 5.8).

### 42b) 4-Methylamine-1-(2-methyl-4-pyridyl)piperidine

A solution of sodium cyanoborohydride (0.47 g) in methanol (5 ml) was added dropwise to a solution of the 1-(2-methyl-4-pyridyl)-4-piperidone (0.96 g) obtained in Example 42a), a 40% aqueous methylamine solution (1.6 g) and acetic acid (0.86 ml) in methanol (10 ml) under ice-cooling. The reaction mixture was stirred at 0°C for 1.5 hours, sodium cyanoborohydrate (0.47 g) was additionally added, and the mixture was stirred at room temperature for 1.5 days. The reaction mixture was concentrated under reduced pressure, a small amount of water was added to the residue, and the solution was made alkaline with potassium carbonate, and extracted with THF. The extract was dried over anhydrous magnesium sulfate, the solvent was distilled off, and the residue was purified by silica gel column to obtain the title compound as pale yellow oil (1.0 g, 97%).
NMR (CDCl₃) δ: 1.30-1.48 (2H, m), 1.92-2.02 (2H, m), 2.44 (3H, s), 2.47 (3H, s), 2.54-2.70 (1H, m), 2.84-2.98 (2H, m), 6.49-6.54 (2H, m), 8.14 (1H, d, J = 6.0).

### 42c) 4-(6-Chloro-2-naphthyl)sulfonyl-N-methyl-N-[1-(2-methyl-4-pyridyl)-4-piperidinyl]propanamide

A solution of 3-(6-chloro-2-naphthyl)sulfonylpropionic acid (0.45 g) obtained in Example 27b), 4-methylamine-1-(2-methyl-4-pyridyl)piperidine (0.41 g) obtained in Example 42b) and DMTMM (0.56 g) in THF (50 ml) was stirred at room temperature for 16 hours. The reaction mixture was concentrated to dryness, made alkaline with an aqueous sodium carbonate solution, extracted with methylene chloride, and the extract was dried over anhydrous sodium sulfate. The solvent was distilled off, and the residue was purified by basic silica gel column to obtain the title compound as a colorless powder (0.30 g, 38%).
NMR (CDCl₃) δ: 1.52-1.95 (4H, m), 2.50 (3H, s), 2.75-3.15 (4H, m), 2.84 (3H, s), 3.50-3.65 (2H, m), 4.45-4.80 (1H, m), 6.50-6.65 (2H, m), 7.60 (1H, dd, J = 2.2 and 8.8), 7.90-8.00 (4H, m), 8.20 (1H, d, J = 6.4), 8.45 (1H, s).

| Elemental Analysis: C₂₅H₂₈ClN₃O₃S·1.75H₂O | | | |
|---|---|---|---|
| Calcd (%) | C, 58.02; | H, 6.13; | N, 8.12 |
| Found (%) | C, 57.77; | H, 5.96; | N, 8.08 |

### Example 43

### 2-(6-Chloro-3-naphthyl)sulfonyl-N'-[1-(4-pyridyl)-4-piperidinyl]acetphydrazide

### 43a) tert-Rutyl 2-[(6-chloro-2-naphthyl)sulfonylacetyl]-carbazimate

According to the same manner as that of Example 42b), the title compound was obtained as colorless crystals (82%) from 2-(6-chloro-2-naphtyl)sulfonylacetic acid obtained in Example 35c) and tert-butyl carbazimate.
NMR (CDCl₃+DMSO-d₆) δ: 1.46 (9H, s), 4.19 (2H, s), 7.07 (1H, bs), 7.56 (1H, dd, J = 1.4 and 8.8), 7.80-8.05 (4H, m), 8.62 (1H, s), 9.65 (1H, bs).

### 43b) [2-(6-Chloro-2-naphthyl)sulfonylaceto]hydrazide

A mixture of tert-butyl 2-[(6-chloro-2-naphthyl)-sulfonylacetyl]carbazimate (0.6 g), toluene (1 ml) and trifuloroacetic acid (1 ml) was stirred at room temperature for 1 hour. The reaction mixture was concentrated to dryness, water was added, pH was adjusted to 8 with 1 N sodium hydroxide, the precipitated precipitates were filtered, washed with water, and dried to obtain the title compound (0.41 g, 93%).
NMR (DMSO-d₆) δ: 3.34 (2H, bs), 4.30 (2H, s), 7.72 (1H, dd, J = 2.2 and 8.8), 7.95 (1H, dd, J = 2.0 and 8.8), 8.10-8.35 (3H, m), 8.58 (1H, s), 9.38 (1H, bs).

### 43c) 2-(6-Chloro-2-naphthyl)sulfonyl-N'-[1-(4-pyridyl)-4 -piperidyl]acetohydrazide

A solution of [2-(6-chloro-2-naphthyl)sulfonylaceto]-hydrazide obtained in Example 43b) and 1-(4-pyridyl)-4-piperidone (0.10 g) in ethanol (10 ml) was heated to reflux for 8 hours, After cooling, methanol (10 ml) and acetic acid (0.2 g) were added thereto. Sodium cyanoborohydride (0.2 g) was added under ice-cooling, and the mixture was further stirred at room temperature for 16 hours. The reaction mixture was concentrated, water was added to the residue, and pH was adjusted to 2 with 1 N hydrochloric acid. The precipitates were filtered, and purified by CHP-20 column to obtain the title compound as a colorless powder (0.19g).
NMR (DMSO-d₆) δ: 1.20-1.55 (2H, m), 1.70-1.90 (2H, m), 3.00-3.30 (3H, m), 3.90-4.20 (2H, m), 4.50 (2H, s), 7.18 (2H, d, J = 7.4), 7.74 (1H, dd, J = 2.2 and 8.8), 7.98 (1H, dd, J = 1.8 and 8.4), 8.10-8.35 (5H, m), 8.61 (1H, s).

| Elemental Analysis: C₂₂H₂₃ClN₄O₃S·HCl·1.75H₂O | | | |
|---|---|---|---|
| Calcd (%) | C, 50.14; | H, 5.26; | N, 10.63 |
| Found (%) | C, 50.10; | H, 5.25; | N, 10.58 |

### Example 44

### 2-[(6-Chloro-2-naphthyl)sulfonyl]-N'-methyl-N'-[1-(4-pyridyl)-4-piperidyl]acetohydrazide

According to the same manner as that of Example 29, the title compound was obtained as colorless powder (36%) from 2-(6-chloro-2-naphthyl)sulfonyl-N'-[1-(4-pyridyl)-4-piperidyl]acetohydrazide obtained in Example 43c).
NMR (CDCl₃) δ: 1.30-2.05 (4H, m), 2.61 and 2.69 (total 3H, each s), 2.70-3.00 (3H, m), 3.75-4.00 (2H, m), 4.11 (1H, s), 4.32 (0.5H, d, J = 14), 4.75 (0.5H, d, J = 14), 6.60-6.72 (2H, m), 7.44 (1H, dd, J = 1.8 and 8.8), 7.80-8.05 (4H, m), 8.25 (1H, bs), 8.48 and 8.51 (total 1H, each s).

| Elemental Analysis: C₂₃H₂₅ClN₄O₃S·0.75H₂O | | | |
|---|---|---|---|
| Calcd (%) | C, 56.78; | H, 5.49; | N, 11.52 |
| Found (%) | C, 56.91; | H, 5.43; | N, 11.74 |

### Example 45

### Ethyl 4-[N-[3-(6-chloro-2-naphthyl)sulfonylpropionyl]-N-[1-(4-pyridyl)-4-piperidyl]amino]acetate

### 45a) Ethyl 4-[1-(4-pyridyl)-4-piperidyl]amino]butyrate

According to the same manner as that of Example 30a), the title compound was obtained as pale yellow oil (85%) from ethyl 1-(4-pyridyl)-4-piperidone and ethyl 4-aminobutyrate hydrochloride.
NMR (CDCl₃) δ: 1.13 (3H, t, J = 7.2), 1.15-1.50 (2H, m), 1.60-1.90 (4H, m), 2.25 (2H, t, J = 7.3), 2.45-2.90 (5H, m), 3.60-3.80 (2H, m), 4.01 (2H, q, J = 7.2), 6.44-6.60 (2H, m), 8.05-8.20 (2H, m).

### 45b) Ethyl 4-[N-[3-(6-chloro-2-naphthyl)sulfonylpropionyl]-N-[1-(4-pyridyl)-4-piperidyl]amino]butyrate

According to the same manner as that of Example 42b), the title compound was obtained as a colorless powder (13%) from 3-(6-chloro-2-naphthyl)sulfonylpropionic acid obtained in Example 27b) and ethyl 4-[1-(4-pyridyl)-4-piperidyl]aminobutyrate obtained in Example 45a).
NMR (CDCl₃) δ: 1.26 (1.5H, t, J = 7.0), 1.27 (1.5H, t, J = 7.0), 1.55-2.00 (4H, m), 2.20-2.40 (2H, m), 2.75-3.05 (4H, m), 3.07-3.30 (2H, m), 3.50-3.68 (2H, m), 3.70-4.20 (6H, m), 4.22-4.50 (1H, m), 6.60-6.75 (2H, m), 7.59 (1H, dd, J = 1.8 and 8.8), 7.85-8.05 (4H, m), 8.20-8.35 (2H, m), 8.50 (1H, s).

| Elemental Analysis: C₂₉H₃₄ClN₃O₅S·0.5H₂O | | | |
|---|---|---|---|
| Calcd (%) | C, 59.94; | H, 6.07; | N, 7.23 |
| Found (%) | C, 59.96; | H, 6.12; | N, 7.47 |

### Example 46

### Benzyl 2-[N-[3-(6-chloro-2-naphthyl)sulfonylpropionyl]-N-[1-(4-pyridyl)-4-piperidyl]amino]ethylcarbamate

### 46a) Benzyl 2-[1-(4-pyridyl)-4-piperidyl]aminoethylcarbamate

According to the same manner as that of Example 30a), the title compound was obtained as colorless oil (91%) from 1-(4-pyridyl)-4-piperidone and benzyl 2-aminoethylcarbamate hydrochloride.
NMR (CDCl₃) δ: 1.20-1.60 (2H, m), 1.85-2.00 (2H, m), 2.60-3.00 (5H, m), 3.29 (2H, q, J = 5.4), 3.70-3.90 (2H, m), 5.11 (2H, s), 5.20 (1H, bs), 6.65 (2H, d, J = 5.2), 7.30-7.40 (5H, m), 8.24 (2H, d, J = 5.2).

### 46b) Benzyl 2-[N-[3-(6-chloro-2-naphthyl)sulfonylpropionyl]-N-[1-(4-pyridyl)-4-piperidyl]amino]ethylcarbamate

According to the same manner as that of Example 42b), the title compound was obtained as a colorless powder (9%) from 3-(6-chloro-2-naphthyl)sulfonylpropionic acid obtained in Example 27b) and benzyl 2-[1-(4-pyridyl)-4-piperidyl]aminoethylcarbamate obtained in Example 46a).
NMR (CDCl₃) δ: 1.40-2.00 (4H, m), 2.70-3.10 (4H, m), 3.10-3.65 (6H, m), 3.70-4.70 (3H, m), 5.06 and 5.10 (total 2H, each s), 5.20-5.40 (1H, m), 6.50-6.75 (2H, m), 7.20-7.40 (5H, m), 7.50-7.65 (1H, m), 7.85-8.00 (4H, m), 8.20-8.36 (2H, m), 8.48 (1H, s).

| Elemental Analysis: C₃₃H₃₅ClN₄O₅S·0.5H₂O | | | |
|---|---|---|---|
| Calcd (%) | C, 61.53; | H, 5.63; | N, 8.70 |
| Found (%) | C, 61.66; | H, 5.64; | N, 9.00 |

### Example 47

### 3-[(4-Biphenyl)sulfonyl]-N-methyl-N-[1-(4-pyridyl)-4-piperidyl]propanamide

### 47a) Methyl 3-(4-bromophenyl)thiopropionate

According to the same manner as that of Example 27a), the tile compound was obtained as colorless prisms (97%) from methyl 4-bromothiphenol and methyl acrylate.
NMR (CDCl₃) δ: 2.62 (2H, t, J = 7.4), 3.15 (2H, t, J = 7.4), 3.69 (3H, s, Me), 7.22 (2H, d, J = 8.5), 7.42 (2H, d, J = 8.5).

### 47b) 3-(4-biphenyl)thiopropionic acid

A mixture of methyl 3-(4-bromophenyl)thiopropionate (2.75g) obtained in Example 47a), phenylboric acid (1.7g), a 2 M aqueous sodium carbonate solution (40 ml) and dimethoxyethane (DME) (20 ml) was refluxed for 30 minutes under the argon atmosphere. The mixture was cooled to room temperature, tetrakis(triphenylphosphono)palladium (0.29 g) was added, and the mixture was refluxed for 2.5 days. The reaction mixture was cooled to room temperature, and made acidic with concentrated hydrochloric acid. The precipitated precipitates were filtered, dissolved in ethyl acetate, purified by silica gel column, and recrystallized from ethyl acetate/hexane to obtain the title compound as pale yellow leaflets (1.64 g, 64%).
NMR (CDCl₃) δ: 2.71 (2H, t, J = 7.3), 3.20 (2H, t, J = 7.3), 7.28-7.60 (9H, m).

### 47c) 3-(4-Biphenyl)sulfonylpropionic acid

A solution of 3-(4-biphenyl)thiopropionic acid (0.52 g) obtained in Example 47b) and 30% hydrogen peroxide in water (0.4 ml) in acetic acid (5 ml) was refluxed for 1 hour, water was added, and the precipitated precipitates were filtered. Recrystallization from ethyl acetate/hexane gave the title compound (0.31 g, 53%).
NMR (CDCl₃) δ: 2.08 (2H, t, J = 7.6), 3.43 (2H, t, J = 7.6), 7.46-7.54 (3H, m), 7.59-7.64 (2H, m), 7.78 (2H, d, J = 8.4), 7.97 (2H, d, J = 8.4).

### 47d) 3-[(4-Biphenyl)sulfonyl]-N-methyl-N-[1-(4-pyridyl)-4-piperidyl]propanamide

According to the same manner as that of Example 30b), the title compound was obtained as a colorless solid (33%) from 3-(4-biphenyl)sulfonylpropionic acid obtained in Example 47c) and 4-methylamino-1-(4-pyridyl)piperidine obtained in Example 30a).
NMR (CDCl₃) δ: 1.61-1.92 (4H, m), 2.74-3.03 (7H, m), 3.54 (2H, t, J = 7.7), 3.85-4.05 (2H, m), 4.57-4.72 (1H, m), 6.64 (2H, d, J = 6.5), 7.44-7.55 (3H, m), 7.58-7.64 (2H, m), 7.79 (2H, d, J = 8.4), 8.00 (2H, d, J = 8.4), 8.26 (2H, d, J = 6.5).

| Elemental Analysis: C₂₆H₂₉N₃O₃S·0.3H₂O | | | |
|---|---|---|---|
| Calcd (%) | C, 66.58; | H, 6.36; | N, 8.96 |
| Found (%) | C, 66.65; | H, 6.14; | N, 8.99 |

### Example 48

### 3-(5-Benzofuranyl)sulfonyl-N-methyl-N-[1-(4-pyridyl)-4-piperidinyl]propanamide

### 48a) 5-Hydroxybenzofuran

A mixture of 5-methoxybenzofuran (Barker P. et al., Synthetic Communications, 1980, *19*, 257) (3.95 g) and pyridine hydrochloride (8.7 g) was stirred at 180°C for 6.5 hours. The reaction mixture was diluted with water, made acidic with 1 N hydrochloric acid, and extracted with ethyl acetate. The extract was washed with water, dried over anhydrous magnesium sulfate and the solvent was distilled off under reduced pressure. The residue was purified by silica gel column to obtain the title compound as a pale yellow solid (2.93 g, 87%).
NMR (CDCl₃) δ: 4.88 (1H, s, OH), 6.67 (1H, dd, J = 2.2 and 0.7), 6.81 (1H, dd, J = 8.8 and 2.5), 7.01 (1H, d, J = 2.5), 7.35 (1H, dd, J = 8.8 and 0.7), 7.59 (1H, d, J = 2.2).

### 48b) 5-(N,N-Dimethylthiocarbamoyl)oxybenzofuran

5-Hydroxybenzofuran (2.93 g) obtained in Example 48a), dimethylthiocarbamoyl chloride (5.4 g) and 1,4-diazabicyclo[2.2.2]octane (4.9 g) were added to DMF (6 ml), and the mixture was stirred at room temperature for 3 hours. The reaction mixture was diluted with water, made acidic with 1 N hydrochloric acid, and extracted with ethyl acetate. The extract was washed with water, and dried over anhydrous magnesium sulfate. The solvent was distilled off, and the residue was purified by silica gel column to obtain the title compound as pale yellow oil (4.8 g, quantitative).
NMR (CDCl₃) δ: 3.38 (3H, s, Me), 3.48 (3H, s, Me), 6.76 (1H, dd, J = 2.2 and 0.8), 7.00 (1H, dd, J = 8.8 and 2.6), 7.27 (1H, d, J = 2.6), 7.49 (1H, d, J = 8.8), 7.64 (1H, d, J = 2.2).

### 48c) 5-(N,N-Dimethylcarbamoyl)thiobenzofuran

5-(N,N-Dimethylthiocarbamoyl)oxybenzofuran (4.4 g) obtained in Example 48b) was stirred at 250 to 260°C for 8 hours under the argon atmosphere, and purified by silica gel column to obtain the title compound as pale orange prisms (1.7 g, 39%).
NMR (CDCl₃) δ: 3.07 (6H, m, Me₂N), 6.76 (1H, dd, J = 2.2 and 1.4), 7.40 (1H, dd, J = 8.5 and 1.8), 7.52 (1H, d, J = 8.5), 7.64 (1H, d, J = 2.2), 7.76 (1H, d, J = 1.8).

### 48d) 5-Mercaptobenzofuran

A solution of 5-(N,N-dimethylcarbamoyl)thiobenzofuran (1.7 g) obtained in Example 48c) and potassium hydroxide (3.3 g) in methanol (33 ml) was refluxed for 3.5 hours. The reaction mixture was cooled to room temperature, made acidic with concentrated hydrochloric acid, and water was added, and the solution was extracted with ethyl acetate. The extract was washed with water, and dried over anhydrous magnesium sulfate. The solvent was distilled off, and the residue was purified by silica gel column to obtain the title compound as orange oil (1.12 g, 97%).
NMR (CDCl₃) δ: 3.54 (1H, s, SH), 6.69 (1H, dd, J = 2.2 and 1.2), 7.25 (1H, dd, J = 8.4 and 1.8), 7.39 (1H, d, J = 8.4), 7.58 (1H, d, J = 1.8), 7.61 (1H, d, J = 2.2).

### 48e) Methyl3-(5-benzofuranyl)thiopropioate

According to the same manner as that of Example 27a), the title compound was obtained as pale yellow oil (85%) from methyl 5-mercaptobenzofuran obtained in Example 48d) and metyl acrylate.
NMR (CDCl₃) δ: 2.60 (2H, t, J = 7.3), 3.14 (2H, t, J = 7.3), 3.67 (3H, s, Me), 6.74 (1H, dd, J = 2.2 and 0.6), 7.37 (1H, dd, J = 8.8 and 1.8), 7.45 (1H, d, J = 8.8), 7.63 (1H, d, J = 2.2), 7.70 (1H, d, J = 1.8).

### 48f) Methyl 3-(5-benzofuranyl)solfonylpropioate

According to the same manner as that of Example 24b), the title compound was obtained as pale yellow oil (91%) from methyl 3-(5-benzofuranyl)thiopropioate obtained in Example 48e).
NMR (CDCl₃) δ: 2.78 (2H, t, J = 7.7), 3.47 (2H, t, J = 7.7), 3.62 (3H, s, Me), 6.92 (1H, dd, J = 2.4 and 0.8), 7.68 (1H, d, J = 8.5), 7.80 (1H, d, J = 2.4), 7.86 (1H, dd, J = 2.0 and 8.5), 8.23 (1H, d, J = 2.0).

### 48g) 3-(5-Benzofuranyl)sulfonylpropionic acid

According to the same manner as that of Example 40c), the title compound was obtained from methyl 3-(5-benzofuranyl)sulfonylpropioate (76%) obtained in Example 48f).
NMR (CDCl₃) δ: 2.80 (2H, t, J = 7.5), 3.45 (2H, t, J = 7.5), 6.92 (1H, dd, J = 1.2 and 8.6), 7.68 (1H, d, J = 8.6), 7.79 (1H, d, J = 2.2), 7.86 (1H, dd, J = 2.0 and 8.6), 8.23 (1H, d, J = 2.0).

### 48h) 3-(1-Benzofuran-5-yl)sulfonyl-N-methyl-N-[1-(4-pyridyl)-4-piperidinyl]propanamide

According to the same manner as that of Example 30b), the title compound was obtained as colorless prisms (36%) from 3-(5-benzofuranyl)sulfonylpropionic acid obtained in Example 48f) and 4-methylamino-1-(4-pyridyl)piperidine obtained in Example 30a).
NMR (CDCl₃) δ: 1.58-1.89 (4H, m), 2.77-3.03 (7H, m), 3.53 (2H, t, J = 7.8), 3.91-3.99 (2H, m), 4.52-4.72 (1H, m), 6.65 (2H, d, J = 6.6), 6.92 (1H, d, J = 3.2), 7.68 (1H, d, J = 8.7), 7.80 (1H, d, J = 2.0), 7.88 (1H, dd, J = 8.7 and 2.0), 8.24-8.32 (3H, m).

| Elemental Analysis: C₂₂H₂₅N₃O₄S·0.25 hexane | | | |
|---|---|---|---|
| Calcd (%) | C, 62.85; | H, 6.40; | N, 9.36 |
| Found (%) | C, 62.64; | H, 6.56; | N, 9.05 |

### Example 49

### 3-(5-Benzofuranyl)sulfonyl-N-methyl-N-[1-(2-methyl-4-pyridyl)-4-piperidyl]propanamide

According to the same manner as that of Example 30b), the title compound was obtained as a colorless powder (21%) from 3-(5-benofuranyl)sulfonylpropionic acid obtained in Example 48f) and 4-methylamino-1-(2-methyl-4-pyridyl)piperidine obtained in Example 42b).
NMR (CDCl₃) δ: 1.59-1.78 (4H, m), 2.44 (2.3H, s), 2.47 (0.7H, s), 2.74-2.98 (7H, m), 3.53 (2H, t, J = 7.9), 3.91-4.03 (2H, m), 4.62 (1H, m), 6.49-6.56 (2H, m), 6.92 (1H, d, J = 1.7), 7.68 (1H, d, J = 8.4), 7.80-7.90 (2H, m), 8.16 (1H, d, J = 5.8), 8.24 (1H, d, J = 1.7).

| Elemental Analysis: C₂₃H₂₇N₃O₄S·0.3H₂O | | | |
|---|---|---|---|
| Calcd (%) | C, 61.81; | H, 6.22; | N, 9.40 |
| Found (%) | C, 61.69; | H, 6.47; | N, 9.43 |

### Example 50

### 4-(6-Chloro-2-naphthyl)sulfonyl-N-methyl-N-[1-(2-methyl-4-pyridyl)-4-piperidyl]propanamide hydrochloride

WSC (2.1 g) was added to a solution of 3-(6-chloro-2-naphthyl)sulfonylpropionic acid (2.2 g) obtained in Example 27b) in DMF (10 ml) under ice-cooling, the mixture was stirred at 0°C for 30 minutes, a solution of 4-methylamine-1-(2-methyl-4-pyridyl)piperidine (1.52 g) obtained in Example 42b) in DMF (5 ml) was added, and the mixture was stirred at room temperature for 18 hours. The solvent was distilled off under reduced pressure, a small amount of water and potassium carbonate were added to the residue to make alkaline, the mixture was extracted with ethyl acetate, and the extract was dried over anhydrous magnesium sulfate. The solvent was distilled off, and the residue was purified by basic silica gel column. The product was dissolved in methanol, a 4 N hydrogen chloride solution in ethyl acetate (4 ml) was added, the mixture was stirred, and the solvent was distilled off to obtain the title compound as a colorless powder (1.86 g, 46%).
NMR (DMSO-d₆) δ: 1.45-1.80 (4H, m), 2.45 (3H, Me), 2.64-2.73 (3.5H, m), 2.86-2.94 (1H, m), 3.05-3.40 (3H, m), 3.59-3.69 (2H, m), 4.20-4.54 (2.5H, m), 7.03-7.13 (2H, m), 7.74 (1H, dd, J = 2.2 and 8.6), 7.97-8.03 (1H, m), 8.08-8.27 (4H, m), 8.66 (1H, s).

| Elemental Analysis: C₂₅H₂₈N₃O₃SCl·HCl·0.5H₂O·0.2EtOAc | | | |
|---|---|---|---|
| Calcd (%) | C, 56.43; | H, 5.80; | N, 7.65 |
| Found (%) | C, 56.45; | H, 5.77; | N, 7.75 |

### Example 51

### tert-Butyl 2-[N-[3-(6-chloro-2-naphthyl)sulfonylpropionyl]-N-[1-(2-methyl-4-pyridyl)-4-piperidyl]amino]ethylcarbamate

### 51a) tert-Butyl 2-[1-(2-methyl-4-pyridyl)-4-piperidyl]aminoethylcarbamate

According to the same manner as that of Example 30a), the title compound was obtained as yellow oil (98%) from 1-(2-methyl-4-pyridyl)-4-piperidone obtained in Example 42a) and tert-butyl 1-aminoethylcarbamate.
NMR (CDCl₃) δ: 1.27-1.41 (2H, m), 1.45 (9H, s), 1.92-1.97 (2H, m), 2.44 (3H, s), 2.63-2.97 (5H, m), 3.18-3.26 (2H, m), 3.48 (1H, m), 3.79-3.86 (2H, m), 4.93 (1H, br s), 6.43-6.55 (2H, m), 8.14 (1H, d, J = 5.8).

### 51b) tert-Butyl 2-[N-[3-(6-chloro-2-naphthyl)sulfonylpropionyl]-N-[1-(2-methyl-4-pyridyl)-4-piperidyl]amino]ethylcarbamate

According to the same manner as that of Example 30b), the title compound was obtained as a colorless powder (16%) from 3-(6-chloro-2-naphthyl)sulfonylpropionic acid obtained in Example 27b) and tert-butyl 2-[1-(2-methyl-4-pyridyl)-4-piperidyl]amino]ethylcarbamate obtained in Example 30b).
NMR (CDCl₃) δ: 1.40 and 1.45 (9H, each s), 1.81 (3H, br s), 2.44 and 2.47 (3H, each s), 2.84-3.05 (4H, m), 3.17-3.28 (4H, m), 3.54-3.64 (2H, m), 3.87-4.38 (3H, m), 4.75-4.90 (1H, m), 6.47-6.55 (2H, m), 7.58-7.64 (1H, m), 7.95-7.98 (4H, m), 8.15-8.22 (1H, m), 8.50 (1H, s).

| Elemental Analysis: C₃₁H₃₉ClN₄O₅S·H₂O | | | |
|---|---|---|---|
| Calcd (%) | C, 58.80; | H, 6.53; | N, 8.85 |
| Found (%) | C, 59.03; | H, 6.31; | N, 8.72 |

### Example 52

### N-(2-Acetylaminoethyl)-3-(6-chloro-2-naphthyl)sulfonyl-N-[1-(2-methyl-4-pyridyl)-4-piperidyl]propanamide

A mixture of tert-butyl 2-[N-[3-(6-chloro-2-naphthyl)sulfonylpropionyl]-N-[1-(2-methyl-4-pyridyl)-4-piperidyl]amino]ethylcarbamate (0.13 g) obtained in Example 41b), trifluoroacetic acid (4 ml) and toluene (5 ml) was stirred at room temperature for 2 hours, and the reaction mixture was concentrated under reduced pressure. The residue was dissolved in methylene chloride (10 ml), triethylamine (0.21 g) and acetic anhydride (0.11 g) were added, and the mixture was stirred at room temperature for 3 hours. The reaction mixture was washed with sodium bicarbonate water, and dried over anhydrous sodium sulfate. The solvent was distilled off, and the residue was purified by silica gel column to obtain the title compound as a colorless solid (0.10g, 85%).
NMR (CDCl₃) δ: 1.52-1.85 (4H, m), 1.92 and 2.01 (3H, each s), 2.45 and 2.47 (3H, each s), 2.80-3.06 (4H, m), 3.30-3.34 (4H, m), 3.55-3.62 (2H, m), 3.83-4.03 (3H, m), 6.29 (1H, br s), 6.48-6.56 (2H, m), 7.59-7.64 (1H, m), 7.79-8.00 (4H, m), 8.15-8.22 (1H, m), 8.50 and 8.53 (1H, each s).

| Elemental Analysis: C₂₈H₃₃ClN₄O₄S·0.5H₂O·0.1EtOAc | | | |
|---|---|---|---|
| Calcd (%) | C, 59.33; | H, 6.10; | N, 9.74 |
| Found (%) | C, 59.20; | H, 6.26; | N, 9.51 |

### Example 53

### N-(2-Aminoethyl)-3-(6-chloro-2-naphthyl)sulfonyl-N-[1-(2-methyl-4-pyridyl)-4-piperidyl]propanamide ditrifluoroacetate

### 53a) tert-Butyl 2-[1-(4-pyridyl)-4-piperidyl]-aminoethylcarbamate

According to the same manner as that of Example 30a), the title compound was obtained as a pale yellow oil (93%) from 1-(4-pyridyl)-4-piperidone and tert-butyl 2-aminoethylcarbamate.
NMR (CDCl₃) δ: 1.25-1.50 (2H, m), 1.45 (9H, s), 1.90-2.05 (2H, m), 2.65-3.05 (5H, m), 3.15-3.30 (2H, m), 3.75-3.92 (2H, m), 4.91 (1H, br s), 6.60-6.75 (2H, m), 8.20-8.28 (2H, m).

### 53b) tert-Butyl 2-[N-[3-(6-chloro-2-naphthyl)sulfonylpropionyl]-N-[1-(4-pyridyl)-4-piperidyl]amino]ethylcarbamate

WSC (0.3 g) was added to a solution of 3-(6-chloro-2-naphthyl)sulfonylpropionic acid (0.3 g) obtained in Example 27b) and tert-butyl 2-[1-(4-pyridyl)-4-piperidyl]-aminoethylcarbamate (0.37 g) obtained in Example 53a) in acetonitrile (30 ml), and the mixture was stirred at room temperature for 16 hours. The reaction mixture was concentrated, made alkaline with an aqueous sodium carbonate solution, extracted with ethyl acetate, and the extract was dried over anhydrous magnesium sulfate. The solvent was distilled off, and the residue was purified by basic silica gel column to obtain the title compound (0.34 g, 56%), which was used as it was in the next reaction.

### 53c) N-(2-Aminoethyl)-3-(6-chloro-2-naphthyl)sulfonyl-N-[1-(2-methyl-4-pyridyl)-4-piperidyl]propanamide ditrifluoroacetate

According to the same manner as that of Example 25a), the title compound was obtained as a colorless powder (88%) from tert-butyl 2-[N-[3-(6-chloro-2-naphthyl)-sulfonylpropionyl]-N-[1-(4-pyridyl)-4-piperidyl]amino]-ethylcarbamate.
NMR (CD₃OD) δ: 1.64-2.05 (4H, m), 2.97 (2H, t, J = 6.3), 3.05 (2H, t, J = 7.2), 3.15-3.40 (2H, m), 3.47 (2H, t, J = 6.3), 3.70 (2H, t, J = 7.2), 4.10-4.48 (3H, m), 7.18 (2H, d, J = 7.8), 7.66 (1H, dd, J = 2.2 and 8.8), 7.90-8.20 (6H, m), 8.59 (1H, s).

| Elemental Analysis: C₂₅H₂₉ClN₄O₃S·2CF₃CO₂H·2H₂O | | | |
|---|---|---|---|
| Calcd (%) | C, 45.52; | H, 4.61; | N, 7.32 |
| Found (%) | C, 45.67; | H, 4.60; | N, 7.32 |

### Example 54

### N-(2-Acetylaminoethyl)-3-(6-chloro-2-naphthyl)sulfonyl-N-[1-(4-pyridyl)-4-piperidyl]propanamide

According to the same manner as that of Example 52), the title compound was obtained as a colorless powder (66%) from tert-butyl 2-[N-[3-(6-chloro-2-naphthyl)-sulfonylpropionyl]-N-[1-(2-methyl-4-pyridyl)-4-piperidyl]amino]ethylcarbamate obtained in Example 53b) and acetic anhydride.
NMR (CD₃OD) δ: 1.56-2.00 (4H, m), 1.99 and 2.03 (3H, each s), 2.78-3.10 (4H, m), 3.20-3.40 (4H, m), 3.50-3.70 (2H, m), 3.70-4.50 (3H, m), 6.30-6.50 (1H, m), 6.60-6.75 (2H, m), 7.55-7.65 (1H, m), 7.90-8.00 (4H, m), 8.18-8.38 (2H, m), 8.49 and 8.52 (1H, each s).

| Elemental Analysis: C₂₇H₃₁ClN₄O₄S·0.5H₂O | | | |
|---|---|---|---|
| Calcd (%) | C, 58.74; | H, 5.84; | N, 10.15 |
| Found (%) | C, 59.02; | H, 5.94; | N, 10.26 |

### Example 55

### 3-(6-Chloro-2-naphthyl)sulfonyl-N-(2-methylsulfonylaminoethyl)-N-[1-(4-pyridyl)-4-piperidyl]propanamide

According to the same manner as that of Example 52), the title compound was obtained as a colorless powder (66%) from tert-butyl 2-[N-[3-(6-chloro-2-naphthyl)-sulfonylpropionyl]-N-[1-(2-methyl-4-pyridyl)-4-piperidyl]amino]ethylcarbamate obtained in Example 53b) and methanesulfonyl chloride.
NMR (CD₃OD) δ: 1.60-2.00 (4H, m), 2.80-3.10 (4H, m), 2.90 (3H, s), 3.10-3.65 (6H, m), 3.80-4.50 (3H, m), 5.30-5.70 (1H, m), 6.58-6.72 (2H, m), 7.56-7.68 (1H, m), 7.88-8.05 (4H, m), 8.20-8.35 (2H, m), 8.50 (1H, s).

| Elemental Analysis: C₂₆H₃₁ClN₄O₅S₂·0.5H₂O | | | |
|---|---|---|---|
| Calcd (%) | C, 53.10; | H, 5.48; | N, 9.53 |
| Found (%) | C, 53.14; | H, 5.34; | N, 9.60 |

### Example 56

### 2-[(6-Chloro-2-naphthyl)thio]-N-methyl-N-[1-(4-pyridyl)-4-piperidyl]ethanesulfonamide

### 56a) N-Methyl-N-[1-(4-pyridyl)-4-piperidyl]ethene-sulfonamide

A solution of 2-chloroethanesulfonyl chloride (2.45 g) in methylene chloride (3 ml) was added dropwise to a solution of 4-methylamino-1-(4-pyridyl)piperidine (1.89 g) obtained in Example 30a) and triethylamine (4.04 g) in methylene chloride (40 ml) under ice-cooling. The reaction mixture was stirred at 0°C for 1 hour, diluted with an aqueous sodium bicarbonate solution, and extracted with chloroform. The extract was dried over anhydrous sodium sulfate, the solvent was distilled off, and the residue was purified by silica gel column to obtain the title compound as a colorless solid (1.0 g, 36%).
NMR (CDCl₃) δ: 1.60-1.85 (4H, m), 2.71 (3H, s), 2.92 (2H, m), 3.90-4.05 (3H, m), 5.96 (1H, d, J = 9.6), 6.24 (1H, d, J = 16.4), 6.44 (1H, dd, J = 9.6 and 16.4), 6.65 (2H, d, J = 6.6), 8.28 (2H, d, J = 6.6).

### 56b) 2-[(6-Chloro-2-naphthyl)thiol-N-methyl-N-[1-(4-pyridyl)-4-piperidyl]ethanesulfonamide

A solution of 6-chloro-2-mercaptonaphthlene (0.22 g) obtained in Example 1d) in methylene chloride (3 ml) was added dropwise to a solution of N-methyl-N-[1-(4-piridyl)-4-piperidyl]ethanesulfonamide (0.32 g) obtained in Example 56a) in methylene chloride (10 ml)-methanol (3 ml) under ice-cooling, and the mixture was further stirred at room temperature for 10 hours. The reaction mixture was concentrated, and the residue was purified by silica gel column to obtain the title compound as a colorless solid (0.27g, 51%).
NMR (CDCl₃) δ: 1.60-1.90 (4H, m), 2.74 (3H, s), 2.83 (2H, m), 3.21 (2H, m), 3.36 (2H, m), 3.80-3.95 (3H, m), 6.62 (2H, d, J = 6.6), 7.47 (2H, d, J = 8.8), 7.74 (2H, d, J = 8.8), 7.82 (2H, s), 8.27 (2H, d, J = 6.6).

| Elemental Analysis: C₂₃H₂₆N₃O₂S₂Cl·0.25H₂O | | | |
|---|---|---|---|
| Calcd (%) | C, 57.48; | H, 5.56; | N, 8.74 |
| Found (%) | C, 57.46; | H, 5.71; | N, 8.54 |

### Example 57

### 2-[[(6-Chloro-2-naphthyl)sulfonyl]-N-methyl-N-[1-(4-pyridyl)-4-piperidyl]ethanesulfonamide

According to the same manner as that of Example 2b), the title compound was obtained as a colorless solid (50 mg, 20%) from 2-[(6-chloro-2-naphthyl)thio]-N-methyl-N-[1-(4-pyridyl)-4-piperidyl]ethanesulfonamide (0.24 g) obtained in Example 56b).
NMR (CDCl₃) δ: 1.70-1.90 (4H, m), 2.80 (3H, s), 2.95 (2H, m), 3.42 (2H, m), 3.54 (2H, m), 3.85-4.05 (3H, m), 6.69 (2H, d, J = 6.6), 7.65 (1H, dd, J = 1.8 and 8.8), 7.85-8.05 (4H, m), 8.19 (2H, d, J = 6.6), 8.50 (1H, s).

| Elemental Analysis: C₂₃H₂₆N₃O₄S₂Cl·0.5H₂O | | | |
|---|---|---|---|
| Calcd (%) | C, 53.43; | H, 5.26; | N, 8.13 |
| Found (%) | C, 55.36; | H, 5.51; | N, 8.14 |

### Example 58

### N-Methyl-N-[1-(2-methyl-4-pyridyl)-4-piperidyl]-4- (4-vinylphenyl)sulfonylbutaneamide

### 58a) Ethyl 4-(4-bromophenyl)sulfonylbutyrate

Sodium ethoxide (1.02 g) and ethyl 4-bromobutyrate (2.34 g) were added to a solution of 4-bromophenol (1.80 g) in ethanol (50 ml), and the mixture was stirred at room temperature for 16 hours. The reaction mixture was concentrated to dryness, dilute hydrochloric acid was added to the residue to adjust to pH 1, which was extracted with ethyl acetate. The extract was dried over anhydrous sodium sulfate, and concentrated. Hexane-ethyl acetate was added to the residue, the precipitated insolubles were filtered, and the filtrate was concentrated. The residue was dissolved in ethyl acetate (100 ml), and mCPBA (5 g) was added under water-cooling. The reaction mixture was washed with aqueous saturated sodium bicarbonate twice, dried over anhydrous sodium sulfate, concentrated, ice-cooled hexane was added to the residue to crystallize to obtain the title compound as colorless crystals (2.38 g, 71%).
NMR(CDCl₃) δ: 1.24(3H, t, J = 7.2), 1.92-2.10 (2H, m), 2.46 (2H, t, J = 7.0), 3.12-3.25 (2H, m), 4.12 (2H, q, J = 7.2), 7.70-7.85 (4H, m).

### 58b) Ethyl 4-(4-vinylphenyl)sulfonylbutyrate

Tributyl(vinyl)tin (1.83 ml) was added to a mixture of ethyl 4-(4-bromophenyl)sulfonylbutyrate (1.78 g) obtained in Example 58a), dichlorobis(triphenylphosphine)palladium (II) (0.09 g) and lithium chloride (1.92 g) in DMF (50 ml) under an argon stream. The mixture was stirred at 90°C for 1 hour, the reaction mixture was cooled, poured into ice-water, extracted with ethyl acetate, and washed with saturated brine. The extract was dried over anhydrous sodium sulfate, concentrated, and the residue was purified by silica gel column to obtain the title compound as pale yellow oil (1.34 g, 75%).
NMR(CDCl₃) δ: 1.23 (3H, t, J = 7.1), 1.90-2.10 (2H, m), 2.45 (2H, t, J = 7.0), 3.10-3.25 (2H, m), 4.11 (2H, q, J = 7.1), 5.47 (1H, d, J = 11.0), 5.92 (1H, d, J = 17.6), 6.77 (1H, dd, J = 11.0 and 17.6), 7.43 (2H, d, J = 6.6), 7.87 (2H, d, J = 6.6).

### 58c) 4-(4-Vinylphenyl)sulfonylbutyric acid

According to the same manner as that of Example 12b), the title compound was obtained as colorless crystals (0.93 g, 77%) from ethyl 4-(4-vinylphenyl)sulfonylbutyrate (1.34 g) obtained in Example 58b).
NMR (CDCl₃) δ: 1.92-2.15 (2H, m), 2.53 (2H, t, J = 7.0), 3.10-3.30 (2H, m), 5.48 (1H, d, J = 10.6), 5.92 (1H, d, J = 17.6), 6.77 (1H, dd, J = 10.6 and 17.6), 7.58 (1H, dd, J = 1.8 and 6.6), 7.8 (2H, dd, J = 2.0 and 6.6).

### 58d) N-Methyl-N-[1-(2-methyl-4-pyridyl)-4-piperidyl]-4-(4-vinylphenyl)sulfonylbutaneamide

According to the same manner as that of Example 42c), the title compound was obtained as a colorless powder (26%) from 4-(4-vinylphenyl)sulfonylbutyric acid obtained in Example 58c) and 4-methylamino-1-(2-methyl-4-pyrdyl)piperidine obtained in Example 42c).
NMR (CDCl₃) δ: 1.55-2.26 (6H, m), 2.45 (3H, s), 2.46-2.70 (2H, m), 2.79 (3H, s), 2.80-3.10 (2H, m), 3.25 (2H, t, J = 7.3), 3.75-4.05 (2.3H, m), 4.65-4.80 (0.7H, m), 5.47 (1H, d, J = 11.0), 5.91 (1H, d, J = 17.6), 6.45-6.65 (2H, m), 6.77 (1H, dd, J = 11.0, 17.6), 7.57 (2H, d, J = 8.3), 7.86 (2H, d, J = 8.3), 8.10-8.25 (1H, m).

| Elemental Analysis: C₂₄H₃₁N₃O₃S·1.1H₂O | | | |
|---|---|---|---|
| Calcd (%) | C, 62.47; | H, 7.25; | N, 9.11 |
| Found (%) | C, 62.21; | H, 7.25; | N, 9.15 |

### Example 59

### N-Methyl-N-[1-(4-pyridyl)-4-piperidyl]-4-(4-vinylpheny)sulfonylpropanamide

### 59a) tert-Butyl 3-(4-bromophenyl)thiopropionate

According to the same manner as that of Example 27a), the title compound was obtained as colorless needles (93%) from 4-bromophenol and ethyl 4-bromopropionate.
NMR (CDCl₃) δ: 1.45 (9H, s), 2.52 (2H, t, J = 7.4). 3.11 (2H, d, J = 7.4), 7.10-7.28 (2H, m), 7.35-7.48 (2H, m).

### 59b) tert-Butyl 3-(4-bromophenyl)sulfonylpropionate

According to the same manner as that of Example 24b), the title compound was obtained as colorless crystals (79%) from tert-butyl 3-(4-bromophenyl)thiopropionate obtained in Example 59a).
NMR (CDCl₃) δ: 1.41 (9H, s), 2.65 (2H, t, J = 7.7), 3.86 (2H, t, J = 7.7), 7.70-7.90 (4H, m).

### 59c) tert-Butyl 4-(4-vinylphenyl)sulfonylpropionate

According to the same manner as that of Example 58b), the title compound was obtained as colorless crystals (82%) from tert-butyl 3-(4-bromophenyl)sulfonylpropionate obtained in Example 59b).
NMR (CDCl₃) δ: 1.40 (9H, s), 2.65 (2H, t, J = 7.9). 3.39 (2H, t, J = 7.9), 5.48 (1H, d, J = 11.0), 5.92 (1H, d, J = 17.6), 6.77 (1H, dd, J = 11.0 and 17.6), 7.58 (2H, d, J = 8.8), 7.86 (2H, d, J = 8.8).

### 59d) 4-(4-Vinylphenyl)sulfonylpropionic acid

tert-Butyl 4-(4-vinylphenyl)sulfonylpropionate (1.45 g) obtained in Example 59c) was dissolved in trifluoroacetic acid (4 ml), and the mixture was stirred at room temperature for 2 hours. Toluene was added, concentrated to dryness, and toluene was added again to crystallize to obtain the title compound as colorless crystals (1.15 g, 97%).
NMR (CDCl₃) δ: 2.80 (2H, t, J = 7.7), 3.41 (2H, t, J = 7.7), 5.48 (1H, d, J = 11.0), 5.92 (1H, d, J = 17.6), 6.77 (2H, dd, J = 11.0 and 17.6), 7.58 (2H, d, J = 8.4), 7.86 (2H, d, J = 8.4), 9.34 (1H, br s).

### 59e) N-Methyl-N-[1-(4-pyridyl)-4-piperidyl]-4-(4-vinylphenyl)sulfonylpropanamide

According to the same manner as that of Example 42c), the title compound was obtained as a colorless powder (36%) from 4-(4-vinylphenyl)sulfonylpropionic acid obtained in Example 59d) and 4-methylamino-1-(4-pyridyl)piperidine obtained in Example 30a).
NMR (CDCl₃) δ: 1.57-2.00 (4H, m), 2.70-3.06 (4H, m), 2.83 (3H, s), 3.49 (2H, t, J = 7.7), 3.80-4.10 (2H, m), 4.50-4.80 (1H, m), 5.49 (1H, d, J = 11.0), 5.93 (1H, d, J = 17.6), 6.60-6.72 (2H, m), 6.78 (1H, dd, J = 11.0 and 17.6), 7.59 (2H, d, J = 8.0), 7.88 (2H, d, J = 8.0), 8.20-8.37 (2H, m).

| Elemental Analysis: C₂₂H₂₇N₃O₃S·0.5H₂O | | | |
|---|---|---|---|
| Calcd (%) | C, 62.53; | H, 6.68; | N, 9.94 |
| Found (%) | C, 62.68; | H, 6.71; | N, 10.54 |

### Example 60

### N-Methyl-N-[1-(2-methyl-4-pyridyl-4-piperidyl)-4-(4-vinylphenyl)sulfonylpropanamide

According to the same manner as that of Example 42c), the title compound was obtained as a colorless powder (31%) from 4-(4-vinylphenyl)sulfonylpropionic acid obtained in Example 59d) and 4-methylamino-1-(2-methyl-4-pyridyl)piperidine obtained in Example 42b).
NMR (CDCl₃) δ: 1.55-2.00 (4H,m), 2.45 (3H, s), 2.70-3.05 (4H, m), 2.83 (3H, s), 3.40-3.56 (2H, m), 3.80-4.10 (2H, m), 4.50-4.76 (1H, m), 5.49 (1H, d, J = 11.0), 5.93 (1H, d, J = 17.6), 6.44-6.60 (2H, m), 6.78 (1H, dd, J = 11.0 and 17.6), 7.59 (2H, J = 8.8), 7.88 (2H, d, J = 8.8), 8.16 (1H, d, J = 6.0).

| Elemental Analysis: C₂₃H₂₉N₃O₃S·H₂O | | | |
|---|---|---|---|
| Calcd (%) | C, 62.00; | H, 7.01; | N, 9.43 |
| Found (%) | C, 62.19; | H, 6.95; | N, 9.59 |

### Example 61

### 3-[N-[3-(6-Chloro-2-naphthyl)sulfonylpropionyl]-N-[1-(4-pyridyl)-4-piperidyl]amino]propionic acid

### 61a) Benzyl 3-(1-tert-butoxycarbonyl-4-piperidyl)aminopropionate

According to the same manner as that of Example 30a), the title compound was obtained as colorless oil (88%) from tert-butyl 4-oxo-1-piperidinecarboxylate and benzyl 3-aminopropionate tuluenesulfonate.
NMR (CDCl₃) δ: 1.10-1.30 (2H, m), 1.45 (9H, s), 1.72-1.90 (2H, m), 2.50-2.90 (3H, m), 2.57 (2H, t, J = 6.4), 2.94 (2H, t, J = 6.4), 3.90-4.10 (2H, m), 5.14 (2H, s), 7.35 (5H, s).

### 61b) Benzyl 3- [N- (1-tert-butoxycarbonyl-4-piperidyl)-N-[3-(6-chloro-2-naphthyl)sulfonylpropionyl]amino]propionate

According to the same manner as that of Example 42b), the title compound was obtained as colorless oil (71%) from benzyl 3-(1-tert-butoxycarbonyl-4-piperidyl)aminopropionate obtained in Example 61a) and 3-(6-chloro-2-naphthyl)-sulfonylpropionic acid obtained in Example 27b).
NMR (CDCl₃) δ: 1.45 (4.5H, s), 1.48 (4.5H, s), 1.50-2.10 (4H, m), 2.40-3.00 (6H, m), 3.35-4.35 (7H, m), 5.08 (1H, s), 5.14 (1H, s), 7.28-7.42 (5H, m), 7.52-7.62 (1H, m), 7.85-8.00 (4H, m), 8.47 (1H, s).

### 61c) Benzyl 3-[N-[3-(6-chloro-2-naphthyl)sulfonylpropionyl]-N-[1-(4-pyridyl)-4-piperidyl]amino]propionate

According to the same manner as that of Example 41c), the title compound was obtained as a colorless powder (33%) from benzyl 3-[N-(1-tert-butoxycarbonyl-4-piperidinyl)-N-[3-(6-chloro-2-naphthyl)sulfonylpropionyl]amino]propionate obtained in Example 61b) and 4-bromopyridine hydrochloride.

### 61d) Benzyl 3-[N-(1-tert-butoxycarbonyl-4-piperidinyl)-N-[3-(6-chloro-2-naphthyl)sulfonylpropionyl]amino]propionate

A solution of benzyl 3-[N-[3-(6-chloro-2-naphthyl)sulfonylpropionyl]-N-[1-(4-pyridyl)-4-piperidyl]amino]propionate obtained in Example 61c) in 25% hydrogen bromide acetic acid solution (2 ml) was stirred at room temperature for 2 hours. The reaction mixture was concentrated to dryness, water was added to the residue, the mixture was washed with ether, the aqueous layer was concentrated, and the residue was purified by CHP-20 column to obtain the title compound as a colorless powder (0.09 g, 88%).
NMR (DMSO-d₆) δ: 1.20-1.76 (4H, m), 2.00-2.20 (1H, m), 2.23-2.55 (1H, m), 2.60-3.00 (4H, m), 3.05-3.22 (1H, m), 3.30-3.45 (1H, m), 3.55-3.75 (2H, m), 3.78-4.10(3H, m), 6.73 (1H, d, J = 6.2), 6.81 (1H, d, J = 6.6), 7.64-7.78 (1H, m), 7.90-8.05 (1H, m), 8.05-8.35 (5H, m), 8.61(1H, s), 8.66 (1H, s).

| Elemental Analysis: C₂₆H₂₈ClN₃O₅S·0.5H₂O | | | |
|---|---|---|---|
| Calcd (%) | C, 57.93; | H, 5.42; | N, 7.80 |
| Found (%) | C, 57.79; | H, 5.45; | N, 7.55 |

### Example 62

### 1-[4-(6-Chloro-2-naphthyl)sulfonyl]butanoyl]-4-(4-pyridyl)piperazine

According to the same manner as that of Example 42c), the title compound was obtained as colorless crystals (63%) from 4-(6-chloro-2-naphthyl)sulfonylbutyric acid obtained in Example 24c) and 1-(4-pyridyl)piperazine.
NMR (CDCl₃) δ: 2.10-2.20 (2H, m), 2.62 (2H, t, J = 6.8), 3.25-3.45 (4H, m), 3.33 (2H, t, J = 7.2), 3.55-3.80 (4H, m), 6.65 (2H, d, J = 6.4), 7.59 (1H, dd, J = 2.0 and 8.8), 7.80-8.00 (4H, m), 8.32 (2H, d, J = 6.4), 8.47 (1H, s).

| Elemental Analysis: C₂₃H₂₄ClN₃O₃S·0.1H₂O | | | |
|---|---|---|---|
| Calcd (%) | C, 60.08; | H, 5.31; | N, 9.14 |
| Found (%) | C, 59.92; | H, 5.33; | N, 9.22 |

### Example 63

### 1-[4-(6-Chloro-2-naphthyl)sulfonyl]propionyl]-4-(4 pyridyl)piperazine

According to the same manner as that of Example 42c), the title compound was obtained as colorless crystals (48%) from 4-(6-chloro-2-naphthyl)sulfonylpropionic acid obtained in Example 27b) and 1-(4-pyridyl)piperazine.
NMR (CDCl₃) δ: 2.85-3.00 (2H, m), 3.27 (2H, t, J = 5.4), 3.38 (2H, t, J = 5.4), 3.50-3.75 (6H, m), 6.64 (2H, d, J = 6.6), 7.58 (1H, dd, J = 2.2 and 8.8), 7.88-8.00 (4H, m), 8.32 (2H, d, J = 6.6), 8.48 (1H, s).

| Elemental Analysis: C₂₂H₂₂ClN₃O₃S·0.1H₂O | | | |
|---|---|---|---|
| Calcd (%) | C, 59.28; | H, 5.02; | N, 9.43 |
| Found (%) | C, 59.16; | H, 5.00; | N, 9.37 |

### Example 64

### 4-(6-Bromo-2-naphthyl)sulfonyl-N-methyl-N-[1-(2-methyl-4-pyridyl)-4-piperidyl]propanamide

### 64a) 6-Bromonaphthalene-2-sulfonyl chloride

Sodium nitrite (41.4 g) was added to a suspension of 6-aminonaphthalene-2-sulfonic acid (111.6 g) in 23.5% hydrobromic acid (500 ml) at -5 to 0°C over 40 minutes. The reaction mixture was stirred at 0°C for 30 minutes, and added to a solution of copper bromide (78.9 g) in 47% hydrobromic acid (100 ml) in portions at 60 to 70°C. The reaction mixture was stirred at room temperature for 30 minutes, and ice-cooled. The formed precipitates were filtered, and washed with cold water and diisopropyl ether to obtain 6-bromonaphthalene-2-sulfonic acid (116 g).

Thionyl chloride (109 ml) was added dropwise to a suspension of the resulting 6-bromonaphthalene-2-sulfonic acid (116 g) in DMF (300 ml). The reaction mixture was stirred at room temperature for 1.5 hours, and poured into ice-water-ethyl acetate. The precipitates were filtered, and purified by silica gel column. The filtrate was extracted with ethyl acetate, washed with water, and dried over anhydrous magnesium sulfate. This solution was purified by silica gel column, combined with the previous product, and washed with hexane to obtain the title compound as colorless crystals (99.5 g, 65.1%).
NMR (CDCl₃) δ: 7.78 (1H, dd, J = 2.2 and 8.8), 7.90-8.07 (3H, m), 8.16 (1H, d, J = 1.4), 8.58 (1H, s).

### 64b) Methyl 3-(6-bromo-2-naphthyl)sulfonylpropionate

A solution of 6-bromonaphthalene-2-sulfonyl chloride (17.0 g) obtained in Example 64a) in THF (100ml) was added dropwise to a suspension of sodium borohydride (4.21 g) in THF (200ml) at room temperature under a nitrogen stream. The reaction mixture was stirred at 40°C for 4 hours, and slowly poured into ice (250 g) while stirring. Then, 6 N hydrochloric acid (83 ml) was added dropwise, the mixture was extracted with ethyl acetate, washed with saturated brine, and dried over anhydrous magnesium sulfate. The filtrate was concentrated under reduced pressure to obtain the pale yellow solid, which was suspended in ethyl acetate (100 ml), triethylamine (8.44 ml) and methyl acrylate (5.26 ml) were added, and heated to reflux for 15 hours. The reaction mixture was concentrated to dryness, ethanol (150 ml) was added to the residue, the mixture was concentrated to dryness again, and dissolved in hot ethanol (150 ml). The insolubles were filtered, the filtrate was stirred at room temperature for 3 hours, and stirred further for 1 hour under ice-cooling. The precipitated crystals were filtered, and dried to obtain the title compound as pale yellow crystals (14.2 g, 72%).
NMR (CDCl₃) δ: 2.80 (2H, t, J = 7.7), 3.51 (2H, t, J = 7.7), 3.60 (3H, s), 7.73 (1H, dd, J = 1.8 and 8.8), 7.85-7.98 (4H, m), 8.13 (1H, m).

### 64c) 3-(6-Bromo-2-naphthyl)sulfonylpropionic acid

Concentrated sulfuric acid (8 ml) and water (8 ml) were added to a solution of methyl 3-(6-bromo-2-naphthyl)-sulfonylpropionate (14.1 g) obtained in Example 64b) in acetic acid (80 ml), followed by heating to reflux for 1 hour. After allowed to cool to room temperature, the reaction mixture was poured into ice (300 g), and the mixture was stirred for 30 minutes under ice-cooling. The precipitated crystals were filtered, washed with cold water, and dried to obtain the title compound as a gray powder (13.5 g, 99%).
NMR (CDCl₃) δ: 2.82 (2H, t, J = 7.5), 3.48 (2H, t, J = 7.5), 7.73 (1H, dd, J = 2.0 and 8.8), 7.84-7.97 (3H, m), 8.46 (1H, s), 8.92 (1H, br).

### 64d) 4-(6-Bromo-2-naphthyl)sulfonyl-N-methyl-N-[1-(2-methyl-4-pyridyl)-4-piperidyl]propanamide

Triethylamine (10.93 g) and WSC (10.35 g) were added to a solution of the 3-(6-bromo-2-naphthyl)sulfonylpropionic acid (12.36 g) obtained in Example 64c) and HOBt (8.27 g) in DMF (200 ml) at room temperature. Then, 4-methylamine-1-(2-methyl-4-pyridyl)piperidine (7.39 g) obtained in Example 42b) was added at room temperature. The reaction mixture was stirred at room temperature for 3 hours, and concentrated under reduced pressure. A 5% aqueous potassium carbonate solution was added to the resulting residue, the mixture was extracted with ethyl acetate, dried over anhydrous sodium sulfate, and the solvent was distilled off under reduced pressure. The residue was purified by silica gel column. The resulting pale yellow product was crystallized with ethyl acetate, and recrystallization procedures were repeated using ethanol, ethyl acetate-methanol, ethanol, and methyl acetate-methanol in this order to obtain the title compound as a white powder (5.2 g, 27%).
NMR (CDCl₃) δ: 1.57-1.88 (4H, m), 2.45 and 2.47 (3H, each s), 2.76-3.03 (7H, m), 3.55-3.60 (2H, m), 3.83-4.03 (2H, m), 4.54-4.62 (1H, m), 6.47-6.57 (2H, m), 7.73 (1H, dd, J = 1.5 and 8.7), 7.87-7.97 (3H, m), 8.14-8.22 (2H, m), 8.48 (1H, s).

| Elemental Analysis: C₂₅H₂₈BrN₃O₃S | | | |
|---|---|---|---|
| Calcd (%) | C, 56.60; | H, 5.32; | N, 7.92 |
| Found (%) | C, 56.42; | H, 5.09; | N, 7.86 |

### Example 65

### 3-(6-Bromo-2-naphthyl)sulfonyl-N-methyl-N-[1-(4-pyridyl)-4-piperidyl]propanamide

WSC (358 mg) was added to a solution of 3-(6-bromo-2-naphthyl)sulfonylpropionic acid (343 mg) obtained in Example 64c) and 4-methylamine-1-(2-methyl-4-pyridyl)-piperidine (200 mg) obtained in Example 42b) in DMF (20 ml), and the mixture was stirred at room temperature for 15 hours. The reaction mixture was concentrated under reduced pressure, aqueous sodium bicarbonate solution was added to the residue, and the mixture was extracted with methylene chloride. The extract was dried over anhydrous sodium sulfate, and the solvent was distilled off. The resulting residue was purified by silica gel column to obtain the title compound as colorless crystals (145 mg, 28%).
NMR (CDCl₃) δ: 1.50-1.85 (4H, m), 2.76 and 2.83 (3H, s), 2.80-3.03 (4H, m), 3.58 (2H, t, J = 7.7), 3.93 (2H, m), 4.59 (1H, m), 6.63-6.70 (2H, m), 7.73 (1H, dd, J = 1.8 and 8.8), 7.80-7.95 (3H, m), 8.13 (1H, s), 8.20-8.35 (2H, m), 8.47 (1H, s).

| Elemental Analysis: C₂₄H₂₆N₃O₃SBr·0.5H₂O | | | |
|---|---|---|---|
| Calcd (%) | C, 54.86; | H, 5.18; | N, 8.00 |
| Found (%) | C, 54.98; | H, 5.20; | N, 8.04 |

### Example 66

### 3-[N-[3-(6-Chloro-2-naphthyl)sulfonylpropanoyl]-N-[1-(2-methyl-4-pyridyl)-4-piperidyl]amino]propionic acid

### 66a) Benzyl 3-[N-[1-(2-methyl-4-pyridyl)-4-piperidyl]amino]propionate

According to the same manner as that of Example 30a), the title compound was obtained as yellow oil (64%) from β-alanine benzyl ester paratoluenesulfonate and 1-(2-methyl-4-pyridyl)-4-piperidone obtained in Example 42a).
NMR (CDCl₃+D₂O) δ: 1.22-1.50 (2H, m), 1.80-2.02 (2H, m), 2.45 (3H, s), 2.57 (2H, t, J = 6.4), 2.58-2.80 (1H, m), 2.82-3.02 (4H, m), 3.72-3.88 (2H, m), 5.14 (2H, s), 6.46-6.58 (2H, m), 7.28-7.42 (5H, m), 8.11 (1H, d, J = 5.6).

### 66b) Benzyl 3-[N-[3-(6-chloro-2-naphthyl)sulfonylpropanoyl]-N-[1-(2-methyl-4-pyridyl)-4-piperidyl]amino]propionate

According to the same manner as that of Example 53b), the title compound was obtained as colorless oil (35%) from benzyl 3-[N-[1-(2-methyl-4-pyridyl)-4-piperidyl]amino]-propionate obtained in Example 66a) and 3-(6-chloro-2-naphthyl)sulfonylpropionic acid (0.3g) obtained in Example 27b).
NMR (CDCl₃) δ: 1.48-1.88 (4H, m), 2.44 and 2.47 (3H, s, each), 2.38-3.06 (6H, m), 3.36-4.06 (7H, m), 5.07 and 5.12 (2H, s, each), 6.40-6.58 (2H, m), 7.24-7.46 (5H, m), 7.52-7.66 (1H, m), 7.86-8.00 (4H, m), 8.10-8.24 (1H, m), 8.47 (1H, s).

### 66c) 3-[N-[3-(6-Chloro-2-naphthyl)sulfonylpropanoyl]-N-[1-(2-methyl-4-pyridyl)-4-piperidyl]amino]propionic acid

According to the same manner as that of Example 61d), the title compound was obtained as a colorless powder (98%) from benzyl 3-[N-[3-(6-chloro-2-naphthyl)sulfonylpropanoyl]-N-[1-(2-methyl-4-pyridyl)-4-piperidyl]amino]-propionate obtained in Example 66b).
NMR (DMSO₆+D₂O) δ: 1.22-1.72 (4H, m), 2.00-2.16 (1H, m), 2.30 and 2.32 (3H, s, each), 2.34-2.54 (1H, m), 2.54-2.98 (4H, m), 3.02-3.20 (1H, m), 3.28-3.46 (1H, m), 3.54-3.72 (2H, m), 3.74-4.06 (3H, m), 6.52-6.74 (2H, m), 7.68-7.78 (1H, m), 7.90-8.06 (2H, m), 8.10-8.32 (3H, m), 8.61 and 8.66 (1H, s, each).

| Elemental Analysis: C₂₇H₃₀N₃O₅SCl·0.5H₂O·0.2EtOH | | | |
|---|---|---|---|
| Calcd (%) | C, 58.53; | H, 5.77; | N, 7.47 |
| Found (%) | C, 58.36; | H, 5.96; | N, 7.22 |

### Example 67

### Ethyl 3-[N-[3-(6-chloro-2-naphthyl)sulfonylpropanoyl]-N-[1-(2-methyl-4-pyridyl)-4-piperidyl]amino]propionate

A solution of 3-[N-[3-(6-chloro-2-naphthyl)sulfonylpropanoyl]-N-[1-(2-methyl-4-pyridyl)-4-piperidyl]amino]propionic acid (0.22 g) obtained in Example 66c) and concentrated sulfuric acid (0.22 ml) in ethanol (4.0 ml) was stirred at room temperature for 6 hours, and concentrated under reduced pressure. The residue was purified by CHP-20 column to obtain the title compound as a colorless powder (0.18 g, 77%).
NMR (CD₃OD) δ: 1.10-1.25 (3H, m), 1.40-1.85 (4H, m), 2.15-2.30 (1H, m), 2.37 and 2.39 (3H, s, each), 2.43-2.62 (1H, m), 2.65-3.02 (4H, m), 3.20-3.38 (1H, m), 3.45-3.75 (3H, m), 3.80-4.15 (5H, m), 6.54-6.74 (2H, m), 7.65 (1H, dt, J = 2.2 and 8.8), 7.88-8.18 (5H, m), 8.54 and 8.57 (1H, s, each).

| Elemental Analysis: C₂₉H₃₄N₃O₅SCl·0.5H₂O | | | |
|---|---|---|---|
| Calcd (%) | C, 59.94; | H, 6.07; | N, 7.23 |
| Found (%) | C, 60.03; | H, 5.80; | N, 7.00 |

### Example 68

### 3-(6-Chloro-2-naphthyl)sulfonyl-N-[1-(2-methyl-4-pydiryl)-4-piperidyl]-N-[3-oxy-3-(1-oxothiomorpholin-4-yl) propyl]propionamide

A solution of the 2-[N-[3-(6-chloro-2-naphthyl)sulfonylpropanoyl]-N-[1-(2-methyl-4-pyridyl)-4-piperidyl]amino]propionic acid (0.25 g) obtained in Example 66c), thiomorpholine 1-oxide trifluoroacetate (0.14 g), WSC (0.13 g) and diisopropylamine (0.24 ml) in DMF (5.0 ml) was stirred at room temperature for 24 hours, and concentrated under reduced pressure. The residue was dissolved in methylene chloride, washed with aqueous sodium bicarbonate solution, and dried over anhydrous magnesium sulfate. The solvent was distilled off, and the residue was purified by basic gel column to obtain the title compound as a colorless powder.
NMR (CD₃OD) δ: 1.40-1.90 (4H, m), 2.10-2.53 (4H, m), 2.62-3.40 (10H, m), 3.45-3.75 (4H, m), 3.78-4.40 (6H, m), 6.57-6.75 (2H, m), 7.60-7.75 (1H, m), 7.90-8.20 (5H, m), 8.53-8.63 (1H, m).

| Elemental Analysis: C₃₁H₃₇N₄O₅S₂Cl·0.7H₂O | | | |
|---|---|---|---|
| Calcd (%) | C, 56.60; | H, 5.88; | N, 8.52 |
| Found (%) | C, 56.65; | H, 6.22; | N, 8.89 |

### Example 69

### 3-(6-Chloro-2-naphthyl)sulfonyl-2,N-dimethyl-N-[1-(2-methyl-4-pyridyl)-4-piperidyl]propanamide

### 69a) Ethyl 3-(6-chloro-2-naphthyl)thio-2-methylpropionate

A solution of 6-chloro-2-mercaptonaphthalene (2.0 g) obtained in Example 1d), ethyl methacrylate (2.22 ml) and sodium ethoxide (0.1 g) in ethanol (40 ml) was stirred at 50°C for 16 hours, the insolubles were filtered, and the filtrate was adjusted to pH 5 with 1 N hydrochloric acid. The solution was concentrated under reduced pressure, and the residue was purified by silica gel column to obtain the title compound as a brown powder (1.6 g, 73%).
NMR (CDCl₃) δ: 1.19-1.34 (6H, m), 2.62-2.82 (1H, m), 3.02 (1H, dd, J = 7.0 and 13.2), 3.37 (1H, dd, J = 7.0 and 13.2), 4.12 (1H, q, J = 7.0), 7.36-7.50 (2H, m), 7.62-7.79 (4H, m).

### 69b) 3-(6-Chloro-2-naphthyl)sulfonyl-2-methylpropionic acid

According to the same manner as Example 27b), the title compound was obtained as a brown powder (49%) from ethyl 3-(6-chloro-2-napthyl)thio-2-methylpropionate obtained in Example 69a).
NMR (CDCl₃) δ: 1.36 (3H, t, J = 7.0), 2.96-3.24 (2H, m), 3.64-3.82 (1H, m), 7.59 (1H, dd, J = 1.8 and 8.8), 7.84-8.00 (4H, m), 8.47 (1H, s).

### 69c) tert-Butyl 4-[N-[3-(6-chloro-2-naphthyl)sulfonyl-2-methylpropionyl]-N-methylamino]piperidine-1-carboxylate

According to the same manner as that of Example 42c), the title compound was obtained as colorless crystals from 3-(6-chloro-2-naphthyl)sulfonyl-2-methylpropionate obtained in Example 69b) and tert-butyl 4-methylaminopiperidine-1-carboxylate.
NMR (CDCl₃) δ: 1.10-1.90 (7H, m), 1.46 and 1.48 (3H, s, each), 2.50-2.95 (2H, m), 2.65 and 2.90 (9H, s, each), 3.00-3.20 (1H, m), 3.30-3.65 (1H, m), 3.70-4.50 (4H, m), 7.58 (1H, dd, J = 1.8 and 8.8), 7.84-7.98 (4H, m), 8.40-8.48 (1H, m) .

### 69d) 3-(6-Chloro-2-naphtyl)sulfonyl-2,N-dimethyl-N-[1-(2-methyl-4-pyridyl)-4-piperidyl]propanamine

A mixture of tert-butyl 4-[N-[3-(6-chloro-2-naphthyl)sulfonyl-2-methylpropionyl]-N-methylaimno]piperidine-1-carboxylate (0.47 g) obtained in Example 69c), trifluoroacetic acid (4 ml) and toluene (4 ml) was stirred at room temperature for 1 hour, and concentrated under reduced pressure. The resulting residue, 4-chloro-2-methylpyridine (0.14 g), sodium acetate (0.09 g) and acetic acid (4.0 ml) were mixed, and the mixture was stirred at 130°C for 2 hours. The reaction mixture was concentrated under reduced pressure, methylene chloride was added to the residue, washed with a 10% aqueous sodium carbonate solution, and dried over anhydrous magnesium sulfate. The solvent was distilled off, and the residue was purified by silica gel column to obtain the title compound as a colorless powder (0.15 g).
NMR (CDCl₃) δ: 1.26 (3H, d, J = 7.0), 1.20-2.00 (4H, m), 2.45 (3H, s), 2.60-4.10 (7H, m), 2.90 (1H, s), 4.40-4.60 (1H, m), 6.44-6.58 (2H, m), 7.59 (1H, dd, J = 1.8 and 8.8), 7.84-7.98 (4H, m), 8.10-8.22 (1H, m), 8.45 (1H, s).

| Elemental Analysis: C₂₆H₃₀N₃O₃SCl·0.5H₂O | | | |
|---|---|---|---|
| Calcd (%) | C, 61.34; | H, 6.14; | N, 8.25 |
| Found (%) | C, 61.66; | H, 6.19; | N, 8.12 |

### Example 70

### N-(2-Aminoethyl)-3-(6-chloro-2-naphthyl)sulfonyl-N-[1-(2-methyl-4-pyridyl)-4-piperidyl]propanamide dihydrochloride

A solution of 4 N hydrogen chloride in ethyl acetate (3.0ml) was added to a solution of tert-butyl 2-[N-[3-(6-chloro-2-naphthyl)sulfonylpropionyl]-N-[1-(2-methyl-4-pyridyl)-4-piperidyl]amino]ethylcarbamate (0.15 g) obtained in Example 51) in ethyl acetate (1 ml), the mixture was stirred at room temperature for 13 hours, concentrated under reduced pressure, the residue was filtered, and dried to obtain the title compound as a colorless powder (0.16 g, quantitative).
NMR (DMSO-d₆) δ: 1.58-1.79 (4H, m), 2.46-2.49 (3H, m), 2.69-2.94 (4H, m), 3.27-3.65 (6H, m), 4.01-4.34 (3H, m), 7.08-7.14 (2H, m), 7.73-8.32 (6H, m), 8.66-8.69 (1H, m).

| Elemental Analysis: C₂₆H₃₃N₄ClO₃S·2HCl·3H₂O | | | |
|---|---|---|---|
| Calcd (%) | C, 48.64; | H, 6.12; | N, 8.73 |
| Found (%) | C, 48.54; | H, 5.96; | N, 8.47 |

### Example 71

### 3-(6-Chloro-2-naphthyl)sulfonyl-N-(2-dimethylaminoethyl)-N-[1-(2-methyl-4-pyridyl)-4-piperidyl]propanamide

According to the same manner as that of Example 24d), the title compound was obtained as a colorless powder (73%) from N-(2-aminoethyl)-3-(6-chloro-2-naphtyl)sulfonyl-N-[1-(2-methyl-4-pyridyl)-4-piperidyl]propanamide obtained in Example 70 and formalin.
NMR (CDCl₃) δ: 1.60-1.63 (2H, m), 1.70-1.80 (2H, m), 2.19 and 2.21 (6H, each s), 2.29-2.40 (2H, m), 2.44 and 2.47 (3H, each s), 2.84-3.03 (4H, m), 3.22-3.30 (2H, m), 3.56-3.63 (2H, m), 3.87-4.36 (3H, m), 6.47-6.56 (2H, m), 7.57-7.63 (1H, m), 7.92-7.97 (4H, m), 8.14-8.21 (1H, m), 8.47 (1H, s).

| Elemental Analysis: C₂₈H₃₅N₄ClO₃S·0.5H₂O | | | |
|---|---|---|---|
| Calcd (%) | C, 60.91; | H, 6.57; | N, 10.15 |
| Found (%) | C, 61.02; | H, 6.82; | N, 10.08 |

### Example 72

### 3-(6-Chloro-2-naphthyl)sulfonyl-N-(2-methoxyethyl)-N-[1-(2-methyl-4-pyridyl)-4-piperidyl]propanamide

### 72a) N-(2-Methoxyethyl)-1-(2-methyl-4-pyridyl)-4-aminopiperidine

According to the same manner as that of Example 24d), the title compound was obtained as colorless oil (71%) from 1-(2-methyl-4-pyridyl)-4-piperidone obtained in Example 42a) and 2-methoxyethylamine.
NMR (CDCl₃) δ: 1.32-1.51 (2H, m), 1.93-1.99 (2H, m), 2.44 (3H, s), 2.64-2.97 (5H, m), 3.37 (3H, m), 3.49-3.54 (2H, m), 3.79-3.87 (2H, m), 6.49-6.55 (2H, m), 8.14 (1H, d, J = 5.8).

### 72b) 3-(6-Chloro-2-naphthyl)sulfonyl-N-(2-methoxyethyl)-N-[1-(2-methyl-4-pyridyl)-4-piperidyl]propanamide

According to the same manner as that of Example 53b), the title compound was obtained as a colorless powder (10%) from N-(2-methoxyethyl)-1-(2-methyl-4-pyridyl)-4-aminopiperidine obtained in Example 72a) and 3-(6-chloro-2-naphthyl)sulfonylpropionic acid obtained in Example 27b).
NMR (CDCl₃) δ: 1.62-1.93 (4H, m), 2.44 and 2.47 (3H, each s), 2.77-3.24 (8H, m), 3.34 and 3.39 (3H, each s), 3.53-3.61 (2H, m), 3.86-4.32 (3H, m), 6.46-6.54 (2H, m), 7.52-7.62 (1H, m), 7.89-7.97 (4H, m), 8.17-8.28 (1H, m), 8.48 (1H, s).

| Elemental Analysis: C₂₇H₃₂N₃ClO₄S·H₂O | | | |
|---|---|---|---|
| Calcd (%) | C, 59.17; | H, 6.25; | N, 7.67 |
| Found (%) | C, 59.06; | H, 6.04; | N, 7.43 |

### Example 73

### tert-Butyl 2-[N-[3-(6-chloro-2-naphthyl)sulfonylpropionyl]-N-[1-(2-methyl-4-pyridyl)-4-piepridyl]amino]ethyl(methyl)-carbamate

### 73a) tert-Butyl 2-aminoethyl(methyl)carbamate

A solution of di-tert-butyl dicarbonate (13.62 g) in THF (100 ml) was added to a solution of N-methylethylenediamine (15.41 g) in THF (400 ml) at 0°C over 1 hour, and the mixture was further stirred at room temperature for 17 hours. The reaction mixture was concentrated under reduced pressure, the residue was diluted with saturated brine, and extracted with ethyl acetate. The extract was dried over anhydrous sodium sulfate, the solvent was distilled off, and the residue was purified by silica gel column to obtain the title compound as colorless oil (1.29 g, 10%).
NMR (CDCl₃) δ: 1.26 (2H, br s), 1.46 (9H, s), 2.82 (2H, t, J = 6.6), 2.88 (3H, s), 3.27 (2H, t, J = 6.4).

### 73b) tert-Butyl methyl [2-[1-(2-methyl-4-pyridyl)-4-piperidyl]aminoethyl]carbamate

According to the same manner as that of Example 24d), the title compound was obtained as colorless oil (73%) from 1-(2-methyl-4-pyridyl)-4-piperidone obtained in Example 42a) and the tert-butyl 2-aminoethyl(methyl)carbamate obtained in Example 73a).
NMR (CDCl₃) δ: 1.31-1.56 (2H, m), 1.46 (9H, s), 1.90-1.97 (2H, m), 2.44 (3H, s), 2.69-2.99 (8H, m), 3.30 (2H, t, J = 6.6), 3.78-3.85 (2H, m), 6.48-6.54 (2H, m), 8.14 (1H, d, J = 5.8).

### 73c) tert-Butyl 2-[N-[3-(6-chloro-2-naphthyl)sulfonylpropionyl]-N-[1-(2-methyl-4-pyridyl)-4 piperidyl]amino]ethyl(methyl)carbamate

According to the same manner as that of Example 53b), the title compound was obtained as a colorless powder (55%) from tert-butyl methyl [2-[[1-(2-methyl-4-pyridyl)-4-piperidyl]amino]ethyl]carbamate obtained in Example 73b) and 3-(6-chloro-2-naphthyl)sulfonylpropionic acid obtained in Example 27b).
NMR (CDCl₃) δ: 1.38-1.45 (9H, m), 1.67-1.77 (3H, m), 2.44 and 2.47 (3H, each s), 2.83-3.10 (7H, m), 3.18-3.30 (4H, m), 3.53-4.40 (6H, m), 6.47-6.55 (2H, m), 7.56-7.63 (1H, m), 7.90-7.97 (4H, m), 8.14-8.21 (1H, m), 8.48 (1H, br s).

| Elemental Analysis: C₃₂H₄₁N₄ClO₅S·0.9H₂O | | | |
|---|---|---|---|
| Calcd (%) | C, 59.55; | H, 6.68; | N, 8.68 |
| Found (%) | C, 59.71; | H, 6.98; | N, 8.78 |

### Example 74

### 3-(6-Chloro-2-napthyl)sulfonyl-N-[2-(methylamino)ethyl]-N-[1-(2-methyl-4-pydiryl)-4-piperidyl]propanamide dihydrochloride

According to the same manner as that of Example 70), the title compound was obtained as a colorless powder (quantitative) from tert-butyl 2-[N-[3-(6-chloro-2-naphthyl)sulfonylpropionyl]-N-[1-(2-methyl-4-pyridyl)-4-piperidyl]amino]ethyl(methyl)carbamate obtained in Example 73c).
NMR (DMSO-d₆) δ: 1.60-1.80 (4H, m), 2.40-2.47 (3H, m), 2.60-2.71 (3H, m), 2.78-3.08 (4H, m), 3.19-3.66 (6H, m), 4.08-4.34 (3H, m), 7.09-7.22 (2H, m), 7.73-7.77 (1H, m), 7.99-8.04 (1H, m), 8.13-8.32 (4H, m), 8.64-8.69 (1H, m).

| Elemental Analysis: C₂₇H₃₅N₄Cl₃O₃S·H₂O·EtOAc | | | |
|---|---|---|---|
| Calcd (%) | C, 52.58; | H, 6.41; | N, 7.91 |
| Found (%) | C, 52.33; | H, 6.42; | N, 7.99 |

### Example 75

### N-[2-(N-Acetyl-N-methylamino)ethyl]-3-(6-chloro-2-naphthyl)sulfonyl-N-[1-(2-methyl-4-pyridyl)-4-piperidyl]propanamide

A solution of 3-(6-chloro-2-napthyl)sulfonyl-N-[2-(methylamino)ethyl]-N-[1-(2-methyl-4-pyridyl)-4-piperidyl]propanamide dihydrochloride (0.20 g) obtained in Example 74), acetic anhydride (0.20 g) and triethylamine (0.40 g) in methylene chloride (10 ml) was stirred at room temperature for 20 hours, washed with saturated sodium bicarbonate water, and dried over anhydrous sodium sulfate. The solvent was distilled off, and the residue was purified by silica gel column to obtain the title compound as a colorless powder (0.13 g, 69%).
NMR (CDCl₃) δ: 1.63-1.94 (4H, m), 2.02-2.10 (3H, m), 2.44 and 2.47 (3H, each s), 2.79-4.02 (13H, m), 6.47-6.55 (2H, m), 7.58-7.63 (1H, m), 7.94-7.99 (4H, m), 8.14-8.24 (1H, m), 8.49 and 8.53 (1H, each s).

| Elemental Analysis: C₂₉H₃₅N₄ClO₄S·H₂O·0.2EtOAc | | | |
|---|---|---|---|
| Calcd (%) | C, 58.99; | H, 6.41; | N, 9.23 |
| Found (%) | C, 58.92; | H, 6.29; | N, 9.14 |

### Example 76

### 3-(7-Bromo-2H-chromen-3-yl)sulfonyl-N-methyl-N-[1-(4-pyridyl)-4-piperidyl]propanamide

### 76a) 3-(7-Bromo-2H-chromen-3-yl)sulfonylpropionic acid

3-(7-Bromo-2H-chromen-3-yl)sulfinyl chloride (3.10 g) was added to a solution of sodium sulfite (1.39 g) and sodium carbonate (1.68 g) in water (25 ml) at 75°C, the mixture was stirred at that temperature for 1.5 hours, a solution of sodium hydroxide (1.0 g) in water (1 ml) and bromosuccinic acid (4.93 g) were added, and mixture was stirred at 110°C for 20 hours. The precipitates formed were filtered, washed with water, and dried to obtain the title compound as a colorless powder (2.59 g, 75%).
NMR (DMSO-d₆) δ: 2.63 (2H, t, J = 7.0), 3.50 (2H, t, J = 7.0), 5.06 (2H, d, J = 1.0), 7.19 (1H, d, J = 1.8), 7.24 (1H, dd, J = 1.8 and 8.0), 7.41 (1H, d, J = 8.0), 7.48 (1H, s) .

### 76b) 3-(7-Bromo-2H-chromen-3-yl)sulfonyl-N-methyl-N-[1-(4-pyridyl)-4-piperidyl]propanamide

DMTMM (0.42 g) was added to a solution of 3-(7-bromo-2H-chromen-3-yl)sulfonylpropionic acid (0.35 g) obtained in Example 76a) and 4-methylamino-1-(4-pyridyl)piperidine (0.20 g) obtained in Example 30a) in DMF (20 ml), the mixture was stirred at room temperature for 15 hours. The reaction mixture was concentrated under reduced pressure, sodium bicarbonate water was added to the residue, and the mixture was extracted with methylene chloride. The extract was dried over anhydrous sodium sulfate, the solvent was distilled off under reduced pressure, and the residue was purified by silica gel column to obtain the title compound as colorless crystals (29%).
NMR (CDCl₃) δ: 1.50-1.80 (4H, m), 2.84 (3H, s), 2.75-3.00 (4H, m), 3.50 (2H, t, J = 7.0), 3.93 (2H, m), 4.63 (1H, m), 5.04 (2H, d, J = 1.2), 6.65 (2H, d, J = 6.6), 7.04 (1H, d, J = 8.2), 7.10 (1H, d, J = 1.8), 7.14 (1H, dd, J = 1.8 and 8.2), 7.31 (1H, s), 8.26 (2H, d, J = 6.6).

| Elemental Analysis: C₂₃H₂₆N₃O₄ SBr·0.5H₂O | | | |
|---|---|---|---|
| Calcd (%) | C, 52.18; | H, 5.14; | N, 7.94 |
| Found (%) | C, 52.05; | H, 5.02; | N, 7.78 |

### Example 77

### 3-(7-Bromo-2H-chromen-3-yl)sulfonyl-N-methyl-N-[1-(2-methyl-4-pyridyl)-4-piperidyl]propanamide

According to the same manner as that of Example 76b), the title compound was obtained as colorless crystals (53%) from 3-(7-bromo-2H-chromen-3-yl)sulfonylpropionic acid obtained in Example 76a) and 4-methylamino-1-(2-methyl-4-pyridyl)piperidine obtained in Example 42b).
NMR (CDCl₃) δ: 1.50-1.90 (4H, m), 2.46 (3H, s), 2.83 (3H, s), 2.75-3.00 (4H, m), 3.49 (2H, t, J = 7.2), 3.93 (2H, m), 4.62 (1H, m), 5.04 (2H, d, J = 1.2), 6.45-6.60 (2H, m), 7.04 (1H, d, J = 8.2), 7.11 (1H, d, J = 1.8), 7.14 (1H, dd, J = 1.8 and 8.2), 7.30 (1H, s), 8.15 (1H, d, J = 6.2).

| Elemental Analysis: C₂₄H₂₈N₃O₄SBr·0.5H₂O | | | |
|---|---|---|---|
| Calcd (%) | C, 53.04; | H, 5.38; | N, 7.73 |
| Found (%) | C, 52.87; | H, 5.41; | N, 7.61 |

### Example 78

### 3-(5-Chloro-3-methylbenzothiophen-2-yl)sulfonyl-N-methyl-N-[1-(2-methyl-4-pyridyl)-4-piperidyl]propanamide

### 78a) 3-(5-Chloro-3-methylbenzothiophen-2-yl)sulfonylpropionic acid

According to the same manner as that of Example 76a), the title compound (30%) was obtained from 5-chloro-3-methylbenzothiophene-2-sulfinyl chloride
NMR (CDCl₃+CD₃OD) δ: 2.72 (3H, s), 2.83 (2H, t, J = 7.6), 3.59 (2H, d, J = 7.6), 7.49 (1H, dd, J = 1.8 and 8.8), 7.80 (1H, d, J = 8.8), 7.83 (1H, d, J = 1.8).

### 78b) 3-(5-Chloro-3-methylbenzothiophen-2-yl)sulfonyl-N-methyl-N-[1-(2-methyl-4-pyridyl)-4-piperidyl]propanamide

According to the same manner as that of Example 76b), the title compound was obtained as colorless crystals (46%) from 3-(5-chloro-3-methylbenzothiophen-2-yl)-sulfonylpropionic acid obtained in Example 78a) and 4-methylamino-1-(2-methyl-4-pyridyl)piperidine obtained in Example 42b).
NMR (CDCl₃) δ: 1.42-1.90 (4H, m), 2.45 and 2.47 (3H, s), 2.74 (3H, s), 2.75 and 2.82 (3H, s), 2.80-3.05 (4H, m), 3.68 (2H, t, J = 7.7), 3.92 (2H, m), 4.54 (1H, m), 6.45-6.60 (2H, m), 7.51 (1H, dd, J = 1.8 and 8.8), 7.80 (1H, d, J = 8.8), 7.85 (1H, d, J = 1.8), 8.15 and 8.20 (1H, d, J = 6.0).

| Elemental Analysis: C₂₄H₂₈N₃O₃S₂Cl·0.25H₂O | | | |
|---|---|---|---|
| Calcd (%) | C, 56.46; | H, 5.63; | N, 8.23 |
| Found (%) | C, 56.45; | H, 5.75; | N, 8.40 |

### Example 79

### 3-(4'-Chlorobiphenyl-4-yl)sulfonyl-N-methyl-N-[1-(2-methyl-4-pyridyl)-4-piperidyl]propanamide

### 79a) 3-(4'-Chlorobiphenyl-4-yl)sulfonylpropionic acid

According to the same manner as that of Example 76a), the title compound was obtained as a colorless solid (54%) from 4'-chlorobiphenyl-4-sulfinyl chloride.
NMR (DMSO-d₆) δ: 2.56 (2H, t, J = 7.6), 3.58 (2H, d, J = 7.6), 7.52 (2H, d, J = 8.8), 7.71 (2H, d, J = 8.8), 7.93 (2H, d, J = 8.8), 8.10 (2H, d, J = 8.8).

### 79b) 3-(4'-Chlorobiphenyl-4-yl)sulfonyl-N-methyl-N-[1-(2-methyl-4-pyridyl)-4-piperidyl]propanamide

According to the same manner as that of Example 76b), the title compound was obtained as colorless crystals (49%) from 3-(4'-chlorobiphenyl-4-yl)sulfonylpropionic acid obtained in Example 79a) and 4-methylamino-1-(2-methyl-4-pyridyl)piperidine obtained in Example 42b).
NMR (CDCl₃) δ: 1.55-2.00 (4H, m), 2.45 and 2.47 (3H, s), 2.78 and 2.85 (3H, s), 2.75-3.05 (4H, m), 3.53 (2H, t, J = 7.7), 3.94 (2H, m), 4.65 (1H, m), 6.45-6.60 (2H, m), 7.47 (2H, d, J = 8.8), 7.56 (2H, d, J = 8.8), 7.75 (2H, d, J = 8.4), 8.00 (2H, d, J = 8.4), 8.16 (1H, d, J = 6.2).

| Elemental Analysis: C₂₇H₃₀N₃O₃SCl | | | |
|---|---|---|---|
| Calcd (%) | C, 63.33; | H, 5.91; | N, 8.21 |
| Found (%) | C, 63.05; | H, 6.14; | N, 8.44 |

### Example 80

### 3-[(E)-2-(4-Bromophenyl)ethenyl]sulfonyl-N-methyl-N-[1-(2-methyl-4-pyridyl)-4-piperidyl]propanamide

### 80a) 3-[(E)-2-(4-Bromophenyl)ethenyl]sulfonylpropionic acid

According to the same manner as that of Example 76a), the title compound was obtained as a colorless solid (34%) from 3-[(E)-2-(4-bromophenyl)ethenyl]sulfinyl chloride.
NMR (DMSO-d₆) δ: 2.66 (2H, t, J = 7.2), 3.41 (2H, d, J = 7.2), 7.50 (2H, s), 7.70 (4H, s).

### 80b) 3-[(E)-2-(4-Bromophenyl)ethenyl]sulfonyl-N-methyl-N-[1-(2-methyl-4-pyridyl)-4-piperidyl]propanamide

According to the same manner as that of Example 76b), the title compound was obtained as colorless crystals (67%) from 3-[(E)-2-(4-bromophenyl)ethenyl]sulfonylpropionic acid obtained in Example 80a) and 4-methylamino-1-(2-methyl-4-pyridyl)piperidine obtained in Example 42b).
NMR (CDCl₃) δ: 1.50-1.90 (4H, m), 2.46 (3H, s), 2.77 and 2.84 (3H, s), 2.75-3.05 (4H, m), 3.50 (2H, t, J = 7.1), 3.93 (2H, m), 4.63 (1H, m), 6.45-6.60 (2H, m), 6.88 (1H, d, J = 15.8), 7.39 (2H, d, J = 8.4), 7.53 (1H, d, J = 15.8), 7.58 (2H, d, J = 8.4), 8.15 (1H, d, J = 6.6).

| Elemental Analysis: C₂₃H₂₈N₃O₃SBr·0.9H₂O | | | |
|---|---|---|---|
| Calcd (%) | C, 52.85; | H, 5.75; | N, 8.04 |
| Found (%) | C, 52.99; | H, 5.67; | N, 7.70 |

### Example 81

### (E)-3-(6-Chloro-2-naphthyl)sulfonyl-N-methyl-N-[1-(2-methyl-4-pyridyl)-4-piperidyl]propeneamide

### 81a) Sodium 6-chloronaphthalene-2-sulfinate

6-Chloronaphthalene-2-sulfonyl chloride (15.7 g) obtained in Example 1c) was added to a solution of sodium sulfite (15.1 g) and sodium carbonate (10.1 g) in water (150 ml) at 70°C, and the mixture was stirred at that temperature for 2 hours. The reaction mixture was allowed to stand at room temperature overnight, the precipitates formed were filtered, and washed with a small amount of water and acetone to obtain the title compound (12.6 g, 84%).
NMR (DMSO-d₆) δ: 7.50 (1H, dd, J = 2.2 and 8.8), 7.72 (1H, dd, J = 8.2 and 1.4), 7.86 (1H, d, J = 8.2), 7.95-8.02 (3H, m).

### 81b) (E)-3-(6-Chloro-2-naphthyl)sulfonylacrylic acid

A solution of sodium hydroxide (0.20 g) in water (1 ml) and 2,3-dibromosuccinic acid (1.38 g) were added to a solution of sodium 6-chloronaphthalene-2-sulfinate (0.50 g) obtained in Example 81a) in water (20 ml) at 60°C, and the mixture was stirred at 110°C for 20 hours. The precipitates were filtered, washed with water, and dried to obtain the title compound as a colorless solid (0.24 g, 40%).
NMR (DMSO-d₆) δ: 6.78 (1H, d, J = 15.0), 7.75 (1H, dd, J = 2.0 and 8.8), 7.77 (1H, d, J = 15.0), 7.94 (1H, dd, J = 2.0 and 8.8), 8.15-8.35 (3H, m), 8.56 (1H, s).

### 81c) (E)-3-(6-Chloro-2-naphthyl)sulfonyl-N-methyl-N-[1-(2-methyl-4-pyridyl)-4-piperidyl]propeneamide

According to the same manner as that of Example 76b), the title compound was obtained as colorless crystals (37%) from (E)-3-(6-chloro-2-naphthyl)sulfonylacrylic acid obtained in Example 81b) and 4-methylamino-1-(2-methyl-4-pyridyl)piperidine obtained in Example 42b).
NMR (CDCl₃) δ: 1.50-1.90 (4H, m), 2.45 and 2.48 (3H, s), 2.87 and 2.99 (3H, s), 2.75-3.05 (2H, m), 3.97 (2H, m), 4.70 (1H, m), 6.45-6.60 (2H, m), 7.32 (1H, d, J = 14.6), 7.48 (1H, d, J = 14.6), 7.60 (1H, dd, J = 1.8 and 8.8), 7.80-8.00 (4H, m), 8.17 (1H, d, J = 6.0), 8.49 (1H, s).

| Elemental Analysis: C₂₅H₂₆N₃O₃SCl·1.3H₂O | | | |
|---|---|---|---|
| Calcd (%) | C, 59.17; | H, 5.68; | N, 8.28 |
| Found (%) | C, 58.91; | H, 5.76; | N, 8.67 |

### Example 82

### 3-(6-Chloro-2-naphthyl)sulfonyl-N-methyl-N-[[1-(4-pyridyl)-4-piperidyl]methyl]propanamide

According to the same manner as that of Example 76b), the title compound was obtained as colorless crystals (58%) from 4-[4-(N-methylamino)methyl-1-pyperidyl]pyridine obtained in Example 1b) and 3-(6-chloro-2-naphthyl)-sulfonylpropionic acid obtained in Example 27b).
NMR (CDCl₃) δ: 1.26 (2H, m), 1.60-1.80 (3H, m), 2.73 (2H, t, J = 7.5), 2.79 (2H, m), 3.12 (2H, t, J = 6.2), 3.55 (2H, t, J = 7.5), 3.85 (2H, m), 5.96 (1H, m), 6.62 (2H, d, J = 6.6), 7.60 (1H, dd, J = 1.8 and 8.8), 7.85-8.00 (4H, m), 8.23 (2H, d, J = 6.6), 8.47 (1H, s).

| Elemental Analysis: C₂₄H₂₆N₃O₃SCl·0.5H₂O | | | |
|---|---|---|---|
| Calcd (%) | C, 59.93; | H, 5.66; | N, 8.74 |
| Found (%) | C, 60.17; | H, 5.87; | N, 8.62 |

### Example 83

### 3-(6-Chloro-2-naphthyl)sulfonyl-2,2,N-trimethyl-N-[1-(2-methyl-4-pyridyl)-4-piperidyl]propanamide

### 83a) tert-Butyl 4-[N-[3-(6-chloro-2-naphthyl)thio-2,2-dimethylpropionyl]-N-methylamino)piperidine-1-carboxylate

tert-Butyl 4-[N-(3-chloro-2,2-dimethylpropionyl)-N-methylamino]piperidine-1-carboxylate (0.70 g), 6-chloro-2-mercaptonaphthalene (0.71 g) obtained in Example 1d), and sodium methoxide (0.11 g) were added to methanol (14 ml), the mixture was stirred at 70°C for 24 hours, the insolubles were filtered, and the filtrate was concentrated under reduced pressure. The residue was diluted with ethyl acetate, washed with water, and dried over anhydrous magnesium sulfate. The solvent was distilled off, and the residue was purified by silica gel column to obtain the title compound (0.86 g, 84%).
NMR (CDCl₃) δ: 1.44 (9H, s), 1.46 (6H, s), 1.20-1.90 (4H, m), 2.60-2.90 (2H, m), 2.89 (3H, s), 3.34 (2H, s), 4.05-4.55 (3H, m), 7.36-7.52 (2H, m), 7.61-7.71 (2H, m), 7.72-7.80 (2H, m).

### 83b) tert-Butyl 4-[N-[3-(6-chloro-2-naphthyl)sulfoynl-2,2-dimethylpropionyl]-N-methylamino]piperidine-1-carboxylate

According to the same manner as that of Examples 1f), the title compound was obtained as colorless crystals (68%) from tert-butyl 4-[N-[3-(6-chloro-2-naphthyl)thio-2,2-dimethylpropionyl]-N-methylamino]piperidine-1-carboxylate obtained in Example 83a).
NMR (CDCl₃) δ: 1.47 (9H, s), 1.59 (6H, s), 1.40-1.70 (4H, m), 2.65-2.90 (2H, m), 2.93 (3H, s), 3.67 (2H, s), 4.10-4.50 (3H, m), 7.67 (1H, dd, J = 2.0 and 8.4), 7.86-8.08 (4H, m), 8.48 (1H, s).

### 83c) 3-(6-Chloro-2-naphthyl)sulfonyl-2,2,N-trimethyl-N-[1-(2-methyl-4-pyridyl)-4-piperidyl]propanamide

tert-Butyl 4-[N-[3-(6-chloro-2-naphthyl)sulfoynl-2,2-dimethylpropionyl]-N-methylamino]piperidine-1-carboxylate (0.62 g) obtained in Example 83b) and trifluoroacetic acid (3.1 ml) were added to toluene (3.1 ml), the mixture was stirred at room temperature for 1 hour, and concentrated under reduced pressure. The residue was dissolved in acetic acid (3.1 ml), sodium acetate (0.19 g) was added, and the mixture was stirred at 130°C for 2 hours. The reaction mixture was concentrated under reduced pressure, the residue was diluted with methylene chloride, washed with a 10% aqueous sodium carbonate solution, and dried over magnesium sulfate. The solvent was distilled off, and the residue was purified by silica gel column to obtain the title compound as a brown amorphous powder (0.18 g, 29%).
NMR (CDCl₃) δ: 1.59 (6H, s), 1.60-1.90 (4H, m), 2.46 (3H, s), 2.80-3.05 (5H, m), 3.68 (2H, s), 3.97 (2H, d, J = 12.4), 4.35-4.65 (1H, m), 6.46-6.60 (2H, m), 7.57 (1H, dd, J = 2.2 and 8.8), 7.86-8.06 (4H, m), 8.17 (1H, d, J = 5.8), 8.48 (1H, s).

| Elemental Analysis: C₂₇H₃₂N₃O₃SCl·0.5H₂O | | | |
|---|---|---|---|
| Calcd (%) | C, 62.00; | H, 6.36; | N, 8.03 |
| Found (%) | C, 61.96; | H, 6.38; | N, 7.98 |

### Example 84

### 3-(6-Chloro-2-naphthyl)sulfonyl-2-methyl-N-[1-(2-methyl-4-pyridyl)-4-piperidyl]propionamide

### 84a) N-(1-Benzyl-4-piperidyl)-3-(6-chloro-2-naphthyl)sulfonyl-2-methylpropionamide

According to the same manner as that of Example 76b), the title compound was obtained as a pale yellow powder (73%) from 3-(6-bromo-2-naphthyl)sulfonyl-2-methylpropionic acid obtained in Example 69b) and 4-amino-1-benzylpiperidine.
NMR (CDCl₃) δ: 1.30 (3H, d, J = 7.0), 1.24-1.54 (2H, m), 1.64-2.16 (4H, m), 2.66-3.00 (3H, m), 3.09 (1H, dd, J = 4.0 and 14.0), 3.47 (2H, s), 3.44-3.70 (1H, m), 3.80 (1H, dd, J = 8.0 and 14.0), 5.53 (1H, d, J = 8.2), 7.20-7.38 (5H, m), 7.57 (1H, dd, J = 2.2 and 8.8), 7.84-7.96 (4H, m), 8.45 (1H, s).

### 84b) 3-(6-Chloro-2-naphthyl)sulfonyl-2-methyl-N-[1-(2-methyl-4-pyridyl)-4-piperidyl]propionamide

1-Chloroethyl chlorocarbonate (0.16 ml) was added to a solution of N-(1-benzyl-4-piperidyl)-3-(6-chloro-2-naphthyl)sulfonyl-2-methylpropionamide (0.34 g) obtained in Example 84a) in 1,2-dichloroethane (2.0 ml) at 0°C, the mixture was stirred at 70°C for 6 hours, methanol (2.0 ml) was added, and the mixture was stirred at 70°C for 1 hour. The reaction mixture was concentrated under reduced pressure, the residue, 4-chloro-2-methylpyridine (0.18 g) and sodium acetate (0.11 g) were added to acetic acid (2.0 ml), and the mixture was stirred at 130°C for 2 hours. The reaction mixture was concentrated under reduced pressure, the residue was diluted with methylene chloride, washed with a 10% aqueous sodium carbonate solution, and dried over anhydrous magnesium sulfate. The solvent was distilled off, and the residue was purified with silica gel column to obtain the title compound as a brown amorphous powder (0.10 g, 29%).

| Elemental Analysis: C₂₅H₂₈N₃O₃SCl·0.5H₂O | | | |
|---|---|---|---|
| Calcd (%) | C, 60.66; | H, 5.90; | N, 8.49 |
| Found (%) | C, 60.94; | H, 5.90; | N, 8.53 |

### Example 85

### 2-[3-(6-Chloro-2-naphthyl)sulfonylpropyl]-8-(2-methyl-4-pyridyl)-2,8-diazaspiro[4.5]decane-1,3-dione

### 85a) Ethyl (1-benzyl-4-piperidine)cyanoacetate hydrochloride

1-Benzyl-4-piperidone (10 g), ethyl cyanoacetate (9.67 ml), ammonium acetate (1.39 g) and acetic acid (2.6 ml) were added to toluene (50 ml) and refluxed for 7 hours while removing the produced water with Dean-Stark. The solvent was distilled off, the residue was dissolved in ethyl acetate, washed with an aqueous sodium bicarbonate solution, and dried over anhydrous sulfate. The solvent was distilled off, and the residue was treated with 4 N hydrogen chloride in ethyl acetate to obtain the title compound (8.5 g, 50%).
NMR (CD₃OD) δ: 1.33 (3H, t, J = 7.0), 2.60-3.40 (5H, m), 3.52-3.80 (2H, m), 4.00-4.28 (1H, m), 4.30 (2H, q, J = 7.0), 4.39 (2H, s), 7.42-7.65 (5H, m).

### 85b) Ethyl 1-benzyl-4-ethoxycarbonylmethylpiperidine-4-carboxylate

A solution of potassium cyanide (2.59 g) in water (10 ml) was added to a suspension of ethyl (1-benzyl-4-piperididene)cyanoacetate hydrochloride (8.5 g) obtained in Example 85a) in ethanol (43 ml), the mixture was stirred at 80°C for 1 hour, and the solvent was distilled off. Concentrated hydrochloric acid (60 ml) was added to the residue, refluxed for 24 hours, and concentrated under reduced pressure. Ethanol (50 ml) was dissolved in ethanol, concentrated sulfuric acid (10 ml) was added, and the mixture was refluxed for 20 hours. The reaction mixture was concentrated under reduced pressure, water was added to the residue, neutralized with sodium bicarbonate water, extracted with methylene chloride, dried over magnesium sulfate, and concentrated to obtain the title compound as brown oil (7.16 g).
NMR (CDCl₃) δ: 1.52-1.70 (2H, m), 2.05-2.40 (4H, m), 2.45-2.65 (2H, m), 2.59 (2H, s), 3.47 (2H, s), 4.09 (2H, q, J = 7.2), 4.19 (2H, q, J = 6.8), 7.10-7.35 (5H, m).

### 85c) 8-Benzyl-2-[3-(6-chloro-2-naphthyl)sulfonylpropyl]-2,8-diazaspiro[4.5]decane-1,3-dione

A solution of ethyl 1-benzyl-4-ethoxycarbonylmethylpiperidine-4-carboxylate (1.12 g) obtained in Example 85b) in concentrated hydrochloric acid (10 ml) was refluxed for 16 hours, and concentrated under reduced pressure. The residue was dissolved in DMF (20 ml), DCC (0.70 g) was added, the mixture was stirred at room temperature for 1 hours, 3-(6-chloro-2-naphthyl)sulfonylpropylamine hydrochloride (0.98 g) and triethylaminde (0.94 ml) were added, and the mixture was stirred at room temperature for 3 hours. The insolubles were filtered, the filtrate was concentrated under reduced pressure, and the residue and sodium acetate (0.64 g) was stirred in acetic anhydride (13 ml) at 100°C for 1 hour. The insolubles were filtered, the filtrate was concentrated under reduced pressure, and the residue was purified by silica gel column to obtain the title compound as a colorless amorphous material (1.20 g, 75%).
NMR (CDCl₃) δ: 1.36-1.54 (2H, m), 1.86-2.22 (6H, m), 2.54 (2H, s), 2.76-2.94 (2H, m), 3.10-3.26 (2H, m), 3.52 (2H, s), 3.59 (2H, t, J = 6.8), 7.18-7.38 (5H, m), 7.59 (1H, dd, J = 2.0 and 8.8), 7.80-8.06 (4H, m), 8.45 (1H, s).

### 85d) 2-[3-(6-Chloro-2-naphthyl)sulfonylpropyl]-8-(2-methyl-4-pyridyl)-2,8-diazaspiro[4.5]decane-1,3-dione

According to the same manner as that of Example 84b), the title compound was obtained as a brown powder (11%) from 2-[3-(6-chloro-2-naphthyl)sulfonylpropyl]-2,8-diazaspiro[4.5]decane-1,3-dione obtained in Example 85d).
NMR (CDCl₃) δ: 1.54-1.76 (2H, m), 1.98-2.20 (4H, m), 2.47 (3H, s), 2.65 (2H, s), 2.94-3.14 (2H, m), 3.23 (2H, t, J = 7.2), 3.63 (2H, t, J = 6.6), 3.76-3.92 (2H, m), 6.48-6.60 (2H, m), 7.61 (1H, dd, J = 2.2 and 8.8), 7.82-8.02 (4H, m), 8.20 (1H, d, J = 5.8), 8.46 (1H, s).

| Elemental Analysis: C₂₇H₂₈N₃O₄SCl·2.8H₂O | | | |
|---|---|---|---|
| Calcd (%) | C, 56.25; | H, 5.87; | N, 7.29 |
| Found (%) | C, 55.99; | H, 5.48; | N, 6.95 |

### Example 86

### (E)-4-(6-Chloro-2-naphthyl)sulfonyl-N-methyl-N-[1-(2-methyl-4-pyridyl)-4-piperidyl]-2-buteneamide

### 86a) Ethyl (E)-4-(6-chloro-2-naphthyl) sulfonyl-2-butenoate

Sodium 6-chloronaphthalene-2-sulfinate (1.0 g) obtained in Example 81a) and ethyl 3-bromochrotonate (0.85 g) were added to DMF (15 ml), the mixture was stirred at room temperature for 14 hours, ice-water was added, and extracted with ethyl acetate. The extract was dried over anhydrous sodium sulfate, and concentrated under reduced pressure. The residue was purified by silica gel column, and recrystallized from ethyl acetate/hexane to obtain the title compound as colorless crystals (0.49 g, 43%).
NMR(CDCl₃) δ: 1.26 (3H,t, J = 7.2), 4.62 (2H, dd, J = 1.2 and 7.7), 4.17 (2H, q, J = 7.2), 5.87 (1H, d, J = 15.8), 6.82 (1H, dt, J = 7.7 and 15.8), 7.60 (1H, dd, J = 1.8 and 8.8), 7.80-8.00 (4H, m), 8.44 (1H, s).

### 86b) (E)-4-(6-Chloro-2-naphthyl)sulfonyl-2-butenoic acid

Concentrated sulfuric acid (0.6 ml) was added to a solution of ethyl (E)-4-(6-chloro-2-naphthyl)sulfonyl-2-butenoate (0.58 g) obtained in Example 86a) in acetic acid (6 ml), the mixture was stirred at 110°C for 3 hours, and concentrated under reduced pressure. Water was added to the residue, and extracted with ethyl acetate. The extract was dried over anhydrous sodium sulfate, concentrated under reduced pressure, and the residue was recrystallized from ethyl acetate/hexane to obtain the title compound as colorless crystal (0.42 g, 78%).
NMR (CDCl₃) δ: 4.05 (2H, d-like), 5.88 (1H, d, J = 15.8), 6.82-7.02 (1H, m), 7.61 (1H, dd, J = 1.8 and 8.8), 7.80-8.00 (4H, m), 8.44 (1H, d, J = 1.4).

### 86c) (E)-4-(6-Chloro-2-naphthyl)sulfonyl-N-methyl-N-[1-(2-methyl-4-pyridyl)-4-piperidyl]-2-buteneamide

According to the same manner as that of Example 76b), the title compound (36%) was obtained from (E)-4-(6-chloro-2-naphthyl)sulfonyl-2-butenoic acid obtained in Example 86b).
NMR (CDCl₃) δ: 1.50-1.80 (4H, m), 2.46 (3H, s), 2.74 (3H, s), 2.80-3.10 (1H, m), 6.30-6.80(4H, m), 7.60 (1H, dd, J = 1.8 and 8.8), 7.85-8.00 (4H, m), 8.17 (1H, d, J = 5.6), 8.45 (1H, s).

| Elemental Analysis: C₂₆H₂₈ClN₃O₃S | | | |
|---|---|---|---|
| Calcd (%) | C, 62.70; | H, 5.67; | N, 8.44 |
| Found (%) | C, 62.64; | H, 5.64; | N, 8.31 |

### Example 87

### 1-[2-(6-Chloro-2-naphthyl)sulfonylacetyl]-4-(4-pyridyl)piperadine

According to the same manner as that of Example 76b), the title compound was obtained as colorless crystals (42%) from 2-(6-chloro-2-naphthyl)sulfonylacetic acid obtained in Example 35c) and 1-(4-pyridyl)piperazine.
NMR (DMSO-d₆) δ: 3.10-3.80 (8H, m), 4.84 (2H, s), 6.80 (2H, d, J = 6.6), 7.71 (1H, dd, J = 2.2 and 8.6), 8.00 (1H, dd, J = 1.4 and 9.2), 8.05-8.40 (3H, m), 8.17 (2H, d, J = 6.6), 8.63 (1H, s).

### Example 88

### 3-[2-(6-Chloro-2-naphthyl)sulfonylethyl]-2,4-dioxo-8-(2-methyl-4-pyridyl)-1,3,8-triazaspiro[4.5]decane

### 88a) tert-Butyl 3-(2-bromoethyl)-2,4-dioxo-1,3,8-triazaspiro[4.5]decane-8-carboxylate

tert-Butyl 2,4-dioxo-1,3,8-triazaspiro]4.5]decane-8-carboxylate (Wysong, C.N.; Yokum, T.S.; Morales, G.A.; Gundry, R.L.; McLaughlin, M.L.; Hammer, R.P. J. Org. Chem., 1996, 61, 7650) (2.7 g), 2-bromoethanol (1.5 g) and triphenylphosphine (3 g) were dissolved in THF (50 ml), and the solution was stirred at 0°C under nitrogen atmosphere. Then, a 40% solution of diethyl azocarboxylate in toluene (5 ml) was added dropwise. The reaction mixture was stirred at room temperature for 2 hours, concentrated under reduced pressure, and water was added to the residue, followed by extraction with ethyl acetate. The extract was washed with water and saturated brine, and dried over anhydrous sodium sulfate. The solvent was distilled off, and the residue was purified by silica gel column to obtain a 1:1 mixture (5.01 g) of the title compound and 1,2-hydrazine diethyl dicarboxylate. This mixture was used in the next reaction as it was.
NMR (CDCl₃) δ: 1.49 (9H, s), 1.50-1.70 (2H, m), 1.95-2.15 (2H, m), 3.10-3.35 (2H, m), 3.61 (2H, t, J = 6.4), 3.93 (2H, t, J = 6.4), 3.93-4.10 (2H, m).

### 88b) tert-Butyl 3-[2-(6-chloro-2-naphthyl)sulfonylethyl]-2,4-dioxo-1,3,8-triazaspiro[4.5]decane-8-carboxylate

According to the manner as that of Example 3a), tert-butyl 3-[2-[(6-chloro-2-naphthyl)thio]ethyl]-2,4-dioxo-1,3,8-triazaspiro]4.5]decabe-8-carboxylate was obtained from tert-butyl 3-(2-bromoethyl)-2,4-dioxo-1,3,8-triazaspiro[4.5]decane-8-carboxylate obtained in Example 88a) and 6-chloro-2-mercaptonaphthalene obtained in Example 1d). This compound was oxidized with mCPBA as in Example 7d) to obtain the title compound as colorless crystals (17%).
NMR (CDCl₃) δ: 1.48 (9H, s), 1.60-1.72 (2H, m), 1.90-2.10 (2H, m), 3.10-3.30 (2H, m), 3.80-4.10 (4H, m), 6.42 (1H, s), 7.60 (1H, dd, J = 1.8 and 8.8), 7.90-8.00 (4H, m), 8.54 (1H,s).

### 88c) 3-[2-(6-Chloro-2-naphthyl)sulfonylethyl]-8-(2-methyl-4-pyridyl)-1,3,8-triazaspiro[4.5]decane-2,4-dione

According to the same manner as that of Example 83c), the title compound was obtained as a colorless powder (13%) from tert-butyl 3-[2-(6-chloro-2-naphthyl)sulfonylethyl]-2,4-dioxo-1,3,8-triazaspiro[4.5]decane-8-carboxylate obtained in Example 88b).
NMR (CDCl₃) δ: 1.65-1.90 (2H, m), 2.00-2.30 (2H, m), 2.45 (3H, s), 3.20-3.45 (2H, m), 3.45-3.70 (2H, m), 3.70-4.00 (4H, m), 6.40-6.70 (2H, m), 7.50-7.75 (2H, m), 7.85-8.10 (4H, m), 8.13 (1H, d, J = 5.4), 8.54 (1H, s).

| Elemental Analysis: C₂₅H₂₅ClN₄O₄S·0.25MeOH·H₂O | | | |
|---|---|---|---|
| Calcd (%) | C, 56.26; | H, 5.24; | N, 10.39 |
| Found (%) | C, 56.01; | H, 5.14; | N, 10.54 |

### Example 89

### 3-[2-(6-Chloro-2-naphthyl)sulfonylpropyl]-8-(2-methyl-4-pyridyl)-1,3,8-triazaspiro[4,5]decane-2,4-dione

### 89a) 8-(2-Methyl-4-pyridyl)-1,3,8-triazaspiro[4.5]decane-2,4-dione

1-(2-Methyl-4-pyridyl)-4-piperidone (1.9 g) obtained in Example 42a), ammonium carbonate (3.18 g) and sodium cyanide (0.72 g) were suspended in ethanol (15 ml)-water (15 ml), and the suspension was stirred at 50 to 55°C for 15 hours. The reaction mixture was cooled, water (10 ml) was added, the precipitated crystals were filtered, and dried to obtain the title compound (1.7 g, 66%).
NMR (DMSO-d₆) δ: 1.28-1.90 (4H, m), 2.32 (3H, s), 3.10-3.40 (2H, m), 3.70-3.94 (2H, m), 6.60-6.80 (2H, m), 8.03 (1H, d, J = 5.8), 8.59 (1H, s), 10.73 (1H, s).

### 89b) 3-[2-(6-Chloro-2-naphthyl)sulfonylpropyl]-8-(2-methyl-4-pyridyl)-1,3,8-triazaspiro[4.5]decane-2,4-dione

8-(2-Methyl-4-pyridyl)-1,3,8-triazaspiro[4.5]decane-2,4-dione (0.26 g) obtained in Example 89a), 3-(6-chloro-2-naphthyl)sulfonylpropanol (0.28 g) obtained in Example 18b) and triphenylphosphine (0.29 g) were suspended in DMF (10 ml), a 40% diethyl azodicarboxylate solution in toluene was added dropwise thereto. The reaction mixture was stirred at room temperature for 15 hours, concentrated under reduced pressure, water was added to the residue, and the mixture was extracted with methylene chloride. The extract was dried over anhydrous sodium sulfate, the solvent was distilled off, and the residue was purified by silica gel column to obtain the title compound as a pale yellow powder (30 mg, 5%).
NMR (CDCl₃) δ: 1.65-1.80 (2H, m), 2.00-2.40 (2H, m), 2.45 (3H, s), 3.16-3.38 (4H, m), 3.61 (2H, t, J = 6.6), 3.74-3.95 (2H, m), 6.45-6.6 (2H, m), 6.99 (1H, s), 7.60 (1H, dd, J = 1.8 and 8.8), 7.85-8.00 (4H, m), 8.18 (1H, d, J = 6.0), 8.47 (1H, s).

| Elemental Analysis: C₂₆H₂₇ClN₄O₄S·0.8H₂O | | | |
|---|---|---|---|
| Calcd (%) | C, 57.67; | H, 5.32; | N, 10.35 |
| Found (%) | C, 57.63; | H, 5.55; | N, 10.07 |

### Example 90

### 4-[3-(6-Chloro-2-naphthyl)sulfonylpropyl]-8-(2-methyl-4-pyridyl)-1-thia-4,8-diazaspiro[4.5]decane-3-one

### 90a) 6-Chloro-2-(3-chloropropyl)sulfonylnaphthalene and 6-chloro-2-(3-bromopropyl)sulfonylnaphthalene

Sodium 6-chloronaphthalene-2-sulfinate (2.49 g) obtained in Example 81a) and 1-bromo-3-chloropropane (7.87 g) were added to DMF (30 ml), and the mixture was stirred at 70°C for 18 hours. The reaction mixture was diluted with water, and extracted with ethyl acetate. The extract was washed with water, dried over anhydrous magnesium sulfate, and the solvent was distilled off to obtain the residue, which was purified by silica gel column to obtain a 1:1 mixture (1.70 g, 52%) of the two title compounds.
NMR (CDCl₃) δ: 2.19-2.41 (4H, m), 3.31-3.38 (2H, m), 3.49 (1H, t, J = 6.2), 3.64 (1H, t, J = 6.2), 7.60 (1H, dd, J = 2.0 and 9.0), 7.88-7.98 (4H, m), 8.48 (1H, s).

### 90b) tert-Butyl 3-oxo-1-thia-4,8-diazaspiro[4.5]decane-8-carboxylate

tert-Butyl 4-oxopiperidine-1-carboxylate (4.0 g), thioglycolic acid (2.2 g), ammonium carbonate (1.17 g) and anhydrous magnesium sulfate (1.41 g) were added to toluene (60 ml), and the mixture was refluxed for 2.5 hours. The reaction mixture was diluted with water, and extracted with ethyl acetate. The extract was washed with water, dried over anhydrous magnesium sulfate, and concentrated under reduced pressure. The resulting solid was washed with hexane to obtain the title compound as the colorless crystals (2.79 g, 51%).
NMR (CDCl₃) δ: 1.46 (9H, s), 1.87-1.95 (4H, m), 3.06-3.20 (2H,m), 3.58 (2H, s), 3.87-3.97 (2H, m), 6.81 (1H, br s).

### 90c) tert-Butyl 4-[3-(6-chloro-2-naphthyl)sulfonylpropyl]-3-oxo-1-thia-4,8-diazaspiro[4.5]decane-8-carboxylate

Sodium hydride (60% oily; 0.23 g) was added to a solution of tert-butyl 3-oxo-1-thia-4,8-diazaspiro[4.5]decane-8-carboxylate (1.42 g) obtained in Example 90b) in DMF (20 ml) under ice-cooling, the mixture was stirred at 0°C for 1 hour, and the 1:1 mixture (1.70 g) of 6-chloro-2-(3-chloropropyl)sulfonylnaphthalene and 6-chloro-2-(3-bromopropyl)sulfonylnaphthalene obtained in Example 90a) was added. The reaction mixture was stirred at room temperature for 6 days, diluted with water, and extracted with ethyl acetate. The extract was washed with water, dried over anhydrous magnesium sulfate, the solvent was distilled off, and the residue was purified by silica gel column to obtain the title compound as colorless prisms (1.26 g, 45%).
NMR (200MHz, CDCl₃) δ: 1.49 (9H, s), 1.67-1.73 (2H, m), 1.94-2.09 (4H, m), 2.89-3.02 (2H, m), 3.23 (2H, t, J = 7.5), 3.40 (2H, t, J = 7.5), 3.50 (2H, s), 4.14-4.23 (2H, m), 7.60 (1H, dd, J = 2.0 and 8.6), 7.87-8.00 (4H, m), 8.48 (1H, s).

### 90d) 4-[3-(6-Chloro-2-naphthyl)sulfonylpropyl]-1-thia-4,8-diazaspiro[4.5]decane-3-one trifluoroacetate

A solution of tert-butyl 4-[3-(6-chloro-2-naphthyl)sulfonylpropyl]-3-oxo-1-thia-4,8-diazaspiro[4.5]decane-8-carboxylate (0.10 g) obtained in Example 90c) in trifluoroacetic acid (1 ml) was stirred at room temperature for 1 hour, concentrated under reduced pressure, and the residue was recrystallized from ethanol/ether to obtain the title compound as a colorless powder (0.11 g, quantitative).
NMR (DMSO-d₆) δ: 1.89-1.96 (2H, m), 2.05-2.15 (2H, m), 2.58-2.69 (2H, m), 3.11-3.29 (4H, m), 3.44-3.58 (6H, m), 7.60 (1H, dd, J = 2.0 and 8.6), 7.87-8.00 (4H, m), 8.49 (1H, s).

### 90e) 4-[3-(6-Chloro-2-naphthyl)sulfonylpropyl]-8-(2-methyl-4-pyridyl)-1-thia-4,8-diazaspiro[4.5]decane-3-one

A solution of 4-[3-(6-chloro-2-naphthyl)-sulfonylpropyl]-1-thia-4,8-diazaspiro[4.5]decane-3-one trifluoroacetate (0.21 g) obtained in Example 90d), 4-chloro-2-methylpyridine (65 mg) and triethylamine (65 mg) in ethanol (5 ml) was heated at 140°C for 3 hours in a sealed tube. The reaction mixture was concentrated under reduced pressure, and the residue was purified by silica gel column to obtain the title compound as a colorless powder (0.16 g, 83%),
NMR (CDCl₃) δ: 1.80-1.-86 (2H, m), 2.08-2.21 (2H, m), 2.48 (3H, s), 3.03-3.15 (2H, m), 3.23 (2H, t, J = 7.7), 3.38 (2H, t, J = 7.4), 3.54 (2H, s), 3.87-3.94 (2H, m), 6.51-6.57 (2H, m), 7.58 (1H, dd, J = 2.2 and 8.8), 7.86-7.96 (4H, m), 8.22 (1H, d, J = 6.0), 8.47 (1H, s).

| Elemental Analysis: C₂₅H₂₈N₃ClO₃S₂ | | | |
|---|---|---|---|
| Calcd (%) | C, 57.84; | H, 5.57; | N, 7.84 |
| Found (%) | C, 58.01; | H, 5.43; | N, 7.63 |

### Example 91

### 3-[3-(6-Chloro-2-naphthyl)sulfonylpropyl]-1-methyl-8-(2-methyl-4-pyridyl)-1,3,8-triazaspiro[4,5]decane-2,4-dione

### 91a) tert-Butyl 4-cyano-4-(methylamino)piperidine-1-carboxylate

N-Boc-4-piperidone (19.92 g) and methylamine (6.76 g) were dissolved in methanol (20 ml) and water (20 ml), and an aqueous solution (12 ml) of sodium cyanide (4.9 g) was added dropwise thereto with ice-cooling. The solution was stirred at room temperature for 18 hours, the reaction mixture was diluted with water, and extracted with ethyl acetate. The extract was washed successively with water and saturated brine, and dried over anhydrous sodium sulfate. The solvent was distilled off to obtain the title compound (25 g).
NMR (CDCl₃) δ: 1.45 (9H, s), 1.50-1.72 (2H, m), 2.53 (3H, s), 3.12-3.32 (2H, m), 3.80-4.00 (2H, m). IR (KBr): 2230, 1698, 1422 cm⁻¹.

### 91b) tert-Butyl 4-cyano-4-(1-methylureido)piperidine-1-carboxylate

A solution of potassium cyanide (6.55 g) in water (10 ml) was added dropwise to a solution of tert-butyl 4-cyano-4-(methylamino)piperidine-1-carboxylate (12.5 g) obtained in Example 91a) in acetic acid (30 ml) at room temperature. The reaction mixture was stirred at 50°C for 1 hour, diluted with water, and extracted with ethyl acetate. The extract was washed successively with water and saturated brine, and dried over anhydrous sodium sulfate. The solvent was distilled off, and the residue was purified by silica gel column to obtain the title compound as colorless crystals (6.7 g, 47%).
NMR (CDCl₃) δ: 1.46 (9H, s), 1.80-1.95(2H, m), 2.32-2.50 (2H, m), 2.93 (3H, s), 3.10-3.28 (2H, m), 4.05-4.28 (2H, m), 4.84 (2H, s). IR (KBr): 1696, 1420 cm⁻¹.

### 91c) tert-Butyl 1-methyl-2,4-dioxa-1,3,8-triazaspiro[4.5]decane-8-carboxylate

A mixture of tert-butyl 4-cyano-4-(1-methylureido)piperidine-1-carboxylate obtained in Example 91b) and 10% hydrochloric acid (10 ml) was stirred at room temperature for 10 minutes, diluted with water, adjusted to pH 3 with aqueous ammonia, and extracted with ethyl acetate. The extract was washed with water, and dried over anhydrous sodium sulfate. The solvent was distilled off to obtain the title compound as colorless crystals (2.17 g, 56%).
NMR (CDCl₃) δ: 1.24-1.32 (2H, m), 1.48 (9H, s), 1.66-1.75 (2H, m), 1.80-1.93 (2H, m), 2.82 (3H, s), 3.40-3.59 (2H, m), 4.00-4.25 (2H, m), 8.03 (1H, s). IR (KBr): 1767, 1716, 1700 cm⁻¹.

### 91d) tert-Butyl 3-[3-(6-chloro-2-naphthyl)sulfonylpropyl]-1-methyl-2,4-dioxo-1,3,8-triazaspiro[4.5]decane-8-carboxylate

According to the same manner as that of Example 88a), the title compound was obtained as a colorless powder (71%) from tert-butyl 1-methyl-2,4-dioxa-1,3,8-triazaspiro[4.5]decane-8-carboxylate obtained in Example 91c) and 3-(6-chloro-2-naphthyl)sulfonylpropanol obtained in Example 18b).
NMR (CDCl₃) δ: 1.48 (9H, s), 1.50-1.60 (2H, m), 1.78-1.92(2H, m), 2.00-2.13 (2H, m), 2.81 (3H, s), 3.18-3.27 (2H, m), 3.35-3.53 (2H, m), 3.59 (2H, t, J = 6.8), 3.98-4.20 (2H, m), 7.60 (1H, dd, J = 2.0 and 8.8), 7.84-8.00 (4H, m), 8.47 (1H, s).

### 91e) 3-[3-(6-Chloro-2-naphthyl)sulfonylpropyl]-1-methyl-8-(2-methyl-4-pyridyl)-1,3,8-triazaspiro[4.5]decane-2,4-dione

tert-Butyl 3-[3-(6-chloro-2-naphthyl)sulfonylpropyl]-1-methyl-2,4-dioxo-1,3,8-triazaspiro[4.5]decane-8-carboxylate (0.39 g) obtained in Example 91d) was dissolved in toluene (2 ml) and trifluoroacetic acid (4 ml), the solution was stirred at room temperature for 2 hours, toluene was added to the reaction mixture, and concentrated to dryness. The residue was dissolved in ethanol (20 ml), triethylamine (0.7 ml) and 4-chloro-2-methylpyridine (0.25 g) were added, and the mixture was heated at 150°C for 16 hours in a sealed tube. The reaction mixture was concentrated, and the residue was made alkaline with an aqueous sodium carbonate solution, followed by extraction with ethyl acetate. The extract was dried over anhydrous sodium sulfate. The solvent was distilled off, and the residue was purified by silica gel column to obtain the title compound as a colorless powder (0.27 g, 67%).
NMR (CDCl₃) δ: 1.60-1.75 (2H, m), 1.90-2.15 (4H, m), 2.47 (3H, s), 2.79 (3H, s), 3.20-3.30 (2H, m), 3.62 (2H, t, J = 6.4), 3.75-3.90 (2H, m), 6.50-6.59 (2H, m), 7.60 (2H, dd, J = 2.0 and 8.8), 7.85-8.00 (4H, m), 8.19 (1H, d, J = 6.0), 8.47 (1H, s).

| Elemental Analysis: C₂₇H₂₉N₄ClO₄S | | | |
|---|---|---|---|
| Calcd (%) | C, 58.00; | H, 5.59; | N, 10.02 |
| Found (%) | C, 58.22; | H, 5.55; | N, 9.88 |

### Example 92

### Ethyl 4-[N-[3-(6-chloro-2-naphthyl)sulfonylpropyl]-N-methylcarbamoyl]-1-(2-methyl-4-pyridyl)-4-piperidylcarbamate

### 92a) 4-Amino-1-(tert-butoxycarbonyl)-4-piperidinecarboxylic acid

Di-tert-butyl dicarbonate (14.4 g) was added dropwise to a solution of tert-butyl 2,4-dioxo-1,3,8-triazaspiro[4.5]decane-8-carboxylate (8.08 g), N,N-dimethylaminopyridine (37 mg) and triethylamine (3.05 g) in THF (200ml), the mixture was stirred at room temperature for 5 hours, and the reaction mixture was concentrated under reduced pressure. Water was added to the residue, and the precipitated crystals were filtered and washed with water. The resulting solid was dissolved in dimethoxyethane (200 ml), a 1 N aqueous sodium hydroxide solution (250 ml), followed by stirring at room temperature for 24 hours. The insolubles were filtered, the filtrate was washed with ether, and adjusted to pH 5 with an aqueous potassium hydrogensulfate solution. The precipitated crystals were filtered, washed with water, and dried to obtain the title compound (5.63 g, 76%). The filtrate was concentrated to obtain further the title compound (1.17 g, 16%).
NMR (CDCl₃+DCl one drop) δ: 1.47 (9H, s), 1.68-1.90 (2H, m), 2.10-2.20 (2H, m), 3.45-3.80 (4H, m).

### 92b) 1-(tert-Butoxycarbonyl)-4- (ethoxycarbonylamino)-4-piperidinecarboxylic acid

Ethyl chlorocarbonate (0.95 ml) was added dropwise to a solution of 4-amino-1-(tert-butoxycarbonyl)-4-piperidinecarboxylic acid (0.98 g) obtained in Example 92a) and pyridine (0.8 g) in methylene chloride (50 ml) at -30°C, and the mixture was stirred further at room temperature for 5 hours. The reaction mixture was concentrated, the residue was diluted with water, adjusted to pH 3 with an aqueous potassium hydrogensulfate, and extracted with ethyl acetate. The extract was washed with water, and dried over anhydrous sodium sulfate. The solvent was distilled off under reduced pressure, and the residue was purified by silica gel column to obtain colorless oil (0.9 g).

A 1 N aqueous sodium hydroxide solution (6 ml) was added to a solution of the resulting oil in ethanol (5 ml), the mixture was stirred at room temperature for 18 hours, and the reaction mixture was concentrated under reduced pressure. The residue was diluted with water, the mixture was adjusted to pH 3 with an aqueous potassium hydrogensulfate solution, and extracted with ethyl acetate. The extract was washed with water, and dried over anhydrous sodium sulfate. The solvent was distilled off to obtain the title compound (0.61 g, 48%).
NMR (CDCl₃) δ: 1.25 (3H, t, J = 7.1), 1.46 (9H, s), 1.90-2.20 (4H, m), 3.00-3.22 (2H, m), 3.76-3.98 (2H, m), 4.13 (2H, q, J = 7.1), 5.11 (1H, bs), 7.47 (1H, bs). IR (KBr): 1698, 1674, 1534, 1433 cm⁻¹.

### 92c) tert-Butyl 4-[N-[3-(6-chloro-2-naphtyl)sulfonylpropyl]-N-methylcarbamoyl]-4-(ethoxycarbonylamino)piperidine-1-carboxylate

According to the same manner as that of Example 30b), the title compound was obtained as a colorless powder (94%) from 1-(tert-butoxycarbonyl)-4-(ethoxycarbonylamino)-4-piperidinecarboxylic acid obtained in Example 92b) and 2-(2-methylaminoethylsulfonyl)-6-chloronaphthalene.
NMR (CDCl₃) δ: 1.23 (3H, t, J = 7.0), 1.44 (9H, s), 1.70-1.92 (2H, m), 1.92-2.10 (4H, m), 3.12 (3H, s), 3.12-3.85 (8H, m), 4.12 (2H, q, J = 7.0), 4.97 (1H, s), 7.57 (1H, dd, J = 1.8 and 8.8), 7.84-8.00 (4H, m), 8.49 (1H, s).

### 92d) Ethyl 4-[N-[3-(6-chloro-2-naphthyl)sulfonylpropyl]-N-methylcarbamoyl]-1-(2-methyl-4-pyridyl)-4-piperidylcarbamate

According to the same manner as that or Example 91e), the title compound was obtained as a colorless powder (94%) from tert-butyl 4-[N-[3-(6-chloro-2-naphthyl)-sulfonylpropyl]-N-methylcarbamoyl]-4-(ethoxycarbonylamino)piperidine-1-carboxylate obtained in Example 92c).
NMR (CDCl₃) δ: 1.23 (3H, t, J = 7.1), 1.88-2.20 (4H, m), 2.20-2.42 (2H, m), 2.44 (3H, s), 3.10-3.40 (4H, m), 3.15 (3H, s), 3.42-3.66 (4H, m), 4.11 (2H,q, J = 7.1), 5.06 (1H, s), 6.40-6.55 (2H, m), 7.57 (1H, dd, J = 1.8 and 8.8), 7.83-8.00 (4H, m), 8.16 (1H, d, J = 5.8), 8.49 (1H, s).

| Elemental Analysis: C₂₉H₃₅ClN₄O₅S·0.5H₂O | | | |
|---|---|---|---|
| Calcd (%) | C, 58.43; | H, 6.09; | N, 9.40 |
| Found (%) | C, 58.65; | H, 6.05; | N, 9.14 |

### Example 93

### 4-Amino-N-[3-(6-chloro-2-naphthyl)sulfonylpropyl]-N-methyl-1-(2-methyl-4-pyridyl)-4-piperidylcarboxamide

### 93a) 1-(tert-Butoxycarbonyl)-4-(tert-butoxycarbonylamino)-4-piperidinecarboxylic acid

Di-tert-butyl dicarbonate (1.09 g) was added dropwise to a solution of 4-amino-1-(tert-butoxycarbonyl)-4-piperidinecarboxylic acid (2.45 g) obtained in Example 92a), and the mixture was stirred at room temperature for 5 hours. The water was added to the reaction mixture, and the mixture was adjusted to pH 3 with an aqueous potassium hydrogensulfate solution, and extracted with ethyl acetate. The extract was washed with water, and dried over anhydrous sodium sulfate. The solvent was distilled off to obtain the title compound (0.61 g, 74%).
NMR (CDCl₃) δ: 1.44 (9H, s), 1.46 (9H, s), 1.90-2.10 (2H, m), 2.60-2.90 (2H, m), 3.08-3.22 (2H, m), 3.75-3.90 (2H, m).

### 93b) tert-Butyl 4-(tert-butoxycarbonylaimno)-4-[N-[3-(6-chloro-2-naphthyl)sulfonylpropyl]-N-methylcarbamoyl]piperidine-1-carboxylate

According to the same manner as that of Example 30b), the title compound was obtained as a colorless powder (84%) from 1-(tert-butoxycarbonyl)-4-(tert-butoxycarbonylamino)-4-piperidinecarboxylic acid obtained in Example 93a) and 2-(2-methylaminoethylsulfonyl)-6-chloronaphthalene.
NMR (CDCl₃) δ: 1.44 (18H, s), 1.70-1.82 (2H, m), 2.00-2.20 (4H, m), 3.10-3.80 (8H, m), 3.15 (3H, s), 4.83 (1H, s), 7.55-7.62 (1H, m), 7.85-8.00 (4H, m), 8.47 (1H, s).

### 93c) 4-Amino-N-[3-(6-chloro-2-naphthyl)sulfonylpropyl]-N-methyl-1-(2-methyl-4-pyridyl)-4-piperidylcarboxamide

According to the same manner as that of Example 91e), the title compound was obtained as a colorless powder (63%) from tert-butyl 4-(tert-butoxycarbonylamino)-4-[N-[3-(6-chloro-2-naphthyl)sulfonylpropyl]-N-methylcarbamoyl]piperidine-1-carboxylate obtained in Example 93b).
NMR (CDCl₃) δ: 1.50-1.70 (2H, m), 1.96-2.10 (2H, m), 2.20-2.30 (2H, m), 2.44 (3H, s), 3.10-3.20 (2H, m), 3.25 (3H, brs), 3.30-3.45 (4H, m), 3.48-3.70 (2H, m), 6.45-6.55 (2H, m), 7.59 (1H, dd, J = 2.0 and 8.8), 7.85-8.00 (4H, m), 8.15 (1H, d, J = 6.4), 8.46 (1H, s).

| Elemental Analysis: C₂₆H₃₁ClN₄O₃S·0.75H₂O | | | |
|---|---|---|---|
| Calcd (%) | C, 59.08; | H, 6.20; | N, 10.60 |
| Found (%) | C, 59.09; | H, 6.17; | N, 10.52 |

### Example 94

### 3-(7-Chloro-2-naphthyl)sulfonyl-N-methyl-N-[1-(2-methyl-4-pyridyl)-4-piperidyl]propanamide

### 94a) 3-(7-Chloro-2-naphthyl)sulfonylpropanecarboxylic acid

According to the same manner as that of Example 76a), the title compound was obtained as a colorless solid (53%) from 7-chloronaphthalene-2-sulfonyl chloride.
NMR (DMSO-d₆) δ: 2.58 (2H, t, J = 7.3), 3.62 (2H, d, J = 7.3), 7.78 (1H, dd, J = 2.2 and 8.8), 7.95 (1H, dd, J = 2.0 and 8.8), 8.16 (1H, d, J = 8.8), 8.25 (1H, d, J = 8.8), 8.40 (1H, d, J = 2.0), 8.59 (1H, s).

### 94b) 3-(7-Chloro-2-naphthyl)sulfonyl-N-methyl-N-[1-(2-methyl-4-pyridyl)-4-piperidyl]propanamide

According to the same manner as that of Example 76c), the title compound was obtained as colorless crystals (58%) from 3-(7-chloro-2-naphthyl)sulfonylpropanecarboxylic acid obtained in Example 94a) and 4-methylamino-1-(4-pyridyl)piperidine obtained in Example 30a).
NMR (CDCl₃) δ: 1.50-1.95 (4H, m), 2.45 and 2.48 (3H, s), 2.76 and 2.83 (3H, s), 2.80-3.05 (4H, m), 3.57 (2H, t, J = 7.7), 3.93 (2H, m), 4.59 (1H, m), 6.45-6.60 (2H, m), 7.64 (1H, dd, J = 2.2 and 8.8), 7.85-8.05 (4H, m), 8.15 (1H, d, J = 6.0), 8.42 (1H, s).

| Elemental Analysis: C₂₅H₂₈N₃O₃SCl·0.5H₂O | | | |
|---|---|---|---|
| Calcd (%) | C, 60.66; | H, 5.90; | N, 8.49 |
| Found (%) | C, 60.82; | H, 5.72; | N, 8.53 |

### Example 95

### 3-(5-Chloro-2-naphthyl)sulfonyl-N-methyl-N-[1-(2-methyl-4-pyridyl)-4-piperidyl]propanamide

### 95a) 3-(5-Chloro-2-naphthyl)sulfonylpropanecarboxylic acid

According to the same manner as that of Example 76a), the title compound was obtained as a colorless solid (64%) from 5-chloronaphthalane-2-sulfonyl chloride.
NMR (DMSO-d₆) δ: 2.59 (2H, t, J = 7.3), 3.64 (2H, d, J = 7.3), 7.71 (1H, t, J = 8.0), 7.96 (1H, d, J = 7.8), 8.09 (1H, dd, J = 2.0 and 8.8), 8.28 (1H, d, J = 8.4), 8.42 (1H, d, J = 8.8), 8.71 (1H, d, J = 2.0).

### 95b) 3-(5-Chloro-2-naphthyl)sulfonyl-N-methyl-N-[1-(2-methyl-4-pyridyl)-4-piperidyl]propanamide

According to the same manner as that of Example 76c), the title compound was obtained as a colorless powder (64%) from 3-(5-chloro-2-naphthyl)sulfonylpropanecarboxylic acid obtained in Example 95a) and 4-methylamino-1-(4-pyridyl)piperidine obtained in Example 30a).
NMR (CDCl₃) δ: 1.50-1.95 (4H, m), 2.45 and 2.47 (3H, s), 2.76 and 2.82 (3H, s), 2.80-3.05 (4H, m), 3.60 (2H, t, J = 7.5), 3.93 (2H, m), 4.58 (1H, m), 6.45-6.60 (2H, m), 7.58 (1H, t, J = 8.0), 7.80 (1H, d, J = 7.6), 7.95 (1H, d, J = 8.4), 8.01 (1H, dd, J = 1.8 and 8.8), 8.15 (1H, d, J = 5.8), 8.48 (1H, d, J = 8.8), 8.53 (1H, d, J = 1.8).

| Elemental Analysis: C₂₅H₂₈N₃O₃SCl·0.5H₂O | | | |
|---|---|---|---|
| Calcd (%) | C, 60.66; | H, 5.90; | N, 8.49 |
| Found (%) | C, 60.54; | H, 6.15; | N, 8.56 |

### Example 96

### 3-(6-Methoxy-2-naphthyl)sulfonyl-N-methyl-N-[1-(2-methyl-4-pyridyl)-4-piperidyl]propanamide

### 96a) 3-(6-Methoxy-2-naphthyl)sulfonylpropanecarboxylic acid

According to the same manner as that of Example 76a), the title compound was obtained as a colorless solid (68%) from 6-methoxynaphthalene-2-sulfonyl chloride.
NMR (DMSO-d₆) δ: 2.56 (2H, t, J = 7.4), 3.57 (2H, d, J = 7.4), 3.93 (3H, s), 7.34 (1H, dd, J = 2.2 and 8.8), 7.50 (1H, d, J = 2.2), 7.84 (1H, d, J = 8.8), 8.06 (1H, d, J = 8.8), 8.14 (1H, d, J = 8.8), 8.48 (1H, s).

### 96b) 3-(6-Methoxy-2-naphthyl)sulfonyl-N-methyl-N-[1-(2-methyl-4-pyridyl)-4-piperidyl]propanamide

According to the same manner as that of Example 76c), the title compound was obtained as colorless crystals (63%) from 3-(6-methoxy-2-naphthyl)sulfonylpropanecarboxylic acid obtained in Example 96a) and 4-methylamino-1-(4-pyridyl)piperidine obtained in Example 30a).
NMR (CDCl₃) δ: 1.50-1.95 (4H, m), 2.45 and 2.47 (3H, s), 2.75 and 2.82 (3H, s), 2.80-3.05 (4H, m), 3.56 (2H, t, J = 7.7), 3.93 (2H, m), 3.97 (3H, s), 4.60 (1H, m), 6.45-6.60 (2H, m), 7.21 (1H, d, J = 2.2), 7.29 (1H, dd, J = 2.2 and 8.8), 7.80-7.95 (3H, m), 8.15 and 8.19 (1H, each d, J = 6.0), 8.41 (1H, s).

| Elemental Analysis: C₂₆H₃₁N₃O₄S·0.5H₂O | | | |
|---|---|---|---|
| Calcd (%) | C, 63.65; | H, 6.57; | N, 8.56 |
| Found (%) | C, 63.87; | H, 6.40; | N, 8.61 |

### Example 97

### 3-(6-Fluoro-2-naphthyl)sulfonyl-N-methyl-N-[1-(2-methyl-4-pyridyl)-4-piperidyl]propanamide

### 97a) 3-(6-Fluoro-2-naphthyl)sulfonylpropanecarboxylic acid

According to the same manner as that of Example 76a), the title compound was obtained as colorless crystals (21%) from 6-fluoronaphthalene-2-sulfonyl chloride.
NMR (DMSO-d₆) δ: 2.57 (2H, t, J = 7.3), 3.61 (2H, d, J = 7.3), 7.65 (1H, dt, J = 2.6 and 8.8), 7.88-8.00 (2H, m), 8.19 (1H, d, J = 8.8), 8.35 (1H, dd, J = 5.8 and 9.2), 8.64 (1H, s).

### 97b) 3-(6-Fluoro-2-naphthyl)sulfonyl-N-methyl-N-[1-(2-methyl-4-pyridyl)-4-piperidyl]propanamide

According to the same manner as that of Example 76c), the title compound was obtained as colorless crystals (67%) form 3-(6-fluoro-2-naphthyl)sulfonylpropanecarboxylic acid obtained in Example 97a) and 4-methylamino-1-(4-pyridyl)piperidine obtained in Example 30a).
NMR (CDCl₃) δ: 1.50-1.95 (4H, m), 2.44 and 2.47 (3H, s), 2.75 and 2.83 (3H, s), 2.80-3.05 (4H, m), 3.58 (2H, t, J = 7.7), 3.93 (2H, m), 4.60 (1H, m), 6.45-6.60 (2H, m), 7.44 (1H, dt, J = 2.4 and 8.6), 7.57 (1H, dd, J = 2.2 and 9.6), 7.88-8.08 (3H, m), 8.14 (1H, d, J = 5.8), 8.51 (1H, s).

| Elemental Analysis: C₂₅H₂₈N₃O₃SF·0.25H₂O | | | |
|---|---|---|---|
| Calcd (%) | C, 63.34; | H, 6.06; | N, 8.86 |
| Found (%) | C, 63.22; | H, 6.17; | N, 8.59 |

### Example 98

### 3-(6-Methyl-2-naphthyl)sulfonyl-N-methyl-N-[1-(2-methyl-4-pyridyl)-4-piperidyl]propanamide

### 98a) 3-(6-Methyl-2-naphthyl)sulfonylpropanecarboxylic acid

According to the same manner as that of Example 76a), the title compound was obtained as a colorless solid (50%) from 6-methylnaphthalene-2-sulfonyl chloride.
NMR (DMSO-d₆) δ: 2.54 (3H, s), 2.56 (2H, t, J = 7.4), 3.59 (2H, d, J = 7.4), 7.56 (1H, dd, J = 1.4 and 8.4), 7.86 (1H, dd, J = 2.0 and 8.6), 7.87 (1H, s), 8.08 (1H, d, J = 8.6), 8.12 (1H, d, J = 8.4), 8.52 (1H, s).

### 98b) 3-(6-Methyl-2-naphthyl)sulfonyl-N-methyl-N-[1-(2-methyl-4-pyridyl)-4-piperidyl]propanamide

According to the same manner as that of Example 76c), the title compound was obtained as colorless crystals (58%) from 3-(6-methyl-2-naphthyl)sulfonylpropanecarboxylic acid obtained in Example 98a) and 4-methylamino-1-(4-pyridyl)piperidine obtained in Example 30a).
NMR (CDCl₃) δ: 1.50-1.95 (4H, m), 2.44 and 2.46 (3H, s), 2.57 (3H, s), 2.73 and 2.80 (3H, s), 2.80-3.03 (4H, m), 3.57 (2H, t, J = 7.7), 3.91 (2H, m), 4.58 (1H, m), 6.45-6.60 (2H, m), 7.48 (1H, dd, J = 1.6 and 8.4), 7.71 (1H, s), 7.80-7.95 (3H, m), 8.14 (1H, d, J = 6.2), 8.45 (1H, s).

| Elemental Analysis: C₂₆H₃₁N₃O₃S | | | |
|---|---|---|---|
| Calcd (%) | C, 67.07; | H, 6.71; | N, 9.02 |
| Found (%) | C, 66.77; | H, 6.64; | N, 8.97 |

### Example 99

### 4-[3-(6-Chloro-2-naphthyl)sulfonylpropanoyl]-8-(2-methyl-4-pyridyl)-1-thia-4,8-diazaspiro[4,5]decane

### 99a) 8-(2-Methyl-4-pyridyl)-1-thia-4,8-diazaspiro[4,5]decane

1-(2-Methyl-4-pyridyl)-4-piperidone (0.80 g) obtained in Example 42a) and cysteamine hydrochloride (0.72 g) were added to ethanol (15 ml), the mixture was refluxed for 5 hours, and the solvent was distilled off. The residue was diluted with water, made alkaline with potassium carbonate, and extracted with ethyl acetate and THF. The extract was dried over anhydrous magnesium sulfate, and concentrated under reduced pressure. The residue was purified by silica gel column, and recrystallized from ethyl acetate/hexane to obtain the title compound (0.52 g, 50%).
NMR (CDCl₃) δ: 1.96-2.01 (4H, m), 2.45 (3H, s), 3.02 (2H, t, J = 6.2), 3.17-3.44 (4H, m), 3.64-3.76 (2H, m), 6.50-6.57 (2H, m), 8.17 (1H, d, J = 6.0)

### 99b) 4-[3-(6-Chloro-2-naphthyl)sulfonylpropanoyl]-8-(2-methyl-4-pyridyl)-1-thia-4,8-diazaspiro[4,5]decane

2-Chloro-1,3-dimethylimidazolium (0.19 g) was added to a solution of 3-(6-chloro-2-naphthyl)sulfonylpropane-carboxylic acid (0.30 g) obtained in Example 27b), 8-(2-methyl-4-pyridyl)-1-thia-4,8-diazaspiro[4,5]decane (0.25 g) obtained in Example 99a) and diisopropylethylamine (0.14 g) in methylene chloride (10 ml), and the mixture was stirred at room temperature for 9 hours. The reaction mixture was washed with water, and dried over anhydrous magnesium sulfate. The solvent was concentrated under reduced pressure, and the residue was purified by silica gel column purified to obtain the title compound as a colorless powder (0.13 g, 24%).
NMR (CDCl₃) δ: 1.57 (2H, d, J = 9.7), 2.45 (3H, s), 2.79-3.08 (8H, m), 3.53 (2H, t, J = 7.5), 3.84 (2H, d, J = 9.7), 3.96 (2H, t, J = 6.1), 6.42-6.47 (2H, m), 7.59 (1H, dd, J = 2.2 and 8.8), 7.91-7.96 (4H, m), 8.11 (1H, d, J = 6.2), 8.45 (1H, s).

| Elemental Analysis: C₂₆H₂₈N₃O₃S₂Cl·H₂O | | | |
|---|---|---|---|
| Calcd (%) | C, 56.97; | H, 5.52; | N, 7.67 |
| Found (%) | C, 57.18; | H, 5.58; | N, 7.53 |

### Example 100

### 3-(6-Chloro-2-quinolyl)sulfonyl-N-methyl-N-[1-(2-methyl-4-pyridyl)-4-piperidyl]propanaimde hydrochloride

### 100a) Methyl 3-(6-chloro-2-quinolyl)thiopropanecarboxylate

2,6-Dichloroquinoline (G. B. Bachman et al., J. Org. Chem., 1944, *9*, 302) (1.35 g), methyl 3-mercaptopropionate (0.98 g) and potassium carbonate (1.03 g) were added to DMF (10 ml), and the mixture was stirred at 60°C for 13 hours. The reaction mixture was diluted with water, and extracted with ethyl acetate. The extract was washed with water, and dried over anhydrous magnesium sulfate. The solvent was distilled off under reduced pressure, and the residue was purified by silica gel column to obtain the title compound as a pale yellow solid (1.25 g, 65%).
NMR (CDCl₃) δ: 2.89 (2H, t, J = 7.0), 3.57 (2H, t, J = 7.0), 3.72 (3H, s), 7.20 (1H, d, J = 8.8), 7.57 (1H, dd, J = 2.2 and 8.8), 7.34 (1H, d, J = 2.2), 7.80 (1H, d, J = 8.8), 7.86 (1H, d, J = 8.8).

### 100b) Methyl 3-(6-chloro-2-quinolyl)sulfonylpropane-carboxylate

According to the same manner as that of Example 7d), the title compound was obtained as colorless leaflets (59%) from methyl 3-(6-chloro-2-quinolyl)thiopropane-carboxylate obtained in Example 100a).
NMR (CDCl₃) δ: 2.93 (2H, d, J = 7.8), 3.67 (3H, s), 3.88 (2H, t, J = 7.8), 7.80 (1H, dd, J = 2.2 and 9.1), 7.94 (1H, d, J = 2.2), 8.14 (1H, d, J = 8.6), 8.16 (1H, d, J = 9.1), 8.37 (1H, d, J = 8.6).

### 100c) 3-(6-chloro-2-quinolyl)sulfonylpropanecarboxylic acid

According to the same manner as that of Example 38c), the title compound (78%) was obtained from methyl 3-(6-chloro-2-quinolyl)sulfonylpropanecarboxylate obtained in Example 100b).
NMR (DMSO-d₆) δ: 2.72 (2H, t, J = 7.3), 3.82 (2H, t, J = 7.3), 7.99 (1H, dd, J = 2.3 and 8.9), 8.16-8.25 (2H, m), 8.38 (1H, d, J = 2.3), 8.76 (1H, d, J = 8.9).

### 100d) 3-(6-Chloro-2-quinolyl)sulfonyl-N-methyl-N-[1-(2-methyl-4-pyridyl)-4-piperidyl]propanamide hydrochloride

According to the same manner as that of Example 99b), the title compound was obtained as a pale yellow powder (7.8%) from 3-(6-chloro-2-quinolyl)sulfonylpropane-carboxylic acid obtained in Example 100b) and 4-methylamino-1-(4-pyridyl)piperidine obtained in Example 30a).
NMR (DMSO-d₆) δ: 1.58-1.77 (4H, m), 2.46 (3H, s), 2.55 (1H, s, 1/3Me), 2.76 (2H, s, 2/3Me), 2.83 (4/3H, t, J = 7.5), 3.01 (2/3H, t, J = 6.9), 3.1-3.3 (2H, m), 3.80-3.93 (2H, m), 4.0-4.4 (2H, m), 4.51 (1H, m), 7.06-7.12 (2H, m), 7.99 (1H, dd, J = 2.2 and 9.2), 8.12-8.25 (3H, m), 8.39 (1H, d, J = 2.2), 8.76 (1H, d, J = 8.6).

| Elemental Analysis: C₂₄H₂₇N₄O₃SCl·HCl·0.7H₂O | | | |
|---|---|---|---|
| Calcd (%) | C, 53.69; | H, 5.80; | N, 10.09 |
| Found (%) | C, 53.77; | H, 5.53; | N, 10.49 |

### Example 101

### 1-[3-(6-Chloro-2-naphthyl)sulfonylpropyl]-3-methyl-8-(2-methyl-4-pyridyl)-1,3,8-triazaspiro[4.5]decane-2,4-dione

### 101a) tert-Butyl 3-methyl-2,4-dioxo-1,3,8-triazaspiro[4.5]decane-8-carhoxylate

A mixture of tert-butyl 2,4-dioxo-1,3,8-triazaspiro[4.5]decane-8-carboxylate (2.92 g) and potassium carbonate (1.64 g) in DMF (40 ml) was stirred at room temperature for 10 minutes, methyl iodide (1 ml) was added, and the mixture was stirred for 17 hours. The solvent was distilled off under reduced pressure, the residue was diluted with water, and extracted with ethyl acetate. The extract was washed with water, and dried over anhydrous sodium sulfate. The solvent was distilled off under reduced pressure to obtain the title compound as colorless crystals (2.91 g, 95%).
NMR (CDCl₃) δ: 1.47 (9H, s), 1.55-1.65 (2H, m), 1.95-2.06 (2H, m), 3.02(3H, s), 3.15-3.30 (2H, m), 3.95-4.10 (2H, m), 7.08 (1H, s).

### 101b) tert-Butyl 1-(3-bromopropyl)-3-methyl-2,4-dioxo-1,3,8-triazaspiro[4.5]decane-8-carboxylate

Sodium hydride (60% oily: 0.2 g) was added to a solution of tert-butyl 3-methyl-2,4-dioxo-1,3,8-triazaspiro[4.5]decane-8-carboxylate (1.34 g) obtained in Example 101a) in DMF (15 ml), the mixture was stirred for 1 hour, 1,3-dibromopropane (1.43 g) was added, and the mixture was stirred for 4 hours. The reaction mixture was diluted with water, and extracted with ethyl acetate. The extract was washed with water, and dried over anhydrous sodium sulfate. The solvent was distilled off, and the residue was purified by silica gel column to obtain the title compound (1.35 g, 70%).
NMR (CDCl₃) δ: 1.48 (9H, s), 1.58-1.68 (2H, m), 1.80-1.93 (2H, m), 2.10-2.20 (2H, m), 3.00 (3H, s), 3.34 (2H, t, J = 7.2), 3.40-3.58 (2H, m), 3.59 (2H, t, J = 6.0), 4.00-4.20 (2H, m).

### 101c) tert-Butyl 1-[3-(6-chloro-2-naphthyl)sulfonylpropyl]-3-methyl-2,4-dioxa-1,3,8-triazaspiro[4.5]decane-8-carboxylate

According to the same manner as that of Example 3a), tert-butyl 1-[3-(6-chloro-2-naphthyl)thiopropyl]-3-methyl-2,4-dioxa-1,3,8-triazaspiro[4.5]decane-8-carboxylate was obtained from tert-butyl 1-(3-bromopropyl)-3-methyl-2,4-dioxo-1,3,8-triazaspiro[4.5]decane-8-carboxylate obtained in Example 101b) and 6-chloro-2-mercaptonaphthalene obtained in Example 1d). The present compound was oxidized with mCPBA as in Example 7d) to obtain the title compound (0.71 g, 64%).
NMR (CDCl₃) δ: 1.50 (9H, s), 1.57-1.66 (2H, m), 1.75-1.90 (2H, m), 2.04-2.16 (2H, m), 2.97 (3H, s), 3.24(2H, d, J = 7.4), 3.38(2H, t, J = 7.2), 3.40-3.55 (2H, m), 4.00-4.25 (2H, m), 7.60 (1H, dd, J = 1.6 and 8.4), 7.76-8.00 (4H, m), 8.48 (1H, s).

### 101d) 1-[3-(6-Chloro-2-naphthyl)sulfonylpropyl]-3-methyl-8-(2-methyl-4-pyridyl)-1,3,8-triazaspiro[4.5]decane-2,4-dione

According to the same manner as that of Example 91c), the title compound was obtained as a colorless powder (0.76 g, 88%) from the tert-butyl 1-[3-(6-chloro-2-naphthyl)sulfonylpropyl]-3-methyl-2,4-dioxa-1,3,8-triazaspiro[4.5]decane-8-carboxylate obtained in Example 101c).
NMR (CDCl₃) δ: 1.65-1.85 (2H, m), 1.89-2.00 (2H, m), 2.05-2.15 (2H, m), 2.47 (3H, s), 3.00 (3H, s), 3.22 (2H, t, J = 7.4), 3.33 (2H, t, J = 7.4), 3.60-3.72 (2H, m), 3.78-3.87 (2H, m), 6.40-6.60 (2H, m), 7.58 (1H, dd, J = 2.0 and 8.8), 7.70-8.00 (4H, m), 8.20 (1H, d, J = 6.0), 8.45 (1H, s).

| Elemental Analysis: C₂₇H₂₉ClN₄O₄S·0.5MeOH | | | |
|---|---|---|---|
| Calcd (%) | C, 59.29; | H, 5.61; | N, 10.06 |
| Found (%) | C, 59.32; | H, 5.77; | N, 10.16 |

### Example 102

### 4-[3-(6-Chloro-2-naphthyl)sulfonylpropyl]-8-(2-methyl-4-pyridyl)-1-thia-4,8-diazaspiro[4.5]decane-3-one 1-oxide

### 102a) tert-Butyl 4-[3-(6-chloro-2-naphthyl)sulfonylpropyl]-3-oxo-1-thia-4,8-diazaspiro[4.5]decane-8-carboxylate 1-oxide

tert-Butyl 4-[3-(6-chloro-2-naphthyl)sulfonylpropyl]-3-oxo-1-thia-4,8-diazaspiro[4.5]decane-8-carboxylate obtained in Example 90c) was oxidized using chloroform as the reaction solvent as in Example 7d) to obtain the title compound (97%) as a colorless powder.
NMR (CDCl₃) δ: 1.34-1.41 (1H, m), 1.51 (9H, s), 1.99-2.30 (5H, m), 2.98-3.71 (8H, m), 4.21-4.35 (2H, m), 7.60 (1H, dd, J = 2.0 and 8.6), 7.86-8.01 (4H, m), 8.48 (1H, s).

### 102b) 4-[3-(6-Chloro-2-naphthyl)sulfonylpropyl]-8-(2-methyl-4-pyridyl)-1-thia-4,8-diazaspiro[4.5]decane-3-one 1-oxide

A 4 N hydrogen chloride in ethyl acetate (10 ml) was added to a solution of tert-butyl 4-[3-(6-chloro-2-naphthyl)sulfonylpropyl]-3-oxo-1-thia-4,8-diazaspiro[4.5]decane-8-carboxylate 1-oxide (0.30 g) obtained in Example 102a) in ethyl acetate (5 ml), and the mixture was stirred at room temperature for 1 hour. The residue obtained by distillation of the solvent under the reduced pressure, 4-chloro-2-methylpyridine (69 mg) and triethylamine (109 mg) were added to ethanol (10 ml), which was heated at 150°C for 15 hours in a sealed tube. The reaction mixture was concentrated under reduced pressure, and the residue was purified by silica gel column to obtain the title compound as a colorless powder (70 mg, 24%).
NMR (CDCl₃) δ: 1.75-1.93 (2H, m), 2.07-2.31 (4H, m), 2.48 (3H, s), 2.93-3.75 (8H, m), 3.88-3.95 (2H, m), 6.51-6.58 (2H, m), 7.57 (1H, dd, J = 2.0 and 9.0), 7.85-7.95 (4H, m), 8.22 (1H, d, J = 6.2), 8.47 (1H, br s).

| Elemental Analysis: C₂₆H₂₈N₃ClO₄S₂·0.5Et₂O | | | |
|---|---|---|---|
| Calcd (%) | C, 57.67; | H, 5.70; | N, 7.21 |
| Found (%) | C, 57.88; | H, 5.64; | N, 7.04 |

### Example 103

### 4-[3-(6-Chloro-2-napthyl)sulfonylpropyl]-8-(2-methyl-4-pyridyl)-1-thia-4,8-diazaspiro[4.5]decane-3-one 1,1-dioxide

### 103a) tert-Butyl 4-[3-(6-chloro-2-naphthyl)sulfonylpropyl]-3-oxo-1-thia-4,8-diazaspiro[4.5]decane-8-carboxylate 1,1-dioxide

tert-Butyl 4-[3-(6-chloro-2-naphthyl)sulfonylpropyl]-3-oxo-1-thia-4,8-diazaspiro[4.5]decane-8-carboxylate obtained in Example 90c) was oxidized as in Example 7d) using chloroform as the reaction solvent and using 6.4 equivalents of mCPBA to obtain the title compound (45%) as a colorless powder.
NMR (CDCl₃) δ: 1.45 (9H, s), 1.93-2.28 (6H, m), 3.17-3.55 (6H, m), 3.80 (2H, s), 4.18-4.24 (2H, m), 7.56-8.07 (5H, m), 8.48 (1H, s).

### 103b) 4-[3-(6-Chloro-2-naphthyl)sulfonylpropyl]-8-(2-methyl-4-pyridyl)-1-thia-4,8-diazaspiro[4.5]decane-3-one 1,1-dioxide

According to the same manner as that or Example 83c), the title compound was obtained as a brown powder (14%) from tert-butyl 4-[3-(6-chloro-2-naphthyl)sulfonylpropyl]-3-oxo-1-thia-4,8-diazaspiro[4.5]decane-8-carboxylate 1,1-dioxide obtained in Example 103a).
NMR (CDCl₃) δ: 2.01-2.21 (4H, m), 2.35-2.43 (2H, m), 2.50 (3H, s), 3.18 (2H, t, J = 7.3), 3.52-3.60 (6H, m), 3.91-3.97 (2H, m), 6.54-6.58 (2H ,m), 7.59 (1H, dd, J = 2.0 and 9.0), 7.83-7.97 (4H, m), 8.22 (1H, d, J = 5.8), 8.45 (1H, d, J = 1.0).

### Example 104

### 2-[3-(6-Chloro-2-naphthyl)sulfonylpropyl]-8-(2-methyl-4-pyridyl)-2,8-diazaspiro[4.5]decane-1-one

### 104a) tert-Butyl 2-[3-(6-chloro-2-naphthyl)sulfonylpropyl]-1,3-oxo-2,8-diazaspiro[4.5]decane-8-carboxylate

1-Chloroethyl chlorocarbonate (0.75 ml) was added dropwise to a solution of 2-[3-(6-chloro-2-naphthyl)sulfonylpropyl]-2,8-diazaspiro[4.5]decane-1,3-dione (3.47g) obtained in Example 85c) in 1,2-dichloroethane (35 ml) at 0°C, the mixture was stirred at 70°C for 1 hour, methanol (35 ml) was added, and the mixture was further stirred at 70°C for 1 hour. Di-tert-butyl dicarbonate (1.67 ml) was added to a mixture of the residue obtained by concentration of the reaction mixture under reduced pressure, potassium carbonate (1.10 g), water (30 ml) and ethyl acetate (30 ml) were added, and the mixture was stirred at room temperature for 2 hours. The organic phase was separated, and dried over anhydrous magnesium sulfate. The solvent was distilled off under reduced pressure to obtain the title compound as the colorless powder (3.14 g, 89%).
NMR (CDCl₃) δ: 1.38-1.58 (2H, m), 1.47 (9H, s), 1.84-2.14 (4H, m), 2.59 (2H, s), 2.84-3.06 (2H, m), 3.21 (2H, t, J = 7.4), 3.61 (2H, t, J = 6.8), 3.92-4.16 (2H, m), 7.60 (1H, dd, J = 2.0 and 8.8), 7.87 (1H, dd, J = 1.8 and 8.8), 7.90-8.00 (3H, m), 8.45 (1H, d, J = 1.0).

### 104b) tert-Butyl 2-[3-(6-chloro-2-naphthyl)sulfonylpropyl]-1-oxo-2,8-diazaspiro[4.5]decane-8-carboxylate

Boron trifluoride diethyl ether complex (0.52 ml) was added to a solution of tert-butyl 2-[3-(6-chloro-2-naphthyl)sulfonylpropyl]-1,3-oxo-2,8-diazaspiro[4.5]decane-8-carboxylate (0.50 g) obtained in Example 104a) and sodium borohydride (0.12 g) in THF (10 ml) at 0°C, and the mixture was stirred at that temperature for 2 hours. The water was added to the reaction mixture slowly, which was extracted with ethyl acetate. The extract was washed with 5% potassium hydrogensulfate, and dried over anhydrous magnesium sulfate. The solvent was distilled off under reduced pressure, and the residue was purified by silica gel column to obtain the title compound as a colorless powder (0.38 g, 78%).
NMR (CDCl₃) δ: 1.20-1.50 (2H, m), 1.45 (9H, s), 1.68-1.90 (2H, m), 1.90-2.12 (4H, m), 2.86-3.06 (2H, m), 3.06-3.22 (2H, m), 3.26-3.44 (4H, m), 3.86-4.08 (2H, m), 7.60 (1H, dd, J = 2.0 and 8.8), 7.87 (1H, dd, J = 1.8 and 8.8), 7.88-8.00 (3H, m), 8.45 (1H, s).

### 104c) 2-[3-(6-Chloro-2-naphthyl)sulfonylpropyl]-8-(2-methyl-4-pyridyl)-2,8-diazaspiro[4.5]decane-1-one

According to the same manner as that of Example 83c), the title compound was obtained as a colorless powder (31%) from tert-butyl 2-[3-(6-chloro-2-naphthyl)sulfonylpropyl]-1-oxo-2,8-diazaspiro[4.5]decane-8-carboxylate obtained in Example 104b).
NMR (CDCl₃) δ: 1.40-1.58 (2H, m), 1.82-2.14 (6H, m), 2.45 (3H, s), 2.96-3.22 (4H, m), 3.28-3.46 (4H, m), 3.68-3.88 (2H, m), 6.44-6.58 (2H, m), 7.60 (1H, dd, J = 1.8 and 8.8), 7.88 (1H, dd, J = 1.4 and 8.8), 7.88-8.00 (3H, m), 8.15 (1H, d, J = 5.8), 8.46 (1H, d, J = 0.8).

| Elemental Analysis: C₂₇H₃₀N₃O₃SCl·1.5H₂O | | | |
|---|---|---|---|
| Calcd (%) | C, 60.16; | H, 6.17; | N, 7.79 |
| Found (%) | C, 60.06; | H, 5.90; | N, 7.73 |

### Example 105

### 3-(6-Chloro-3,4-dihydro-2-naphthyl)sulfonyl-N-methyl-N-[1-(2-methyl-4-pyridyl)-4-piperidyl]propanamide

### 105a) 3-(6-chloro-3,4-dihydro-2-naphthyl)sulfonylpropionic acid

According to the same manner as that or Example 76a), the title compound was obtained as colorless crystals (15%) from 6-chloro-3,4-dihydronaphthalene-2-sulfonyl chloride.
NMR (DMSO-d₆) δ: 2.55-2.68 (4H, m), 2.96 (2H, t, J = 8.0), 3.42 (2H, t, J = 7.1), 7.19 (1H, s), 7.30-7.44 (2H, m), 7.49 (1H, d, J = 8.0).

### 105b) 3-(6-Chloro-3,4-dihydro-2-naphthyl)sulfonyl-N-methyl-N- [1-(2-methyl-4-pyridyl)-4-piperidyl]prpanamide

According to the same manner as that of Example 76b), the title compound was obtained as the crystals (61%) from 3-(6-chloro-3,4-dihydro-2-naphthyl)sulfonylpropanecarboxylic acid obtained in Example 105a) and 4-methylamino-1-(4-pyridyl)piperidine obtained in Example 30a).
NMR (CDCl₃) δ: 1.50-1.90 (4H, m), 2.46 and 2.47 (3H, s), 2.65-3.04 (11H, m), 3.44 (2H, t, J = 5.0), 3.80-4.05 (2H, m), 4.62 (1H, m), 6.47-6.58 (2H, m), 7.15-7.27 (3H, m), 7.40 (1H, s), 8.14-8.21 (1H, m).

| Elemental Analysis: C₂₅H₃₀ClN₃O₃S·H₂O | | | |
|---|---|---|---|
| Calcd (%) | C, 60.41; | H, 6.29; | N, 8.45 |
| Found (%) | C, 60.55; | H, 6.31; | N, 8.35 |

### Example 106

### 3-(6-Chloro-2-naphthyl)sulfonyl-N-[[4-hydroxy-1-(2-methyl-4-pyridyl)-4-piperidyl]methyl]propanamide

### 106a) N-[1-(tert-Butoxycarbonyl)-4-hydroxy-4-piperidyl]methyl-3- (6-chloro-2-naphthyl) sulfonylpropanamide

According to the same manner as that described in Example 76h), the title compound was obtained from 3-(6-chloro-2-naphthyl)sulfonylpropanecarboxylic acid obtained in Example 27b) and tert-butyl 4-(aminomethyl)-4-hydroxypiperidine-1-carhoxylate as a solid (91%).
NMR (CDCl₃) δ: 1.34-1.65 (4H, m), 1.44 (9H, s), 2.79 (2H, t, J = 7.3), 2.87 (1H, m), 3.05-3.30 (4H, m), 3.56 (2H, t, J = 7.3), 3.76 (2H, m), 6.46 (1H, m), 7.59 (1H, dd, J = 1.8 and 9.2), 7.85-8.00 (4H, m), 8.47 (1H, s).

### 106b) 3-(6-Chloro-2-naphthyl)sulfonyl-N-[[4-hydroxy-1-(2-methyl-4-pyridyl)-4-piperidyl]methyl]propanamide

According to the same manner as that described in Example 102b), the title compound was obtained from N-[1-tert-butoxycarbonyl)-4-hydroxy-4-piperidyl]methyl-3-(6-chloro-2-naphthyl)sulfonylpropanamide obtained in Example 106a) as a solid (13%).
NMR (CDCl₃) δ: 1.40-1.80 (4H, m), 2.42 (3H, s), 2.79 (2H, t, J = 7.2), 3.20-3.65 (8H, m), 6.45-6.60 (3H, m), 7.60 (1H, dd, J = 2.0 and 8.8), 7.85-8.00 (4H, m), 8.10 (1H, d, J = 5.8), 8.47 (1H, s).

| Elemental Analysis: C₂₅H₂₈N₃O₄S·1.25H₂O | | | |
|---|---|---|---|
| Calcd (%) | C, 57.24; | H, 5.86; | N, 8.01 |
| Found (%) | C, 57.24; | H, 5.86; | N, 7.72 |

### Example 107

### Methyl 4-[3-(6-chloro-2-naphthyl)sulfonylpropanoyl]amino-1-(2-methyl-4-pyridyl)piperidine-4-carboxylate

### 107a) 4-(Benzyloxycarbonylamino)-1-(tert-butoxycarbonyl)-piperidine-4-carboxylic acid

Sodium bicarbonate (0.86 g), water (20 ml) and dioxane (20 ml) were added to a solution of 4-amino-1-(tert-butoxycarbonyl)-4-piperidinecarboxylic acid (2.45 g) obtained in Example 92a) in 1 N aqueous sodium hydroxide solution (10 ml), and benzyl chloroformate (1.72 g) was added dropwise thereto. The mixture was stirred at room temperature for 16 hours. The reaction mixture was concentrated under reduced pressure and the residue was diluted with water and adjusted to pH 3 with an aqueous solution of potassium hydrogen sulfate. The mixture was extracted with ethyl acetate. The extract was washed with water and dried over anhydrous sodium sulfate. The solvent was distilled off under reduced pressure to obtain the title compound as colorless crystals (2.89 g).
NMR (CDCl₃) δ: 1.45 (9H, s), 1.90-2.20 (4H, m), 3.00-3.22 (2H, m), 3.74-3.95 (2H, m), 5.10 (2H, s), 5.18 (1H, bs), 7.27-7.40 (5H, m).

### 107b) Methyl 4-(benzyloxycarbonylamino)-1-(tert-butoxycarbonyl)piperidine-4-carboxylate

Sodium carbonate (0.56 g) and methyl iodide (0.67 ml) were added to a solution of 4-(benzyloxycarbonylamino)-1-(tert-butoxycarbonyl)piperidine-4-carboxylic acid (1 g) in DMF (10 ml) and the mixture was stirred at room temperature for 6 hours. Ice-water was added to the reaction mixture and the mixture was extracted with ethyl acetate. The extract was dried over anhydrous sodium sulfate and the solvent was distilled off. The residue was purified by a silica gel column to obtain the title compound as colorless crystals (0.8 g, 77%).
NMR (CDCl₃) δ: 1.45 (9H, s), 1.95-2.20 (4H, m), 3.00-3.20 (2H, m), 3.69 (3H, s), 5.03 (1H, s), 5.11 (2H, s), 7.30-7.40 (5H, m).

### 107c) Methyl 4-[3-(6-chloro-2-naphthyl)sulfonylpropanoyl]-amino-1-(tert-butoxycarbonyl)-piperidine-4-carboxylate

10% Palladium-carbon (0.18 g) was added to methyl 4-(benzyloxycarbonylamino)-1-(tert-butoxycarbonyl)piperidine-4-carboxylate obtained in Example 107b) in methanol (50 ml) and the mixture was subjected to hydrogenolysis at ordinary temperature and ordinary pressure for 30 minutes. The catalyst was filtered off and the filtrate was concentrated under reduced pressure. According to the same manner as that described in Example 30b), the title compound was obtained from the resultant amine and 3-(6-chloro-2-naphtyl)sulfonylpropanecarboxylic acid as a colorless powder (quantitative).
NMR (CDCl₃) δ: 1.45 (9H, s), 1.85-2.08 (4H, m), 2.79(2H, t, J = 7.4), 3.09-3.20 (2H, m), 3.51 (2H, t, J = 7.4), 3.69 (3H, s), 3.70-3.82 (2H, m), 6.22 (1H, s), 7.60 (1H, dd, J = 2.0 and 8.8), 7.85-8.00 (4H, m), 8.47 (1H, s).

### 107d) Methyl 4-[3-(6-chloro-2-naphthyl)sulfonylpropanoyl]-amino-1-(2-methyl-4-pyridyl)piperidine-4-carboxylate

Accoding to the same manner as that described in Example 83c), the title compound was obtained from methyl 4-[3-(6-chloro-2-naphthyl)sulfonylpropanoyl]amino-1-(tert-butoxycarbonyl)piperidine-4-carobxylate obtained in Example 107c) as a colorless powder (40%).
NMR (CDCl₃) δ: 2.00-2.10 (2H, m), 2.12-2.22 (2H, m), 2.43 (3H, s), 2.81 (2H, t, J = 7.4), 3.20-3.40 (2H, m), 3.10-3.23 (2H, m), 3.50-3.60 (4H, m), 3.71 (3H, s), 6.42-6.52 (2H, m), 6.63 (1H, s), 7.85-8.00 (4H, m), 8.13 (1H, d, J = 6.0), 8.44 (1H, s).

| Elemental Analysis: C₂₆H₂₈ClN₃O₅S·0.5H₂O | | | |
|---|---|---|---|
| Calcd (%) | C, 57.93; | H, 5.42; | N, 7.20 |
| Found (%) | C, 58.10; | H, 5.32; | N, 7.71 |

### Example 108

### 1-[4-(6-Chloro-2-naphthyl)sulfonylbutyl]-3-methyl-8-(2-methyl-4-pyridyl)-1,3,8-triazaspiro[4.5]decane-2,4-dione

### 108a) tert-Butyl 1-(4-bromobutyl)-3-methyl-2,4-dioxo-1,3,8-triazaspiro[4.5]decane-8-carboxylate

According to the same manner as that described in Example 101b), the title compound was obtained from tert-butyl 3-methyl-2,4-dioxo-1,3,8-triazaspiro[4.5]decane-8-carboxylate obtained in Example 101a) and 1,4-dibromobutane as colorless crystals (70%).
NMR (CDCl₃) δ: 1.49 (9H, s), 1.60-1.70 (2H, m), 1.75-1.95 (6H, m), 3.01 (3H, s), 3.22 (2H, t, J = 7.6), 3.40-3.58 (2H, m), 3.43 (2H, t, J = 6.0), 4.00-4.20 (2H, m).

### 108b) tert-Butyl 1-[4-(6-chloro-2-naphthyl)sulfonylbutyl]-3-methyl-2,4-dioxa-1,3,8-triazaspiro[4.5]decane-8-carhoxylate

According to the same manner as that described in Example 101c), the title compound was obtained from tert-butyL 1-(4-bromobutyl)-3-methyl-2.4-dioxo-1,3,8-tyiazaspiro[4.5]decane-8-carboxylate obtained in Example 108a) as a colorless powder (92%).
NMR (CDCl₃) δ: 1.48 (9H, s), 1.54-1.88 (8H, m), 2.96 (3H, s), 3.10-3.25 (4H, m), 3.35-3.55 (2H, m), 3.95-4.25 (2H, m), 7.60 (1H, dd, J = 1.6 and 8.8), 7.85-8.00 (4H, m) 8.47 (1H, s).

### 108c) 1-[4-(6-Chloro-2-naphthyl)sulfonylbutyl]-3-methyl-8-(2-methyl-4-pyridyl)-1,3,8-triazaspiro[4.5]-decane-2,4-dione

According to the same manner as that described in Example 91e), the title compound was obtained from tert-Butyl 1-[4-(6-chloro-2-naphthyl)sulfonylbutyl]-3-methyl-2,4-dioxa-1,3,8-triazaspiro[4.5]decane-8-carboxylate obtained in Example 108b) as a colorless powder (81%).
NMR (CDCl₃) δ: 1.65-1.85 (6H, m), 1.85-2.00 (2H, m), 2.46 (3H, s), 2.98 (3H, s), 3.10-3.25 (4H, m), 3.60-3.75 (2H, m), 3.78-3.90 (2H, m), 6.48-6.64 (2H, m), 7.55-7.65 (1H, m), 7.85-8.00 (4H, m), 8.19 (1H, d, J = 5.6), 8.44 (1H, s).

| Elemental Analysis: C₂₈H₃₁ClN₄O₄S | | | |
|---|---|---|---|
| Calcd (%) | C, 60.58; | H, 5.63; | N, 10.09 |
| Found (%) | C, 60.38; | H, 5.75; | N, 9.90 |

### Example 109

### 1-[2-(6-chloro-2-naphthyl)sulfonylethyl]-3-methyl-8-(2-methyl-4-pyridyl)-1,3,8-triazaspiro[4.5]-decane-2,4-dione

### 109a) tert-Butyl 1-(ethoxycarbonylmethyl)-3-methyl-2,4-dioxo-1,3,8-triazaspiro[4.5]decane-8-carboxylate

According to the same manner as that described in Example 101b), the title compound was obtained from tert-butyl 3-methyl-2,4-dioxo-1,3,8-triazaspiro[4.5]decane-8-carboxylate obtained in Example 101a) and ethyl chloroformate as colorless crystals (94%).
NMR (CDCl₃) δ: 1.30 (3H, t, J = 7.0), 1.47 (9H, s), 1.60-1.72 (2H, m), 1.72-1.82 (2H, m), 3.05 (3H, s), 3.40-3.60 (2H, m), 3.97 (2H, s), 4.00-4.20 (2H, m), 4.22 (2H, q, J = 7.0).

### 109b) tert-Butyl 1-(2-hydroxyethyl)-3-methyl-2,4-dioxo-1,3,8-triazaspiro[4.5]decane-8-carboxylate

Sodium borohydride (0.35 g) was added to a solution of tert-Butyl 1-(ethoxycarbonylmethyl)-3-methyl-2,4-dioxo-1,3,8-triazaspiro[4.5]decane-8-carboxylate (1.69 g) obtained in Example 109a) in THF (80 ml) and methanol (8 ml) was added dropwise thereto under reflux. The reaction mixture was concentrated under reduced pressure, the residue was adjusted to pH 2 with an aqueous solution of potassium hydrogen sulfate, and the mixture was extracted with ethyl acetate. The extract was washed with water and then dried over anhydrous sodium sulfate. The solvent was distilled off and the residue was purified by a silica gel column to obtain the title compound as a colorless powder (0.61 g, 40%).
NMR (CDCl₃) δ: 1.45 (9H, s), 1.60-1.70 (2H, m), 1.75-1.90 (2H, m), 2.98-3.04 (1H, m), 3.03 (3H, s), 3.38 (2H, t, J = 5.2), 3.38-3.60 (2H, m), 3.78-3.85 (2H, m), 3.95-4.25 (2H, m).

### 109c) tert-Butyl 3-methyl-1-[2-(4-methylphenyl)-sulfonyloxyethyl]-2,4-dioxo-1,3,8-triazaspiro[4.5]decane-8-carboxylate

Triethylamine (0.77 ml) and toluenesulfonyl chloride (0.70 g) were added to a solution of tert-Butyl 1-(2-hydroxyethyl)-3-methyl-2,4-dioxo-1,3,8-triazaspiro[4.5]decane-8-carboxylate in methylene chloride (60 ml) and the mixture was stirred at room temperature for 20 hours. The solvent was distilled off under reduced pressure, water was added to the residue and the mixture was extracted with ethyl acetate. The extract was washed with an aqueous solution of sodium bicarbonate and dried over anhydrous sodium sulfate. The solvent was distilled off and the residue was purified by a silica gel column to obtain the title compound as a colorless powder (0.60 g, 68%).
NMR (CDCl₃) δ: 1.49 (9H, s), 1.55-1.68 (2H, m), 1.73-1.85 (2H, m), 2.46 (3H, s), 2.97 (3H, s), 3.37-3.57 (4H, m), 3.97-4.25 (4H, m), 7.36 (2H, d, J = 8.4), 7.77 (2H, d, J = 8.4).

### 109d) tert-Butyl 1-[2-(6-chloro-2-naphthyl)sulfonylethyl]-3-methyl-2,4-dioxa-1,3,8-triazaspiro[4.5]decane-8-carboxylate

According to the same manner as that described in Example 101c), the title compound was obtained from tert-butyl 3-methyl-1-[2-(4-methylphenyl)-sulfonyloxyethyl]-2,4-dioxo-1,3,8-triazaspiro[4.5]decane-8-carboxylate obtained in Example 109c) as a colorless powder (quantitative).
NMR (CDCl₃) δ: 1.50 (9H, s), 1.60-1.70 (2H, m), 1.80-1.92 (2H, m), 2.94 (3H, s), 3.35-3.75 (6H, m), 4.00-4.25 (2H, m), 7.61 (1H, dd, J = 2.0 and 8.8), 7.90-8.00 (4H, m), 8.49 (1H, s).

### 109e) 1-[2-(6-Chloro-2-naphthyl)sulfonylethyl]-3-methyl-8-(2-methyl-4-pyridyl)-1,3,8-triazaspiro[4.5]decane-2,4-dione

According to the same manner as that described in Example 91e), the title compound was obtained from tert-butyl 1-[2-(6-chloro-2-naphthyl)sulfonylethyl]-3-methyl-2,4-dioxa-1,3,8-triazaspiro[4.5]decane-8-carboxylate obtained in Example 109d) as a colorless powder (56%). NMR (CDCl₃) δ: 1.70-1.90 (2H, m), 1.95-2.10 (2H, m), 2.48 (3H, s), 2.96 (3H, s), 3.55-3.70 (6H, m), 3.80-3.90 (2H, m), 6.50-6.60 (2H, m), 7.60 (1H, dd, J = 1.6 and 8.8), 7.85-8.00 (4H, m), 8.21 (1H, d, J = 5.6), 8.46 (1H, s).

| Elemental Analysis: C₂₆H₂₇ClN₄O₄S·0.4H₂O | | | |
|---|---|---|---|
| Calcd (%) | C, 58.45; | H, 5.24; | N, 10.49 |
| Found (%) | C, 58.63; | H, 5.55; | N, 10.54 |

### Example 110

### 2-[2-(6-Chloro-2-naphthyl)sulfonylethyl]-8-(2-methyl-4-pyridyl)-2,8-diazaspiro[4.5]decane-1,3-dione

### 110a) 8-Benzyl-2-[2-(6-chloro-2-naphthyl)sulfonylethyl]-2,8-diazaspiro[4.5]decane-1,3-dione

According to the same manner as that described in Example 85c), the title compound was obtained from 3-(6-chloro-2-naphthyl)sulfonylpropylamine hydrochloride as pale yellow oil (quantitative).
NMR (CDCl₃) δ: 1.00-2.25 (6H, m), 2.54 (2H, s), 2.75-2.95 (2H, m), 3.45-3.60 (4H, m), 3.85 (2H, t, J = 6.0), 7.20-7.38 (5H, m), 7.60 (1H, dd, J = 2.0 and 8.8), 7.86-8.06 (4H, m), 8.51 (1H, s).

### 110b) 2-[2-(6-Chloro-2-naphthyl)sulfonylethyl]-8-(2-methyl-4-pyridyl)-2,8-diazaspiro[4.5]decane-1,3-dione

1-Chloroethyl chlorocarbonate (0.062 ml) was added to a solution of 8-benzyl-2-[2-(6-chloro-2-naphthyl)sulfonylethyl]-2,8-diazaspiro[4.5]decane-1,3-dione (0.28 g) in 1,2-dichloroethane (2.8 ml) at 0°C and the mixture was stirred at 70°C for 1 hour. Methanol (2.0 ml) was added to the reaction mixture and the mixture was stirred 70°C for further 1 hour. The solvent was distilled off under reduced pressure and the resultant residue, 4-chloro-2-methylpyridine (0.14 g) and triethylamine (0,76 ml) were dissolved in ethanol (5.0 ml). The mixture was heated at 150°C for 4 hours. The reaction mixture was concentrated under reduced pressure and the residue was purified by silica gel column to obtain the title compound as a pale yellow powder (8 mg).
NMR (CDCl₃) δ: 1.58-1.78 (2H, m), 2.04-2.26 (2H, m), 2.49 (3H, s), 2.66 (2H, s), 3.00-3.20 (2H, m), 3.50-3.62 (2H, m), 3.76-3.96 (4H, m), 6.50-6.62 (2H, m), 7.61 (1H, dd, J = 1.8 and 8.8), 7.88-8.06 (4H, m), 8.49 (1H, d, J = 5.8), 8.52 (1H, s).

| Elemental Analysis: C₂₆H₂₇ClN₄O₄S·0.4H₂O | | | |
|---|---|---|---|
| Calcd (%) | C, 58.45; | H, 5.24; | N, 10.49 |
| Found (%) | C, 58.63; | H, 5.55; | N, 10.54 |

### Example 111

### 2-[2-(6-Chloro-2-naphthyl)sulfonylethyl]-8-(2-methyl-4-pyridyl)-2,8-diazaspiro[4.5]decane-1-one

### 111a) tert-Butyl 2-[2-(6-chloro-2-naphthyl)sulfonylethyl]-1,3-oxo-2,8-diazaspiro[4.5]decane-8-carboxylate

According to the same manner as that in Example 104a), the title compound was obtained from 8-benzyl-2-[2-(6-chloro-2-naphthyl)sulfonylethyl]-2,8-diazaspiro[4.5]decane-1,3-dione obtained in Example 110a) as a colorless powder (quantitative).
NMR (CDCl₃) δ: 1.47 (9H, s), 1.40-1.62 (2H, m), 1.88-2.08 (2H, m), 2.60 (2H, s), 2.86-3.08 (2H, m), 3.48-3.60 (2H, m), 3.80-3.92 (2H, m), 3.94-4.16 (2H, m), 7.61 (1H, dd, J = 2.2 and 8.8), 7.88-8.04 (4H, m), 8.51 (1H, s).

### 111b) tert-Butyl 2-[2-(6-chloro-2-naphthyl)sulfonylethyl]-1-oxo-2,8-diazaspiro[4,5]decane-8-carboxylate

According to the same manner as that described in Example 104b), the title compound was obtained from tert-butyl 2-[2-(6-chloro-2-naphthyl)sulfonylethyl]-1,3-oxo-2,8-diazaspiro[4.5]decane-8-calboxylate obtained in Example 111a) as a colorless powder (quantitative).
NMR (CDCl₃) δ: 1.24-1.40 (2H, m), 1.45 (9H, s), 1.66-1.86 (2H, m), 1.92 (2H, t, J =7.0), 2.84-3.04 (2H, m), 3.45 (4H, q, J =6.6), 3.71 (2H, t, J = 6.6), 3.84-4.02 (2H, m), 7.59 (1H, dd, J = 1.4 and 8.8), 7.85-8.00 (4H, m), 8.47 (1H, s).

### 111c) 2-[2-(6-Chloro-2-naphthyl)sulfonylethyl]-8-(2-methyl-4-pyridyl)-2,8-diazaspiro[4.5]decane-1-one

According to the same manner as that described in Example 91e), the title compound was obtained from tert-Butyl 2-[2-(6-chloro-2-naphthyl)sulfonylethyl]-1-oxo-2,8-diazaspiro[4.5]decane-8-carboxylate obtained in Example 111b) as a brown powder (31%).
NMR (CDCl₃) δ: 1.40-1.56 (2H, m), 1.82-2.04 (4H, m), 2.44 (3H, s), 2.96-3.16 (2H, m), 3.38-3.56 (4H, m), 3.66-3.86 (4H, m), 6.44-6.56 (2H, m), 7.60 (1H, dd, J = 2.2 and 8.8), 7.85-8.20 (4H, m), 8.15 (1H, d, J = 5.8), 8.49 (1H, s).

| Elemental Analysis: C₂₆H₂₈N₃O₃SCl·H₂O | | | |
|---|---|---|---|
| Calcd (%) | C, 60.51; | H, 5.86; | N, 8.14 |
| Found (%) | C, 60.84; | H, 5.85; | N, 8.34 |

### Example 112

### 1-[3-(6-Chloro-2-naphthyl)sulfonylpropyl]-7-(2-methyl-4-pyridyl)-1,7-diazaspiro[3.5]nonane-2-one

### 112a) tert-Butyl 4-methylenepiperidine-1-carboxylate

1.6 N Solution of butyl lithium in hexane (54.9 ml) was added to a solution of methyltriphenylphosphonium bromide (31.4 g) in THF (315 ml) and the mixture was stirred for 30 minutes. To the mixture was added dropwise a solution of tert-butyl 4-oxopiperidine-1-carboxylate (3.5 g) in THF (50 ml) at -15°C. The reaction mixture was stirred at 0°C for 1 minute and water was slowly added thereto. The mixture was extracted with ethyl acetate, the extract was dried over anhydrous magnesium sulfate and the solvent was distilled off to obtain the title compound as yellow oil (3.23 g, 93%).
NMR (CDCl₃) δ: 1.47 (9H, s), 2.18 (4H, t, J = 5.8), 3.42 (4H, t, J = 5.8), 4,74 (2H, s).

### 112b) tert-Butyl 2-oxo-1,7-diazaspiro[3.5]nonane-7-carboxylte

Chlorosulfonyl isocyanate (1.35 ml) was added to a solution of tert-butyl 4-methylenepiperidine-1-carboxylate (3.23 g) obtained in Example 112a) in ether (50 ml) at 0°C and the mixture was stirred at the same temperature for 2 hours. The reaction mixture was slowly poured into a mixture of an aqueous solution of 25% sodium sulfite (50 ml) and ether (25 ml) with maintaining at slightly alkaline with 10% aqueous solution of potassium hydroxide. The organic layer was separated and the aqueous layer was extracted with ethyl acetate. The extract was combined with the organic layer, the layer was dried over anhydrous magnesium sulfate and the solvent was distilled off. The residue was purified by a silica gel column to obtain colorless oil (2.23 g, 57%).
NMR (CDCl₃) δ: 1.47 (9H, s), 1.78 (4H, t, J = 5.8), 2.73 (2H, d, J = 1.4), 3.14-3.34 (2H, m), 3.56-3.74 (2H, m), 6.02-6.30 (1H, br).

### 112c) 7-(2-Mehtyl-4-pyridyl)-1,7-diazaspiro[3.5]nonane-2-one

According to the same manner as that described in Example 91e), the title compound was obtained from tert-butyl 2-oxo-1,7-diazaspiro[3.5]nonane-7-carboxylate obtained in Example 112b) as a brown powder (43%).
NMR (CDCl₃) δ: 1.90 (4H, t, J = 5.6), 2.45 (3H, s), 2.79 (2H, d, J = 1.8), 3.14-3.34 (2H, m), 3.44-3.64 (2H, m), 6.38-6.48 (1H, br), 6.48-6.60 (2H, m), 8.17 (1H, d, J = 5.8).

### 112d) 1-[3-(6-Chloro-2-naphthyl)sulfonylpropyl]-7-(2-methyl-4-pyridyl-1,7-diazaspiro[3.5]nonane-2-one

Powdered potassium hydroxide (48 mg) was added to a mixture of 7-(2-mehtyl-4-pyridyl)-1,7-diazaspiro[3.5]nonane-2-one (0.18 g) obtained in Example 112c) and a 1 : 1 mixture of 6-chloro-2-(3-chloropropyl)sulfonylnaphthalene and 6-chloro-2-(3-bromopropyl)sulfonylhaphthalene (0.27 g) obtained in Example 25 mg), and tetrabutylammonium bromide (25 mg) in THF (5.4 ml). The mixture was stirred at room temperature for 24 hours. The solvent was distilled off under reduced pressure. The residue was dissolved in methylene chloride, washed with 10% aqueous solution of sodium carbonate and dried over anhydrous magnesium sulfate. The solvent was distilled off under reduced pressure and the residue was purified by a basic silica gel column to obtain a pale red powder (28 mg, 7%).
NMR (CDCl₃) δ: 1.54-1.74 (2H, m), 1.86-2.12 (4H, m), 2.47 (3H, s), 2.77 (2H, s), 2.72-2.96 (2H, m), 3.16-3.32 (4H, m), 3.84-3.98 (2H, m), 6.46-6.58 (2H, m), 7.60 (1H, dd, J = 1.8 and 9.2), 7.84 (4H, m), 8.20 (1H, d, J = 5.8), 8.46 (1H, s).

| Elemental Analysis: C₂₆H₂₈N₃O₃SCl·1.5H₂O | | | |
|---|---|---|---|
| Calcd (%) | C, 59.47; | H, 5.95; | N, 8.00 |
| Found (%) | C, 59.27; | H, 5.64; | N, 8.03 |

### Example 113

### (S)-4-[[2-[(6-Chloro-2-naphthyl)sulfonylmethyl]-1-pyrrolidyl]carbonyl]-1-(2-methyl-4-pyridyl)piperidine

### 113a) Benzyl (S)-2-hydroxymethylpyrrolidine-1-carboxylate

Borane-THF complex (80 ml) was added to a solution of N-benzyloxycarbonylproline (10 g) in THF (100 ml) at 0°C and the mixture was stirred at room temperature for 18 hours. The reaction mixture was diluted with ethyl acetate and then the mixture was washed with water and dried over anhydrous magnesium sulfate. The solvent was distilled off under reduced pressure and the residue was purified by a silica gel column to obtain the title compound as a colorless powder (9.4 g, 99%).
NMR (CDCl₃) δ: 1.30-2.15 (4H, m), 3.30-3.80 (4H, m), 3.86-4.10 (1H, m), 4.30-4.48 (1H, m), 5.15 (2H, s), 7.25-7.45 (5H, m).

### 113b) Benzyl (S)-2-methylsulfonyloxymethylpyrrolidine-1-carboxylate

Methanesulfonyl chloride (0.64 ml) was added to a solution of benzyl (S)-2-hydroxymethylpyrrolidine-1-carboxylate (1.5 g) obtained in Example 113a) and triethylamine (1.3 ml) in ethyl acetate (30 ml) at 0°C and the mixture was stirred at room temperature for 1 hour. The reaction mixture was washed with water and dried over anhydrous magnesium sulfate. The solvent was distilled off under reduced pressure to obtain a colorless powder (2.0 g, quantitative).
NMR (CDCl₃) δ: 1.75-2.15 (4H, m), 2.75-3.00 (3H, m), 3.35-3.55 (2H, m), 4.00-4.45 (3H, m), 5.00-5.30 (2H, m), 7.25-7.50 (5H, m).

### 113c) Benzyl (S)-2-(6-chloro-2-naphthyl)thiomethylpyrrolidine-1-carboxylate

A solution of benzyl (S)-2-methylsulfonyloxy-methylpyrrolidine-1-carboxylate (2.0 g) obtained in Example 113b), 6-chloro-2-mercaptonaphthalene (1.2 g) obtained in Example 1d) and sodium methoxide (0.34 g) in methanol (20 ml) was stirred at 50°C for 4 hours and then the mixture was concentrated under reduced pressure. The residue was diluted with ethyl acetate, washed with water and dried over anhydrous magnesium sulfate. The solvent was distilled off and the residue was purified by a silica gel column to obtain the title compound as colorless crystals (1.4 g, 52%).
NMR (CDCl₃) δ: 1.70-2.10 (4H, m), 2.65-3.00 (1H, m), 3.30-3.70 (3H, m), 3.95-4.25 (1H, m), 5.00-5.20 (2H, m), 7.20-8.00 (11H, m).

### 113d) Benzyl (S)-2-(6-chloro-2-naphthyl)sulfonylmethyl-pyrrolidine-1-carboxylate

According to the same manner as that described in Example 7d), the title compound was obtained from benzyl (S)-2-(6-chloro-2-naphthyl)thiomethyl-pyrrolidine-1-carboxylate obtained in Example 113c) as a colorless powder (quantitative).
NMR (CDCl₃) δ: 1.80-2.45 (4H, m), 3.00-3.28 (1H, m), 3.30-3.45 (2H, m), 3.50-4.05 (1H, m), 4.08-4.25 (1H, m), 4.80-5.08 (2H, m), 7.05-7.40 (4H, m), 7.50-8.00 (6H, m), 8.36 and 8.49 (total 1H, s for each).

### Example 113e) (S)-2-(6-Chloro-2-naphthyl)sulfonylmethyl-pyrrolidine hydrobromide

Benzyl (S)-2-(6-chloro-2-naphthyl)sulfonylmethyl-pyrrolidine-1-carboxylate (1.5 g) obtained in Example 113d) was dissolved in 25% solution of hydrogen bromide in acetic acid and the mixture was stirred at room temperature for 1 hour. The precipitate was collected by filtration and washed with ether to obtain a colorless powder (1.1 g, 86%).
NMR (CDCl₃) δ: 1.64-2.18 (3H, m), 2.20-2.40 (1H, m), 3.38 (2H, dd, J = 6.6 and 7.8), 3.62-3.80 (1H, m), 3.86-4.06 (2H, m), 7.69 (1H, dd, J = 2.2 and 8.8), 8.02 (1H, dd, J = 2.0 and 8.8), 8.08-8.20 (3H, m), 8.67 (1H, d, J = 1.6).

### 113f) Ethyl 1-(2-methyl-4-pyridyl)piperidine-4-carboxylate

A solution of ethyl isonipecotinate(2.5. g) and 4-chloro-2-methylpyridine (3.1 g) in acetic acid (70 ml) was stirred at 130°C for 5 hours and the mixture concentrated under reduced pressure. The residue was diluted with methylene chloride, washed with 10% aqueous solution of sodium carbonate and dried over anhydrous magnesium sulfate. The solvent was distilled off and the residue was purified by a silica gel column to obtain the title compound as a yellow powder (5.2 g, 95%).
NMR (CDCl₃) δ: 1.27 (3H, t, J = 7.1), 1.8-2.0 (4H, m), 2.44 (3H, s), 2.48-2.59 (1H, m), 2.87-3.01 (2H, m), 3.81 (2H, dd, J = 13.6 and 4.2), 4.16 (2H, q, J = 7.19), 6.50 (1H, dd, J = 5.8 and 2.4), 6.59 (1H, d, J = 2.4), 8.16 (1H, d, J = 5.8).

### 113g) 1-(2-Methyl-4-pyridyl)piperidine-4-carboxylic acid

A solution of ethyl 1-(2-methyl-4-pyridyl)piperidine-4-carboxylate (5.2 g) obtained in Example 113f), sodium hydroxide (1.3 g) and water (10 ml) in methanol (52 ml) was stirred at room temperature for 1 hour and the mixture was neutralized with 1 N hydrochloric acid and concentrated under reduced pressure. The residue was dissolved in ethanol and insolubles were removed by filtration. The filtrate was concentrated to dryness to obtain the title compound as a colorless powder (4.2 g, 83%).

### 113h) (S)-4-[[2-[6-(chloro-2-naphthyl)sulfonylmethyl]-1-pyrrolidyl]carbonyl]-1-(2-methyl-4-pyridyl)piperidine

DMTMM (0.35 g) was added to a mixture of (S)-2-(6-chloro-2-naphthyl)sulfonylmethylpyrrolidine hydrobromide (0.20 g) obtained in Example 113e), 1-(2-methyl-4-pyridyl)piperidine-4-carboxylic acid obtained in Example 113g) and diisopropylethylamine (0.27 ml) in DMF (4.0 ml) and the mixture was stirred at room temperature for 12 hours. The reaction mixture was concentrated under reduced pressure and the residue was diluted with methylene chloride, washed with 10% aqueous solution of sodium carbonate and then dried over anhydrous magnesium sulfate. The solvent was distilled off under reduced pressure and the residue was purified by a silica gel column to obtain the title compound as a yellow powder (46%).
NMR (CDCl₃) δ: 1.60-1.92 (4H, m), 1.92-2.64 (5H, m), 2.43 (3H, s), 2.72-2.94 (2H, m), 3.15 (1H, dd, J = 9.8 and 13.6), 3.34-3.64 (2H, m), 3.74-3.98 (3H, m), 4.26-4.42 (1H, m), 6.38-6.56 (2H, m), 7.57 (1H, dd, J = 1.8 and 8.8), 7.84-8.00 (4H, m), 8.12 (1H, d, J = 5.8), 8.48 (1H, s).

| Elemental analysis: C₂₇H₃₀N₃O₃SCl·0.5H₂O | | | |
|---|---|---|---|
| Calcd (%) | C, 62.23; | H, 6.00; | N, 8.06 |
| Found (%) | C, 61.98; | H, 5.86; | N, 8.27 |

### Example 114

### 3-(5-Chloro-2-benzothiazolyl)sulfonyl-N-methyl-N-[1-(2-methyl-4-pyridyl)-4-piperidyl]propanamide

### 114a) 3-(5-Chloro-2-bonzothiazolyl)thiopropanecarboxylic acid

5-Chloro-2-mercaptobenzothiazole (1.84 g) and 3-bromopropionic acid (1.53 g) were added to an solution of potassium hydroxide (1 g) in 70% aqueous ethanol (50 ml) and the mixture was refluxed for 3 hours. The reaction mixture was concentrated under reduced pressure, diluted with water, adjusted to pH 3 with acetic acid and then extracted with ethyl acetate. The extract was washed with water and dried over anhydrous sodium sulfate. The solvent was distilled off and the residue was purified by a silica gel column to obtain the title compound as colorless crystals (1.52 g, 59%).
NMR (CDCl₃+DMSO-d₆) δ: 2.87 (2H, t, J = 7.0), 3.53 (2H, t, J = 7.0), 7.12 (1H, dd, J = 1.6 and 8.4), 7.39 (1H, d, J = 8.4), 7.47 (1H, s).

### 114b) tert-Butyl 4-[N-[3-(5-chloro-2-benzothiazolyl)thiopropionyl]-N-methylamino]piperidine-1-carboxylate

According to the same manner as that described in Example 30b), the title compound was obtained from 3-(5-chloro-2-benzothiazolyl)thiopropanecarboxylic acid obtained in Example 114a) as colorless crystals (28%).
NMR (CDCl₃) δ: 1.46 (9H, s), 1.46-1.75 (4H, m), 2.60-3.00 (4H, m), 2.83 (3H, s), 3.60-3.75 (2H, m), 4.10-4.30 (2H, m), 4.60-4.72 (1H, m), 7.25-7.30 (1H, m), 7.64 (1H, d, J = 8.8), 7.80-7.84 (1H, m).

### 114c) 3-(5-Chloro-2-benzothiazolyl)sulfonyl-N-methyl-N-[1-(2-methyl-4-pyridyl)-4-piperidyl]propanamide

The title compound was obtained from tert-butyl 4-[N-[3-(5-chloro-2-benzothiazolyl)thiopropionyl]-N-methylamino]piperidine-1-carboxylate obtained in Example 114b) by oxidation according to the same manner as that described in Example 7d) followed by the same manner as that described in Example 83c) as a pale yellow powder (0.6%).
NMR (CDCl₃) δ: 1.56-1.70 (2H, m), 1.90-2.20 (2H, m), 2.40-2.50 (2H, m), 2.46 (3H, s), 2.75-3.10 (4H, m), 2.86 (3H, s), 3.90-4.10 (2H, m), 4.55-4.65 (1H, m), 6.50-6.60 (2H, m), 7.59 (1H, dd, J = 2.0 and 8.8), 7.95 (1H, d, J = 8.8), 8.17 (1H, d, J = 6.0), 8.20 (1H, d, J = 2.0).

### Example 115

### 3-(6-Chloro-2-naphthyl)sulfonyl-N-methyl-N-[1-(2-methyl-4-pyridyl)-3-piperidyl]propanamide

### 115a) tert-Butyl 3-hydroxypiperidine-1-carboxylate

Di-tert-butyl dicarboxylate (26.19 g) was added dropwise to a solution of 3-hydroxypiperidine (10.1 g) in water (50 ml)-acetonitrile (100 ml) at room temperature. The reaction mixture was stirred for 1 hour and then concentrated under reduced pressure, followed by addition of water and extraction with ethyl acetate. The extract was washed with eater and dried over anhydrous sodium sulfate. The solvent was distilled off and the residue was crystallized from hexane to obtain the title compound as colorless crystals (14.13 g, 70%).
NMR (CDCl₃) δ: 1.40-1.60 (2H, m), 1.46 (9H, s), 1.70-1.80 (1H, m), 1.84-1.94 (1H, m), 3.00-3.18 (2H, m), 3.48-3.60 (1H, m), 3.66-3.78 (2H, m).

### 115b) tert-Butyl 3-oxopiperidine-1-carboxylate

DMSO (5.42 ml) was added dropwise to a solution of oxalyl chloride (5.01 ml) in methylene chloride (150 ml) at -78°C and the mixture was stirred for 10 minutes. Then, a solution of tert-butyl 3-hydroxypiperidine-1-carboxylate (5.78 g) obtained in Example 115a) in methylene chloride (10 ml) was added dropwise thereto. After stirring the mixture the same temperature for 10 minutes, the temperature was raised to -45°C and the mixture was stirred for further 1 hour. Trimethylamine (30 ml) was added to the reaction mixture and the mixture was stirred at 0°C for 20 minutes. An aqueous saturated ammonium chloride solution was added to the mixture and the mixture was extracted with methylene chloride. The extract was dried over anhydrous sodium sulfate and concentrated and the residue was purified by a silica gel column to obtain the title compound as colorless crystals (4.5 g, 78%).
NMR (CDCl₃) δ: 1.47 (9H, s), 1.93-2.01 (2H, m), 2.46 (2H, t, J = 6.8), 3.58 (2H, t, J = 6.0), 4.00 (2H, s).

### 115c) tert-Butyl 3-(methylamino)piperidine-1-carboxylic acid

According to the same manner as that described in Example 42b), the title compound was obtained from tert-butyl 3-oxopiperidine-1-carboxylic acid obtained in Example 115b) as pale yellow oil (80%).
NMR (CDCl₃) δ: 1.20-1.50 (4H, m), 1.46 (9H, s), 1.60-1.72 (1H, m), 1.87-1.97 (1H, m), 2.40-2.50 (1H, m), 3.70-3.83 (1H, m).

### 115d) tert-Butyl 3[N-[3-(6-chloro-2-naphthyl)sulfonyl-propionyl]-N-methylamino]piperidine-1-carboxylate

According to the same manner as that described in Example 30b), the title compound was obtained from tert-butyl 3-(methylamino)piperidine-1-carboxylic acid obtained in Example 115c) as a colorless powder (72%).
NMR (CDCl₃) δ: 1.40-1.90 (5H, m), 1.43 and 1.49 (total 9H, each s), 2.44-3.00 (4H, m), 2.77 and 2.89 (total 3H, each s), 3.50-3.66 (2H, m), 3.85-4.35 (2H, m), 7.56-7.62 (1H, m), 7.90-8.00 (4H, m), 8.48 and 8.49 (total 1H, each s).

### 115e) 3-(6-Chloro-2-naphthyl)sulfonyl-N-methyl-N-[1-(2-methyl-4-pyridyl)-3-piperidyl]propanamide

According to the same manner as that described in Example 83c), the title compound was obtained from tert-butyl 3[N-[3-(6-chloro-2-naphthyl)sulfonylpropionyl]-N-methylamino]piperidine-1-carboxylate as a colorless powder (51%).
NMR (CDCl₃) δ: 1.55-2.00 (4H, m), 2.42 (2.25H, s), 2.48 (0.75H, s), 2.65-2.98 (4H, m), 2.85 (0.75H, s), 2.95 (2.25H, s), 3.45-3.90 (4H, m), 4.34-4.46 (1H, m), 6.42-6.60 (2H, m), 7.56-7.65 (1H, m), 7.88-8.00 (4H, m), 8.13 (0.75H, d, J = 6.0), 8.21 (0.25H, d, J = 6.0), 8.46 (0.25H, s), 8.49 (0.75H, s).

| Elemental Analysis: C₂₅H₂₈ClN₃O₃S·0.5H₂O | | | |
|---|---|---|---|
| Calcd(%) | C, 60.60; | H, 5.90; | N, 8.49 |
| Found(%) | C, 60.84; | H, 5.98; | N, 8.63 |

### Example 116

### N-[1-(2-Amino-4-pyridyl)-4-piperidyl]-3-(6-chloro-2-naphthyl)sulfonyl-N-methylpropanamide

### 116a) 8-(2-Amino-4-pyridyl)-1,4-dioxa-8-azaspiro[4.5]decane

According to the same manner as that described in Example 90e), the title compound was obtained from 2-amino-4-chloropyridine and 1,4-dioxa-8-azaspiro[4.5]decane as a colorless solid (25%).
NMR (CDCl₃) δ: 1.75 (4H, t, J = 6.0), 3.42 (2H, t, J = 6.0), 3.99 (4H, s), 4.21 (2H, br), 5.88 (1H, d, J = 2.2), 6.20 (1H, dd, J = 2.2 and 6.2), 7.80 (1H, d, J = 6.2).

### 116b) 1-(2-Amino-4-pyridyl)-4-piperidinone

4 N Hydrochloric acid (6 ml) was added to a solution of 8-(2-amino-4-pyridyl)-1,4-dioxa-8-azaspiro[4.5]decane (0.46 g) obtained in Example 116a) in acetone (8 ml) and the mixture was stirred for 12 hours. The reaction mixture was neutralized with an aqueous saturated sodium bicarbonate solution and concentrated under reduced pressure. The residue was dissolved in 1 N aqueous sodium hydroxide solution to which chloroform and potassium carbonate were added. The organic layer was separated and dried over anhydrous magnesium sulfate. The solvent was distilled off to obtain the title compound (0.29 g, 79%).
NMR (CDCl₃) δ: 2.53 (4H, t, J = 6.0), 3.68 (4H, t, J = 6.0), 5.91 (1H, d, J = 2.2), 6.23 (1H, dd, J = 2.2 and 6.2), 7.87 (1H, d, J = 6.2).

### 116c) 2-Amino-4-(4-methylamino-1-piperidino)pyridine

According to the same manner as that described in Example 42b), the title compound was obtained from 1-(2-amino-4-pyridyl)-4-piperidinone obtained in Example 116b) as a colorless solid (66%).
NMR (CDCl₃) δ: 1.34 (2H, m), 1.95 (2H, m), 2.46 (3H, s), 2.58 (1H, m), 2.87 (2H, m), 3.76 (2H, m), 4.19 (2H, br), 4.63 (2H, s), 5.87 (1H, d, J = 2.4), 6.20 (1H, dd, J = 2.4 and 6.2), 7.80 (1H, d, J = 6.2).

### 116d) N-[1-(2-Amino-4-pyridyl)-4-piperidyl]-3-(6-chloro-2-naphthyl)sulfonyl-N-methylpropanamide

According to the same manner as that described in Example 65), the title compound was obtained from 2-amino-4-(4-methylamino-1-piperidino)pyridine obtained in Example 116c) as a white amorphous solid (37%).
NMR (CDCl₃) δ: 1.56-1.75 (4H, m), 2.82 (3H, s), 2.82-2.96 (4H, m), 3.56 (2H, m), 3.81 (2H, m), 4.24 (2H, br), 4.57 (1H, m), 5.84 (1H, d, J = 2.6), 6.16 (1H, dd, J = 2.6 and 6.6), 7.60 (1H, m), 7.78-7.94 (5H, m), 8.48 (1H, s).

| Elemental Analysis: C₂₄H₂₇N₄O₃SCl·0.5H₂O | | | |
|---|---|---|---|
| Calcd (%) | C, 58.11; | H, 5.69; | N, 11.30 |
| Found (%) | C, 58.38; | H, 5.91; | N, 11.56 |

### Example 117

### 3-(6-Chloro-2-naphthyl)sulfonyl-N-[1-(2-hydorxymethyl-4-pyridyl)-4-piperidyl]-N-methylpropanamide

### 117a) 2-Hydroxymethyl-4-(4-methylaminopiperidino)pyridine

According to the same manner as that described in Example 65), the title compound was obtained from 1-(2-hydroxymethyl-4-pyridyl)-4-piperidinone as light brown oil (74%).
NMR (CDCl₃) δ: 1.36 (2H, m), 1.98 (2H, m), 2.47 (3H, s), 2.61 (1H, m), 2.94 (2H, m), 3.86 (2H, m), 4.63 (2H, s), 6.60 (2H, m), 8.19 (1H, d, J = 6.0).

### 117b) 3-(6-Chloro-2-naphthyl)sulfonyl-N-[1-(2-hydroxymethyl-4-pyridyl)-4-piperidyl]-N-methylpropanamide

According to the same manner as that described in Example 76b), the title compound was obtained from 2-hydroxymethyl-4-(4-methylaminopiperidino)pyridine obtained in Example 117a) as a colorless powder (40%).
NMR (CDCl₃) δ: 1.56-1.79 (4H, m), 2.83 (3H, s), 2.86-2.99 (4H, m), 3.57 (2H, dd, J = 3.2 and 8.2), 3.95 (2H, m), 4.63 (2H, s), 4.65 (1H, m), 6.56-6.63 (2H, m), 7.60 (1H, dd, J = 2.2 and 8.2), 7.93-7.97 (4H, m), 8.20 (1H, d, J = 6.6), 8.49 (1H, s).

| Elemental Analysis: C₂₅H₂₈N₃O₄SCl·0.5H₂O | | | |
|---|---|---|---|
| Calcd (%) | C, 58.76; | H, 5.72; | N, 8.22 |
| Found (%) | C, 58.89; | H, 5.92; | N, 8.02 |

### Example 118

### 3-(6-Chloro-2-naphthyl)sulfonyl-N-[1-(2,6-dimethyl-4-pyridyl)-4-piperidyl]-N-propanamide

### 118a) 8-(2,6-Dimethyl-4-pyridyl)-1,4-dioxa-8-azaspiro[4.5]-decane

According to the same manner as described in Example 90e), the title compound was obtained from 2,6-dimethyl-4-chloropyridine as a colorless solid (71%).
NMR (CDCl₃) δ: 1.76 (4H, m), 2.42 (6H, s), 3.46 (4H, m), 3.99 (4H, s), 6.41 (2H, s).

### 118b) 1-(2,6-Dimethyl-4-pyridyl)-4-piperidinone

According to the same manner as that described in Example 116b), the title compound was obtained form 8-(2,6-dimethyl-4-pyridyl)-1,4-dioxa-8-azaspiro[4.5]-decane obtained in Example 118a) as a pale yellow solid (42%).
NMR (CDCl₃) δ: 2.45 (6H, s), 2.54 (4H, t, J = 6.2), 3.72 (4H, t, J = 6.2), 6.45 (2H, s).

### 118c) 2,6-Dimethyl-4-(4-methylamino-1-piperidino)pyridine

According to the same manner as that described in Example 42b), the title compound was obtained from 1-(2,6-dimethyl-4-pyridyl)-4-piperidinone obtained in Example 118b) as a colorless solid (55%).
NMR (CDCl₃) δ: 1.40 (2H, m), 2.00 (2H, m), 2.47 (9H, s), 2.69 (1H, m), 2.98-3.12 (4H, m), 3.88 (1H, m), 6.43 (2H, s).

### 118d) 3-(6-Chloro-2-naphthyl)sulfonyl-N-[1-(2,6-dimethyl-4-pyridyl)-4-piperidyl]-N-methylpropanamide

According to the same manner as that described in Example 76b), the title compound was obtained from 2,6-dimethyl-4-(4-methylamino-1-piperidino)pyridine obtained in Example 118c) as a white solid (38%).
NMR (CDCl₃) δ: 1.56-1.68 (4H, m), 2.44 (6H, s), 2.83 (3H, s), 2.88-3.04 (4H, m), 3.57 (2H, dd, J = 7.0 and 8.0), 3.94 (2H, m), 4.60 (1H, m), 6.38 (2H, s), 7.60 (1H, dd, J = 2.2 and 8.8), 7.93-7.97 (4H, m), 8.48 (1H, s).

| Elemental Analysis: C₂₄H₂₇N₄O₃SCl·1.5H₂O | | | |
|---|---|---|---|
| Calcd (%) | C, 59.25; | H, 6.31; | N, 7.97 |
| Found (%) | C, 59.34; | H, 6.19; | N, 8.33 |

### Example 119

### N-[3-(6-Chloro-2-naphthyl)sulfonylpropyl]-N-methyl-1-(2-methyl-4-pyridyl)-4-piperidinecarboxamide

### 119a) tert-Butyl N-[3-(6-chloro-2-naphthyl)sulfonylpropyl]-N-methylcarbamate

According to the same manner as that described in Example 6c), the title compound was obtained from tert-butyl N-[3-(6-chloro-2-naphthyl)sulfonylpropyl]carbamate obtained in Example 9c) as colorless crystals (61%).
NMR (CDCl₃) δ: 1.39 (9H, s), 1.90-2.04 (2H, m), 2.80 (3H, s), 3.12-3.20 (2H, m), 3.31 (2H, t, J = 6.7), 7.60 (1H, dd, J = 1.8 and 9.0), 7.86-7.97 (4H, m), 8.46 (1H, s).

### 119b) 6-Chloro-2-(3-methylaminopropyl)sulufonylnaphthalene trifluoroacetate

According to the same manner as that described in Example 90d), the title compound was obtained from tert-butyl N-[3-(6-chloro-2-naphthyl)sulfonylpropyl]-N-methylcarbamate obtained in Example 119a) as colorless crystals (98%).
NMR (DMSO-d₆) δ: 1.81-1.97 (2H, m), 2,52 (3H, s), 2.97 (2H, m), 3.53 (2H, t, J = 7.8), 7.76 (1H, dd, J = 2.0 and 8.8), 7.97 (1H, dd, J = 8.6 and 1.8), 8.22 (1H, d, J = 8.6), 8.23-8.33 (2H, m), 8.46 (2H, br), 8.65 (1H, br).

### 119c) N-[3-(6-Chloro-2-naphthyl)sulfonylpropyl]-N-methyl-(tert-butoxycarbonyl)-4-piperidinecarboxamide

According to the same manner as that described in Example 30b), the title compound was obtained from 6-chloro-2-(3-methylaminopropyl)sulufonylnaphthalene trifluoroacetate obtained n Example 119b) as a colorless solid (81%).
NMR (CDCl₃) δ: 1.45 (9H, s), 1.62 (2H, m), 2.01 (2H, m), 2.56-2.82 (4H, m), 3.04 (3H, s), 3.14 (2H, m), 3.48 (2H, t, J = 7.0), 4.12 (2H, br), 7.59 (1H, dd, J = 1.8 and 9.2), 7.85-7.97 (4H, m), 8.45 (1H, br).

### 119d) N-[3-(6-Chloro-2-naphthyl)sulfonylpropyl]-N-methyl-1-(2-methyl-4-pyridyl)-4-piperidinecarboxamide

According to the same manner as that described in Example 91e), the title compound was obtained from N-[3-(6-chloro-2-naphthyl)sulfonylpropyl]-N-methyl-(tert-butoxycarbonyl)-4-piperidinecarboxamide obtained in Example 119c) as a colorless solid (89%).
NMR (CDCl₃) δ: 1.74-2.13 (4H, m), 2.45 (3H, s), 2.63-2.95 (4H, m), 3.08 (3H, s), 3.12-3.19 (2H, m), 3.46-3.53 (2H, m), 3.86-3.92 (2H, m), 6.47-6.51 (2H, m), 7.59 (1H, dd, J = 1.8 and 8.6), 7.85-7.96 (4H, m), 8.15 (1H, d, J = 6.2), 8.46 (1H, s).

| Elemental Analysis: C₂₆H₂₉N₃O₃SCl·0.3H₂O | | | |
|---|---|---|---|
| Calcd (%) | C, 59.10; | H, 5.91; | N, 8.33 |
| Found (%) | C, 61.81; | H, 5.89; | N, 8.13 |

### Example 120

### 1-[3-(6-Chloro-2-naphthyl)sulfonylpropanoyl]-4-(2-methyl-4-pyridyl)-1,4-diazepane

### 120a) 1-[3-(6-Chloro-2-naphthyl)sulfonylpropanyl]-1,4-diazepane

According to the same manner as that described in Example 30b), the title compound was obtained from 3-(6-chloro-2-naphthyl)sulfonylpropionic acid and 1,4-diazepane as brown oil (41%).
NMR (CDCl₃) δ: 1.77-1.87 (2H, m), 2.78-3.00 (6H, m), 3.46-3.62 (6H, m), 7.59 (1H, dd, J = 2.0 and 9.0), 7.93-7.97 (4H, m), 8.48 (1H, s).

### 120b) 1-[3-(6-Chloro-2-naphthyl)sulfonylpropanoyl]-4-(2-methyl-4-pyridyl)-1,4-diazepane

A solution of 1-[3-(6-chloro-2-naphthyl)sulfonyl-propanyl]-1,4-azepane (0.16 g) obtained in Example 120a), 4-chloro-2-methylpyridine (0.11 g) and sodium acetate (41 mg) in acetic acid (5 ml) was stirred at 110°C for 13 hours and then concentrated under reduced pressure. The residue was diluted with an aqueous potassium carbonate solution and extracted with ethyl acetate. The extract was dried over anhydrous sodium sulfate. The solvent was distilled off and the residue was purified by a silica gel column to obtain the title compound as a dark red powder (60 mg, 30%).
NMR (CDCl₃) δ: 1.79-2.05 (2H, m), 2.45 (3H, s), 2.78-3.00 (4H, m), 3.36-3.71 (8H, m), 6.34-6.38 (2H, m), 7.60. (1H, dd, J = 1.8 and 8.8), 7.93-7.97 (5H, m), 8.13 (1H, d, J = 5.8), 8.48 (1H, s).

| Elemental Analysis: C₂₄H₂₆N₃ClO₃S·H₂O | | | |
|---|---|---|---|
| Calcd (%) | C, 58.83; | H, 5.76; | N, 8.58 |
| Found (%) | C, 58.58; | H, 5.86; | N, 8.29 |

### Example 121

### (R)-4-[[2-(6-Chloro-2-naphthyl)sulfonylmethyl-1-pyrrolidyl]carbonyl]-1-(2-methyl-4-pyridyl)piperidine

### 121a) Benzyl (R)-2-hydroxymethylpyrrolidine-1-carboxylate

Benzyl chlorocarbonate (8.4 ml) was added to a mixture of D-prolinol (5.4 g), sodium carbonate (7.4 g), water (50 ml) and ethyl acetate (50 ml) at 0°C and the mixture was stirred at room temperature for 1 hour. The organic phase was separated, washed with an aqueous sodium carbonate solution and dried over anhydrous magnesium sulfate. The solvent was distilled off under reduced pressure and the residue was purified by a silica gel column to obtain the title compound as colorless oil (quantitative).
NMR (CDCl₃) δ: 1.52-2.10 (4H, m), 3.40 (1H, m), 3.50-3.74 (3H, m), 4.01 (1H, m), 4.38 (1H, m), 5.15 (2H, s), 7.28-7.46 (5H, m).

### 121b) Benzyl (R)-2-methylsulfonyloxymethylpyrrolidine-1-carboxylate

According to the same manner as that described in Example 113b), the title compound was obtained from benzyl (R)-2-hydroxymethylpyrrolidine-1-carboxylate obtained in Example 121a) as a colorless powder (quantitative).
NMR (CDCl₃) δ: 1.78-2.15 (4H, m), 2.84 and 2.93 (total 3H, s for each), 3.35-3.55 (2H, m), 4.00-4.45 (3H, m), 5.14 (2H, s), 7.25-7.50 (5H, m).

### 121c) Benzyl (R)-2-(6-chloro-2-naphthyl)thiomethyl-pyrrolidine-1-carboxylate

According to the same manner as that described in Example 113c), the title compound was obtained from benzyl (R)-2-methylsulfonyloxymethylpyrrolidine-1-carboxylate obtained in Example 121b) as colorless oil (60%).
NMR (CDCl₃) δ: 1.70-2.15 (4H, m), 2.65-3.00 (1H, m), 3.30-3.79 (3H, m), 3.95-4.25 (1H, m), 4.95-5.20 (2H, m), 7.24-8.02 (11H, m).

### 121d) Benzyl (R)-2-(6-chloro-2-naphthyl)sulfonylmethyl-pyrrolidine-1-carboxylate

According to the same manner as that described in Example 7d), the title compound was obtained from benzyl (R)-2-(6-chloro-2-naphthyl)thiomethylpyrrolidine-1-carboxylate obtained in Example 121c) as colorless syrup (96%).
NMR (CDCl₃) δ: 1.75-2.50 (4H, m), 3.00-3.28 (1H, m), 3.28-3.47 (2H, m), 3.50-4.03 (1H, m), 4.08-4.25 (1H, m), 4.80-5.08 (2H, m), 7.04-7.40 (5H, m), 7.52-8.08 (5H, m), 8.36 and 8.49 (total 1H, s for each).

### 121e) (R)-2-(6-Chloro-2-naphthyl)sulfonylmethylpyrrolidine hydrobromide

According to the same manner as that described in Example 113e), the title compound was obtained from benzyl (R)-2-(6-chloro-2-naphthyl)sulfonylmethyl-pyrrolidine-1-carboxylate as a colorless powder (93%).
NMR (CDCl₃) δ: 1.55-2.00 (3H, m), 2.14 (1H, m), 3.21 (2H, t, J = 7.2), 3.75 (1H, m), 3.90 (1H, m), 4.04 (1H, dd, J = 4.5 and 14.4 ), 7.78 (1H, m), 8.05 (1H, m), 8.21-8.34 (3H, m), 8.73 (1H, s).

### 121f) (R)-4-[[2-(6-chloro-2-naphthyl)sulfonylmethyl-1-pyrrolidyl]carbonyl]-1-(2-methyl-4-pyridyl)piperidine

According to the same manner as that described in Example 113g), the title compound was obtained from (R)-2-(6-chloro-2-naphthyl)sulfonylmethylpyrrolidine hydrobromide obtained in Example 121e) as a yellow powder (26%).
NMR (CDCl₃) δ: 1.54-2.64 (9H, m), 2.43 (3H, s), 2.70-2.94 (2H, m), 3.15 (1H, dd, J = 10.0 and 13.8), 3.36-3.68 (2H, m), 3.76 (3H, m), 4.26-4.44 (1H, m), 6.36-6.56 (2H, m), 7.57 (1H, dd, J = 2.2 and 8.8), 7.78-8.06 (4H, m), 8.14 (1H, d, J = 5.8), 8.48 (1H, s).

| Elemental Analysis: C₂₇H₃₀N₃O₃SCl·0.2H₂O | | | |
|---|---|---|---|
| Calcd (%) | C, 62.89; | H, 5.94; | N, 8.15 |
| Found (%) | C, 62.88; | H, 5.82; | N, 8.24 |

### Example 122

### (R)-4-[2-[2-(6-Chloro-2-naphthyl)sulfonylmethyl-1-pyrrolidyll-2-oxoethyl]-1-(2-methyl-4-pyridyl)piperidine

### 122a) Ethyl 2-(1-tert-butoxycarbonyl-4-piperidyl)acetate

To a solution of diethyl phosphonoacetate (22.3g) in THF (90ml), sodium hydride (60% oily; 4.3g) was added at 0 °C, and stirred for 30 minutes. A solution of tert-butyl 4-oxopiperidine-1-carboxylate (18g) in THF (90ml) was added, and stirred at room temperature for 2 hours. The reaction mixture was diluted with ethyl acetate, washed with 5% aqueous potassium hydrogen sulfate solution, and dried over anhydrous magnesium sulfate. The solvent was removed under reduced pressure, and the residue was recrystallized from hexane to obtain tert-butyl 4-(2-ethoxy-2-oxoethylidene)-1-piperidinecarboxylate as a yellow solid (12.8g, 53%).

To a solution of the solid (12.8g) obtained in methanol (130ml), 10% palladium-carbon (1.3g) was added, and stirred under hydrogen atmosphere. After completion of the reaction, the catalyst was filtered off, and the filtrate was concentrated under reduced pressure, and then the residue was purified by a silica gel column to obtain the title compound as a colorless oil (12.9g, quantitatively).
NMR (CDCl₃) δ: 1.02-1.33 (2H, m), 1.26 (3H, t, J = 7.0), 1.45 (9H, s), 1.60-1.80 (2H, m), 1.80-2.05 (1H, m), 2.23 (2H, d, J = 7.0), 2.60-2.83 (2H, m), 3.95-4.22 (2H, m), 4.13 (2H, q, J = 7.0).

### 122b) Ethyl 2-[1-(2-methyl-4-pyridyl)-4-piperidyl]acetate

From Ethyl 2-(1-tert-butoxycarbonyl-4-piperidyl)acetate obtained in Example 122a), the title compound was obtained as a colorless oil (51%) according to the same manner as that of Example 91e).
NMR (CDCl₃) δ: 1.28 (3H, t, J = 7.0), 1.20-1.44 (2H, m), 1.74-1.90 (2H, m), 1.92-2.32 (3H, m), 2.45 (3H, s), 2.86 (2H, dt, J = 2.4 and 12.6), 3.78-3.94 (2H, m), 4.16(2H, q, J = 7.0), 6.44-6.58 (2H, m), 8.15(1H, d, J = 6.0).

### 122c) 2-[1-(2-Methyl-4-pyridyl)-4-piperidyl]acetic acid

A mixture of ethyl 2-[1-(2-methyl-4-pyridyl)-4-piperidyl]acetate (5.2g) obtained in Example 122b), sodium hydroxide (1.3g), water (10ml) and methanol (2ml) was stirred at room temperature for 1 hour, then neutralized with 1N HCl, and concentrated under reduced pressure. The residue was purified by a CHP-20 column to obtain the title compound as a colorless powder (43%).
NMR (DMSO-d₆) δ: 1.08-1.24 (2H, m), 1.71 (2H, d, J = 12.9), 1.82-1.98 (1H, m), 2.16 (2H, d, J = 6.6), 2.23 (3H, s), 2.78 (2H, t, J = 12.6), 3.88 (2H, d, J = 13.2), 6.61 (1H, dd, J = 2.4 and 6.0), 6.67 (1H, d, J = 2.4), 7.99 (1H, d, J = 6.0).

### 122d) (R)-4-[2-[2-(6-Chloro-2-naphthyl)sulfonylmethyl-1-pyrrolidyl]-2-oxoethyl]-1-(2-methyl-4-pyridyl)piperidine

From 2-[1-(2-methyl-4-pyridyl)-4-piperidyl]acetic acid obtained in Example 122c) and (R)-2-(6-chloro-2-naphthyl)sulfonylmethylpyrrolidine hydrobromide obtained in Example 121e), the title compound was obtained as a colorless powder (23%) according to the same manner as that of Example 113h).
NMR (CDCl₃) δ: 1.05-1.35 (2H, m), 1.50-3.00 (11H, m), 2.46 (3H, s), 3.05-3.25 (1H, m), 3.25-3.55 (2H, m), 3.67-4.02 (3H, m), 4.25-4.45 (1H, m), 6.44-6.56 (2H, m), 7.58 (1H, dd, J = 2.2 and 8.8), 7.86-8.02 (4H, m), 8.06-8.18 (1H, m), 8.49 (1H, s).

| Elemental Analysis: C₂₈H₃₂N₃O₃SCl·0.5H₂O | | | |
|---|---|---|---|
| Calcd(%) | C, 62.85; | H, 6.22; | N, 7.85 |
| Found(%) | C, 62.65; | H, 6.10; | N, 7.99 |

### Example 123

### (S)-4-[2-[2-(6-Chloro-2-naphthyl)sulfonylmethyl-1-pyrrolidyl]-2-oxoethyl]-1-(2-methyl-4-pyridyl)piperidine

From 2-[1-(2-methyl-4-pyridyl)-4-piperidyl]acetic acid obtained in Example 122c) and (S)-2-(6-chloro-2-naphthyl)sulfonylmethylpyrrolidine hydrobromide obtained in Example 113e), the title compound was obtained as a colorless powder (21%) according to the same manner as that of Example 113h).
NMR (CDCl₃) δ: 1.08-1.35 (2H, m), 1.60-2.60 (9H, m), 2.47 (3H, s), 2.75-2.98 (2H, m), 3.16 (1H, dd, J = 9.8 and 13.6), 3.25-3.55 (2H, m), 3.76-4.05 (3H, m), 4.26-4.45 (1H, m), 6.46-6.56 (2H, m), 7.59 (1H, dd, J = 2.0 and 8.8), 7.86-8.00 (4H, m), 8.08-8.14 (1H, m), 8.49 (1H, s).

### Example 124

### 3-(5-Chloro-2-indolyl)sulfonyl-N-methyl-N-[1-(2-methyl-4-pyridyl)-4-piperidyl]propanamide

### 124a) 3-(5-Chloro-2-indolyl)thiopropionic acid

From 5-chloro-2-indolinethion (Takada, S et al., Chem. Pharm. Bull., 1984, *32*, 877), the title compound was obtained as a light yellow crystal (70%) according to the same manner as that of Example 114a).
NMR (CDCl₃) δ: 2.68 (2H, t, J = 7.0), 3.06 (2H, t, J = 7.0), 6.62 (1H, d, J = 1.2), 7.14 (1H, dd, J = 2.0 and 8.8), 7.24 (1H, d, J = 8.8), 7.52 (1H, d, J = 1.2), 8.35 (1H, s).

### 124b) tert-Butyl 4-[N-[3-(5-chloro-2-indolyl)thiopropionyl]-N-methylamino]piperidine-1 carhoxylate

From 3-(5-chloro-2-indolyl)thiopropionic acid obtained in Example 124a) and tert-butyl 4-methylaminopiperidine-1-carboxylate, the title compound was obtained as a colorless crystal (43%) according to the same manner as that of Example 30b).
NMR (CDCl₃) δ: 1.42-1.63 (4H, m), 1.46 and 1.47 (total 9H, each s), 2.60-2.90 (4H, m), 2.82 and 2.85 (total 3H, each s), 3.18 (2H,t, J = 6.6), 3.50-3.70 (0.2H, m), 4.05-4.35 (2H, m), 4.58-4.75 (0.8H, m), 6.52 (1H, d, J = 2.0), 7.12 (1H, dd, J = 2.0 and 8.8), 7.28 (1H, d, J = 8.8), 7.48 (1H, d, J = 2.0), 9.60 (1H, bs).

### 124c) tert-Butyl 4-[N-[3-(5-chloro-2-indolyl)sulfonylpropionyl]-N-methylamino]piperidine-1-carboxylate

From tert-butyl 4-[N-[3-(5-chloro-2-indolyl)thiopropionyl]-N-methylamino]piperidine-1-carboxylate obtained in Example 124b), the title compound was obtained as a colorless crystal (93%) according to the same manner as that of Example 7d).
NMR (CDCl₃) δ: 1.42-1.72 (4H, m), 1.46 and 1.48 (total 9H, each s), 2.60-3.00 (4H, m), 2.78,2.84 (total 3H, each s), 3.68 (2H, t, J = 7.2), 4.05-4.55 (3H, m), 7.14 (1H, s), 7.33 (1H, dd, J = 2.0 and 8.8), 7.41 (1H, d, J = 8.8), 7.68 (1H, s), 9.82 (0.75H, s), 9.93 (0.25H, s).

### 124d) 3-(5-Chloro-2-indolyl)sulfonyl-N-methyl-N-[1-(2-methyl-4-pyridyl)-4-piperidyl]propanamide

From tert-butyl 4-[N-[3-(5-chloro-2-indolyl)sulfonylpropionyl]-N-methylamino]piperidine-1-carboxylate obtained in Example 124c), the title compound was obtained as a colorless powder (33%) according to the same manner as that of Example 83c).
NMR (CDCl₃) δ: 1.50-1.90 (4H, m), 2.45 (2.25H, s), 2.48 (0.75H, s), 2.78 (0.75H, s), 2.83 (2.25H, s), 3.69 (2H, t, J = 7.2), 3.70-4.05 (2.25H, m), 4.50-4.65 (0.75H, s), 6.45-6.60 (2H, m), 7.14 (0.75H, s), 7.15 (0.25H, s), 7.33 (1H, dd, J = 2.0 and 8.8), 7.41 (1H, d, J = 8.8), 7.69 (1H, d, J = 2.0), 8.16 (0.75H, d, J = 6.0), 8.20 (0.25H, d, J = 6.0).

| Elemental Analysis: C₂₃H₂₇ClN₄O₃S·0.5H₂O | | | |
|---|---|---|---|
| Calcd(%) | C, 57.07; | H, 5.83; | N, 11.58 |
| Found(%) | C, 57.10; | H, 5.92; | N, 11.53 |

### Example 125

### 3-(5-Chloro-1-methyl-2-indolyl)sulfonyl-N-methyl-N-[1-(2-methyl-4-pyridyl)-4-piperidyl]propanamide

### 125a) tert-Butyl 4-[N-[3-(5-chloro-1-methyl-2-indolyl)sulfonylpropionyl]-N-methylamino]piperidine-1-carboxylate

To a solution of tert-butyl 4-[N-[3-(5-chloro-2-indolyl)sulfonylpropionyl]-N-methylamino]piperidine-1-carboxylate (0.35g) obtained in Example 124c) in DMF (10ml), sodium hydride (60% oily; 36mg) was added and stirred for 30 minutes, then methyl iodide (0.1ml) was added and stirred at room temperature for further 16 hours. The reaction mixture was concentrated under reduced pressure, and the residue was diluted with water, and extracted with ethyl acetate. The extract was washed with water, then dried over anhydrous sodium sulfate. The solvent was removed under reduced pressure, and the residue was purified by a silica gel column to obtain the title compound as a colorless powder (0.34g, 90%).
NMR (CDCl₃) δ: 1.15-1.70 (4H, m), 1.45, 1.48 (total 9H, each s), 2.50-2.66 (2H, m), 2.76 (3H, s), 2.80 (2H, t, J = 7.0), 3.66 (2H, t, J = 7.0), 4.00-4.30 (1H, m), 4.03 (3H, s), 7.17 and 7.18 (total 1H, each s), 7.32-7.40 (2H, m), 7.65,7.66 (total 1H, each d, J = 2.0)

### 125b) 3-(5-Chloro-1-methyl-2-indolyl)sulfonyl-N-methyl-N-[1-(2-methyl-4-pyridyl)-4-piperidyl]propanamide

From tert-butyl 4-[N-[3-(5-chloro-1-methyl-2-indolyl)sulfonylpropionyl]-N-methylamino]piperidine-1-carboxylate obtained in Example 125a), the title compound was obtained as a colorless powder (14%) according to the same manner as that of Example 83c).
NMR (CDCl₃) δ: 1.20-1.34 (2H, m), 1.40-1.90 (2H, m), 2.44 and 2.47 (total 3H, each s), 2.67-2.95 (2H, m), 2.75 (3H, s), 2.80 (2H, t, J = 6.8), 3.68 (2H, t, J = 6.8), 3.80-3.90 (2H, m), 4.04 and 4.05 (total 3H, each s), 4.15-4.26 (1H, m), 6.40-6.58 (2H, m), 7.17 and 7.19 (total 1H, each s), 7.33-7.42 (2H, m), 7.66 (1H, d, J = 1.6), 8.15 and 8.19 (total 1H, d, J = 6.0).

| Elemental Analysis: C₂₄H₂₉ClN₄O₃S·0.25H₂O | | | |
|---|---|---|---|
| Calcd(%) | C, 58.41; | H, 6.02; | N, 11.35 |
| Found(%) | C, 58.25; | H, 6.00; | N, 11.17 |

### Example 126

### 4-(6-Chloro-2-naphthyl)sulfonyl-N-methyl-N-[1-(2-methyl-4-pyridyl)-3-piperidyl]butanamide

### 126a) tert-Butyl 3-[N-[4-(6-chloro-2-naphthyl)sulfonylbutanoyl]-N-methylamino]piperidine-1-carboxylate

From 4-(6-chloro-2-naphthyl)sulfonylbutyric acid obtained in Example 24c) and tert-butyl 3-(methylamino)piperidine-1-carboxylate obtained in Example 115c), the title compound was obtained as a colorless powder (80%) according to the same manner as that of Example 30b).
NMR (CDCl₃) δ: 1.44 and 1.47(total 9H, each s), 1.45-1.85 (4H, m), 2.00-2.20 (2H, m), 2.45-2.80 (4.6H, m), 2.80 and 2.84 (total 3H, each s), 3.32 (2H, t, J = 7.2), 3.45-3.60 (0.4H, m), 3.84-4.42 (2H, m), 7.58 (1H, dd, J = 2.0 and 8.8), 7.86-8.00 (4H, m), 8.48 (1H, s).

### 126b) 4-(6-Chloro-2-naphthyl)sulfonyl-N-methyl-N-[1-(2-methyl-4-pyridyl)-3-piperidyl]butanamide

From tert-butyl 3-[N-[4-(6-chloro-2-naphthyl)sulfonylbutanoyl]-N-methylamino]piperidine-1-carboxylate obtained in Example 126a), the title compound was obtained as a colorless powder (44%) according to the same manner as that of Example 83c).
NMR (CDCl₃) δ: 1.60-1.93 (4H, m), 2.06-2.20 (2H, m), 2.43 (2H, s, 2/3Me), 2.46 (1H, s, 1/3Me), 2.50-2.83 (4H, m), 2.88 (1H, s, 1/3NMe), 2.90 (2H, s, 2/3NMe), 3.25-3.45 (2H, m), 3.62-3.92 (2H, m), 4.40-4.58 (1H, m), 6.47-6.60 (2H, m), 7.59 (1H, dd, J = 2.0 and 8.8), 7.86-8.00 (4H, m), 8.13 (2/3H, d, J = 6.4), 8.20 (1/3H, d, J = 6.4), 8.46 (1/3H, s), 8.48 (2/3H, s).

| Elemental Analysis: C₂₆H₃₀ClN₃O₃S·0.5H₂O | | | |
|---|---|---|---|
| Calcd(%) | C, 61.34; | H, 6.14; | N, 8.25 |
| Found(%) | C, 61.19; | H, 6.12; | N, 8.24 |

### Example 127

### 3-(6-Chloro-2-naphthyl)sulfonyl-N-[1-(2-methyl-4-pyridyl)-4-piperidyl]propanamide

### 127a) [1-(2-Methyl4-pyridyl)-4-piperidyl]amine

To a solution of 1-(2-methyl-4-pyridyl)-4-piperidone (0.67 g) obtained in Example 42a) in acetic acid (8ml), ammonium acetate (2.71g) was added, and stirred at 0 °C for 1 hour, then sodium triacetoxyborohydride (0.90g) was added and stirred at room temperature for 15 hours. The reaction mixture was concentrated under reduced pressure, and methylene chloride and aqueous potassium carbonate solution were added to the residue to alkalize it. The organic layer was separated, and dried over sodium sulfate. The solvent was removed under reduced pressure to obtain the title compound as a yellow oil (0.53g, 79%).
NMR (CDCl₃) δ: 1.28-1.65 (4H, m), 1.88-2.00 (3H, m), 2.44 (3H, s), 2.82-2.97 (2H, m), 3.80-3.87 (2H, m), 6.49-6.54 (2H, m), 8.15 (1H, d, J = 5.8).

### 127b) 3-(6-Chloro-2-naphthyl)sulfonyl-N-[1-(2-methyl-4-pyridyl)-4-piperidyl]propanamide

From [1-(2-methyl-4-pyridyl)-4-piperidyl]amine obtained in Example 127a), the title compound was obtained as a colorless powder (15%) according to the same manner as that of Example 76b).
NMR (CDCl₃) δ: 1.35-1.48 (2H, m), 1.89-1.94 (2H, m), 2.44 (3H, s), 2.68 (2H, t, J = 7.5), 2.85-2.97 (2H, m), 3.55 (2H, d, J = 7.5), 3.76-3.83 (2H, m), 3.93 (1H, m), 5.57 (1H, d, J = 7.6), 6.47-6.51 (2H, m), 7.60 (1H, dd, J = 1.8 and 8.8), 7.88-7.96 (4H, m), 8.16 (1H, d, J = 5.8), 8.46 (1H, d, J = 0.8).

| Elemental Analysis: C₂₄H₂₆N₃ClO₃S·0.3H₂O | | | |
|---|---|---|---|
| Calcd(%) | C, 60.38; | H, 5.62; | N, 8.80 |
| Found(%) | C, 60.46; | H, 5.46; | N, 8.90 |

### Example 128

### N-Butyl-3-(6-chloro-2-naphthyl)sulfonyl-N-[1-(2-methyl-4-pyridyl)-4-piperidyl]propanamide

### 128a) N-Butyl-N-[1-(2-methyl-4-pyridyl)-4-piperidyl]amine

From 1-(2-methyl-4-pyridyl)-4-piperidone obtained in Example 42a) and butylamine, the title compound was obtained as a yellow oil (98%) according to the same manner as that of Example 30a).
NMR (CDCl₃) δ: 1.35-1.69 (2H, m), 1.94-2.02 (2H, m), 2.44 (3H, s), 2.63-3.09 (3H, m), 3.79-3.87 (4H, m), 6.48-6.55 (2H, m), 7.25-7.34 (5H, m), 8.14 (1H, d, J = 6.0).

### 128b) N-Butyl-3-(6-chloro-2-naphthyl)sulfonyl-N-[1-(2-methyl-4-pyridyl)-4-piperidyl]propanamide

From N-butyl-N-[1-(2-methyl-4-pyridyl)-4-piperidyl]amine obtained in Example 128a), the title compound was obtained as a light yellow powder (36%) according to the same manner as that of Example 76b).
NMR (CDCl₃) δ: 1.47-1.80 (4H, m), 2.41-2.45 (3H, m), 2.68-3.18 (4H, m), 3.52-3.89 (2H, m), 3.89-3.96 (2H, m), 4.46 and 4.52 (2H, each s), 4.63-4.69 (1H, m), 6.41-6.50 (2H, m), 7.07-7.30 (5H, m), 7.56-7.62 (1H, m), 7.76-7.95 (3H, m), 8.11-8.51 (2H, m).

| Elemental Analysis: C₃₁H₃₂N₃ClO₃S·0.2H₂O | | | |
|---|---|---|---|
| Calcd(%) | C, 65.82; | H, 5.77; | N, 7.43 |
| Found(%) | C, 65.57; | H, 5.98; | N, 7.75 |

### Example 129

### 3-(6-Chloro-2-naphthyl)sulfonyl-N-[1-(2-methyl-4-pyridyl)-4-piperidyl]-N-(2-pyridyl)methylpropanamide

### 129a) N-[1-(2-Methyl-4-pyridyl)-4-piperidyl]-N-(2-pyridyl)methylamine

From 1-(2-methyl-4-pyridyl)-4-piperidone obtained in Example 42a) and N-(2-pyridyl)methylamine, the title compound was obtained as a yellow oil (41%) according to the same manner as that of Example 30a).
NMR (CDCl₃) δ: 1.39-1.56 (2H, m), 1.98-2.08 (2H, m), 2.43 (3H, s), 2.74-2.96 (3H, m), 3.80-3.86 (2H, m), 3.97 (2H, s), 6.50-6.54 (2H, m), 7.17 (1H, t, J = 6.2), 7.27-7.69 (1H, m), 8.14 (1H, d, J = 6.0), 8.55 (1H, d, J = 4.8).

### 129b) 3-(6-Chloro-2-naphthyl)sulfonyl-N-[1-(2-methyl-4-pyridyl)-4-piperidyl]-N-(2-pyridyl)methylpropanamide

From N-[1-(2-methyl-4-pyridyl)-4-piperidyl]-N-(2-pyridyl)methylamine obtained in Example 129a), the title compound was obtained as a colorless powder (50%) according to the same manner as that of Example 76b).
NMR (CDCl₃) δ: 1.45-1.64 (4H, m), 2.42 and 2.45 (3H, each s), 2.79-3.19 (4H, m), 3.57-3.65 (2H, m), 3.80-3.97 (2H, m), 4.52-4.72 (3H, m), 6.41-6.50 (2H, m), 7.09-7.17 (2H, m), 7.56-7.64 (2H, m), 7.84-7.98 (4H, m), 8.10-8.18 (1H, m), 8.40-8.51 (2H, m).

| Elemental Analysis: C₃₀H₃₁N₄ClO₃S·H₂O | | | |
|---|---|---|---|
| Calcd(%) : | C, 62.00; | H, 5.72; | N, 9.64 |
| Found(%) : | C, 62.09; | H, 5.36; | N, 9.63 |

### Example 130

### 1-[3-(6-Chloro-2-naphthyl)sulfonylpropanoyl]-4-(2-methyl-4-pyridyl)piperazine

### 130a) tert-Butyl 4-(2-methyl-4-pyridyl)-1-piperazinecarboxylate

From 1-Boc-piperazine, the title compound was obtained as a brown solid (92%) according to the same manner as that of Example 90e).
NMR (CDCl₃) δ: 1.49 (9H, s), 2.46 (3H, s), 3.28-3.33 (4H, m), 3.53-3.58 (4H, m), 6.48-6.55 (2H, m), 8.19 (1H, d, J = 5.8).

### 130b) 4-(2-Methyl-4-pyridyl)-1-piperazine dihydrochloride

From tert-bytyl 4-(2-methyl-4-pyridyl)-1-piperazinecarboxylate obtained in Example 130a), the title compound was obtained as a brown powder (quantitative) according to the same manner as that of Example 70a).
NMR (CDCl₃) δ: 2.57 (3H, s), 3.42 (4H, t, J = 5.5), 4.01 (4H, t, J = 5.3), 7.14-7.18 (2H, m), 8.13 (1H, dd, J = 2.2 and 8.0).

### 130c) 1-[3-(6-Chloro-2-naphthyl)sulfonylpropanoyl]-4-(2-methyl-4-pyridyl)piperazine

From 4-(2-methyl-4-pyridyl)-1-piperazine dihydrochloride obtained in Example 130b), the title compound was obtained as a colorless powder (55%) according to the same manner as that of Example 30b).
NMR (CDCl₃) δ: 2.48 (3H, s), 2.89-2.97 (2H, m), 3.23-3.28 (2H, m), 3.34-3.39 (2H, m), 3.55-3.71 (6H, m), 6.47-6.53 (2H, m), 7.59 (1H, dd, J = 2.2 and 8.8), 7.92-7.96 (4H, m), 8.22 (1H, d, J = 5.4), 8.48 (1H, s).

| Elemental Analysis: C₂₃H₂₄N₃ClO₃S·0.1H₂O | | | |
|---|---|---|---|
| Calcd(%) | C, 60.08; | H, 5.31; | N, 9.14 |
| Found(%) | C, 59.78; | H, 5.39; | N, 9.42 |

### Example 131

### Methyl 1-[3-(6-chloro-2-naphthyl)sulfonytpropanoyl]-4-(2-methyl-4-pyridyl)-2-piperazinecarboxylate

### 131a) 1-Benzyl 2-methyl 4-(2-methyl-4-pyridyl)piperazine-1,2-dicarboxylate

From 1-benzyl 2-methyl piperazine-1,2-dicarboxylate hydrochloride (JP-A No. 3-232864), the title compound was obtained as a yellow oil (63%) according to the same manner as that of Example 90e).
NMR (CDCl₃) δ: 2.46 (3H, s), 2.46-3.43 (3H, m), 3.67-3.73 (3H, m), 4.00-4.42 (3H, m), 4.80-4.93 (1H, m), 5.17-5.21 (2H, m), 6.49-6.54 (2H, m), 7.34-7.38 (5H, m), 8.20 (1H, d, J = 5.4).

### 131b) Methyl 4-(2-methyl-4-pyridyl)piperazine-2-dicarboxylate

1-Benzyl 2-methyl 4-(2-methyl-4-pyridyl)piperazine-1,2-dicarboxylate (1.0g) obtained in Example 131a) and 10% palladium-carbon (0.15g) were added to methanol (15ml), and hydrogenolyzed at ambient temperature under ambient pressure. The catalyst was filtered off, and the filtrate was concentrated under reduced pressure. The residue was purified by a silica gel column to obtain the title compound as a colorless oil (0.53g, 83%).
NMR (CDCl₃) δ: 2.15 (1H, br), 2.47 (3H, s), 2.86-3.82 (10H, m), 6.53-6.59 (2H, m), 8.18 (1H, d, J = 5.8).

### 131c) Methyl 1-[3-(6-chloro-2-naphthyl)sulfonylpropanoyl]-4-(2-methyl-4-pyridyl)-2-piperazinecarboxylate

From methyl 4-(2-methyl-4-pyridyl)piperazine-2-dicarboxylate obtained in Example 131b), the title compound was obtained as a colorless powder (52%) according to the same manner as that of Example 30b).
NMR (CDCl₃) δ: 2.47 (3H, s), 2.89-3.20 (4H, m), 3.43-3.85 (7H, m), 4.31-4.60 (2H, m), 5.19 (1H, m), 6.49-6.55 (2H, m), 7.60 (1H, dd, J = 1.8 and 8.8), 7.89-7.98 (4H, m), 8.22 (1H, d, J = 6.2), 8.49 (1H, s).

| Elemental Analysis: C₂₅H₂₆N₃ClO₅S·0.5H₂O | | | |
|---|---|---|---|
| Calcd(%) | C, 57.19; | H, 5.18; | N, 8.00 |
| Found(%) | C, 57.43; | H, 5.57; | N, 8.16 |

### Example 132

### (S)-2-(6-Chloro-2-naphthyl)sulfonylmethyl-N-[1-(2-methyl-4-pyridyl)-4-piperidyl]pyrrolidin-1-carboxamide

### 132a) tert-Butyl (S)-4-[2-(6-chloro-2-naphthyl)sulfonylmethyl-1-pyrrolidyl]carbonylaminopiperidine-1-carboxylate

To a solution of 1-tert-butoxycarbonylpiperidine-4-carboxylic acid (0.40 g) and triethylamine (0.34 ml), diphenylphosphoryl azide (0.53 ml) was added at 0°C, then stirred at 80°C for 2hours. The temperature of the reaction mixture was cooled to room temperature, and a solution of (S)-2-(6-chloro-2-naphthyl)sulfonylmethylpyrrolidine (0.64 g) obtained in Example 113e) in toluene (2 ml), and stirred at 80°C for 1 hour. The reaction mixture was washed with 5% aqueous potassium hydrogen sulfate solution, followed by with aqueous saturated sodium bicarbonate solution, and then dried over anhydrous magnesium sulfate. The solvent was removed under reduced pressure, and the residue was purified by a silica gel column to obtain the title compound as a colorless powder (0.88g, quantitative).

### 132b) (S)-2-(6-Chloro-2-naphthyl)sulfonylmethyl-N-[1-(2-methyl-4-pyridyl)-4-piperidyl]pyrrolidin-1-carboxamide

From tert-butyl (S)-4-[2-(6-chloro-2-naphthyl)sulfonylmethyl-1-pyrrolidyl]carbonylaminopiperidine-1-carboxylate obtained in Example 132a), the title compound was obtained as a colorless powder (8%) according to the same manner as that of Example 83c).
NMR (CDCl₃) δ: 1.22-1.52 (2H, m), 1.82-2.34 (6H, m), 2.44 (3H, s), 2.84-3.02 (2H, m), 3.10-3.34 (3H, m), 3.68-3.90 (4H, m), 4.20-4.42 (2H, m), 6.44-6.56 (2H, m), 7.58 (1H, dd, J = 1.8 and 8.8), 7.86-7.98 (4H, m), 8.14 (1H, d, J = 5.8), 8.47 (1H, s).

| Elemental Analysis: C₂₇H₃₁N₄O₃SCl·2H₂O | | | |
|---|---|---|---|
| Calcd(%) | C, 57.59; | H, 6.26; | N, 9.95 |
| Found(%) | C, 57.53; | H, 5.81; | N, 10.28 |

### Example 133

### (S)-N-[2-(6-Chloro-2-naphtyl)sulfonylmethyl-1-pyrrolidyl]-1-(2-methyl-4-pyridyl)piperidine-4-carboxamide

### 133a) tert-Butyl (S)-4- [N-[2-(6-chloro-2-naphthyl)sulfonylmethyl-1-pyrrolidyl]carbamoyl]piperidine-1-carboxylate

To a mixture of (S)-2-(6-chloro-2-naphthyl)sulfonylmethylpyrrolidine hydrobromide (2.4 g) obtained in Example 113e), acetic acid (24 ml) and water (2.4 ml), sodium nitrite (2.12 g) was added at 0°C, and stirred at room temperature for further 2 hours. The solvent was removed under reduced pressure, and the residue was dissolved in ether, and washed with water, and then dried over anhydrous magnesium sulfate. The solvent was removed under reduced pressure to obtain an oil, which was dissolved in acetic acid (18 ml) and water (24 ml), and zinc powder (1.69 g) was added and stirred at room temperature for 2 hours. The insoluble materials were filtered off, and the filtrate was dissolved in methylene chloride, and washed with aqueous saturated sodium bicarbonate solution, and dried over anhydrous magnesium sulfate.

1-tert-Butoxycarbonylpiperidine-4-carboxylic acid (1.41 g), HOBt (1.13 g) and WSC (1.77 g) were added to the methylene chloride solution above obtained, and stirred at room temperature for 2 hours. The reaction mixture was washed with aqueous saturated sodium bicarbonate solution, and dried over anhydrous magnesium sulfate. The solvent was removed under reduced pressure, and the residue was purified by a silica gel column to obtain the title compound as a colorless powder (1.88 g, 57%).
NMR (CDCl₃) δ: 1.44 and 1.46 (total 9H, each s), 1.48-1.98 (7H, m), 2.08-2.58 (2H, m), 2.60-3.02 (3H, m), 3.10-3.36 (2H, m), 3.40-3.62 (2H, m), 3.96-4.26 (2H, m), 5.92 and 6.68 (total 1H, each s), 7.60 (1H, dd, J = 2.1 and 8.7), 7.82-8.00 (4H, m), 8.44 and 8.46 (total 1H, each s).

### 133b) (S)-N-[2-(6-Chloro-2-naphthyl)sulfonylmethyl-1-pyrrolidyl]-1-(2-methyl-4-pyridyl)piperidine-4-carboxamide

From tert-Butyl (S)-4-[N-[2-(6-chloro-2-naphthyl)sulfonylmethyl-1-pyrrolidyl]carbamoyl]piperidine-1-carboxylate obtained in Example 133a), the title compound was obtained as a brown powder (22%) according to the same manner as that of Example 83c).
NMR (CDCl₃) δ: 1.48-1.96 (7H, m), 2.04-2.50 (2H, m), 2.43 (3H, s), 2.70-3.10 (3H, m), 3.16-3.38 (2H, m), 3.42-3.68 (2H, m), 2.74-3.96 (2H, m), 6.40-6.56 (2H, m), 7.59 (1H, dd, J = 1.8 and 8.8), 7.82-8.00 (4H, m), 8.11 (1H, d, J = 5.8), 8.45 (1H, d, J = 1.2).

| Elemental Analysis: C₂₇H₃₁N₄O₃SCl·1.5H₂O | | | |
|---|---|---|---|
| Calcd(%) | C, 58.53; | H, 6.18; | N, 10.11 |
| Found(%) | C, 58.67; | H, 5.86; | N, 10.06 |

### Example 134

### 1-[3-(6-Chloro-2-naphthyl)sulfonylpropanoyl]-N-methyl-4-(2-methyl-4-pyridyl)piperazine-2-carboxamide

### 134a) 1-Benzyloxy-4-(2-methyl-4-pyridyl)piperazine-2-carboxylic acid

A solution of 1-benzyl 2-methyl 4-(2-methyl-4-pyridyl)piperazine-1,2-dicarboxylate (1.30 g) obtained in Example 131a) and 1N aqueous sodium hydroxide (7.0 ml) in methanol (10 ml) was stirred at room temperature for 2 hours, and then neutralized with 1N HCl. The reaction mixture was concentrated under reduced pressure, and the residue was purified by XAD-2 column to obtain the title compound as a light yellow powder (1.39g, quantitative).
NMR (CD₃OD) δ: 2.49 (3H, s), 3.32-4.81 (7H, m), 5.17-5.18 (2H, m), 6.99 (2H, br), 7.33-7.38 (5H, m), 7.97 (1H, d, J = 7.6).

### 134b) Benzyl 2-(N-methylcarbamoyl)-4-(2-methyl-4-pyridyl)piperidine-1-carboxylate

From 1-benzyloxy-4-(2-methyl-4-pyridyl)piperazine-2-carboxylic acid obtained in Example 134a) and methylamine, the title compound was obtained as a colorless powder (81%) according to the same manner as that of Example 76b).
NMR (CDCl₃) δ: 2.45 (3H, s), 2.79 (3H, d, J = 4.8), 2.95-3.61 (4H, m), 4.10-4.79 (3H, m), 5.20 and 5.22 (2H, each s), 6.52-6.59 (2H, m), 7.38 (5H, br), 8.18 (1H, d, J = 5.8).

### 134c) N-Methyl-4-(2-methyl-4-pyridyl)piperidine-2-carboxamide

From benzyl 2-(N-methylcarbamoyl)-4-(2-methyl-4-pyridyl)piperidine-1-carboxylate obtained in Example 134b), the title compound was obtained as a colorless oil (quantitative) according to the same manner as that of Example 129b).
NMR (CDCl₃) δ: 2.45 (3H, s), 2.85 (3H, d, J = 5.1), 2.96-3.17 (4H, m), 3.47-3.54 (3H, m), 3.81-3.86 (1H, m), 6.55 (1H, dd, J = 2.6 and 6.0), 6.60 (1H, d, J = 2.4), 6.99 (1H, br), 8.18 (1H, d, J = 4.0).

### 134d) 1-[3-(6-Chloro-2-naphthyl)sulfonylpropanoyl]-N-methyl-4-(2-methyl-4-pyridyl)piperazine-2-carboxamide

From N-methyl-4-(2-methyl-4-pyridyl)piperidine-2-carboxamide obtained in Example 134c), the title compound was obtained as a colorless powder (30%) according to the same manner as that of Example 76b).
NMR (CDCl₃) δ: 2.47 (3H, s), 2.73-5.22 (14H, m), 6.54-6.61 (2H, m), 6.72-6.74 (1H, m), 7.62 (1H, dd, J = 2.0 and 8.8), 7.88-8.01 (4H, m), 8.20 (1H, d, J = 5.8), 8.48 (1H, s).

| Elemantal Analysis: C₂₅H₂₇N₄ClO₄S·0.5H₂O | | | |
|---|---|---|---|
| Calcd(%) | C, 57.30; | H, 5.39; | N, 10.69 |
| Found(%) | C, 57.47; | H, 5.44; | N, 10.38 |

### Example 135

### Ethyl 2-[1-[3-(6-chloro-2-naphtyl)sulfonylpropanoyl]-4-(2-methyl-4-pyridyl)-2-piperazyl]acetate

### 135a) Ethyl 2-[4-(2-methyl-4-pyridyl)-2-piperazyl]acetate

The title compound was obtained from ethyl 2-(2-piperazyl)acetate (JP-A 3-232864) as described in Example 90e) as yellow oil (74%).
NMR (CDCl₃) δ: 1.29 (3H, t, J = 7.1), 1.64 (1H, br), 2.44-3.70 (12H, m), 4.18 (2H, q, J = 7.1), 6.50-6.54 (2H, m), 8.18 (1H, d, J = 6.2).

### 135b) Ethyl 2-[1-[3-(6-Chloro-2-naphtyl)sulfonyl propanoyl]-4-(2-methyl-4-pyridyl)-2-piperazyl]acetate

The title compound was obtained from ethyl 2-[4-(2-methyl-4-pyridyl)-2-piperazyl]acetate obtained in Example 135a) as described in Example 76b) as yellow powder (50%).
NMR (CDCl₃) δ: 1.18-1.30 (3H, m), 2.46 (3H, s), 2.59-3.07 (6H, m), 3.45-4.96 (9H, m), 6.45-6.49 (2H, m), 7.56-7.62 (1H, m), 7.95-7.97 (4H, m), 8.20 (1H, d, J = 5.8), 8.49 (1H, d, J = 3.0).

| Elemental Analysis: C₂₇H₃₀N₃ClO₅S·0.5H₂O | | | |
|---|---|---|---|
| Calcd (%) | C, 58.63; | H, 5.65; | N, 7.60 |
| Found (%) | C, 58.33; | H, 5.72; | N, 7.56 |

### Example 136

### 2-(6-Chloro-2-naphtyl)sulfonyl-N-[4-(2-methyl-4-pyridyl)-1-piperazyl]acetamide and 1-[2-(6-chloro-2-naphtyl)sulfonylacetyl]-4-(2-methyl-4-pyridyl)piperazine

### 136a) 1-(2-Methyl-4-pyridyl)-4-nitrosopiperazine

An solution of sodium nitrite (0.41 g) in water (2 ml) was added slowly to a solution of 4-(2-methyl-4-pyridyl)-1-piperazine dihydrochloride (1.00 g) in water (10 ml) obtained in Example 130b) at 0°C. The mixture was stirred at the temperature for two hours. A residue obtained by concentrating the reaction mixture under reduced pressure was dissolved in ethanol, and insoluble substances were filtered off. The filtrate was concentrated to obtain the title compound as a brown solid (0.97 g, quantitative).
NMR (CDCl₃) δ: 2.56 (3H, s), 3.93 (4H, s), 3.95-4.01 (2H, m), 4.58-4.64 (2H, m), 7.07-7.11 (2H, m), 8.07-8.11 (1H, m).

### 136b) 4-(2-Methyl-4-pyridyl)-1-piperazylamine

A solution of 1-(2-methyl-4-pyridyl)-4-nitrosopiperazine (0.97 g) obtained in Example 136a) in acetic acid (5 ml) was added to a suspension of zinc dust (1.10 g) in water (5 ml). The mixture was stirred vigorously at room temperature for 15 hours. The reaction mixture was heated to 80°C and filtered while heating. The filtrate was basified with 40% aqueous potassium hydroxide and extracted with methylene chloride. The extract was dried over anhydrous sodium sulfate, and then concentrated under reduced pressure, and the residue was purified on basic silica gel column to obtain the title compound as a yellow oil containing 4-(2-methyl-4-pyridyl)-1-piperazine (0.52 g, 68%).
NMR (CDCl₃) δ: 2.45 (3H, s), 2.74 (2H, t, J = 5.1), 2.97-3.02 (2H, m), 3.26-3.31 (2H, m), 3.38 (2H, t, J = 5.2), 6.49-6.55 (2H, m), 8.19 (1H, d, J = 6.0).

### 136c) 2-(6-Chloro-2-naphtyl)sulfonyl-N-[4-(2-methyl-4-pyridyl)-1-piperazyl]acetamide and 1-[2-(6-chloro-2-naphtyl)sulfonylacetyl]-4-(2-methyl-4-pyridyl)piperazine

The title compound was obtained from 4-(2-methyl-4-pyridyl)-1-piperazylamine containing 4-(2-methyl-4-pyridyl)-1-piperazine obtained in Example 136b) as described in Example 30b).
2-(6-Chloro-2-naphtyl)sulfonyl-N-[4-(2-methyl-4-pyridyl)-1-piperazyl]acetamide: yellow powder (18%), NMR (CDCl₃) δ: 2.47 and 2.48 (3H, each s), 2.65 (1H, m), 2.98-4.55 (10H, m), 6.45-6.55 (2H, m), 7.41-7.97 (5H, m), 8.19-8.24 (1H, m), 8.48-8.50 (1H, m).

| Elemental Analysis: C₂H₂₃N₄ClO₃S·0.5H₂O | | | |
|---|---|---|---|
| Calcd (%) | C, 56.46; | H, 5.17; | N, 11.97 |
| Found (%) | C, 56.19; | H, 5.41; | N, 11.87 |

1-[2-(6-Chloro-2-naphtyl)sulfonylacetyl]-4-(2-methyl-4-pyridyl)piperazine: colorless powder (27%), NMR (CDCl₃) δ: 2.49 (3H, s), 3.34-3.87 (8H, m), 4.36 (2H, s), 6.51-6.57 (2H, m), 7.59 (1H, dd, J = 1.8 and 8.8), 7.88-7.97 (4H, m), 8.24 (1H, d, J = 6.0), 8.48 (1H, m).

| Elemental Analysis: C₂₂H₂₂N₃ClO₃S·0.3H₂O | | | |
|---|---|---|---|
| Calcd (%) | C, 58.80; | H, 5.07; | N, 9.35 |
| Found (%) | C, 58.66; | H, 5.15; | N, 9.72 |

### Example 137

### 3-(6-Chloro-2-naphtyl)sulfonyl-N-[4-(2-methyl-4-pyridyl)-1-piperazyl]propaneamide

The title compound was obtained from 4-(2-methyl-4-pyridyl)-1-piperazylamine containing 4-(2-methyl-4-pyridyl)-1-piperazine obtained in Example 136b) as described in Example 30b) as colorless powder (29%).
NMR (CDCl₃) δ: 2.45 and 2.48 (3H, each s), 2.62-2.69 (2H, m), 2.89-3.14 (6H, m), 3.41-3.59 (4H, m), 3.75 (1H, br), 6.27-6.63 (3H, m), 7.56-7.64 (1H, m), 7.93-7.97 (4H, m), 8.17-8.24 (1H, m), 8.47-8.50 (1H, m).

| Elemental Analysis: C₂₃H₂₅N₄ClO₃S·0.8H₂O | | | |
|---|---|---|---|
| Calcd (%) | C, 56.68; | H, 5.50; | N, 11.49 |
| Found (%) | C, 56.50; | H, 5.23; | N, 11.32 |

### Example 138

### 3-(5-Chloro-2-benzothienyl)sulfonyl-N-methyl-N-[1-(2-methyl-4-pyridyl)-4-piperidyl]propaneamide

### 138a) 5-Chlorobenzothiophene-2-sulfonyl chloride

Sulfuryl chloride (1.36 ml) was added dropwise at 0°C to DMF (1.46 g), and the mixture was stirred at room temperature for 15 minutes, and then 5-chlorobenzothiophene (Pla P. A. et al., J. Heterocyclic Chem., 1988, *25*, 1271) (1.68 g) was added thereto. The resulting mixture was stirred at 90°C for 3 hours. Ice water was added to the reaction mixture, and the mixture was extracted with ethyl acetate. The extract was washed with water, dried over anhydrous sodium sulfate, and then concentrated under reduced pressure. The residue was purified on silica gel column, recrystallized from hexane to obtain the title compound as colorless crystals (0.52 g, 20%).
NMR (CDCl₃) δ: 7.54 (1H, dd, J = 1.8 and 8.8), 7.88 (1H, d, J = 8.8), 8.30 (1H, d, J = 1.8), 8.56 (1H, s).

### 138b) tert-Butyl 3-(5-chloro-2-benzothienyl)sulfonylpropionate

5-Chlorobenzothiophene-2-sulfonyl chloride (0.43 g) obtained in Example 138a) was added to a suspension of sodium borohydride (0.12 g) in THF (10 ml), and the mixture was stirred at 40°C for 7 hours. Ice water was added to the reaction mixture, the mixture was adjusted to pH 12 with 10% hydrochloric acid, and the mixture was extracted with ethyl acetate. The extract was dried over anhydrous sodium sulfate and concentrated. The residue was dissolved in ethyl acetate (50 ml). Triethylamine (1.4 ml) and tert-butyl acrylate (1.03 g) were added thereto. The mixture was refluxed for 20 hours. The reaction mixture was diluted with ethyl acetate, and the mixture was adjusted to pH 2 with dilute hydrochloric acid. The mixture was extracted with ethyl acetate, and the extract was dried over anhydrous sodium sulfate. The solvent was distilled off under reduced pressure, and the residue was purified on silica gel column followed by recrystallization from hexane/ethyl acetate to obtain the title compound as colorless crystals (0.25 g, 44%).
NMR (CDCl₃) δ: 1.39 (9H, s), 2.71 (2H, t, J = 7.7), 3.52 (2H, t, J = 7.7), 7.47 (1H, dd, J = 1.8 and 8.8), 7.85 (1H, d, J = 8.8), 8.26 (1H, d, J = 1.8), 8.39 (1H, s).

### 138c) 3-(5-Chloro-2-benzothienyl)sulfonylpropionic acid

Trifluoroacetic acid (2 ml) was added to a solution of tert-butyl 3-(5-chloro-2-benzothienyl)sulfonylpropionate (0.28 g) obtained in Example 138b) in toluene (2 ml), and the mixture was stirred at room temperature for 1 hour. The reaction mixture was concentrated under reduced pressure, and the residue was crystallized from hexane/ethyl acetate to obtain the title compound as colorless crystals (0.24 g, 98%).
NMR (CDCl₃+DMSO-d₆) δ: 2.75 (2H, t, J = 7.7), 3.55 (2H, t, J = 7.7), 7.47 (1H, dd, J = 2.2 and 8.4), 7.87 (1H, d, J = 8.4), 8.25 (1H, d, J = 2.2), 8.42 (1H, s).

### 138d) 3-(5-Chloro-2-benzothienyl)sulfonyl-N-methyl-N-[1-(2-methyl-4-pyridyl)-4-piperidyl]propaneamide

The title compound was obtained from 3-(5-chloro-2-benzothienyl)sulfonylpropionic acid obtained in Example 138c) and 4-methylamine-1-(2-methyl-4-pyridyl)piperidine obtained in Example 42b) as described in Example 30b) as colorless crystals (50%).
NMR (CDCl₃) δ: 1.40-2.00 (5H, m), 2.44 (2.25H, s), 2.47 (0.75H, s), 2.76 (0.75H, s), 2.78-3.10 (4H,m), 2.84 (2.25H, s), 3.52-3.76 (2H, m), 3.72-4.10 (2.25H, m), 4.48-4.80 (0.25H, m), 6.40-6.60 (2H, m), 7.47 (1H, dd, J = 1.8 and 8.8), 7.86 (1H, d, J = 8.8), 8.16 (1H, d, J = 5.8), 8.25 (1H, d, J = 1.8), 8.40 (0.75H, s), 8.42 (0.25H, s).

| Elemental Analysis: C₂₃H₂₆N₃ClO₃S₂·0.25EtOH·0.25H₂O | | | |
|---|---|---|---|
| Calcd (%) | C, 55.55; | H, 5.55; | N, 8.27 |
| Found (%) | C, 55.58; | H, 5.41; | N, 8.12 |

### Example 139

### 3-(6-Chloro-2-benzothienyl)sulfonyl-N-methyl-N-[1-(2-methyl-4-pyridyl)-4-piperidyl]propaneamide

### 139a) 6-Chlorobenzothiophene-2-sulfonyl chloride

The title compound was obtained from 6-chlorobenzothiophene (WO 98/24784) as described in Example 138a) (10%).
NMR (CDCl₃) δ: 7.60(1H, dd, J = 1.8 and 8.8), 7.95 (1H, d, J = 1.8), 8.24 (1H, d, J = 8.8), 8.50 (1H, s).

### 139b) tert-Butyl 3-(6-chloro-2-benzothienyl)sulfonylpropionate and 3-(6-chloro-2-benzothienyl)sulfonylpropionic acid

The title compound was obtained from 6-chlorobenzothiophene-2-sulfonyl chloride obtained in Example 139a) as described in Example 138b).
tert-Butyl 3-(6-chloro-2-benzothienyl)sulfonylpropionate: colorless crystal (8%), NMR (CDCl₃) δ: 1.39 (9H, s), 2.70 (2H, t, J = 7.7), 3.50 (2H, t, J = 7.7), 7.53 (1H, dd, J = 1.8 and 8.8), 7.92 (1H, d, J = 1.8), 8.19 (1H, d, J = 8.8), 8.33 (1H, s).
3-(6-Chloro-2-benzothienyl)sulfonylpropionic acid: crystal (16%), NMR (CDCl₃+DMSO-d₆) δ: 2.74 (2H, t, J = 7.5), 3.54 (2H, t, J = 7.5), 7.52 (1H, dd, J = 1.8 and 8.8), 7.94 (1H, d, J = 1.8), 8.18 (1H, d, J = 8.8), 8.33(1H, s).

### 139c) 3-(6-Chloro-2-benzothienyl)sulfonyl-N-methyl-N-[1-(2-methyl-4-pyridyl)-4-piperidyl]propaneamide

The title compound was obtained from 3-(6-chloro-2-benzothienyl)sulfonylpropionic acid obtained in Example 139b) as described in Example 30b) as colorless powder (50%).
NMR (CDCl₃) δ: 1.45-2.00 (5H, m), 2.45 and 2.47(total 3H, each s), 2.70-3.10 (4H, m), 2.77 and 2.83 (total 3H, each s), 3.50-3.70 (2H, m), 3.70-4.10 (2.25H, m), 4.48-4.80 (0.75H, m), 6.40-6.60 (2H, m), 7.52 (1H, dd, J = 1.8 and 8.8), 7.93 (1H, d, J = 1.8), 8.10-8.20 (1H, m), 8.19 (1H, d, J = 8.8), 8.34 and 8.35 (total 1H, each s).

| Elemental Analysis: C₂₃H₂₆ClN₃O₃S₂·0.25H₂O | | | |
|---|---|---|---|
| Calcd (%) | C, 55.56; | H, 5.38; | N, 8.46 |
| Found (%) | C, 55.58; | H, 5.41; | N, 8.35 |

### Example 140

### 3-(5-Chloro-2-benzofuranyl)sulfonyl-N-methyl-N-[1-(2-methyl-4-pyridyl)-4-piperidyl]propaneamide

### 140a) 1-Chloro-4-(2,2-diethoxyethoxy)benzene

4-Chlorophenol (12.9 g), potassium carbonate (13.8 g) and 1-bromo-2,2-diethoxyethane (17.7 g) were added to DMF (100 ml). The mixture was stirred at 150°C for 17 hours. DMF was distilled off under reduced pressure. Water was added to the residue, and the mixture was extracted with ethyl acetate. The extract was washed with water, and dried over anhydrous sodium sulfate. The solvent was distilled off under reduced pressure to obtain the title compound (24.5 g, quantitative).
NMR (CDCl₃) δ: 3.46 (6H, s), 3.97 (2H, d, J = 5.0), 4.70 (1H, t, J = 5.0), 6.86 (2H, d, J = 8.8), 7.23 (2H, d, J = 8.8).

### 140b) 5-Chlorobenzofuran

1-Chloro-4-(2,2-diethoxyethoxy)benzene (24.5 g) obtained in Example 140a) was added dropwise to a mixture of polyphosphoric acid (60 g) and 4-chlorobenzene (300 ml) while refluxing, and the resulting mixture was refluxed for 14 hours. Ice water was added to the reaction mixture, and the mixture was extracted with hexane. The extract was washed with water, and dried over anhydrous sodium sulfate. The solvent was distilled off, and the residue was purified on silica gel column to obtain the title compound as a pale yellow oil (10 g, 66%).
NMR (CDCl₃) δ: 6.73 (1H, dd, J = 2.2 and 2.2), 7.25 (1H, dd, J = 2.2 and 8.8), 7.43 (1H, d, J = 8.8), 7.57 (1H, d, J = 2.2), 7.64 (1H, d, J = 2.2).

### 140c) 5-Chlorobenzofuran-2-sulfonyl chloride

Chlorosulfonic acid (6.5 g) was added dropwise to phosphorus pentachloride (4.6 g) under nitrogen flow, the mixture was stirred for 10 minutes, 5-chlorobenzofuran (3.4 g) obtained in Example 140b) was added thereto, and the mixture was stirred at 55° C for 15 minutes. Ice water was added to the reaction mixture, the mixture was extracted with ethyl acetate, and the extract was dried over anhydrous sodium sulfate. The solvent was distilled off, and the residue was purified on silica gel column to obtain the title compound as colorless crystals (2.1 g, 39%).
NMR (CDCl₃) δ: 7.49 (1H, dd, J = 2.2 and 8.8), 7.59 (1H, d, J = 8.8), 7.93 (1H, d, J = 2.2), 8.39 (1H, s).

### 140d) tert-Butyl 3-(5-chloro-2-benzofuran)sulfonylpropionate

The title compound was obtained from 5-chlorobenzofuran-2-sulfonyl chloride obtained in Example 140c) as described in Example 138b) as colorless crystals (11%).
NMR (CDCl₃) δ: 1.39 (9H, s), 2.75 (2H, t, J = 7.7), 3.53 (2H, t, J = 7.7), 7.42 (1H, dd, J = 2.2 and 8.8), 7.54 (1H, d, J = 8.8), 7.87 (1H, d, J = 2.2), 8.21 (1H, s).

### 140e) 3-(5-Chloro-2-benzofuran)sulfonylpropionic acid

The title compound was obtained from tert-butyl 3-(5-chloro-2-benzofuran)sulfonylpropionate obtained in Example 140d) as described in Example 138c) as colorless crystals (79%).
NMR (CDCl₃+DMDO-d₆) δ: 2.80 (2H, t, J = 7.7), 3.57 (2H, t, J = 7.7), 7.42 (1H, dd, J = 2.2 and 8.8), 7.55 (1H, d, J = 8.8), 7.87 (1H, d, J = 2.2), 8.24 (1H, s).

### 140f) 3-(5-Chloro-2-benzofuranyl)sulfonyl-N-methyl-N-[1-(2-methyl-4-pyridyl)-4-piperidyl]propanamide

The title compound was obtained from 3-(5-chloro-2-benzofuran)sulfonylpropionic acid obtained in Example 140e) as described in Example 30b) as colorless powder (35%).
NMR (CDCl₃) δ: 1.50-1.90 (4H, m), 2.45 and 2.47 (total 3H, each s), 2.76 and 2.84 (total 3H, each s), 2.80-3.10 (4H, m), 3.50-3.75 (2H, m), 3.80-4.15 (2H, m), 4.40-4.80 (1H, m), 6.45-6.60 (2H, m), 7.30-7.60 (2H, m), 7.86 (1H, d, J = 2.2), 8.16 (1H, d, J = 6.2), 8.24 and 8.25(total 1H, each s).

| Elemental Analysis: C₂₃H₂₆ClN₃O₄S | | | |
|---|---|---|---|
| Calcd (%) | C, 58.04; | H, 5.51; | N, 8.83 |
| Found (%) | C, 57.89; | H, 5.78; | N, 9.11 |

### Example 141

### N-Methyl-N-[1-(2-methyl-4-pyridyl)-4-piperidyl]-3-[4-(4-pyridyl)phenyl]sulfonylpropanamide

### 141a) tert-Butyl 3-[4-(4-pyridyl)phenyl]sulfonylpropionate

tert-Butyl 3-(4-bromophenyl)sulfonylpropionate obtained in Example 59b) (1.40 g), 4-pyridyl boric acid (0.50 g) and tetrakistriphenylphosphonium palladium (0.25 g) were added to a mixture of 2 M aqueous sodium carbonate solution (4 ml) and dimethoxyethane (15 ml), and the mixture was stirred under argon atmosphere for 15 hours. The reaction mixture was diluted with water, and extracted with ethyl acetate. The extract was dried over anhydrous sodium sulfate, the solvent was distilled off under reduced pressure, and the residue was purified on silica gel column to obtain the title compound as colorless solid (0.19 g, 14%).
NMR (CDCl₃) δ: 1.40 (9H, s), 2.70 (2H, t, J = 7.7), 3.45 (2H, t, J = 7.7), 7.53 (2H, d, J = 6.2), 7.82 (2H, d, J = 8.2), 8.04 (2H, d, J = 8.2), 8.75 (2H, d, J = 6.2).

### 141b) N-Methyl-N-[1-(2-methyl-4-pyridyl)-4-piperidyl]-3-[4-(4-pyridyl)phenyl]sulfonylpropanamide

A solution of tert-butyl 3-[4-(4-pyridyl)phenyl]sulfonylpropionate obtained in Example 141a) (0.19 g) in TFA (5 ml) was stirred at room temperature for 1 hour, and concentrated under reduced pressure. The residue was dissolved in DMF (10 ml), 4-methylamine-1-(2-methyl-4-pyridyl)piperidine obtained in Example 42b) (0.20 g) and DMTMM (0.42 g) were added thereto, and the mixture was stirred at room temperature for 15 hours. The reaction mixture was concentrated under reduced pressure, aqueous sodium bicarbonate was added to the residue, and the mixture was extracted with methylene chloride. The extract was dried over anhydrous sodium sulfate, the solvent was distilled off, and the residue was purified on silica gel column to obtain the title compound as colorless solid (15 mg, 6%).
NMR (CDCl₃) δ: 1.50-1.95 (4H, m), 2.65 and 2.68 (3H, s), 3.12 (2H, m), 3.54 (2H, t, J = 7.6), 4.07 (2H, m), 4.76 (1H, m), 6.50-6.64 (2H, m), 7.54 (2H, d, J = 6.4), 7.84 (2H, d, J = 8.4), 8.00-8.20 (3H, m), 8.75 (2H, d, J = 6.4).

### Example 142

### 3-(6-Chloro-2-indolyl)sulfonyl-N-methyl-N-[1-(2-methyl-4-pyridyl)-4-piperidyl]propanamide

### 142a) 6-Chloro-1,3-dihydro-2H-indole-2-thione

6-Chlorooxyindole(8.38 g) and sodium bicarbonate (8.4 g) were suspended in THF (75 ml), phosphorus pentasulfide (6.75 g) was added thereto, and the mixture was stirred at room temperature for 12 hours. Insoluble substances were filtered off, and the filtrate was concentrated under reduced pressure. Water was added to the residue, the mixture was extracted with chloroform, and the extract was washed with water and saturated aqueous sodium chloride, and concentrated. The residue was recrystallized from chloroform to obtain the title compound as yellow crystals (2.56 g, 28%).
NMR (CDCl₃) δ: 4.04 (2H, s), 6.97 (1H, s), 7.14 (1H, d, J = 8.0), 7.29 (1H, d, J = 8.0), 12.65 (1H, s).

### 142b) 3-(6-Chloro-1H-indol-2-yl)thiopropanic acid

The title compound was obtained from 6-chloro-1,3-dihydro-2H-indole-2-thione obtained in Example 142a) as described in Example 114a) as pale yellow crystal (72%).
NMR (CDCl₃ +DMSO-d₆) δ: 2.63 (2H, t, J = 7.0), 3.07 (2H, t, J = 7.0), 6.60 (1H, dd, J = 0.6 and 1.8), 7.03 (1H, dd, J = 1.8 and 8.4), 7.30-7.35 (1H, m), 7.43 (1H, d, J = 8.4), 9.63 (1H, bs).

### 142c) 3-(6-Chloro-2-indolyl)thio-N-methyl-N-[1-(2-methyl-4-pyridyl)-4-piperidyl]propanamide

The title compound was obtained from 3-(6-chloro-1H-indol-2-yl)thiopropanic acid obtained in Example 142b) as described in Example 30b) as pale yellow crystals (55%).
NMR (CDCl₃) δ: 1.40-2.00 (4H, m), 2.45 (3H, s), 2.50-3.28 (6H, m), 2.79 (3H, s), 3.80-4.10 (2H, m), 4.60-4.90 (1H, m), 6.40-6.60 (2H, m), 7.05 (1H, dd, J = 1.8 and 8.4), 7.36 (1H, s), 7.43 (1H, d, J = 8.4), 8.17 (0.75H, d, J = 5.6), 8.19 (0.25H, d, J = 5.6), 9.97 (1H, bs).

### 142d) 3-(6-Chloro-2-indolyl)sulfonyl-N-methyl-N-[1-(2-methyl-4-pyridyl)-4-piperidyl]propanamide

1 N Hydrochloric acid (0.55 ml) was added to a solution of 3-(6-chloro-2-indolyl)thio-N-methyl-N-[1-(2-methyl-4-pyridyl)-4-piperidyl]propanamide (0.12 g) obtained in Example 142c) in methanol (6 ml), 50% mCPBA (0.14 g) was added thereto, and the mixture was stirred at room temperature for 1.5 hours. The reaction mixture was concentrated under reduced pressure, an aqueous sodium carbonate was added to the residue to basify it, and the mixture was extracted with ethyl acetate. The extract was washed with saturated aqueous sodium bicarbonate, and dried over anhydrous sodium sulfate. The solvent was distilled off under reduced pressure, and the residue was purified on silica gel column. The product was re-crystallized from methanol/ether to obtain the title compound as colorless crystal (0.75 g, 93%).
NMR (CDCl₃) δ: 1.40-1.95 (4H, m), 2.45 (2.25H, s), 2.48 (0.75H, s), 2.70-3.10 (4H, m), 2.78 (0.75H, s), 2.83 (2.25H, s), 3.70 (3H, t, J = 7.3), 3.75-4.10 (2H, m), 4.45-4.80 (1H, m), 6.40-6.60 (2H, m), 7.18 (1H, dd, J = 1.8 and 8.4), 7.19 (1H, d, J = 0.8), 7.46 (1H, d, J = 0.8), 7.63 (1H, d, J = 8.4), 8.17 (0.75H, d, J = 5.8), 8.21(0.25H, d, J = 5.8).

| Elemental Analysis: C₂₃H₂₇ClN₄O₃S·0.25H₂O | | | |
|---|---|---|---|
| Calcd (%) | C, 57.61; | H, 5.78; | N, 11.68 |
| Found (%) | C, 57.63; | H, 5.69; | N, 11.66 |

### Example 143

### Methyl 1-[3-(6-chloro-2-naphtyl)sulfonylpropanoyl]-4-(2-methyl-4-pyridyl)piperazine-2-carboxylate

### 143a) Ethyl 4-(2-methyl-4-pyridyl)piperazine-2-dicarboxylate

The title compound was obtained from ethyl piperazine-2-carboxylate (JP-A 3-232864) as described in Example 90e) as a yellow oil (75%).
NMR (CDCl₃) δ: 1.31 (3H, t, J = 7.1), 2.46 (3H, s), 2.85-3.79 (7H, m), 4.23 (2H, q, J = 7.1), 6.53-6.59 (2H, m), 8.19 (1H, d, J = 5.8).

### 143b) Methyl 1-[3-(6-chloro-2-naphtyl)sulfonylpropanoyl]-4-(2-methyl-4-pyridyl)piperazine-2-carboxylate

The title compound was obtained from ethyl 4-(2-methyl-4-pyridyl)piperazine-2-dicarboxylate obtained in Example 143a) as described in Example 30b) as yellow powder (35%).
NMR (CDCl₃) δ: 1.19-1.25 (3H, m), 2.45 (3H, s), 2.92-3.13 (4H, m), 3.54-3.83 (4H, m), 4.09-5.16 (5H, m), 6.49-6.54 (2H, m), 7.60 (1H, dd, J = 2.0 and 8.8), 7.91-7.97 (4H, m), 8.22 (1H, d, J = 3.8), 8.50 (1H, s).

| Elemental Analysis: C₂₆H₂₈N₃ClO₅S | | | |
|---|---|---|---|
| Calcd (%) | C, 58.92; | H, 5.32; | N, 7.93 |
| Found (%) | C, 58.64; | H, 5.57; | N, 8.22 |

### Example 144

### tert-Butyl 1-[3-(6-chloro-2-naphtyl)sulfonylpropanoyl]-4-(2-methyl-4-pyridyl)piperazine-2-carboxylate

### 144a) tert-Butyl 1-benzyloxy-4-(2-methyl-4-pyridyl)piperazine-2-carboxylate

A solution of 1-benzyl 2-methyl 4-(2-methyl-4-pyridyl)piperazine-1,2-dicarboxylate obtained in Example 131a) (4.91 g) and 1N aqueous sodium hydroxide (50 ml) in ethanol (50 ml) was stirred at room temperature for 4 hours, and 1 N hydrochloric acid (50 ml) was added to the reaction mixture. The mixture was concentrated under reduced pressure. The residue was purified on XAD-2 column, the resulting carboxylate was mixed with N,N-dimethylformamide dineopentylacetal (45 ml), tert-butanol (50 ml) and DMF (40 ml), and the mixture was refluxed for 15 hours. The reaction mixture was concentrated under reduced pressure, the residue was diluted with ethyl acetate, the mixture was washed with aqueous potassium carbonate, and dried over anhydrous sodium sulfate. The solvent was distilled off under reduced pressure to obtain the title compound as a yellow oil (5.10 g, 97%).
NMR (CDCl₃) δ: 1.33 and 1.38 (9H, each s), 2.45 (3H, s), 2.92-5.28 (9H, m), 6.50-6.54 (2H, m), 7.34-7.38 (5H, each s), 8.19 (1H, d, J = 6.2).

### 144b) tert-Butyl 4-(2-methyl-4-pyridyl)piperazine-2-carboxylate

The title compound was obtained from tert-butyl 1-benzyloxy-4-(2-methyl-4-pyridyl)piperazine-2-carboxylate obtained in Example 144a) as described in Example 131b) as gray powder (quantitative).
NMR (CDCl₃) δ: 1.49 (9H, s), 2.53 (3H, s), 2.85-3.77 (8H, m), 6.58-6.62 (2H, m), 8.14-8.17 (1H, m).

### 144c) tert-Butyl 1-[3-(6-chloro-2-naphtyl)sulfonylpropanoyl]-4-(2-methyl-4-pyridyl)piperazine-2-carboxylate

The title compound was obtained from tert-butyl 4-(2-methyl-4-pyridyl)piperazine-2-carboxylate obtained in Example 144b) as described in Example 76b) as yellow powder (75%).
NMR (CDCl₃) δ: 1.36 (9H, s), 2.47 (3H, s), 2.87-5.06 (11H, m), 6.50-6.54 (2H, m), 7.60 (1H, dd, J = 1.8 and 8.8), 7.89-7.98 (4H, m), 8.22 (1H, d, J = 6.0), 8.49 (1H, s).

| Elemental Analysis: C₂₈H₃₂N₃ClO₅S·1.1H₂O | | | |
|---|---|---|---|
| Calcd (%) | C, 58.19; | H, 5.96; | N, 7.27 |
| Found (%) | C, 57.95; | H, 5.86; | N, 7.57 |

### Example 145

### 1-[3-(6-Chloro-2-naphtyl)sulfonylpropanoyl]-4-(2-methyl-4-pyridyl)piperazine-2-carboxylate

tert-Butyl 1-[3-(6-chloro-2-naphtyl)sulfonylpropanoyl]-4-(2-methyl-4-pyridyl)piperazine-2-carboxylate obtained in Example 144c) (0.24 g) was dissolved in trifluoroacetic acid (2 ml), and the solution was stirred at room temperature for 60 hours. The reaction mixture was concentrated under reduced pressure, and the residue was purified on XAD-2 column to obtain the title compound as yellow powder (86%).
NMR (CD₃OD) δ: 2.55 (3H, s), 2.92-4.08 (5H, m), 4.60-5.11 (3H, m), 7.05-7.07 (2H, m), 7.68 (1H, dd, J = 2.0 and 8.8), 7.96-8.17 (5H, m), 8.61 (1H, m).

| Elemental Analysis: C₂₄H₂₄N₃ClO₅S·HCl·0.5H₂O | | | |
|---|---|---|---|
| Calcd (%) | C, 52.65; | H, 4.79; | N, 7.68 |
| Found (%) | C, 52.45; | H, 4.95; | N, 7.52 |

### Example 146

### 4-[1-[3-(6-Chloro-2-naphtyl)sulfonylpropanoyl]-4-(2-methyl-4-pyridyl)-2-piperazyl]carbonylthiomorpholine 1-oxide

The title compound was obtained from 1-[3-(6-chloro-2-naphtyl)sulfonylpropanoyl]-4-(2-methyl-4-pyridyl)-2-piperazinecarboxylate obtained in Example 145) and thiomorpholine 1-oxide trifluoroacetate salt as described in Example 30b) as colorless powder (52%).
NMR (CDCl₃) δ: 2.23-5.02 (22H, m), 6.39-6.42 (2H, m), 7.59 (1H, dd, J = 1.9 and 7.8), 7.89-7.96 (4H, m), 8.21 (1H, d, J = 6.0), 8.47 (1H, s).

| Elemental Analysis: C₂₈H₃₁N₄ClO₅S·0.2C₆H₁₅N·H₂O | | | |
|---|---|---|---|
| Calcd (%) | C, 54.68; | H, 5.66; | N, 9.17 |
| Found (%) | C, 54.43; | H, 5.90; | N, 9.55 |

### Example 147

### tert-Butyl 2-[1-[3-(6-chloro-2-naphtyl)sulfonylpropanoyl]-4-(2-methyl-4-pyridyl)-2-piperazyl]acetate

### 147a) Ethyl 2-[1-Benzyloxycarbonyl-4-(2-methyl-4-pyridyl)-2-piperazyl]acetate

A solution of benzyl chlorocarbonate (3.89 g) in ethyl acetate (10 ml) was added dropwise to a solution of ethyl 2-[4-(2-methyl-4-pyridyl)-2-piperazyl]acetate obtained in Example 135a) (5.00 g) and triethylamine (2.88 g) in ethyl acetate (90 ml) at room temperature. The mixture was stirred at room temperature for 2 hours. The reaction mixture was washed with aqueous sodium bicarbonate, and dried over anhydrous sodium sulfate. The solvent was distilled off under reduced pressure, and the residue was purified on basic silica gel column to obtain the title compound as a yellow oil (7.69 g, quantitative).
NMR (CDCl₃) δ: 1.23 (3H, t, J = 7.1), 2.45 (3H, s), 2.56 (1H, dd, J = 4.8 and 15.6), 2.74 (1H, dd, J = 9.2 and 15.5), 2.95 (1H, dt, J = 3.6 and 11.7), 3.13 (1H, dd, J = 3.5 and 13.4), 3.24-3.30 (1H, m), 3.67-3.71 (1H, m), 3.88 (1H, m), 4.05 (1H, m), 4.11 (2H, q, J = 7.2), 4.71 (1H, m), 5.17 (2H, s), 6.45-6.51 (2H, m), 7.30-7.41 (5H, m), 8.18 (1H, d, J = 6.0).

### 147b) tert-Butyl 2-[1-benzyloxycarbonyl-4-(2-methyl-4-pyridyl)-2-piperazyl]acetate

The title compound was obtained from ethyl 2-[1-benzyloxycarbonyl-4-(2-methyl-4-pyridyl)-2-piperazyl]acetate obtained in Example 147a) as described in Example 144a) as a yellow oil (95%).
NMR (CDCl₃) δ: 0.91 (3H, s), 1.44 (6H, s), 2.44 (3H, s), 2.50-3.30 (5H, m), 3.66-4.14 (3H, m), 4.71 (1H, m), 5.17 (2H, s), 6.44-6.49 (2H, m), 7.33-7.38 (5H, m), 8.17 (1H, d, J = 6.0).

### 147c) tert-Butyl 2-[4-(2-methyl-4-pyridyl)-2-piperazyl]acetate

The title compound was obtained from tert-butyl 2-[1-benzyloxycarbonyl-4-(2-methyl-4-pyridyl)-2-piperazyl]acetate obtained in Example 147b) as described in Example 144b) as a yellow oil (quantitative).
NMR (CDCl₃) δ: 0.95 and 1.47 (9H, each s), 2.15 (1H, br), 2.36-2.65 (6H, m), 2.87-3.20 (4H, m), 3.65-3.69 (2H, m), 6.48-6.53 (2H, m), 8.17 (1H, d, J = 5.8).

### 147d) tert-Butyl 2-[1-[3-(6-chloro-2-naphtyl)sulfonylpropanoyl]-4-(2-methyl-4-pyridyl)-2-piperazyl]acetate

The title compound was obtained from tert-butyl 2-[4-(2-methyl-4-pyridyl)-2-piperazyl]acetate obtained in Example 147c) as described in Example 76b) as yellow powder (70%).
NMR (CDCl₃) δ: 1.38 and 1.41 (9H, each s), 2.46 (3H, s), 2.34-4.93 (13H, m), 6.45-6.50 (2H, m), 7.56-7.61 (1H, m), 7.90-8.02 (4H, m), 8.20 (1H, d, J = 6.0), 8.49 (1H, d, J = 6.6).

| Elemental Analysis: C₂₉H₃₄N₃ClO₅S·0.7H₂O | | | |
|---|---|---|---|
| Calcd (%) | C, 59.57; | H, 6.10; | N, 7.19 |
| Found (%) | C, 59.19; | H, 6.35; | N, 7.58 |

### Example148

### 2-[1-[3-(6-Chloro-2-naphtyl)sulfonylpropanoyl]-4-(2-methyl-4-pyridyl)-2-piperazyl]acetic acid

The title compound was obtained from tert-butyl 2-[1-[3-(6-chloro-2-naphtyl)sulfonylpropanoyl]-4-(2-methyl-4-pyridyl)-2-piperazyl]acetate obtained in Example 147d) as described in Example 145) as colorless powder (93%).
NMR (CDCl₃) δ: 2.28-4.51 (16H, s), 6.83 (2H, m), 7.59-7.64 (1H, m), 7.94-8.16 (5H, m), 8.58 (1H, s).

| Elemental Analysis: C₂₅H₂₆N₃ClO₅S·0.3NH₃·1.1H₂O | | | |
|---|---|---|---|
| Calcd (%) | C, 55.51; | H, 5.42; | N, 8.54 |
| Found (%) | C, 55.21; | H, 5.03; | N, 8.84 |

### Example 149

### [1-[3-(6-Chloro-2-naphtyl)sulfonylpropanoyl]-4-(2-methyl-4-pyridyl)-2-piperazyl]methanol

Sodium borohydride (0.76 g) was added to a solution of ethyl 4-(2-methyl-4-pyridyl)piperazine-2-dicarboxylate (0.50 g) obtained in Example 143a) in methanol (5 ml), the mixture was refluxed for 1 hour, neutralized with 1N hydrochloric acid, and the solvent was distilled off under reduced pressure. On the other hand, HOBt (0.16 g) and WSC (0.21 g) were added to a solution of 3-(6-chloro-2-naphtyl)sulfonylpropionic acid obtained in Example 27b) (0.21 g) in DMF (10 ml), the mixture was stirred at 0°C for 1 hour, and the above-obtained alcohol (0.20 g) and triethylamine (0.29 g) were added thereto. The reaction mixture was stirred at 0°C for 1 hour followed by at room temperature for 15 hours. The reaction mixture was concentrated under reduced pressure, and the residue was diluted with ethyl acetate and aqueous potassium carbonate. The organic layer was separated, and dried over anhydrous sodium sulfate. The residue obtained by distilling off the solvent was purified on basic silica gel column to obtain the title compound as colorless powder (35 mg, 8%).
NMR (CDCl₃) δ: 1.59 (1H, br), 2.44 and 2.46 (3H, each s), 2.78-3.11 (4H, m), 3.45-4.63 (9H, m), 6.44-6.49 (2H, m), 7.60 (1H, d, J = 9.0), 7.91-7.96 (4H, m), 8.13-8.19 (1H, m), 8.49 (1H, s).

| Elemental Analysis: C₂₄H₂₆N₃ClO₄S·0.7H₂O | | | |
|---|---|---|---|
| Calculated (%) | C, 57.58; | H, 5.52; | N, 8.39 |
| Found (%) | C, 57.73; | H, 5.49; | N, 8.07 |

### Example 150

### [1-[3-(6-Chloro-2-naphtyl)sulfonylpropanoyl]-4-(2-methyl-4-pyridyl)-2-piperazyl]ethanol

Ethyl 2-[4-(2-methyl-4-pyridyl)-2-piperazyl]acetate obtained in Example 135a) was reduced to an alcohol as described in Example 149). Subsequently, it was condensed with 3-(6-chloro-2-naphtyl)sulfonylpropionic acid obtained in Example 27b) as described in Example 76b) to obtain the title compound as colorless powder (37%).
NMR (CDCl₃) δ: 1.70-4.77 (18H, m), 6.46-6.51 (2H, m), 7.57-7.62 (1H, m), 7.95-7.98 (4H, m), 8.19-8.23 (1H, m), 8.49 (1H, s).

| Elemental Analysis: C₂₅H₂₈N₃ClO₄S·0.2THF·0.6H₂O | | | |
|---|---|---|---|
| Calcd (%) | C, 58.77; | H, 5.89; | N, 7.97 |
| Found (%) | C, 58.74; | H, 5.90; | N, 7.73 |

### Example 151

### 1-[3-(6-Chloro-2-naphtyl)sulfonylpropanoyl]-4-(2-methyl-4-pyridyl)-2-piperazinecarboxamide

The title compound was obtained from 1-[3-(6-chloro-2-naphtyl)sulfonylpropanoyl]-4-(2-methyl-4-pyridyl)-2-piperazinecarboxylate obtained in Example 145) as described in Example 76b) and 25% aqueous ammonia as pale yellow powder (17%).
NMR (CDCl₃) δ: 2.47 (3H, s), 2.75-4.06 (10H, m), 4.50-4.54 (1H, m), 5.27-5.61 (2H, m), 6.56-6.61 (2H, m), 6.72 (1H, br), 7.60-7.64 (1H, m), 7.90-7.99 (4H, m), 8.21 (1H, d, J = 5.7), 8.49 (1H, s).

| Elemental Analysis: C₂₄H₂₅N₄ClO₄S·0.4THF·0.8H₂O·0.5EtOH | | | |
|---|---|---|---|
| Calcd (%) | C, 56.32; | H, 5.83; | N, 9.88 |
| Found (%) | C, 56.23; | H, 5.34; | N, 9.38 |

### Example 152

### 1-[3-(6-Chloro-2-naphtyl)sulfonylpropanoyl]-N',N'-dimethyl-4-(2-methyl-4-pyridyl)-2-piperazinecarbohydrazide

The title compound was obtained from 1-[3-(6-chloro-2-naphtyl)sulfonylpropanoyl]-4-(2-methyl-4-pyridyl)-2-piperazinecarboxylate obtained in Example 145) and N,N-dimethylhydrazine as described in Example 151) as pale yellow powder (25%).
NMR (CDCl₃) δ: 2.23-6.63 (23H, m), 7.57-7.65 (1H, m), 7.92-7.99 (4H, m), 8.20-8.23 (1H, m), 8.47-8.50 (1H, m).

| Elemental Analysis: C₂₆H₃₀N₅ClO₄S·0.5EtOH·0.5THF·H₂O | | | |
|---|---|---|---|
| Calcd (%) | C, 56.07; | H, 6.33; | N, 11.27 |
| Found (%) | C, 55.69; | H, 5.92; | N, 10.85 |

### Example 153

### Methyl 2-[[1-[3-(6-chloro-2-naphtyl)sulfonylpropanoyl]-4-(2-methyl-4-pyridyl)-2-piperazyl]carbonylamino]acetate

The title compound was obtained from 1-[3-(6-chloro-2-naphtyl)sulfonylpropanoyl]-4-(2-methyl-4-pyridyl)-2-piperazinecarboxylic acid obtained in Example 145) and glycine methyl ester hydrochloride as described in Example 151) as pale yellow powder (65%).
NMR (CDCl₃) δ: 2.47 (3H, s), 2.76-4.53 (15H, m), 5.31 (1H, br), 6.54-6.59 (2H, m), 7.11 (1H, m), 7.62 (1H, dd, J = 1.8 and 8.8), 7.93-8.02 (4H, m), 8.21 (1H, d, J = 5.8), 8.52 (1H, s).

| Elemental Analysis: C₂₇H₂₉N₄ClO₆S·0.2THF·0.75H₂O | | | |
|---|---|---|---|
| Calcd (%) | C, 55.56; | H, 5.38; | N, 9.32 |
| Found (%) | C, 55.55; | H, 5.45; | N, 9.05 |

### Example 154

### Methyl 2-[[2-[1-[3-(6-chloro-2-naphthyl)sulfonylpropanoyl]-4-(2-methyl-4-pyridyl)-2-piperazyl]acetyl]amino]acetate

From 2-[1-[3-(6-chloro-2-naphthyl)sulfonylpropanoyl]-4-(2-methyl-4-pyridyl)-2-piperazyl]acetic acid obtained in Example 148) and glycine methyl ester hydrochloride, the title compound was obtained as a light yellow powder (71%) according to the same manner as that of Example 151).
NMR (CDCl₃) δ: 2.46 (3H, s), 2.59-4.88 (15H, m), 3.73 and 3.78 (3H, each s), 6.13-6.35 (1H, m), 6.54-6.57 (2H, m), 7.57-7.62 (1H, m), 7.94-7.99 (4H, m), 8.20 (1H, d, J=5.8), 8.50 (1H, d, J=4.0).

| Elemental Analysis: C₂₈H₃₁N₄ClO₆S | | | |
|---|---|---|---|
| Calcd(%) | C,57.28; | H,5.32; | N,9.54 |
| Found(%) | C,57.00; | H,5.43; | N,9.32 |

### Example 155

### 1-[3-(6-Chloro-2-naphthyl)sulfonylpropanoyl]-N-[2-(N,N-dimethylamino)ethyl]-N-methyl-4-(2-methyl-4-pyridyl)-2-piperazincarboxamide

From 1-[3-(6-chloro-2-naphtyl)sulfonylpropanoyl]-4-(2-methyl-4-pyridyl)-2-piperazinecarboxylic acid obtained in Example 145 and N,N,N'-trimethylethylenediamine, the title compound was obtained as a light yellow powder (81%) according to the same manner as that of Example 151.
NMR (CDCl₃) δ: 2.16-3.85 (26H, m), 5.01-5.08 (1H, m), 6.43 (2H, m), 7.58 (1H, dd, J = 1.9 and 9.1), 7.92-7.96 (4H, m), 8.18-8.21 (1H, m), 8.47 (1H, s).

| Elemental Analysis: C₂₉H₃₆N₅ClO₄S·0.5THF·1.3H₂O | | | |
|---|---|---|---|
| Calcd(%) | C, 57.67; | H, 6.65; | N, 10.85 |
| Found(%) | C, 57.52; | H, 6.35; | N, 10.69 |

### Example 156

### 4- [[1-[3-(6-Chloro-2-naphthyl)sulfonylpropanoyl]-4-(2-methyl-4-pyridyl)-2-piperazyl]carbonyl]morpholine

From 1-[3-(6-chloro-2-naphtyl)sulfonylpropanoyl]-4-(2-methyl-4-pyridyl)-2-piperazinecarboxylic acid obtained in Example 145) and morpholine, the title compound was obtained as a light yellow powder (73%) according to the same manner as that of Example 151).
NMR (CDCl₃) δ: 2.47 (3H, s), 2.86-4.06 (18H, m), 5.12 (1H, t, J = 4.7), 6.39-6.44 (2H, m), 7.59 (1H, dd, J = 2.0 and 8.9), 7.89-7.95 (4H, m), 8.20 (1H, d, J = 5.7), 8.47 (1H, d, J = 0.9).

| Elemental Analysis: C₂₈H₃₁N₄ClO₅S·0.2Et₂O·0.5H₂O | | | |
|---|---|---|---|
| Calcd(%) | C, 58.14; | H, 5.76; | N, 9.42 |
| Found(%) | C, 57.97; | H, 5.66; | N, 9.17 |

### Example 157

### 4-[[1-[3-(6-Chloro-2-naphthyl)sulfonylpropanoyl]-4-(2-methyl-4-pyridyl) -2-piperazyl]carbonyl]thiomorpholine

From 1-[3-(6-chloro-2-naphtyl)sulfonylpropanoyl]-4-(2-methyl-4-pyridyl)-2-piperazinecarboxylic acid obtained in Example 145) and thiomorpholine, the title compound was obtained as a light yellow powder (65%) according to the same manner as that of Example 151).
NMR (CDCl₃) δ: 2.47 (3H, s), 2.56-4.10 (18H, m), 5.11 (1H, t, J = 4.8), 6.39-6.43 (2H, m), 7.59 (1H, dd, J = 2.2 and 8.8), 7.92-7.96 (4H, m), 8.20 (1H, d, J = 5.8), 8.47 (1H, s).

| Elemental Analysis: C₂₈H₃₁N₄ClO₄S₂·0.5H₂O | | | |
|---|---|---|---|
| Calcd(%) | C, 56.41; | H, 5.41; | N, 9.40 |
| Found(%) | C, 56.56; | H, 5.19; | N, 9.21 |

### Example 158

### 4-[[1-[3-(6-Chloro-2-naphthyl)sulfonylpropanoyl]-4-(2-methyl-4-pyridyl)-2-piperazyl]carbonyl]thiomorpholine 1,1-oxide

From 1-[3-(6-chloro-2-naphtyl)sulfonylpropanoyl]-4-(2-methyl-4-pyridyl)-2-piperazinecarboxylic acid obtained in Example 145) and thiomorpholine 1,1-oxide, the title compound was obtained as a light yellow powder (78%) according to the same manner as that of Example 151).
NMR (CDCl₃) δ: 2.47 (3H, s), 2.89-4.70 (18H, m), 5.00 (1H, t, J = 5.5), 6.38-6.41 (2H, m), 7.59 (1H, dd, J = 2.0 and 9.0), 7.93-7.97 (4H, m), 8.21 (1H, d, J = 5.4), 8.48 (1H, s) .

| Elemental Analysis:C₂₈H₃₁N₄ClO₆S₂·0.5H₂O | | | |
|---|---|---|---|
| Calcd(%) | C, 53.54; | H, 5.13; | N, 8.92 |
| Found(%) | C, 53.25; | H, 5.22; | N, 9.20 |

### Example 159

### 4-[2-[1-[3-(6-Chloro-2-naphthyl)sulfonylpropanoyl]1-1(2-methyl-4-pyridyl)-2-piperazyl]acetyl]thiomorpholine 1-oxide

From 2-[1-[3-(6-chloro-2-naphthyl)sulfonylpropanoyl]-4-(2-methyl-4-pyridyl)-2-piperazyl]acetic acid and thiomorpholine 1-oxide trifluoroacetate obtained in Example 148), the title compound was obtained as a colorless powder (24%) according to the same manner as that of Example 30b).
NMR (CDCl₃) δ: 2.40-4.86 (23H, m), 6.47-6.56 (2H, m), 7.61 (1H, dd, J = 2.0 and 8.9), 7.89-7.98 (4H, m), 8.17-8.22 (1H, m), 8.48 (1H, s).

| Elemental Analysis:C₂₉H₃₃N₄ClO₅S₂·1.2H₂O | | | |
|---|---|---|---|
| Calcd(%) | C, 54.53; | H, 5.59; | N, 8.77 |
| Found(%) | C, 54.43; | H, 5.51; | N, 8.56 |

### Example 160

### 1-[3-(6-Chloro-2-naphthyl)sulfonylpropanoyl]-N,N-dimethyl-4-(2-methyl-4-pyridyl)piperazine-2-carboxamide

### 160a) 1-Benzyloxy-N,N-dimethyl-4-(2-methyl-4-pyridyl)piperazine-2-acrboxamide

From 1-benzyloxy-4-(2-methyl-4-pyridyl)piperazine-2-catrboxylic acid obtained in Example 134a) and dimethylmethylamine, the title compound was obtained as a yellow oil (91%) according to the same manner as that of Example 76b).
NMR (CDCl₃) δ: 2.44 (3H, s), 2.89 (3H, s), 2.96 (3H, s), 2.85-3.10 (2H, m), 3.71-3.88 (4H, m), 5.00-5.17 (3H, m), 6.43 (2H, m), 7.35 (5H, br), 8.16 (1H, d, J = 5.8).

### 160b) N,N-Dimethyl-4-(2-methyl-4-pyridyl)piperazine-2-carboxamide

From 1-benzyloxy-N,N-dimethyl-4-(2-methyl-4-pyridyl)piperazine-2-carboxamide obtained in Example 160a), the title compound was obtained as a yellow oil (quantitative) according to the same manner as that of Example 131b).
NMR (CDCl₃) δ: 1.65 (1H, m), 2.46 (3H, s), 2.79-3.18 (7H, m), 3.70-3.88 (6H, m), 6.49-6.55 (2H, m), 8.19 (1H, d, J = 5.8).

### 160c) 1-[3-(6-Chloro-2-naphthyl)sulfonylpropanoyl]-N,N-dimethyl-4-(2-methyl-4-pyridyl)piperazinecarboxamide

From N,N-dimethyl-4-(2-methyl-4-pyridyl)piperazine-2-carboxamide obtained in Example 160b), the title compound was obtained (58%) as a colorless powder according to the same manner as that of Example 76b).
NMR (CDCl₃) δ: 2.47 (3H, s), 2.89-4.03 (16H, m), 5.10 (1H, t, J = 4.9), 6.38-6.41 (2H, m), 7.59 (1H, dd, J = 2.0 and 9.0), 7.91-7.96 (4H, m), 8.20 (1H, d, J = 6.0), 8.47 (1H, s).

| Elemental Analysis: C₂₆H₂₉N₄O₄SCl·0.5THF·1.5H₂O | | | |
|---|---|---|---|
| Calcd(%) | C, 56.80; | H, 6.13; | N, 9.46 |
| Found(%) | C, 56.92; | H, 5.90; | N, 9.27 |

### Example 161

### 2-[1-[3-(6-Chloro-2-naphthyl)sulfonylpropanoyl]-4-(2-methyl-4-pyridyl)-2-piperazyl]-N-methylacetamide

### 161a) 2-[1-Benzyloxycarbonyl-4-(2-methyl-4-pyridyl)-2-piperazyl]acetic acid

From ethyl 2-[1-benzyloxycarbonyl-4-(2-methyl-4-pyridyl)-2-piperazyl]acetate obtained in Example 147a), the title compound was obtained as a light yellow solid (quantitative) according to the same manner as that of Example 134a).
NMR (200MHz, DMSO-d₆) δ: 2.31 (3H, s), 2.34-2.42 (1H, m), 2.52-2.65 (1H, m), 2.81-2.91 (1H, m), 3.04-3.22 (2H, m), 3.76-3.91 (3H, m), 4.49 (1H, m), 5.10 (2H, s), 6.57 (1H, dd, J = 2.6 and 5.8), 6.64 (1H, d, J = 2.2), 7.31-7.39 (5H, m), 8.03 (1H, d, J = 5.8).

### 161b) 2-[1-Benzyloxycarbonyl-4-(2-methyl-4-pyridyl)-2-piperazyl]-N-methylacetamide

From 2-[1-benzyloxycarbonyl-4-(2-methyl-4-pyridyl)-2-piperazyl]acetic acid obtained in Example 161a) and methylamine, the title compound was obtained as a brown oil (quantitative) according to the same manner as that of Example 76b).
NMR (CDCl₃) δ: 2.45 (3H, s), 2.55-3.34 (6H, m), 3.70-4.14 (6H, m), 4.60 (1H, m), 5.17-5.19 (2H, m), 6.50-6.53 (2H, m), 7.38 (5H, m), 8.18 (1H, d, J = 5.8).

### 161c) N-Methyl-2-[4-(2-methyl-4-pyridyl)-2-piperazyl]acetamide

From 2-[1-benzyloxycarbonyl-4-(2-methyl-4-pyridyl)-2-piperazyl]acetic acid obtained in Example 161a), the title compound was obtained as a yellow oil (quantitative).
NMR (CDCl₃) δ: 1.70 (1H, br), 2.45 (3H, s), 2.55-2.66 (1H, m), 2.80-3.20 (6H, m), 3.70-3.88 (5H, m), 6.48-6.53 (3H, m), 8.18 (1H, d, J = 5.8).

### 161d) 2-[1-[3-(6-Chloro-2-naphthyl)sulfonylpropanoyl]-4-(2-methyl-4-pyridyl)-2-piperazyll-N-methylacetamide

From N-methyl-2-[4-(2-methyl-4-pyridyl)-2-piperazyl]acetamide obtained in Example 161c) and 3-(6-chloro-2-naphthyl)sulfonylpropionic acid obtained in Example 27b), the title compound was obtained as a colorless powder (40%) according to the same manner as that of Example 42c).
NMR (CDCl₃) δ: 2.24-5.01 (16H, m), 2.46 (3H, s), 5.61-5.74 (1H, m), 6.54 (2H, m), 7.57-7.63 (1H, m), 7.95-8.02 (4H, m), 8.20 (1H, d, J = 5.4), 8.50 (1H, d, J = 6.4).

| Elemental Analysis: C₂₆H₂₉N₄O₄SCl·H₂O | | | |
|---|---|---|---|
| Calcd(%) | C, 57.08; | H, 5.71; | N, 10.24 |
| Found(%) | C, 56.98; | H, 5.91; | N, 10.12 |

### Example 162

### 2-[1-[3-(6-Chloro-2-naphthyl)sulfonylpropanoyl]-N,N-dimethyl-4-(2-methyl-4-pyridyl)-2-piperazyl]acetamide

### 162a) 2-[1-Benzyloxycarbonyl-4-(2-methyl-4-pyridyl)-2-piperazyl]-N,N-dimethylacetamide

From 2-[1-benzyloxycarbonyl-4-(2-methyl-4-pyridyl)-2-piperazyl]acetic acid obtained in Example 161a) and dimethylamine the title compound was obtained as a brown oil (96%) according to the same manner as that of Example 76b).
NMR (CDCl₃) δ: 2.44 (3H, s), 2.71-3.20 (9H, m), 3.70-3.75 (2H, m), 3.83-3.88 (2H, m), 4.01-4.14 (1H, m), 4.68 (1H, m), 5.17 (2H, s), 6.49-6.51 (2H, m), 7.36-7.39 (5H, m), 8.16 (1H, d, J = 6.6).

### 162b) N,N-Dimethyl-2-[4-(2-methyl-4-pyridyl)-2-piperazyl]acetamide

From 2-[1-benzyloxycarbonyl-4-(2-methyl-4-pyridyl)-2-piperazyl]-N,N-dimethylacetamide obtained in Example 162a) the title compound was obtained as a yellow oil (95%) according to the same manner as that of Example 131b). NMR (CDCl₃) δ: 1.86 (1H, br), 2.45 (3H, s), 2.58-2.70 (1H, m), 2.89-3.30 (9H, m), 3.67-3.75 (3H, m), 3.83-3.88 (2H, m), 6.50-6.54 (2H, m), 8.17 (1H, d, J = 5.4).

### 162c) 2-[1-[3-(6-Chloro-2-naphthyl)sulfonylpropanoyl]-N,N-dimethyl-4-(2-methyl-4-pyridyl)-2-piperazyl]acetamide

From N,N-dimethyl-2-[4-(2-methyl-4-pyridyl)-2-piperazyl]acetamide obtained in Example 162b) and 3-(6-chloro-2-naphthyl)sulfonylpropionic acid, the title compound was obtained as a colorless powder (40%) according to the same manner as that of Example 76b).
NMR (CDCl₃) δ: 2.46 (3H, s), 2.60-4.93 (19H, m), 6.46-6.54 (2H, m), 7.56-7.63 (1H, m), 7.95-8.02 (4H, in), 8.19 (1H, d, J = 6.2), 8.49 (1H, d, J = 3.4).

| Elemental Analysis: C₂₆H₂₉N₄O₄SCl·H₂O | | | |
|---|---|---|---|
| Calcd(%) | C, 57.80; | H, 5.93; | N, 9.99 |
| Found(%) | C, 57.98; | H, 5.91; | N, 9.84 |

### Example 163

### 3-(6-Chloro-2-naphthyl)sulfonyl-N-methyl-N-[4-(2-methyl-4-pyridyl)-1-piperazyl]propanamide

### 163a) N-[4-(2-methyl-4-pyridyl)-1-piperazyl]formamide

To a solution of 4-(2-methyl-4-pyridyl)-1-piperazylamine(0.40 g) containing 4-(2-methyl-4-pyridyl)-1-piperazine obtained in Example 136b) in formic acid (1 ml), acetic anhydride (0.38 ml) was added, and stirred at room temperature for 16 hours. The reaction mixture was basified with an aqueous solution of potassium carbonate, and extracted with ethyl acetate. The extracted solution was dried over anhydrous sodium sulfate, then the solvent was removed under reduced pressure, and the residue was purified with a silica gel column to obtain the title compound as a colorless powder (0.33 g, 79%).
NMR (CDCl₃) δ: 2.47 (3H, s), 2.89-2.94 (4H, m), 3.42-3.47 (4H, m), 6.50-6.54 (2H, m), 6.66-6.71 (1H, m), 7.98-8.42 (2H, m).

### 163b) 1-(Methylamino)-4-(2-methyl-4-pyridyl)piperazine

To a solution of lithium alminium hydride (0.10 g) in THF (25 ml), a solution of N-[4-(2-methyl-4-pyridyl)-1-piperazyl]formamide (0.30 g) obtained in Example 163a) in THF (5 ml) was added, and refluxed for 3 hours. After addition of ethyl acetate and 1N HCl, the mixture was basified with potassium carbonate. The insoluble material was filtered off, and the filtrate was extracted with THF. The extract was dried over anhydrous sodium sulfate, and the solvent was removed under reduced pressure to obtain the title compound as a colorless oil (0.27g, 96%).
NMR (CDCl₃) δ: 2.45 (3H, s), 2.64 (3H, s), 2.77 (4H, t, J = 5.0), 3.39 (4H, t, J = 5.1), 6.49-6.55 (2H, m), 8.17 (1H, d, J = 5.8).

### 163c) 3-(6-Chloro-2-naphthyl)sulfonyl-N-methyl-N-[4-(2-methyl-4-pyridyl)-1-piperazyl]propanamide

From 1-(methylamino)-4-(2-methyl-4-pyridyl)piperazine obtained in Example 163b) and 3-(6-chloro-2-naphthyl)sulfonylpropionic acid obtained in Example 27b), the title compound was obtained as a colorless powder (25%) according to the same manner as that of Example 76b).
NMR (CDCl₃) δ: 2.49 (3H, s), 2.78-3.17 (11H, m), 3.48-3.55 (2H, m), 3.78-3.83 (2H, m), 6.48-6.56 (2H, m), 7.57 (1H, dd, J = 1.8 and 8.8), 7.91-7.95 (4H, m), 8.23 (1H, d, J = 5.8), 8.48 (1H, s).

| Elemental Analysis: C₂₄H₂₇N₄ClO₃S | | | |
|---|---|---|---|
| Calcd(%) | C, 59.19; | H, 5.59; | N, 11.50 |
| Found(%) | C, 59.04; | H, 5.56; | N, 11.23 |

### Example 164

### 1-[3-(6-Bromo-2-naphthyl)sulfonylpropionyl]-4-(2-methyl-4-pyridyl)piperazine

From 3-(6-bromo-2-naphtyl)sulfonylpropionic acid obtained in Example 64c) and 4-(2-methyl-4-pyridyl)-1-piperazine dihydrochloride obtained in Example 130b), the title compound was obtained (31%) according to the same manner as that of Example 30b).
NMR (CDCl₃) δ: 2.48 (3H, s), 2.93 (2H, t, J = 7.7), 3.23-3.28 (2H, m), 3.34-3.39 (2H, m), 3.55-3.70 (6H, m), 6.48-6.52 (2H, m), 7.71 (1H, dd, J = 1.8 and 8.8), 7.85-7.94 (3H, m), 8.13 (1H, s), 8.22 (1H, d, J = 5.8), 8.47 (1H, s).

| Elemental Analysis: C₂₃H₂₄BrN₃O₃S·0.5H₂O | | | |
|---|---|---|---|
| Calcd(%) | C, 54.01; | H, 4.93; | N, 8.22 |
| Found(%) | C, 54.21; | H, 5.05; | N, 7.95 |

### Example 165

### N-(6-Bromo-2-naphthyl)sulfonylpropyl-1-(1-butoxycarbonyl-4-piperidyl)-N-methylpiperidine-4-carboxamide

### 165a) N-(6-bromo-2-naphtjyl)sulfonylpropyl-1-(1-butoxycarbonyl-4-piperidyl)-N-methylpiperidine-4-carboxamide

From 1-(1-butoxycarbonyl-4-piperidyl)piperidine-4-carboxylic acid (WO9800134) and 6-chloro-2-(3-methylaminopropyl)sulfonylnaphthalene trifluoroacetate obtained in Example 119b), the title compound was obtained as a colorless powder (32%) according to the same manner as that of Example 76b).
NMR (CDCl₃) δ: 1.40-1.44 (3H, m), 1.45 (9H, s), 1.61-1.76 (6H, m), 1.98-2.25 (4H, m), 2.37-2.45 (2H, m), 2.69 (2H, m), 2.86-2.95 (2H, m), 3.02 (3H, s), 3.11-3.17 (2H, m), 3.47 (2H, t, J = 6.9), 4.14 (1H, m), 7.72 (1H, dd, J = 1.8 and 8.7), 7.85-7.95 (4H, m), 8.13 (1H, m), 8.44-8.48 (1H, m).

| Elemental Analysis: C₃₀H₄₂N₃BrO₅S | | | |
|---|---|---|---|
| Calcd(%) | C, 56.60; | H, 6.65; | N, 6.60 |
| Found(%) | C, 56.49; | H, 6.66; | N, 6.42 |

### Example 166

### N-(6-Bromo-2-naphthyl)sulfonylpropyl-1- (4-piperidyl) -N-methylpiperidine-4-carboxamide dihydrochloride

To a solution of N-(6-bromo-2-naphthyl)sulfonylpropyl-1-(1-butoxycarbonyl-4-piperidyl)-N-methylpiperidine-4-carboxamide (0.17 g) obtained in Example 165a) in ethyl acetate (10 ml), a 40% solution of hydrogen chloride in ethanol (5 ml) was added, and stirred at room temperature for 15 hours. The mixture was diluted with ethyl acetate, and the precipitation was filtrated to obtain the title compound as a colorless powder (0.14 g, quantitative).
NMR (DMSO-d₆) δ: 1.75-3.43 (27H, m), 7.84-7.89 (1H, m), 7.95-8.02 (1H, m), 8.18-8.24 (2H, m), 8.44-8.46 (1H, m), 8.63 (1H, m), 9.07 (2H, br).

| Elemental Analysis: C₂₅H₃₆N₃BrCl₂O₃S·0.5H₂O | | | |
|---|---|---|---|
| Calcd(%) | C, 48.55; | H, 6.03; | N, 6.79 |
| Found(%) | C, 48.71; | H, 6.02; | N, 6.71 |

### Example 167

### 3-[(6-Chloro-2-naphythyl)sulfonyl]-N-[1-(imidazo[1,2-a]pyridin-5-yl)-4-piperidinyl]-N-methylpropanamide

### 167a) 5-(1,4-Dioxa-8-azaspiro[4,5]-decan-8-yl)imidazo[1,2-a]pyridine

Under nitrogen atmosphere, 5-chloroimidazo[1,2-a]pyridine (4.58 g) and, 4-dioxa-8-azaspiro[4,5]-decane (12.89 g) were stirred at 125°C for 16 hours. To the reaction mixture, water (100 ml) was added, and the mixture was extracted with chloroform. The extract was washed with brine, dried over anhydrous magnesium sulfate, then the solvent was removed under reduced pressure. The residue was purified to obtain the title compound as a light yellow crystal (6.60 g, 85%).
NMR (CDCl₃) δ: 1.96 (4H, t, J = 6.0), 3.22 (4H, t, J = 4.5), 4.04 (4H, s), 6.32 (1H, d, J = 7.5), 7.18 (1H, dd, J = 9.3 and 7.2), 7.40 (1H, d, J = 8.4), 7.54 (1H, s), 7.65 (1H, s).

### 167b) 5-(4-Methylaminopiperadino)imidazo[1,2-a]pyridine dihydrochloride monohydrate

To a solution of 5-(1,4-dioxa-8-azaspiro[4,5]-decan-8-yl)imidazo[1,2-a]pyridine (6.60 g) obtained in Example 167a) in acetone (25 ml), 4N HCl (14.4 ml) was added, and stirred at 50°C for 6 hours. The solvent was removed under reduced pressure, and adjusted to pH 11 with 1 N aqueous sodium hydroxide. The mixture was saturated with sodium chloride, and extracted with chloroform. The extract was washed with brine, dried over magnesium sulfate, and then the solvent was removed under reduced pressure. The residue was dissolved in acetic acid (50 ml), and added a 40% methylamine-methanol solution (25 ml) dropwise for 30 minutes. The reaction mixture was stirred at room temperature, then sodium triacetoxyborohydride (6.3 g) was added, and stirred at room temperature for 2 hours. The solvent was removed under reduced pressure, and the residue was adjusted to pH 11 at 0 °C with 1 N aqueous sodium hydroxide. The mixture was saturated with sodium chloride, and extracted with chloroform. The extract was washed with brine, and dried over magnesium sulfate, and then the solvent was removed under reduced pressure. The residue was dissolved in ethanol (100 ml), and di-tert-butyl dicarbonate (5.55 g) was added at room temperature, and stirred for 1 hour. The solvent was removed under reduced pressure, and the residue was purified by a silica gel column. The oil obtained was dissolved in ethanol (10 ml), and 12 N HCl (21 ml) was added. The reaction mixture was stirred at room temperature for 1 hour, then the solvent was removed under reduced pressure. To the residue ethanol was added and crystallized to obtain the title compound as white crystals (3.07g, 38%).
NMR (CDCl₃) δ: 1.91-2.07 (2H, m), 2.34-2.39 (2H, m), 2.83 (3H, s), 2.98-3.11 (2H, m), 3.39-3.51 (1H, m), 3.63-3.70 (2H, m), 7.00 (1H, d, J = 7.6), 7.58 (1H, d, J = 8.8), 7-83-7.93 (3H, m).

### 167c) 3-[(6-Chloro-2-naphythyl)sulfonyl]-N-[1-(imidazo[1,2-a]pyridin-5-yl)-4-piperidinyl]-N-methylpropanamide

To a suspension of 3-[(6-chloro-2-naphthyl)sulfonyl]propionic acid (0.30 g) obtained in Example 27b) in acetonitrile (5 ml), HOBt (0.23 g), and then WSC (0.29 g) were added at room temperature, and stirred for 20 minutes. To the reaction mixture, a solution of 5-(4-methylaminopiperadino)imidazo[1,2-a]pyridine dihydrochloride monohydrate (0.36 g) obtained in Example 167b), 1,8-diazabicyclo[5.4.0]-7-undecen (0.36 ml), and triethylamine (0.42 ml) in acetonitrile (5 ml), and stirred at room temperature for 1 hour. The solvent was removed under reduced pressure, water was added to the residue, and extracted with chloroform. The extract was washed with brine and dried over anhydrous magnesium sulfate. The solvent was evaporated in vacuo and the residue purified by a silica gel column and crystallized from acetonitrile/ether to obtain the title compound as white crystals (0.19 g, 37%).
NMR (CDCl₃) δ: 1.69-1.73 (2H, m), 1.85-1.93 (2H, m), 2.77-3.08 (7H, m), 3.48-3.52 (2H, m), 3.57-3.62 (2H, m), 3.77-3.95 (0.3H, m), 4.53-4.68 (0.7H, m), 6.27-6.34 (1H, m), 7.14-7.24 (1H, m), 7.38-7.45 (1H, m), 7.50-7.52 (1H, m), 7.60 (1H, dd, J = 9.0 and 1.8 ), 7.64-7.67 (1H, m), 7.92-7.97 (4H, m), 8.50 (1H, s).

| Elemental Analysis: C₂₆H₂₇ClN₄O₃S | | | |
|---|---|---|---|
| Calcd(%) | C, 61.11; | H, 5.33; | N, 10.96 |
| Found(%) | C, 61.03; | H, 5.37; | N, 11.21 |

### Example 168

### 3-[(6-Bromo-2-naphthyl)sulfonyl]-N-[1-(imidazo[1,2-a]pyridin-5-yl)-4-piperidinyl]-N-methylpropanamide

From 3-[(6-bromo-2-naphthyl)sulfonyl]propionic acid obtained in Example 64c) and 5-(4-methylaminopiperadino)imidazo[1,2-a]pyridine dihydrochloride monohydrate obtained in Example 167b), the title compound was obtained as white crystals (41%) according to the same manner as that of Example 167c).
NMR (CDCl₃) δ: 1.68-1.73 (2H, m), 1.82-2.25 (2H, m), 2.75-3.07 (7H, m), 3.47-3.63 (4H, m), 3.75-3.96 (0.3H, m), 4.48-4.70 (0.7H, m), 6.26-6.34 (1H, m), 7.13-7.26 (1H, m), 7.37-7.48 (1H, m), 7.50-7.52 (1H, m), 7.64-7.66 (1H, m), 7.73 (1H, dd, J = 8.8 and 1.8), 7.88 (1H, d, J = 8.8), 7.92-7.99 (2H, m), 8.13 (1H, s), 8.49 (1H, s)

| Elemental Analysis: C₂₆H₂₇BrN₄O₃S·0.25CH₃CN | | | |
|---|---|---|---|
| Calcd(%) | C, 56.26; | H, 4.94; | N, 10.52 |
| Found(%) | C, 55.97; | H, 4.97; | N, 10.63 |

### Formulation Example 1

An FXa inhibitor (e.g., deep vein thrombosis treating agent, cardiogenic cerebral infarction treating agent, and the like) containing a compound represented by the formula (I) according to the present invention or a salt thereof as an active ingredient can be produced for example by the following formulations.

| 1. Capsule | |
|---|---|
| (1) Compound obtained in Example 42 | 40 mg |
| (2) Lactose | 70 mg |
| (3) Microcrystalline cellulose | 9 mg |
| (4) Magnesium stearate | 1 mg |
| 1 Capsule | 120 mg |

Components (1), (2) and (3) and a half of (4) are mixed and granulated. Then the remainder of Component (4) was added and the entire mass is filled into gelatin capsules.

| 2. Capsule | |
|---|---|
| (1) Compound obtained in Example 54 | 40 mg |
| (2) Lactose | 70 mg |
| (3) Microcrystalline cellulose | 9 mg |
| (4) Magnesium stearate | 1 mg |
| 1 Capsule | 120 mg |

Components (1), (2) and (3) and a half of (4) are mixed and granulated. Then the remainder of Component (4) was added and the entire mass is filled into gelatin capsules.

| 3. Tablet | |
|---|---|
| (1) Compound obtained in Example 42 | 40 mg |
| (2) Lactose | 58 mg |
| (3) Corn starch | 18 mg |
| (4) Microcrystalline cellulose | 3.5 mg |
| (5) Magnesium stearate | 0.5 mg |
| 1 Table | 120 mg |

Components (1), (2) and (3) and a 2/3 of (4) and a half of Component (5) are mixed and granulated. Then the remainders of Components (4) and (5) are added to the granule, and then compressed into tablets.

| 4. Tablet | |
|---|---|
| (1) Compound obtained in Example 54 | 40 mg |
| (2) Lactose | 58 mg |
| (3) Corn starch | 18 mg |
| (4) Microcrystalline cellulose | 3.5 mg |
| (5) Magnesium stearate | 0.5 mg |
| 1 Table | 120 mg |

Components (1), (2) and (3) and a 2/3 of (4) and a half of Component (5) are mixed and granulated. Then the remainders of Components (4) and (5) are added to the granule, and then compressed into tablets.

### Formulation Example 2

50 mg of the compound obtained in Example 50 was dissolved in 50 ml of JP distilled water for injection, and JP distilled water for injection was further added to make 100 ml. This solution was filtered aseptically, and 1 ml aliquots of this solution were dispensed aseptically into injection vials, which were lyophilized and closed tightly.

### Experiment 1

### (1) Human activated coagulation factor X (FXa) inhibiting effect

Method: A cuvette was charged with 225 µl of 0.05 M tris buffer (pH 8.3) containing 0.145 M sodium chloride and 2 mM calcium chloride, 5 µl of a sample (test compound dissolved in dimethyl sulfoxide) and 10 µl of human FXa (0.3 unit/ml), which were reacted at 37°C for 10 minutes and then combined with 10 µl of a substrate (3mM, S-2765) and further reacted at 37°C for 10 minutes. Then the reaction was terminated by adding 25 µl of 50 % aqueous acetic acid, and the change in the absorbance at 405 nm was determined using a spectrophotometer to calculate the concentration at which the FXa effect was inhibited by 50 % (IC₅₀).

### (2) In vitro clotting time measurement

### (2-1) Prothrombin time (PT) measurement:

A PT-test WAKO (WAKO PURE CHEMICAL) was employed together with an automatic coagulometer (STA compact DIAGNOSTICA STAGO). 97 µl of Human normal plasma (fresh human plasma, FFP, SEKISUI KAGAKU KOGYO) was combined with 3 µl of a test substance and pre-incubated at 37°C for 4 minutes. 50 µl of the plasma described above was combined with 100 µl of rabbit brain-derived tissue thromboplastin solution and the time for the clotting was measured. The test substance was used after dissolving in dimethyl sulfoxide (DMSO). A concentration required for 2-fold prolongation of the clotting time was calculated based on the clotting time observed when DMSO was added instead of the test substance.

### (2-2) Intrinsic clotting time (ATPP) measurement:

A STA-APTT-LT (DIAGNOSTICA STAGO) was employed with together with an automatic coagulometer (STA compact DIAGNOSTICA STAGO). 97 µl of human normal plasma (fresh human plasma, FFP, SEKISUI KAGAKU KOGYO) was combined with 3 µl of a test substance. 50 µl of the plasma was combined with 50 µl of an active partial thromboplastin solution and preincubated at 37°C for 4 minutes. 50 µl of 20 mmol/L CaCl₂ was added and the time for the clotting was determined. The test substance was used after dissolving in DMSO. A concentration required for 2-fold prolongation of the clotting time was calculated based on the clotting time observed when DMSO was added instead of the test substance.

### (2-3) Thrombin clotting time (TT) measurement:

An automatic coagulometer (Biomatic B10, Sarstedt) was employed for a measurement. Human plasma-derived thrombin (Sigma) was dissolved in distilled water at 2.3 NIH unit/ml. 97 µl of human normal plasma (fresh human plasma, FFP, SEKISUI KAGAKU KOGYO) was combined with 3 µl of a test substance and pre-incubated at 37°C for 3 minutes. 100 µl of the plasma described above was combined with 200 µl of a thrombin solution, and the time for the clotting was measured. The test substance was used after dissolving in DMSO. A concentration required for 2-fold prolongation of the clotting time was calculated based on the clotting time observed when DMSO was added instead of the test substance.

### (3) Ex vivo clotting time measurement (mice)

### (3-1) Intravenous administration:

Male ICR mice (25 to 35 g, Slc) were employed. An animal received a test substance by a single administration of 5 ml/kg to a tail vein under anesthesia with pentobarbital (50 mg/kg, i.p.). 5 Minutes after the administration, 0.8 ml of the blood was taken from an abdominal aorta with a 1/10 volume of 3.8 % sodium citrate (CYTORAL, YAMANOUCHI), and centrifuged at 3000 rpm for 15 minutes to obtain a plasma. 50 µl of the plasma described above was combined with 100 µl of rabbit brain-derived tissue thromboplastin solution and the time for the clotting was measured. The clotting time was measured using a PT-test WAKO (WAKO PURE CHEMICAL) together with an automatic coagulometer (STA compact DIAGNOSTICA STAGO). The test substance was used after dissolving in physiological saline, and the physiological saline was given instead of the test substance in a control group. The activity of the substance is indicated as a ratio (%) of the clotting time in a treatment group based on the clotting time in the control group.

### (3-2) Oral administration:

Male ICR mice (25 to 35 g, Slc) were employed. 5 ml/kg of a test substance was given by a forcible oral administration to an animal after fasting for 12 hours or longer. 1 hour after administration, a blood was taken from an abdominal aorta under anesthesia with pentobarbital (50 mg/kg, o.p.). The test substance was used after suspending in 0.5 % methyl cellulose, and 0.5 % methyl cellulose was given instead of the test substance in a control group. Otherwise, the procedure similar to that for the intravenous administration described above was employed.

### (4) In vivo antithrombotic effect measurement

### (4-1) Rat arteriovenous shunt method:

A method by Umetsu et al (Thromb. Haemostas., 39, 74-73, (1978)) was employed. Male SD rats (weighing 250 to 350 g) were used under anesthesia with pentobarbital (50 mg/kg) to form an extracorporeal circulation of a polyethylene tube attached with a silk thread between the left jugular vein and the right jugular vein. In order to prevent a blood coagulation, the tube had previously been filled with a physiological saline containing heparin (50 U/ml). The blood was allowed to circulate for 15 minutes, the thrombus depositing on the silk thread during which period was weighed wet. A test substance was given orally or intravenously. When given orally, the test substance was suspended in 0.5 % methyl cellulose, and given (5 ml/kg) 2 hours before initiation of the experiment to an animal while fasting. In a control group, 0.5 % methyl cellulose was given instead of the test substance. When given intravenously, 1 ml/kg was given to a tail vein 5 minutes before initiating the blood circulation. The test substance was used after dissolving in physiological saline, and the physiological saline was given instead of the test substance in a control group. The activity of a test substance is indicated as a ratio (%) of the wet weight of the thrombus in a treatment group based on the wet weight in the control group.

### (4-2) Rat abdominal vena cava partial stasis model

Male Sprague-Dawley rats (250-400 g, NIPPON CLAIR) were employed. An abdominal vena cava thrombus model was established by a modified Finkle's method (Thromb, Haemostas., 79, 431-438, 1998). An abdominal vena cava was exposed carefully under anesthesia with pentobarbital (50 mg/kg, i.p.), and the abdominal vena cava was tied at the renal vein bifurcation and at 1 cm downstream thereof, between which all branches were ligated. A balloon catheter (Fogarty, 2F, Baxter) was inserted from the left femoral vein, and the region between the two ties was injured three times by means of the balloon inflated with 200 to 300 ml of air. The balloon catheter was removed, the thread tied at the renal vein bifurcation was bound together with a 26G needle, and then the needle was removed, whereby establishing a partial stasis. After 30 minutes, another thread was bound, and the thrombus formed between the two threads was isolated carefully, and its wet weight was measured using a hooded analytical balance (BP110S, Sartorius). On the other hand, 2 ml of the blood was taken from an abdominal aorta with a 1/10 volume of 3.8 % sodium citrate (CYTORAL, YAMANOUCHI), and centrifuged at 3000 rpm for 10 minutes to obtain a platelet-poor plasma (PPP). A test substance was given orally or intravenously. When given orally, the test substance was suspended in 0.5 % methyl cellulose, and given (5 ml/kg) 2 hours before initiation of the experiment to an animal while fasting. In a control group, 0.5 % methyl cellulose was given instead of the test substance. When given intravenously, 1 ml/kg was given to a tail vein 5 minutes before initiating the partial stasis. The test substance was used after dissolving in physiological saline, and the physiological saline was given instead of the test substance in a control group. The activity (% inhibition of thrombus formation) of a test substance is indicated as a ratio (%) of the wet weight of the thrombus in a treatment group based on the wet weight in the control group.

### (4-3) Rat deep vein thrombosis (DVT) model

Male SD rats (weighing 250 to 350 g) were employed. A polyethylene tube was inserted into the left femoral vein under anesthesia with pentobarbital (50 mg/kg, i.p.). In the polyethylene tube, a silk thread (5 cm in length) which had previously been attached to a guide wire was inserted, and a physiological saline containing heparin (50 U/ml) was filled in order to prevent a blood coagulation. After inserting the polyethylene to reach the abdominal vena cava, the silk thread was placed in the abdominal vena cava using the guide wire. After placing for 30 minutes, heparin (200 U/kg) was given intravenously from a tail vein. After exsanguinating by cutting a brachial artery, the abdominal region was opened and the silk thread was taken out and examined for the wet weight of the thrombus depositing on the thread (including the weight of the thread). A test substance was given at 1 ml/kg to a tail vein 5 minutes before placing the silk thread. The test substance was used after dissolving in physiological saline, and the physiological saline was given instead of the test substance in a control group. The wet weight only of the thrombus was calculated as (wet weight of thrombus depositing on silk thread) - (wet weight determined after immersing silk thread in heparinized venous blood (11.6 ± 0.2 mg)).

### Results

Table 1 shows IC₅₀ values. Based on the results shown below, it is evident that the compound of the invention has an excellent FXa inhibiting effect.

**Table 1**

| Example No. | IC₅₀ (nM) | Example No. | IC₅₀ (nM) |
|---|---|---|---|
| 5 | 95 | 14 | 60 |
| 31 | 50 | 33 | 55 |
| 34 | 37 | 42 | 61 |
| 52 | 49 | 53 | 95 |
| 54 | 39 | 55 | 40 |
| 64 | 36 | 67 | 25 |
| 68 | 34 | 75 | 59 |
| 118 | 63 | 129 | 66 |

### INDUSTRIAL APPLICABILITY

Compound (I) according to the present invention or a salt thereof has an excellent FXa inhibiting effect, has a less hemorrhagic side effect, is useful as an anticoagulant capable of being absorbed orally, and thus can advantageously be used for preventing and/or treating various diseases attributable to thrombus or infarction.

## Claims

1. A compound represented by the formula (I): 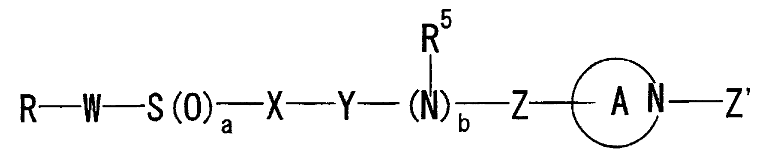 wherein R is an optionally substituted cyclic hydrocarbon group or an optionally substituted heterocyclic group, W is a bond or an optionally substituted divalent chainlike hydrocarbon group, X is an optionally substituted divalent hydrocarbon group, Y and Z are independently -N(R⁶)-, -CO-, -S(O)-, -S(O)₂-, -CH₂-, -N(R⁶)-CO-, -CO-CH₂- or a bond, ring A is an optionally substituted nitrogen-containing heterocyclic ring, R⁵ and R⁶ are independently hydrogen atom, an optionally substituted hydrocarbon group, an optionally substituted alkoxy group, an optionally esterified or amidated carboxyl or an optionally substituted acyl group, R⁵ may bind to a substituent of X or a substituent of ring A to form a ring, Z' is an optionally substituted imidoyl group or an optionally substituted nitrogen-containing heterocyclic group, a is 0, 1 or 2, and b is 0 or 1, or a salt thereof.

2. A prodrug of the compound according to claim 1 or a salt thereof.

3. The compound according to claim 1, wherein R is an optionally substituted aryl group.

4. The compound according to claim 1, wherein R is an aryl group optionally substituted with a substituent selected from a halogen atom, C₁₋₆ alkyl, C₂₋₆ alkenyl, C₂₋₆ alkynyl, optionally substituted amino, nitro, cyano, optionally substituted amidino, and optionally esterified or amidated carboxyl.

5. The compound according to claim 1, wherein R is an optionally substituted heterocyclic group.

6. The compound according to claim 1, wherein R is a heterocyclic group optionally substituted with a substituent selected from a halogen atom, C₁₋₆ alkyl, C₂₋₆ alkenyl, C₂₋₆ alkynyl, optionally substituted amino, nitro, cyano, optionally substituted amidino, and optionally esterified or amidated carboxyl.

7. The compound according to claim 1, wherein R is naphthyl optionally substituted with a halogen atom.

8. The compound according to claim 1, wherein W is a bond.

9. The compound according to claim 1, wherein X is an optionally substituted divalent chainlike hydrocarbon group.

10. The compound according to claim 1, wherein X is an optionally substituted phenylene group.

11. The compound according to claim 1, wherein Y and Z are independently -N(R⁶)- (wherein R⁶ is the same as defined in claim 1), -CO-, -S(O)-, -S(O)₂-, -CH₂- or a bond.

12. The compound according to claim 1, wherein Y is -CO- or -SO₂-, and Z is a bond.

13. The compound according to claim 1, wherein Y is a bond, and Z is -CO-.

14. The compound according to claim 1, wherein ring A is an optionally substituted piperazine ring or an optionally substituted piperidine ring.

15. The compound according to claim 1, wherein Z' is an optionally substituted nitrogen-containing heterocyclic group.

16. The compound according to claim 1, wherein Z' is a nitrogen-containing heterocyclic group optionally substituted with a substituent selected from optionally substituted C₁₋₄ alkyl, and optionally substituted amino.

17. The compound according to claim 1, wherein Z' is an optionally substituted pyridyl group.

18. The compound according to claim 17, wherein Z' is bound to ring A at 4-position of the pyridine ring.

19. The compound according to claim 1, wherein R⁵ is hydrogen atom or optionally substituted C₁₋₆ alkyl.

20. The compound according to claim 1, wherein R⁵ binds to a substituent of ring A to form a ring.

21. The compound according to claim 1, wherein a is 2.

22. The compound according to claim 1, wherein b is 1.

23. A compound selected from the group consisting of N-[3-[(6-chloro-2-naphthyl)sulfonyl]propyl]-N-methyl-1-(4-pyridyl)-4-piperidinecarboxamide, methyl 2-[N-[3-[(6-chloro-2-naphthyl)sulfonyl]propyl]-N-[1-(4-pyridyl)-4-piperidyl]carbonylamino]acetate, 3-[(6-chloro-2-naphtyl)sulfonyl]-N-methyl-N-[1-(4-pyridyl)-4-piperidyl]propanamide, ethyl 2-[N-[3-[(6-chloro-2-naphthyl)sulfonyl]propanoyl]-N-[1-(4-pyridyl)-4-piperidyl]amino]acetate, ethyl 3-[N-[3-[(6-chloro-2-naphthyl)sulfonyl]propanoyl]-N-[1-(4-pyridyl)-4-piperidyl]aminopropionate, 3-[(6-chloro-2-naphthyl)sulfonyl]-N-methyl-N-[1-(2-methyl-4-pyridyl)-4-piperidyl]propanamide, N-[2-(acetylamino)ethyl]-3-[(6-chloro-2-naphthyl)sulfonyl]-N-[1-(2-methyl-4-pyridyl)-4-piperidyl]propanamide, N-(2-aminoethyl)-3-[(6-chloro-2-naphthyl)sulfonyl]-N-[1-(4-pyridyl)-4-piperidyl]propanamide, N- [2-(acetylamino)ethyl]-3-[(6-chloro-2-naphthyl)sulfonyl]-N-[1-(4-pyridyl)-4-piperidyl]propanamide, 3-[(6-chloro-2-naphthyl)sulfonyl]-N-[2-[(methanesulfonyl)amino]ethyl]-N-[1-(4-pyridyl)-4-piperidyl]propanamide, 3-[(6-bromo-2-naphthyl)sulfonyl]-N-methyl-N-[1-(2-methyl-4-pyridyl)-4-piperidyl]propanamide, 3-[(6-chloro-2-naphthyl)sulfonyl]-N-[1-(2-methyl-4-pyridyl)-4-piperidyl]-N-[3-(1-oxide-4-thiomorpholinyl)-3-oxopropyl]propanamide, N-[2-(N-acetyl-N-methylamino)ethyl] -3-[(6-chloro-2-naphthyl)sulfonyl]-N-[1-(2-methyl-4-pyridyl)-4-piperidyl]propanamide, 3-[(6-chloro-2-naphthyl)sulfonyl]-N-methyl-N-[1-(2,6-dimethyl-4-pyridyl)-4-piperidyl]propanamide and 1-[3-[(6-chloro-2-naphthyl)sulfonyl]propanoyl-4-(2-methyl-4-pyridyl)piperazine, or a salt thereof.

24. A prodrug of the compound according to claim 23, or a salt thereof.

25. A pharmaceutical composition comprising the compound according to claim 1, or a salt thereof or a prodrug thereof.

26. The composition according to claim 25 which is an anticoagulant.

27. The composition according to claim 25 which is an activated coagulation factor X inhibiting agent.

28. The composition according to claim 25 which is an agent for preventing and/or treating cardiac infarction, cerebral thrombosis, deep vein thrombosis, pulmonary thrombotic embolus, or thrombotic embolus during operation or after operation.

29. A process for preparing the compound according to claim 1 or a salt thereof, which comprises:
reacting a compound represented by the formula (II):
L¹―Z'
wherein L¹ is a leaving group, and the other symbol is the same as defined in claim 1, or a salt thereof, with a compound represented by the formula (III): 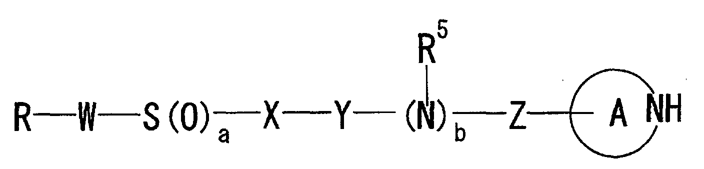 wherein the symbols are the same as defined in claim 1, or a salt thereof, or
reacting a compound represented by the formula (IV):
R-W-S(O)ₐ-X-Y-L²
wherein L² is a leaving group, and the other symbols are the same as defined in claim 1, or a salt thereof, with a compound represented by the formula (V): 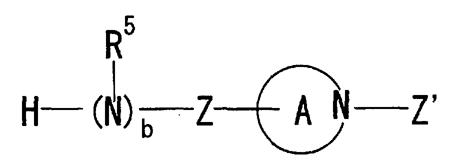 wherein the symbols are the same as defined in claim 1, or a salt thereof, or
reacting a compound represented by the formula (VI): 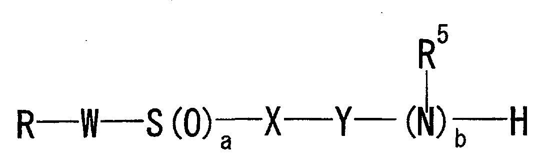 wherein the symbols are the same as defined in claim 1, or a salt thereof, with a compound represented by the formula (VII): 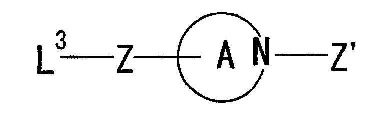 wherein L³ is a leaving group, and the other symbols are the same as defined in claim 1, or a salt thereof, or reacting a compound represented by the formula (Ia): 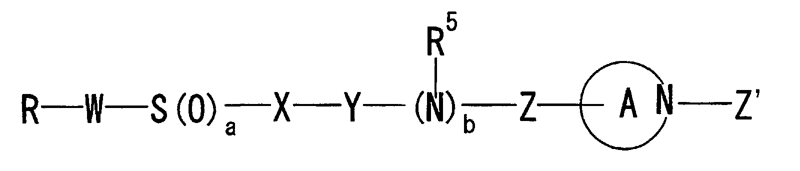 wherein a is 0, and the other symbols are the same as defined in claim 1, or a salt thereof, with an oxidizing reagent, provided that a in the reaction product is 1 or 2, or
reacting a compound represented by the formula (VIII):
R⁵-L⁴
wherein L⁴ is a leaving group, and the other symbol is the same as defined in claim 1, or a salt thereof, with a compound represented by the formula (Ib): 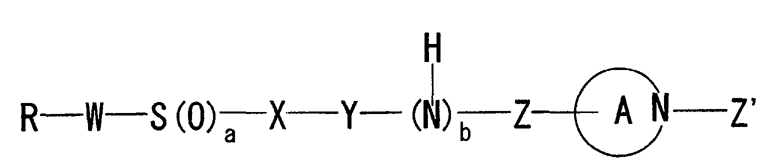 wherein the symbols are the same as defined in claim 1, or a salt thereof, or
reacting a compound represented by the formula (IX):
R-W-S(Oₐ)-M
wherein M is hydrogen atom, an alkali metal, an alkaline earth metal or a leaving group, and the other symbols are the same as defined in claim 1, or a salt thereof, with a compound represented by the formula (X): 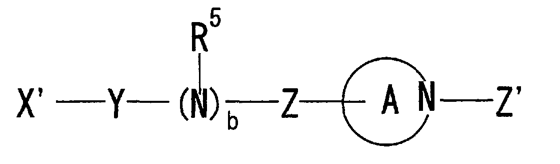 wherein X' is alkenyl or alkynyl, or alkyl having a leaving group, and the other symbols are the same as defined in claim 1, or a salt thereof, and
if necessary, further subjecting the compound obtained in the above reaction to hydrolysis, esterification, amidation, alkylation, acylation, reduction, oxidation and/or deprotection.

30. 3-(6-Halogeno-2-naphthyl)sulfonylpropionic acid, or its ester or its amide or a salt thereof.

31. 3-(6-Chloro-2-naphthyl)sulfonylpropionic acid, or its ester, its amide or a salt thereof.

32. A method for inhibiting blood coagulation in a mammal, which comprises administering an effective amount of the compound according to claim 1 or a salt thereof, or a prodrug thereof to the mammal.

33. A method for inhibiting activated coagulation factor X in a mammal, which comprises an effective amount of the compound according to claim 1 or a salt thereof, or a prodrug thereof to the mammal.

34. A method for preventing and/or treating cardiac infarction, cerebral thrombosis, deep vein thrombosis, pulmonary thrombotic embolus or thrombotic embolus during operation or after operation in a mammal, which comprises administering an effective amount of the compound according to claim 1 or a salt thereof, or a prodrug thereof to the mammal.

35. Use of the compound according to claim 1 or a salt thereof, or a prodrug thereof for manufacturing a medicament for inhibiting blood coagulation.

36. Use of the compound according to claim 1 or a salt thereof, or a prodrug thereof for manufacturing a medicament for inhibiting activated coagulation factor X.

37. Use of the compound according to claim 1 or a salt thereof, or a prodrug thereof for manufacturing a medicament for preventing and/or treating cardiac infarction, cerebral thrombosis, deep vein thrombosis, pulmonary thrombotic embolus, or thrombotic embolus during operation or after operation.
